(19) Europäisches Patentamt
European Patent Office
Office européen des brevets

(11) EP 2 818 471 A1

(12) **EUROPEAN PATENT APPLICATION**

(43) Date of publication:
**31.12.2014 Bulletin 2015/01**

(21) Application number: **13173962.5**

(22) Date of filing: **27.06.2013**

(51) Int Cl.:
***C07D 471/04*** *(2006.01)* ***C07D 487/04*** *(2006.01)*
***A61K 31/437*** *(2006.01)* ***A61K 31/4985*** *(2006.01)*
***A61K 31/5025*** *(2006.01)* ***A61P 3/10*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Max-Planck-Gesellschaft zur Förderung der Wissenschaften e.V.**
**80539 München (DE)**

(72) Inventors:
- **Ullrich, Axel**
**80331 München (DE)**
- **Falcenberg, Mathias**
**82152 Martinsried (DE)**

(74) Representative: **Arth, Hans-Lothar**
**ABK Patent Attorneys**
**Jasminweg 9**
**14052 Berlin (DE)**

(54) **Nitrogen bicyclic compounds as inhibitors for Scyl1 and Grk5**

(57) The present invention relates to compounds assumed to be capable of modulating the activity of the proteins Scyl1 and Grk5, thereby regulating the expression and/or release of insulin as well as to pharmaceutical compositions containing such compounds and the use thereof especially for the treatment of a metabolic disease such as diabetes, obesity and impaired adipogenesis.

**Figure 11**

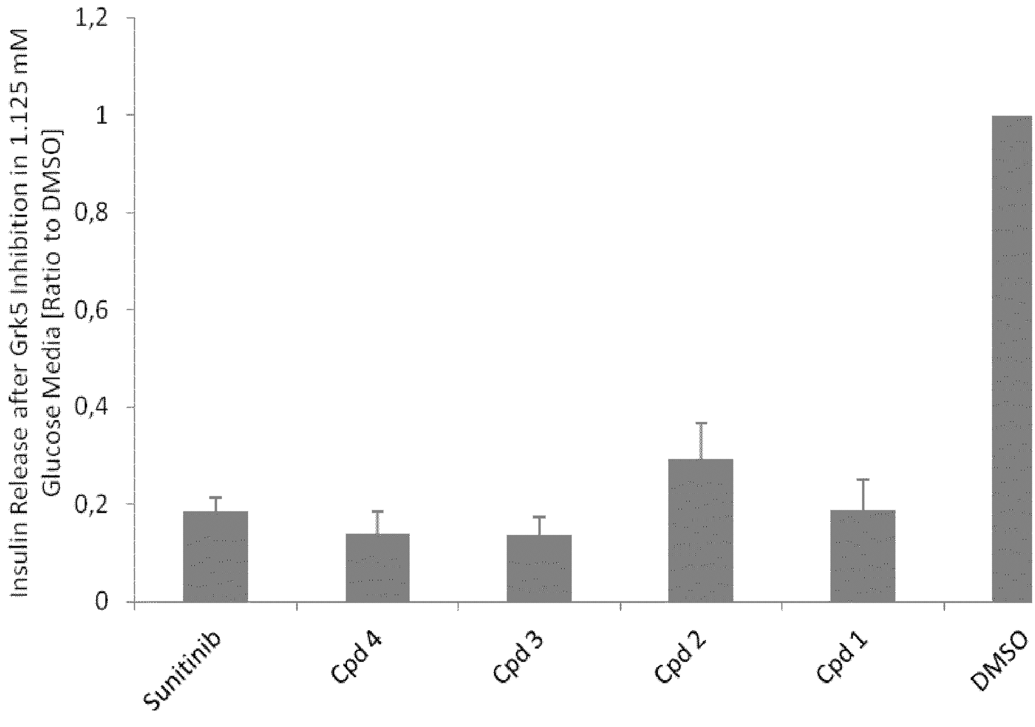


EP 2 818 471 A1

## Description

The present invention relates to compounds assumed to be capable of modulating the activity of the proteins Scyl1 and Grk5, thereby regulating the expression and/or release of insulin as well as to pharmaceutical compositions containing such compounds and the use thereof especially for the treatment of a metabolic disease such as diabetes, obesity and impaired adipogenesis.

## Background of the invention

Diabetes as a leading cause of death in developed countries is a metabolic condition characterized by high blood sugar levels. There are two main types of diabetes: type 1, resulting from insufficient insulin production of the pancreas beta cells, which requires the person to inject insulin; and type 2, resulting from insensitivity of peripheral tissues (such skeletal muscle, liver or adipose tissue) insulin release alterations, and relative insulin deficiency.

Insulin resistance, a condition in which cells fail to respond to the action of insulin, is a key feature in the pathogenesis of metabolic disorders such as type 2 diabetes (T2D) and obesity. Adipocytes are insulin-sensitive cells that take up glucose and store energy in the form of triglycerides. In addition to their storage function, adipocytes have recently been shown to be dynamic endocrine cells that produce and secrete various adipocytokines, such as TNF-a, IL-6, leptin, resistin, and adiponectin. The insulin resistance that accompanies obesity is attributable, at least in part, to changes in adipokine secretion. For example, excess accumulation of adipose tissue is detrimental to many systems, and involved in the pathologies including insulin resistance, type 2 diabetes and fatty infiltration of the liver. In the situation of insulin resistance, the liver inappropriately releases glucose into the blood. The proportion of insulin resistance versus beta cell dysfunction differs individually among patients, with some having primarily insulin resistance and only a minor defect in insulin secretion and others with slight insulin resistance and primarily a lack of insulin secretion. However, the precise mechanisms regulating insulin resistance in physiopathological conditions are not fully understood.

The decreased insulin sensitivity of peripheral tissues in type 2 diabetes which accounts for 90% of all cases of the disease is initially compensated by an increased release of insulin by the beta cells of the pancreas. At a certain stage of the disease, the pancreas cannot maintain the increases release of insulin anymore. As disease progresses, drugs which are currently available and elevate insulin release have lead to beta-cell damage and loss of insulin production.

A number of diseases, including cancer, diabetes and inflammation are linked to perturbation of protein kinase mediated cell signaling pathways. For some time, a new class of multiple kinase drugs has been undergoing clinical trials. Some have been approved for various applications, mostly for the treatment of cancer. The targets of these multiple kinase inhibitors like Imanitib or Sunitinib interact at all stages of signal transduction: from the receptor tyrosine kinases which initiate intracellular signaling to second-messenger generators and kinases involved in signaling cascades and finally to those kinases which regulate the cell cycle governing cellular fate.

Several publications have shown the effect of multi-targeted receptor tyrosine kinase inhibitors like Sunitinib (Sutent®) and Imatinib (Gleevec®) on diabetes during a period of treatment which leads to a remission of diabetes type 1 or 2 in patients. The fact that multi-targeted receptor tyrosine kinase inhibitors like Sunitinib targets many different receptors, results in many of its side effects such as the classic hand-foot syndrome, stomatitis, and other dermatologic toxicities. However, WO 2012/028335 A1 discloses only few kinases which could be identified that affect the insulin release or sensitivity specifically and are suitable as potential targets to develop a more specific treatment strategy. Two of said identified targets were GRK5 and Scyl1.

Scyl1 is an evolutionarily conserved N-terminal protein kinase-like domain protein that plays a role in COP1-mediated retrograde protein trafficking in mammalian cells. Furthermore, loss of Scyl1 function has been shown to result in neurodegenerative disorders in mice.

A potential influence in the in the insulin regulating mechanism may attributed to G-protein-coupled receptor (GPCR) kinase 5 (GRK5). GRK5 is an important member of the threonine/serine kinase family that phosphorylates GPCRs as part of feedback inhibition of GPCRs in signal transduction. The in vivo physiological functions of GRK5 have been ascribed to its kinase activity, phosphorylating and desensitizing specific GPCRs, as well as its kinase independent function and targeting to non-GPCR substrates. GRK5 interacts with IκBα and inhibits NFκB-mediated transcriptional responses. GRK5 also phosphorylates p53 and regulates p53-mediated apoptosis in response to DNA damage. G protein-coupled receptor kinases (GRKs) specifically phosphorylate serine/threonine residues located on the cytoplasmic loops and C terminus of the receptors. Phosphorylation of a GPCR by GRK promotes the high affinity binding of arrestins and induces receptor internalization, and thus feed-back inhibits GPCR responses to extracellular signals. The in vivo physiological functions of various subtypes of GRKs (GRK1-7) have been previously ascribed to phosphorylating and desensitizing specific GPCRs. Wang et al. (Biochem. Biophys Res.Commun. 421 (2012) 312-317) published recently experimental data from evaluation of $GRK^{-/-}$ mice showing that GRK5 deficiency prevents obesity induced by a high-fat diet and suggesting that GRK5 is also an important regulator of adipogenesis and is crucial for the development of diet-induced obesity. A genome-wide association study in Chinese Hans identified two novel T2D loci, including GRK5 (rs10886471: P = 7.1 x 10(-9)). This study could further show that the risk increasing allele rs10886471 was also associated with higher GRK5 mRNA expression levels, higher fasting insulin, but not with fasting glucose, suggesting that it might impair insulin sensitivity.

Thus it is the objective of the present invention to provide compounds which are useful as new anti-diabetic drugs for raising the blood insulin level and enhance an insulin dependent uptake of glucose. This goal is achieved by the compounds according to claim 1 which bind to regulating proteins and the disclosed targets Scyl1 and Grk5. Further advantageous embodiments, aspects and details of the invention are evident from the pending claims, the description, the examples and the figures.

Therefore, it is further the objective of the present invention to provide compounds and/or pharmaceutically acceptable salts thereof which can be used as pharmaceutically active agents, especially for the treatment of diabetes, obesity and impaired adipogenesis as well as compositions comprising at least one of those compounds and/or pharmaceutically acceptable salts thereof as pharmaceutically active ingredients.

The objective of the present invention is solved by the teaching of the independent claims. Further advantageous features, aspects and details of the invention are evident from the dependent claims, the description, the figures, and the examples of the present application.

**Description of the invention**

In order to reveal new negatively regulated kinases which display increased insulin release after inhibition, the inventors had investigated a kinome wide siRNA screen and presented new negatively regulating kinases, in particular Scyl1 and Grk5 as new potential targets for triggering insulin release and therapeutic anti-diabetes targets.

This invention relates to compounds that are assumed to bind to the most promising kinase candidates Scyl1 and Grk5, respectively. The assumed Scyl1 and Grk5 inhibitors displayed an insulin independent increase of 2-NBDG glucose analog uptake as well as an improved release of insulin.

The present invention therefore relates to the compounds of the general formula (I)

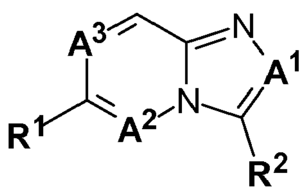

(I)

wherein

**A**$^1$, **A**$^2$ and **A**$^3$ represent C-H, or N, wherein one of **A**$^1$, **A**$^2$ and **A**$^3$ represents N, preferred wherein one of **A**$^1$, **A**$^2$ and **A**$^3$ represents N and the other two of **A**$^1$, **A**$^2$ and **A**$^3$ represent C-H;

**R**$^1$ represents $-(CH_2)_n$-**R**$^3$, or $-NH-(CH_2)_n$-**R**$^3$;

**R**$^2$ represents $-(CH_2)_m$-**R**$^4$, or $-NHCO-(CH_2)_m$-**R**$^4$;

**R**$^3$ and **R**$^4$ are independently of each other

-H, -F, -Cl, -Br, -I, -CN, $-NO_2$, $-NHCH_3$, $-N(CH_3)_2$, $-CH=CH-C_4H_9$, $-CH=CH-C_5H_{11}$, -CH=CH-Ph, $-CH=CH-C_6H_{13}$, $-CH_2-OH$; $-C_2H_4-OH$; $-C_3H_6-OH$, $-C_4H_9-OH$, $C_5H_{10}-OH$, $-C_6H_{12}-OH$, $-C_7H_{14}-OH$, $-C_8H_{16}OH$, $-CH=CH-C_3H_6-OH$, $-CH=CH-C_4H_8-OH$, $-CH(CH_2OH)_2$, $-CH(C_2H_5)-CH_2-OH$, $-CH(CH_3)-C_2H_4-OH$, $-C(CH_3)_2-OH$, $-C(CH_3)_2CH_2-OH$, $-CH(CH_3)OH$, $-CH_2-CH(CH_3)OH$, $-C(OH)(CH_3)-C_2H_5$, $-C(OH)(CH_3)-C_3H_7$, $-CH_2-C(OH)(CH_3)-C_2H_5$, $-CH(CH_3)-CH(CH_3)OH$, $-C(CH_3)_2-C_2H_4OH$, $-CH_2-C(CH_3)_2OH$, $-C(OH)(C_2H_5)_2$, $-C_2H_4-C(OH)(CH_3)_2$, $-C(CH(CH_3)_2)CH_2OH$, $-C_3H_6-C(OH)(CH_3)_2$, $-CH(CH(CH_3)_2)CH_2-OH$, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -OPh, $-OCH_2-Ph$, $-OCPh_3$, -SH, $-SCH_3$, $-SC_2H_5$, $-COCH_3$, $-COC_2H_5$, $-COC_3H_7$, -CO-cyclo-$C_3H_5$, $-COCH(CH_3)_2$, $-COC(CH_3)_3$, -COOH, $-OCF_3$, $-CH_2-OCF_3$, $-C_2H_4-OCF_3$, $-C_3H_6OCF_3$, $-OC_2F_5$, $-COOCH_3$, $-COOC_2H_5$, $-COOC_3H_7$, -COO-cyclo-$C_3H_5$, $-COOCH(CH_3)_2$, $-COOC(CH_3)_3$, $-OOC-CH_3$, $-OOC-C_2H_5$, $-OOC-C_3H_7$, -OOC-cyclo-$C_3H_5$, $-OOC-CH(CH_3)_2$, $-OOC-C(CH_3)_3$, $-CONH_2$, $-CONHCH_3$, $-CONHC_2H_5$, $-CONHC_3H_7$, -CONH-cyclo-$C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, -CON(cyclo-$C_3H_5)_2$, $-CON[CH(CH_3)_2]_2$, $-CON[C(CH_3)_3]_2$, $-NHCOCH_3$, $-NHCOC_2H_5$, $-NHCOC_3H_7$, -NHCO-cyclo-$C_3H_5$, $-NHCO-CH(CH_3)_2$, $-NHCO-C(CH_3)_3$, $-NHCO-OCH_3$, $-NHCO-OC_2H_5$, $-NHCO-OC_3H_7$, -NHCO-O-cyclo-$C_3H_5$,

$-NHCO-OCH(CH_3)_2$, $-NHCO-OC(CH_3)_3$, $-NH_2$, $-NHCH_3$, $-NHC_2H_5$, $-NHC_3H_7$, $-NH\text{-cyclo-}C_3H_5$, $-NH-CH(CH_3)_2$,$-NHC(CH_3)_3$, $-N(CH_3)_2$, $-N(C_2H_5)_2$, $-N(C_3H_7)_2$, $-N(\text{cyclo-}C_3H_5)_2$, $-N[CH(CH_3)_2]_2$, $-N[C(CH_3)_3]_2$, $-C{\equiv}C-\mathbf{R^5}$, $-\mathbf{R^{11}}$, $-\mathbf{R^{12}}$;

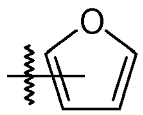 , 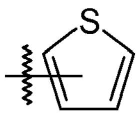 , 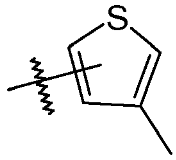 ,

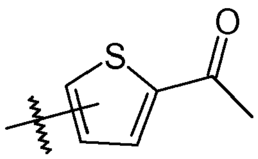 , 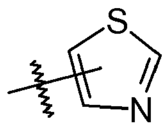 , 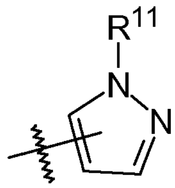 ,

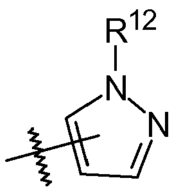 , 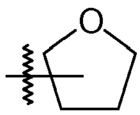 , 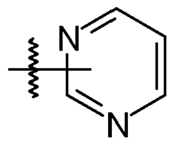 ,

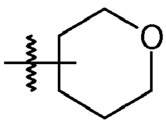 , 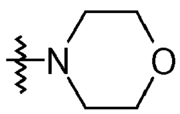 , 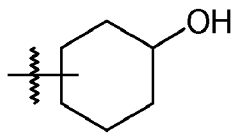 ,

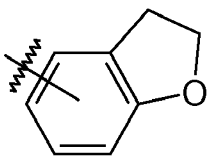 , 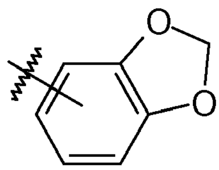 , 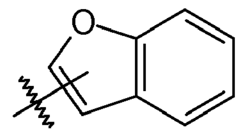 ,

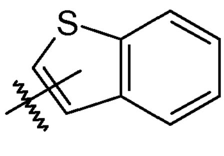 , 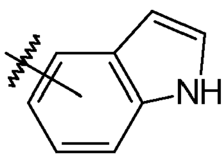 , 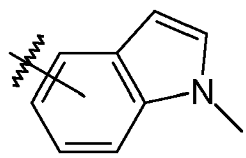 ,

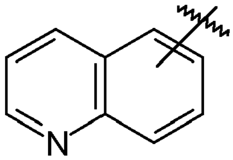 , 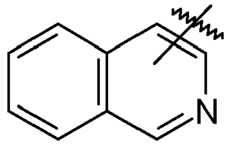 , 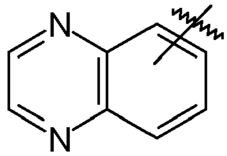 ,

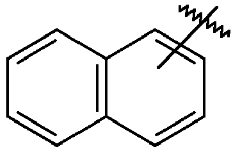 , 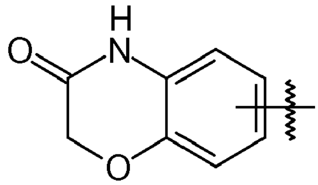 , 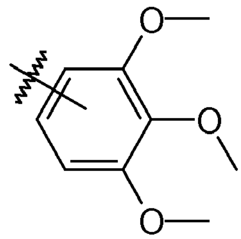 ,

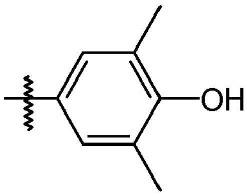 , 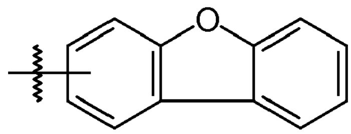 , 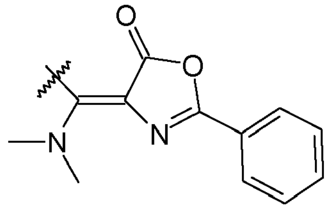 ,

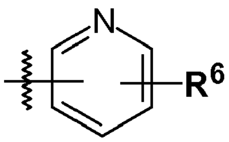 , 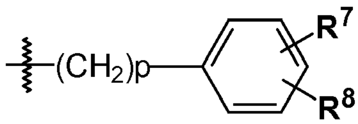 ;

**$R^5$** is -H, $-CH_2OH$, $-CH_2N(R^{13})_2$, -**$R^{13}$**,

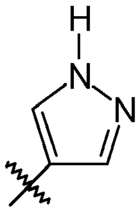 , 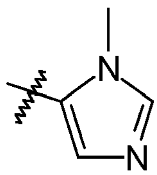 , 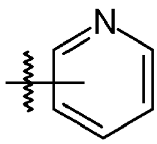 , 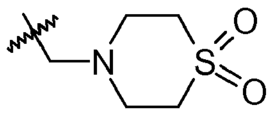 , 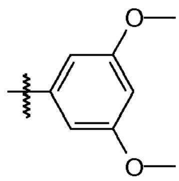 ,

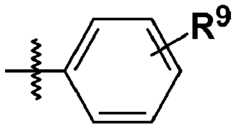 ;

**$R^6$** is -H, $-NH_2$, -OMe, $-O-(CH_2)_3N(CH_3)_2$,

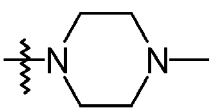 , 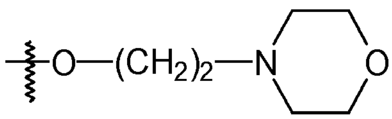 ;

**$R^7$** and **$R^8$** are independently of each other
-H, -F, -Br, -Cl, -OH, -CN, $-NO_2$, -**$R^{14}$**, -**$R^{15}$**, -O**$R^{14}$**, -O**$R^{15}$**, $-CH_2OH$, $-CH_2NH_2$, $-CH_2CN$; $-CH_2N(\mathbf{R^{14}})_2$, $-CH_2N(\mathbf{R^{15}})_2$, $-CH_2NH(\mathbf{R^{14}})$, $-CH_2NH(\mathbf{R^{15}})$, $-O(CH_2)_3N(CH_3)_2$, $-SCH_3$, $-NH_2$, $-NH(\mathbf{R^{14}})$, $-NH(\mathbf{R^{15}})$, $-NHCOCH_3$, $-NHSO_2CH_3$, $-N(\mathbf{R^{14}})_2$, $-N(\mathbf{R^{15}})_2$, $-SO_2CH_3$, $-SO_2NH_2$, $-CH_2CO_2H$, $-C_2H_4CO_2H$, $-CH=CH-CO_2H$, -CO**$R^{10}$**,

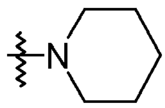, 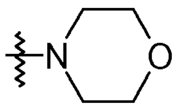, 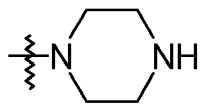, 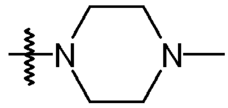,

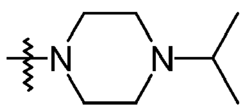, 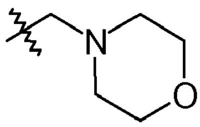;

$\mathbf{R^9}$ is -H, -F, -Br, -Cl, -OH, -CN, -$\mathbf{R^{16}}$, -O$\mathbf{R^{16}}$, $-NHCOCH_3$, $-CON(CH_3)_2$;
$\mathbf{R^{10}}$ is -OH, -$\mathbf{R^{17}}$, -O$\mathbf{R^{17}}$, $-NH_2$, -NH$\mathbf{R^{17}}$, -N($\mathbf{R^{17}}$)$_2$, $-NHC_2H_4OH$, $-NH(CH_2)_qN(\mathbf{R^{17}})_2$,

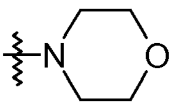, 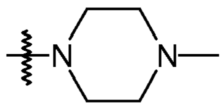, , 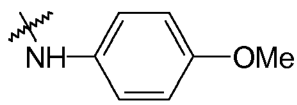, 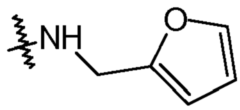;

$\mathbf{R^{11}}$, $\mathbf{R^{12}}$, $\mathbf{R^{13}}$, $\mathbf{R^{14}}$, $\mathbf{R^{15}}$, $\mathbf{R^{16}}$, and $\mathbf{R^{17}}$ are independently of each other

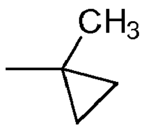,

$C_3H_5$, cyclo-$C_4H_7$, cyclo-$C_5H_9$, cyclo-$C_6H_{11}$, cyclo-$C_7H_{13}$, -H, $-CH_2-OCH_3$, $-C_2H_4-OCH_3$, $-C_3H_6OCH_3$, $-CH_2OC_2H_5$, $-C_2H_4-OC_2H_5$, $-C_3H_6-OC_2H_5$, $-CH_2-OC_3H_7$, $-C_2H_4-OC_3H_7$, $-C_3H_6-OC_3H_7$, $-CH_2$-O-cyclo-$C_3H_5$, $-C_2H_4$-O-cyclo-$C_3H_5$, $-C_3H_6$-O-cyclo-$C_3H_5$, $-CH_2-OCH(CH_3)_2$, $-C_2H_4-OCH(CH_3)_2$, $-C_3H_6-OCH(CH_3)_2$, $-CH_2-OC(CH_3)_3$, $-C_2H_4-OC(CH_3)_3$, $-C_3H_6-OC(CH_3)_3$, $-CH_2-OC_4H_9$, $-C_2H_4-OC_4H_9$, $-C_3H_6-OC_4H_9$, $-CH_2$-OPh, $-C_2H_4$-OPh, $-C_3H_6$-OPh, $-CH_2-OCH_2$-Ph, $-C_2H_4-OCH_2$-Ph, $-C_3H_6-OCH_2$-Ph, $-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, cyclo-$C_8H_{15}$, -Ph, $-CH_2$-Ph, $-CH_2-CH_2$-Ph, -CH=CH-Ph, $-CPh_3$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$,- $CH_2-C(CH_3)_3$, $-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$, $-C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C{\equiv}CH$, $-C{\equiv}C-CH_3$, $-CH_2-C{\equiv}CH$, $-C_2H_4-C{\equiv}CH$, $-CH_2-C{\equiv}C-CH_3$, $-C{\equiv}C-C_2H_5$, $-CH(CH_3)Ph$, $-C(CH_3)_2Ph$;
m, n, p and q are integer from 0 to 3;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

Preferred are compound of the general formula (I)

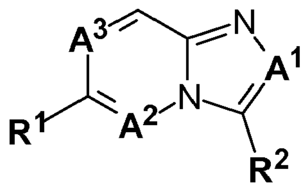

(I)

wherein

**A$^1$**, **A$^2$** and **A$^3$** represent C-H, or N, wherein one of **A$^1$**, **A$^2$** and **A$^3$** represents N, preferred wherein one of **A$^1$**, **A$^2$** and **A$^3$** represents N and the other two of **A$^1$**, **A$^2$** and **A$^3$** represent C-H;

**R$^1$** represents $-(CH_2)_n-$**R$^3$**, or $-NH-(CH_2)_n-$**R$^3$**;

**R$^2$** represents $-(CH_2)_m-$**R$^4$**, or $-NHCO-(CH_2)_m-$**R$^4$**;

**R$^3$** and **R$^4$** are independently of each other
-H, -F, -Br, -Cl, -CN, -OH, $-OCH_3$, $-OC_2H_5$, $-NHCH_3$, $-N(CH_3)_2$, $-CH_3$, $-C_2H_5$, -cyclo-$C_3H_5$, $-C_3H_7$, $-CH(CH_3)_2$, -cyclo-$C_4H_7$, $-C_4H_9$, $-CH_2-CH(CH_3)_2$, $-CH(CH_3)-C_2H_5$, $-C(CH_3)_3$, -cyclo-$C_5H_9$, $-C_5H_{11}$ $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$,- $C_2H_4-CH(CH_3)_2$, -cyclo-$C_6H_{11}$, $-C_6H_{13}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH(CH_3)Ph$, $-CH=CH-C_4H_9$, $-CH=CH-C_5H_{11}$, -CH=CH-Ph, $-CH=CH-C_6H_{13}$, $-CH_2-OH$; $-C_2H_4-OH$; $-C_3H_6-OH$, $-C_4H_9-OH$, $-C_5H_{10}-OH$, $-C_6H_{12}-OH$, $-C_7H_{14}-OH$, $-C_8H_{16}OH$, $-CH=CH-C_3H_6-OH$, $-CH=CH-C_4H_8-OH$, $-CH(CH_2OH)_2$, $-CH(C_2H_5)-CH_2-OH$, $-CH(CH_3)-C_2H_4-OH$, $-C(CH_3)_2-OH$, $-C(CH_3)_2-CH_2-OH$, $-CH(CH_3)OH$, $-CH_2-CH(CH_3)OH$, $-C(OH)(CH_3)-C_2H_5$, $-C(OH)(CH_3)-C_3H_7$, $-CH_2-C(OH)(CH_3)-C_2H_5$, $-CH(CH_3)-CH(CH_3)OH$, $C(CH_3)_2-C_2H_4OH$, $-CH_2-C(CH_3)_2OH$, $-C(OH)(C_2H_5)_2$, $-C_2H_4-C(OH)(CH_3)_2$, $-C(CH(CH_3)_2)CH_2OH$, $-C_3H_6-C(OH)(CH_3)_2$, $-CH(CH(CH_3)_2)CH_2-OH$, $-C \equiv C-$**R$^5$**

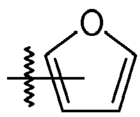 , 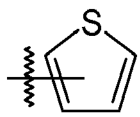 , 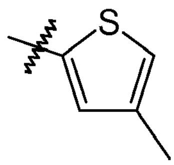 ,

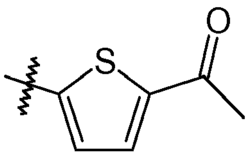 , 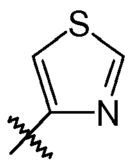 , 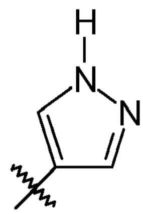 ,

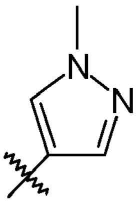,

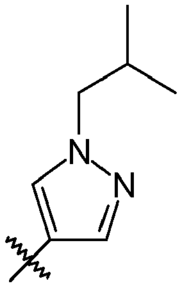,

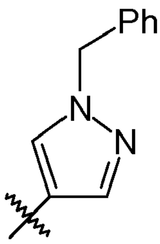,

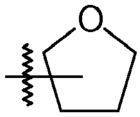,

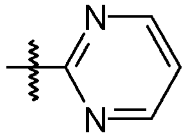,

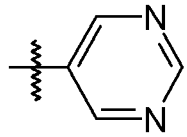,

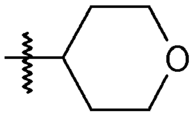,

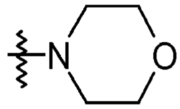,

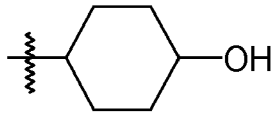,

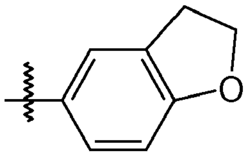,

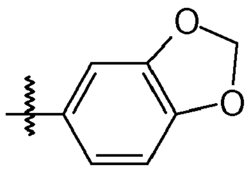,

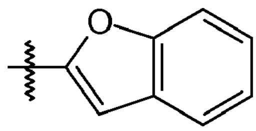,

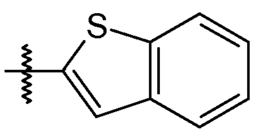,

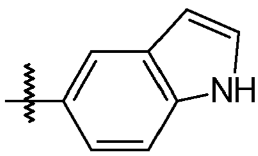,

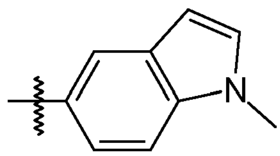,

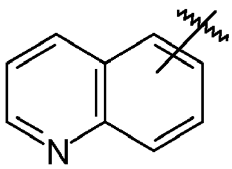,

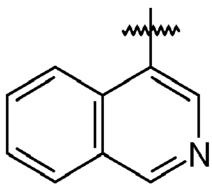,

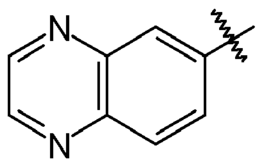,

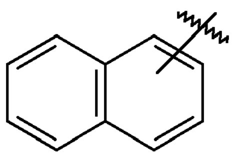,

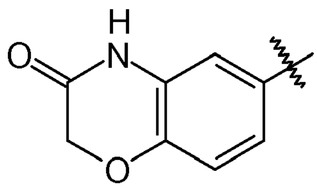,

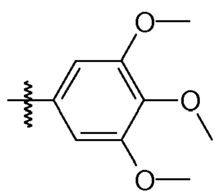,

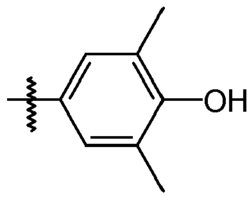, 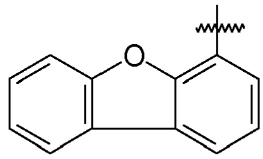, 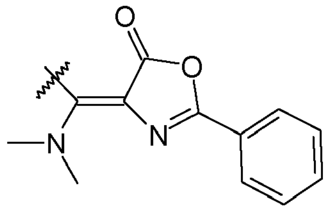,

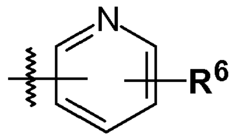, , 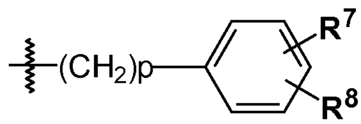;

$R^5$ is -H, $-CH_2OH$, $-CH_2N(CH_3)_2$, $-C(CH_3)_3$,

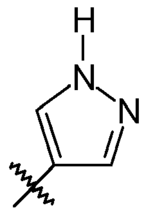, 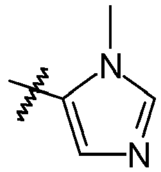, 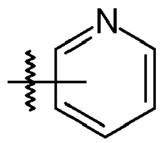, 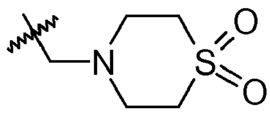, 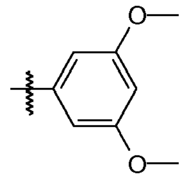,

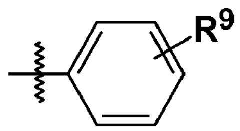;

$R^6$ is -H, $-NH_2$, -OMe, $-O-(CH_2)_3N(CH_3)_2$,

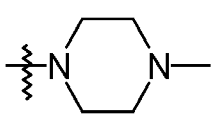, 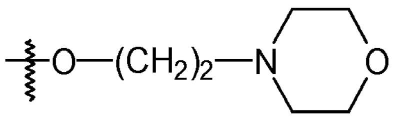;

$R^7$ and $R^8$ are independently of each other
-H, -F, -Br, -Cl, -OH, -CN, $-NO_2$, $-CH_3$, $-CH(CH_3)_2$, $-C(CH_3)_3$, $-OCH_3$, $-OC_2H_5$, $-OCH(CH_3)_2$, $-OC(CH_3)_3$, $-CF_3$, $-OCF_3$, $-CH_2OH$, $-CH_2NH_2$, $-CH_2CN$; $-CH_2N(CH_3)_2$, $-CH_2NHPh$, -OPh, $-O(CH_2)_3N(CH_3)_2$, $-NH_2$, $-NHCH_3$, $-NHCOCH_3$, $-NHSO_2CH_3$, $-N(CH_3)_2$, $-SCH_3$, $-SO_2CH_3$, $-SO_2NH_2$, $-CH_2CO_2H$; $-C_2H_4CO_2H$; $-CH=CH-CO_2H$, -CO**$R^{10}$**,

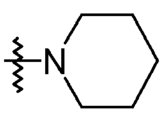, 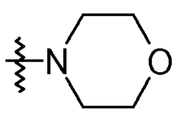, 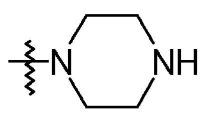, 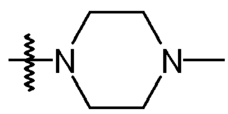,

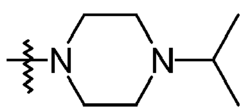, 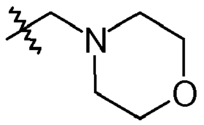;

$\mathbf{R^9}$ is -H, -F, -Br, -Cl, -OH, -CN, $-CH_3$, $-CH(CH_3)_2$, $-OCH_3$, $-OC_2H_5$, $-CF_3$, $-OCF_3$, $-NHCOCH_3$, $-CON(CH_3)_2$;

$\mathbf{R^{10}}$ is -OH, $-CH_3$, $-OCH_3$, $-NH_2$, $-NHCH_3$, $-N(CH_3)_2$, $-NHC_2H_4OH$, $-NHC_2H_4N(CH_3)_2$, $-NH(CH_2)_3N(CH_3)_2$,

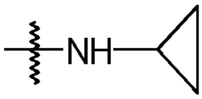,

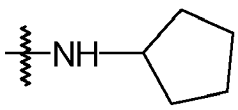, 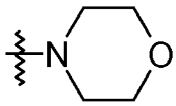, 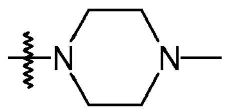, 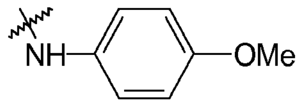,

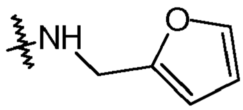;

m, n and p are integer from 0 to 3;

and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

Further preferred are compounds of the general formula (II)

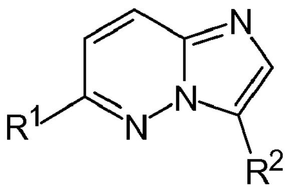

(II)

$\mathbf{R^1}$ represents $-(CH_2)_n-\mathbf{R^3}$, or $-NH-(CH_2)_n-\mathbf{R^3}$;
$\mathbf{R^2}$ represents $-(CH_2)_m-\mathbf{R^4}$;
$\mathbf{R^3}$ and $\mathbf{R^4}$ are independently of each other
-H, -F, -Br, -Cl, -CN, -OH, $-OCH_3$, $-OC_2H_5$, $-NHCH_3$, $-N(CH_3)_2$, $-CH_3$, $-C_2H_5$, -cyclo-$C_3H_5$, $-C_3H_7$, $-CH(CH_3)_2$, - cyclo-$C_4H_7$, $-C_4H_9$, $-CH_2-CH(CH_3)_2$, $-CH(CH_3)-C_2H_5$, $-C(CH_3)_3$, -cyclo-$C_5H_9$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, -cyclo-$C_6H_{11}$, $-C_6H_{13}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH(CH_3)Ph$, $-CH=CH-C_4H_9$, $-CH=CH-C_5H_{11}$, -CH=CH-Ph, $-CH=CH-C_6H_{13}$, $-C=C-C(CH_3)_3$, $-CH_2-OH$; $-C_2H_4-OH$; $-C_3H_6-OH$, $-C_4H_9-OH$, $-C_5H_{10}-OH$, $-C_6H_{12}-OH$, $-C_7H_{14}-OH$, $-C_8H_{16}-OH$, $-CH=CH-C_3H_6-OH$, $-CH=CH-C_4H_8-OH$, $-CH(CH_2OH)_2$, $-CH(C_2H_5)-CH_2-OH$, $-CH(CH_3)-C_2H_4-OH$, $-C(CH_3)_2-OH$, $-C(CH_3)_2-CH_2-OH$, $-CH(CH_3)OH$, $-CH_2-CH(CH_3)OH$, $-C(OH)(CH_3)-C_2H_5$, $-C(OH)(CH_3)-C_3H_7$, $-CH_2-C(OH)(CH_3)-C_2H_5$, $-CH(CH_3)-CH(CH_3)OH$, $-C(CH_3)_2-C_2H_4OH$, $-CH_2-C(CH_3)_2OH$, $-C(OH)(C_2H_5)_2$, $-C_2H_4-C(OH)(CH_3)_2$, $-C(CH(CH_3)_2)CH_2OH$, $-C_3H_6-C(OH)(CH_3)_2$, $-CH(CH(CH_3)_2)CH_2-OH$,

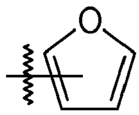,
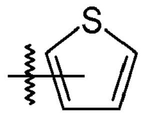,
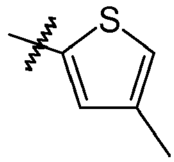,

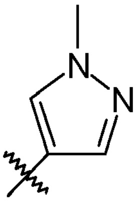,
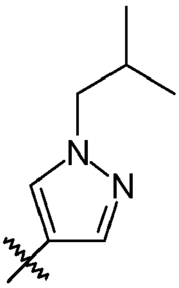,
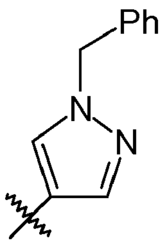,

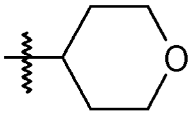,
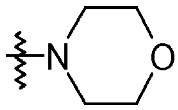,
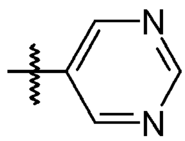,

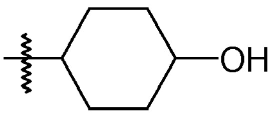,
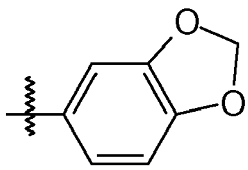,
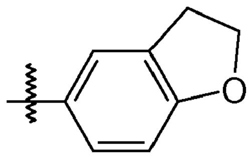,

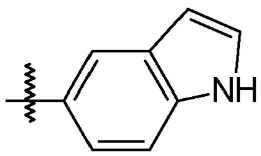,
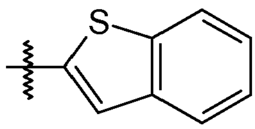,
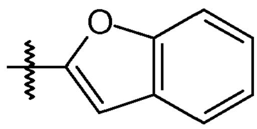,

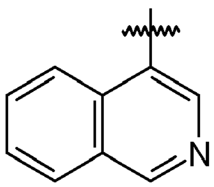,
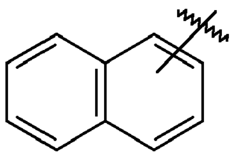,
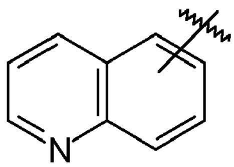,

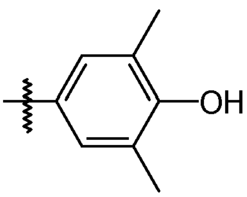,
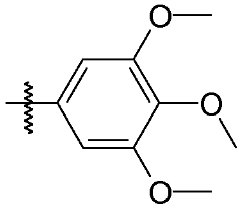,
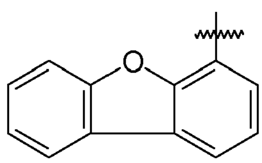,

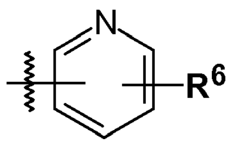 , 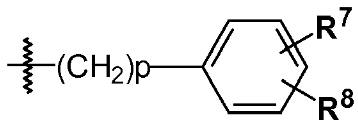 , 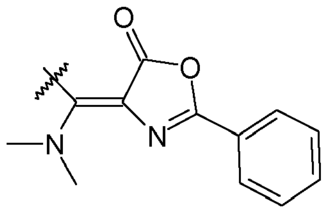 ,

$R^6$ is -H, $-NH_2$, -OMe, $-O-(CH_2)_3N(CH_3)_2$,

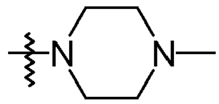

$R^7$ and $R^8$ are independently of each other
-H, -F, -Br, -Cl, -OH, -CN, $-NO_2$, $-CH_3$, $-CH(CH_3)_2$, $-C(CH_3)_3$, $-OCH_3$, $-OC_2H_5$, $-CF_3$, $-OCF_3$, $-CH_2OH$, $-CH_2NH_2$, $-CH_2CN$; $-CH_2N(CH_3)_2$,- OPh, $-SCH_3$, $-NH_2$, $-NHCH_3$, $-NHCOCH_3$, $-NHSO_2CH_3$, $-N(CH_3)_2$, $-SO_2CH_3$, $-SO_2NH_2$, $-C_2H_4CO_2H$, $-CH=CH-CO_2H$, $-O(CH_2)_3N(CH_3)_2$, $-COR^{10}$,

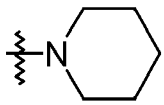 , 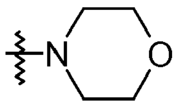 , 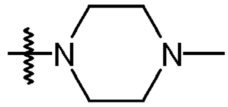 , 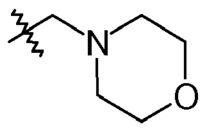 ;

$R^{10}$ is -OH, $-CH_3$, $-OCH_3$, $-NH_2$, $-NHCH_3$, $-NHC_2H_4OH$, $-NHC_2H_4N(CH_3)_2$, -NH $(CH_2)_3N(CH_3)_2$,

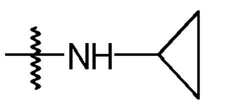 ,

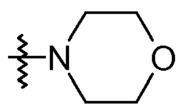 , 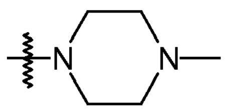 , 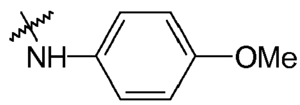 , 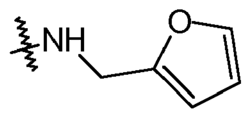 ;

m, n and p are integer from 0 to 3;
and enantiomers, stereoisomeric forms, mixtures of enantiomers, anomers, deoxyforms, diastereomers, mixtures of diastereomers, prodrugs, tautomers, hydrates, solvates and racemates of the above mentioned compounds and pharmaceutically acceptable salts thereof.

Further preferred are compounds of the general formula (III)

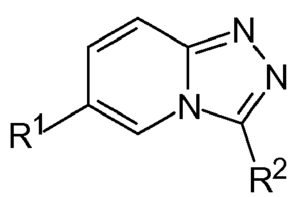

(III)

wherein

**$R^1$** represents $-(CH_2)_n-$**$R^3$**;
**$R^2$** represents $-(CH_2)_m-$**$R^4$**;
**$R^3$** and **$R^4$** are independently of each other
-H, -F, -Br, -Cl, -CN, -OH, $-OCH_3$, $-OC_2H_5$, $-NHCH_3$, $-N(CH_3)_2$, $-CH_3$, $-C_2H_5$, -cyclo-$C_3H_5$, $-C_3H_7$, $-CH(CH_3)_2$, -cyclo-$C_4H_7$, $-C_4H_9$, $-CH_2-CH(CH_3)_2$, -CH(CH3)-$C_2H_5$, $-C(CH_3)_3$, -cyclo-$C_5H_9$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$,- cyclo-$C_6H_{11}$, $-C_6H_{13}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH(CH_3)Ph$, $-CH=CH-C_4H_9$, -CH=CH-$C_5H_{11}$, -CH=CH-Ph, $-CH=CH-C_6H_{13}$, $-CH_2-OH$; $-C_2H_4-OH$; $-C_3H_6-OH$, $-C_4H_9-OH$, $-C_5H_{10}-OH$, $-C_6H_{12}-OH$, $-C_7H_{14}-OH$, $-C_8H_{16}-OH$, $-CH=CH-C_3H_6-OH$, $-CH=CH-C_4H_8-OH$, $-CH(CH_2OH)_2$, $-CH(C_2H_5)-CH_2-OH$ $-CH(CH_3)-C_2H_4-OH$, $-C(CH_3)_2-OH$, $-C(CH_3)_2CH_2-OH$, $-CH(CH_3)OH$, $-CH_2-CH(CH_3)OH$, $-C(OH)(CH_3)-C_2H_5$, $-C(OH)(CH_3)-C_3H_7$, $-CH_2-C(OH)(CH_3)-C_2H_5$, $-CH(CH_3)-CH(CH_3)OH$, $-C(CH_3)_2-C_2H_4OH$, $-CH_2-C(CH_3)_2OH$, $-C(OH)(C_2H_5)_2$, $-C_2H_4-C(OH)(CH_3)_2$, $-C(CH(CH_3)_2)CH_2OH$, $-C_3H_6-C(OH)(CH_3)_2$, $-CH(CH(CH_3)_2)CH_2-OH$, -C=C-**$R^5$**

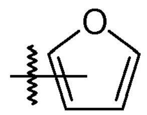,
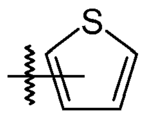,
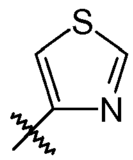,

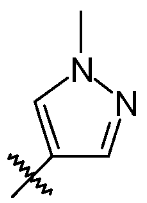,
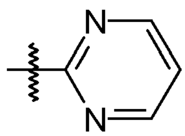,
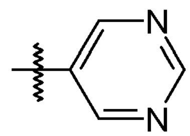,

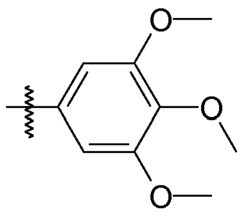,
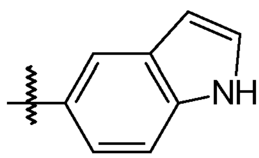,
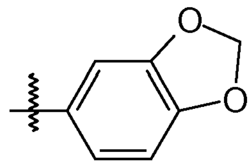,

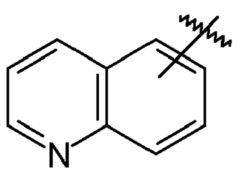,
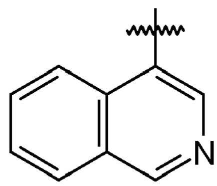,
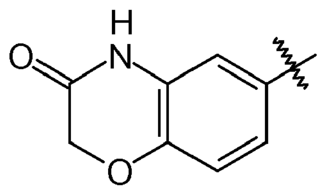,

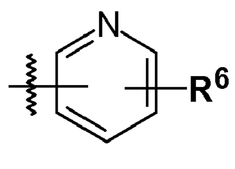,
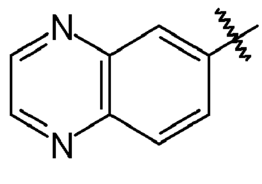,
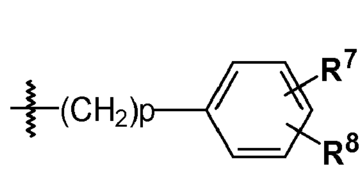;

**$R^5$** is -H, $-CH_2OH$, $-CH_2N(CH_3)_2$,

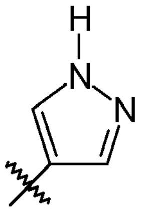, 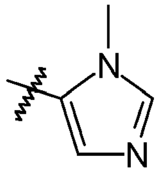, 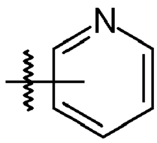, 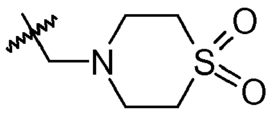, 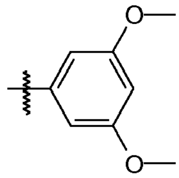,

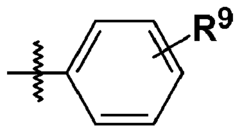;

**$R^6$** is -H, $-NH_2$, -OMe;
**$R^7$** and **$R^8$** are independently of each other
-H, -F, -Cl, -OH, -CN, $-NO_2$, $-CH_3$, $-CH(CH_3)_2$, $-OCH_3$, $-CF_3$, $-OCF_3$, $-CH_2OH$, $-CH_2N(CH_3)_2$, $-CH_2NHPh$, $-O(CH_2)_3N(CH_3)_2$, -OPh, $-SCH_3$, $-NH_2$, $-NHCOCH_3$, $-NHSO_2CH_3$, $-N(CH_3)_2$, $-SO_2CH_3$,

$-COR^{10}$, 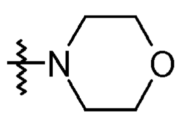, 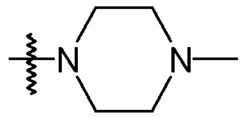;

**$R^9$** is -H, -F, -Br, -Cl, -OH, -CN, $-CH_3$, $-CH(CH_3)_2$, $-OCH_3$, $-OC_2H_5$, $-CF_3$, $-OCF_3$, $-NHCOCH_3$, $-CON(CH_3)_2$;
**$R^{10}$** is -OH, $-CH_3$, $-NH_2$, $-NHCH_3$, $-N(CH_3)_2$, $-NHC_2H_4OH$, $-NHC_2H_4N(CH_3)_2$, $-NH(CH_2)_3N(CH_3)_2$,

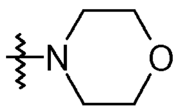, 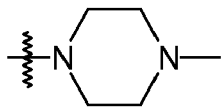;

m, n and pare 0 or 1;

and enantiomers, stereoisomeric forms, mixtures of enantiomers, anomers, deoxyforms, diastereomers, mixtures of diastereomers, prodrugs, tautomers, hydrates, solvates and racemates of the above mentioned compounds and pharmaceutically acceptable salts thereof.

Also, further preferred are compounds of the general formula (IV)

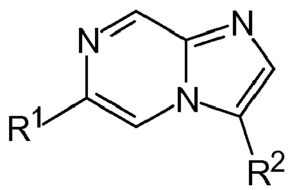

(IV)

wherein

**$R^1$** represents $-(CH_2)_n$-**$R^3$**;
**$R^2$** represents $-(CH_2)_m$-**$R^4$**, or $-NHCO-(CH_2)_m$-**$R^4$**;
**$R^3$** and **$R^4$** are independently of each other
-H, -F, -Br, -Cl, -CN, -OH, $-OCH_3$, $-OC_2H_5$, $-NHCH_3$, $-N(CH_3)_2$, $-CH_3$, $-C_2H_5$, -cyclo-$C_3H_5$, $-C_3H_7$, $-CH(CH_3)_2$, -cyclo-

$C_4H_7$, - $C_4H_9$, $-CH_2-CH(CH_3)_2$, $-CH(CH_3)-C_2H_5$, $-C(CH_3)_3$, -cyclo-$C_5H_9$, $-C_5H_{11}$ $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, -cyclo-$C_6H_{11}$, $-C_6H_{13}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH(CH_3)Ph$, $-CH=CH-C_4H_9$, $-CH=CH-C_5H_{11}$, -CH=CH-Ph, $-CH=CH-C_6H_{13}$, $-CH_2-OH$; $-C_2H_4-OH$; $-C_3H_6-OH$, $-C_4H_9-OH$, $-C_5H_{10}-OH$, $-C_6H_{12}-OH$, $-C_7H_{14}-OH$, $-C_8H_{16}-OH$, $-CH=CH-C_3H_6-OH$, $-CH=CH-C_4H_8-OH$, $-CH(CH_2OH)_2$, $-CH(C_2H_5)-CH_2-OH$, $-CH(CH_3)-C_2H_4-OH$, $-C(CH_3)_2-OH$, $-C(CH_3)_2-CH_2-OH$, $-CH(CH_3)OH$, $-CH_2-CH(CH_3)OH$, $-C(OH)(CH_3)-C_2H_5$, $-C(OH)(CH_3)-C_3H_7$, $-CH_2-C(OH)(CH_3)-C_2H_5$, $-CH(CH_3)-CH(CH_3)OH$, $-C(CH_3)_2-C_2H_4OH$, $-CH_2-C(CH_3)_2OH$, $-C(OH)(C_2H_5)_2$, $-C_2H_4-C(OH)(CH_3)_2$, $-C(CH(CH_3)_2)CH_2OH$, $-C_3H_6-C(OH)(CH_3)_2$, $-CH(CH(CH_3)_2)CH_2-OH$,

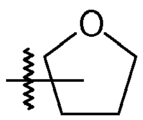
,
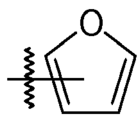
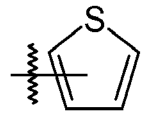
,
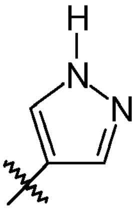
,
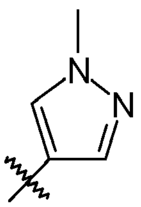
,
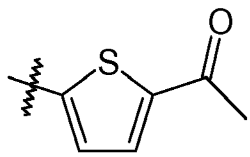
,
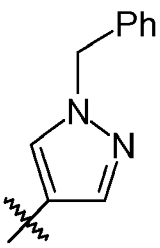
,
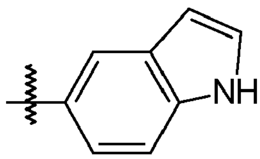
,
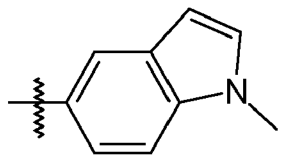
,
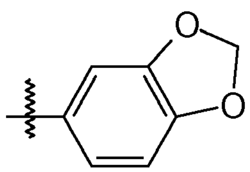
,
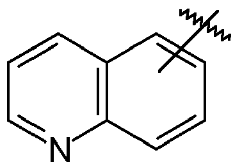
,
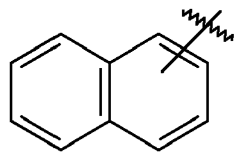
,
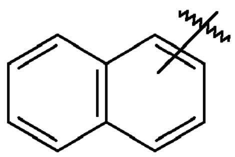
,
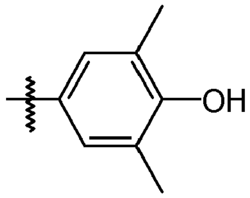
,
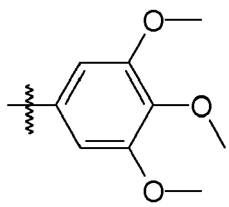
,

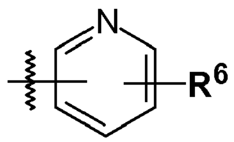 , 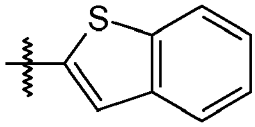 , 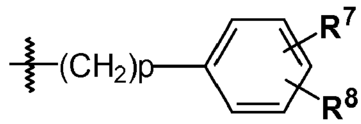 ;

**$R^6$** is -H, $-NH_2$, -OMe, $-O-(CH_2)_3N(CH_3)_2$,

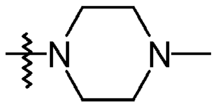 , 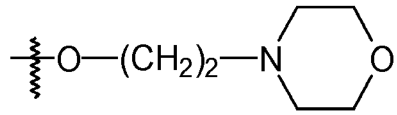 ;

**$R^7$** and **$R^8$** are independently of each other
-H, -F, -Br, -Cl, -OH, -CN, $-NO_2$, $-CH_3$, $-CH(CH_3)_2$, $-C(CH_3)_3$, $-OCH_3$, $-OC_2H_5$, $-OCH(CH_3)_2$, $-CF_3$, $-OCF_3$, $-CH_2OH$, $-CH_2N(CH_3)_2$, $-CH_2NHPh$, -OPh, $-O(CH_2)_3N(CH_3)_2$, $-SCH_3$, $-NH_2$, $-NHCH_3$, $-NHCOCH_3$, $-NHSO_2CH_3$, $-N(CH_3)_2$, $-C_2H_4CO_2H$, -CO**$R^{10}$**,

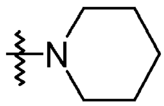 , 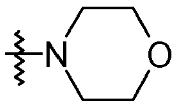 , 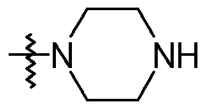 , 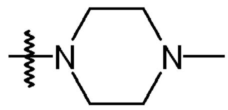 ,

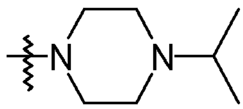 ;

**$R^{10}$** is $-CH_3$, $-NH_2$, $-N(CH_3)_2$, $-NHC_2H_4OH$,
$-NHC_2H_4N(CH_3)_2$, $-NH\ (CH_2)_3N(CH_3)_2$,

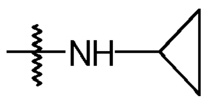 , 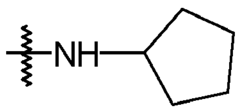 ,

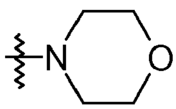 , 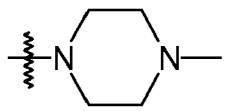 , 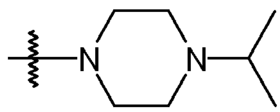 ;

m, n and pare 0 or 1;

and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

More preferred are compounds of the general formula (II)

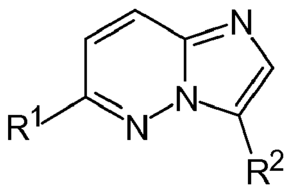

(II)

wherein

$\mathbf{R^1}$ represents $-(CH_2)_n-\mathbf{R^3}$, or $-NH-(CH_2)_n-\mathbf{R^3}$;
$\mathbf{R^2}$ represents $-(CH_2)_m-\mathbf{R^4}$;
$\mathbf{R^3}$ is
-H, -OH, $-OCH_3$, $-OC_2H_{53}$ $-NHCH_3$, $-N(CH_3)_2$, $-CH_3$, $-C_2H_5$, -cyclo-$C_3H_5$, $-C_3H_7$, $-CH(CH_3)_2$, -cyclo-$C_4H_7$, - $C_4H_9$, $-CH_2-CH(CH_3)_2$, $-CH(CH_3)-C_2H_5$, $-C(CH_3)_3$, $-CH_2-OH_3$ $-C_2H_4-OH$, $-C_3H_6-OH$, $-C_4H_9-OH$, $-CH(C_2H_5)-CH_2-OH$, $-CH(CH_3)-C_2H_4-OH$, $-C(CH_3)_2-OH$, $-C(CH_3)CH_2-OH$, $-CH(CH_3)OH$, $-CH_2-CH(CH_3)OH$, $-CH(CH(CH_3)_2)CH_2-OH$,

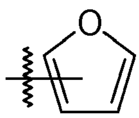,
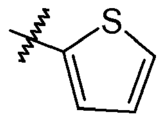,
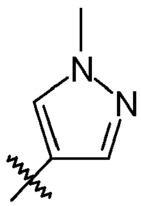,

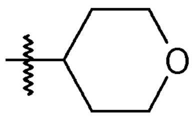,
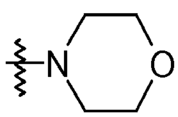,
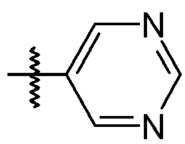,

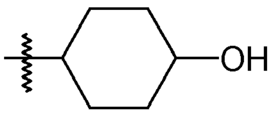,
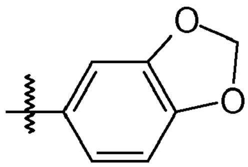,
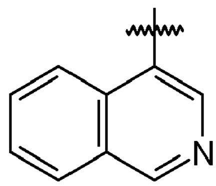,

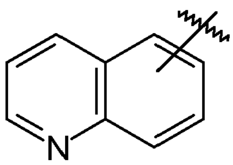,
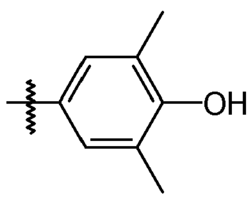,
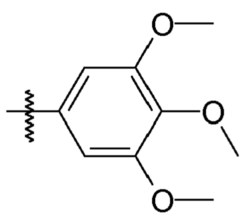,

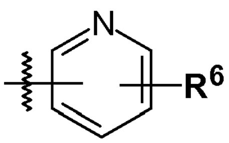,
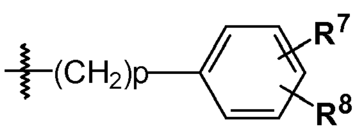, ,

wherein more preferred **$R^6$** is -H, $-NH_2$, -OMe;
more preferred **$R^7$** and **$R^8$** are independently of each other
-H, -F, -Cl, -OH, $-CH_3$, $-CH(CH_3)_2$, $-OCH_3$, $-OC_2H_5$, $-CF_3$, $-OCF_3$, $-CH_2OH$, $-CH_2NH_2$, $-CH_2CN$; $-CH_2N(CH_3)_2$, -OPh, $-NH_2$, $-NHCOCH_3$, $-NHSO_2CH_3$, $-N(CH_3)_2$, $-SO_2CH_3$, $-SO_2NH_2$, -CO**$R^{10}$**,

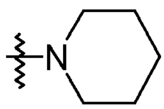 , 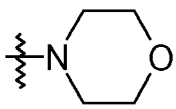 , 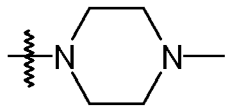 ,

wherein more preferred **$R^{10}$** is -OH, $-NH_2$, $-NHCH_3$, $-NHC_2H_4OH$, $-NHC_2H_4N(CH_3)_2$; and
p is 0 or 1.

More preferred, **$R^4$** is
$-CH=CH-C_4H_9$, -CH=CH-Ph, $-C\equiv C-C(CH_3)_3$, $-CH=CH-C_3H_6$-0H,

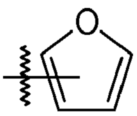 , 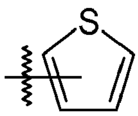 , 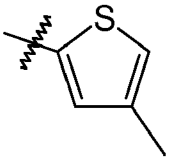 ,

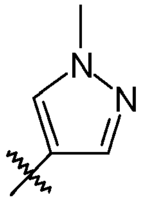 , 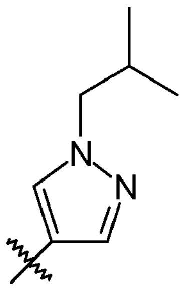 , 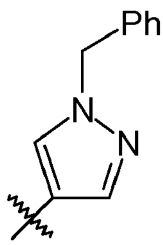 ,

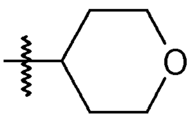 , 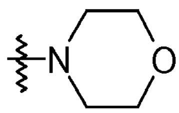 , 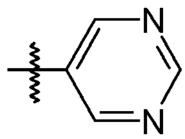 ,

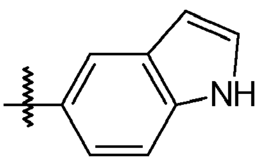 , 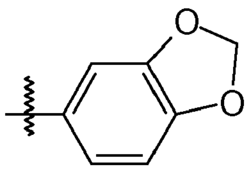 , 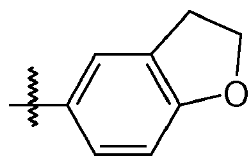 ,

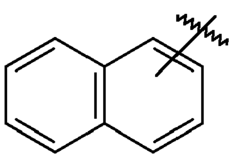 , 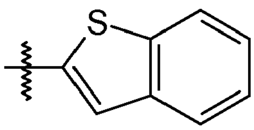 , 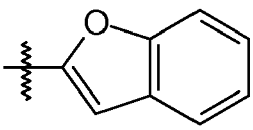 ,

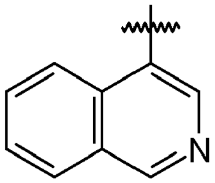 , 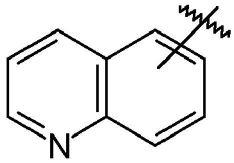 , 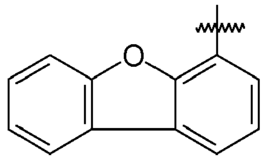 ,

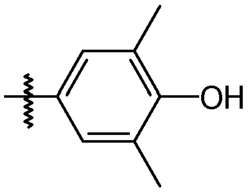 , 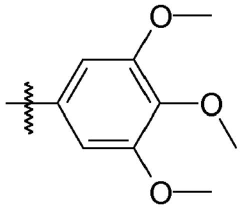 , 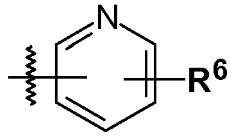 ,

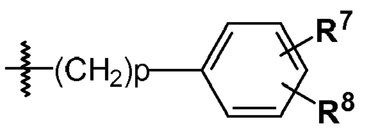 , 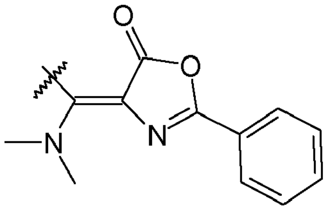 ,

wherein more preferred **$R^6$** is -H, $-NH_2$, -OMe, $-O-(CH_2)_3N(CH_3)_2$,

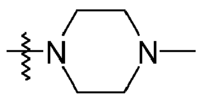 ;

more preferred **$R^7$** and **$R^8$** are independently of each other -H, -F, -Br, -Cl, -OH, -CN, $-CH_3$, $-CH(CH_3)_2$, $-OCH_3$, $-OC_2H_5$, $-CF_3$, $-OCF_3$, $-CH_2OH$, $-CH_2CN$, $-CH_2N(CH_3)_2$, -OPh, $-SCH_3$,- $NH_2$, $-NHCOCH_3$, $-NHSO_2CH_3$, $-N(CH_3)_2$, $-SO_2CH_3$, $-SO_2NH_2$, $-C_2H_4CO_2H$; $-CH=CH-CO_2H$; -CO**$R^{10}$**,

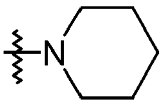 , 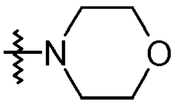 , 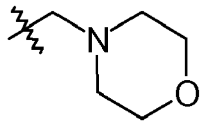 ,

wherein more preferred **$R^{10}$** is -OH, $-CH_3$, $-NH_2$, $-NHCH_3$, $-NHC_2H_4OH$, $-NHC_2H_4N(CH_3)_2$, -NH $(CH_2)_3N(CH_3)_2$,

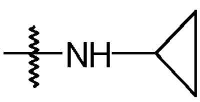 , 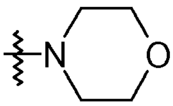 , 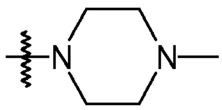 , 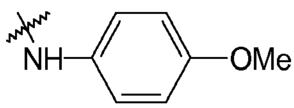 ,

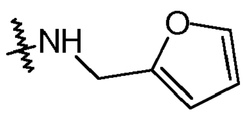 ;

and

p is 0 or 1.

Further more preferred are compounds of the general formula (III)

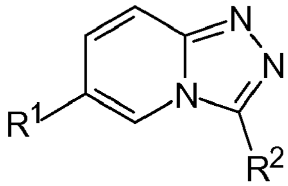

(III)

wherein

$\mathbf{R^1}$ represents $-(CH_2)_n-\mathbf{R^3}$;

$\mathbf{R^2}$ represents $-(CH_2)_m-\mathbf{R^4}$;

$\mathbf{R^3}$ is $-C{\equiv}C-\mathbf{R^5}$,

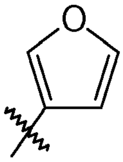 , 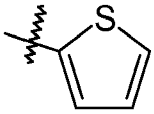 , 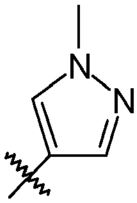 ,

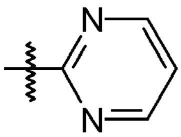 , 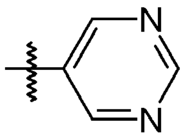 , 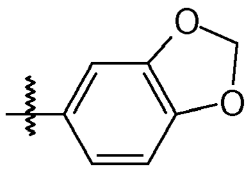 ,

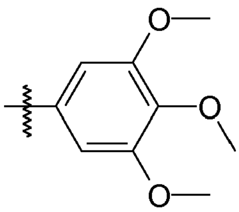 , 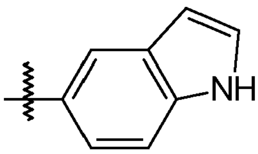 , 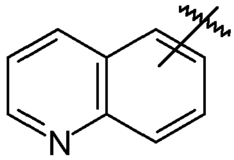 ,

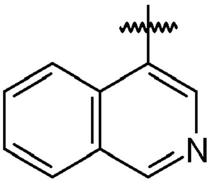 , 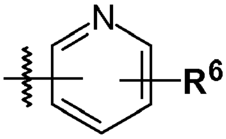 , 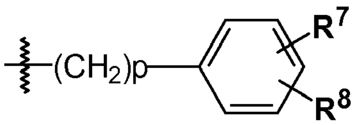 ;

wherein more preferred $\mathbf{R^5}$ is -H, $-CH_2OH$, $-CH_2N(CH_3)_2$,

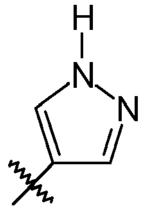 , 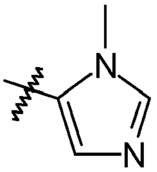 , 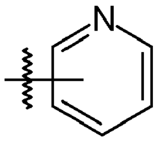 , 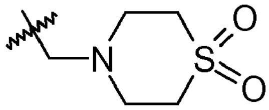 , 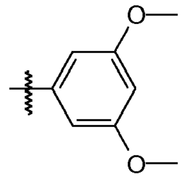 ,

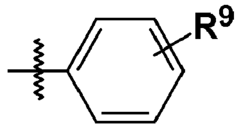 ,

wherein more preferred $\mathbf{R^9}$ is -H, -F, $-OCH_3$, $-CF_3$, $-NHCOCH_3$, $-CON(CH_3)_2$;
more preferred $\mathbf{R^6}$ is -H, $-NH_2$, -OMe;
more preferred $\mathbf{R^7}$ and $\mathbf{R^8}$ are independently of each other -H, -F, $-Cl_3$ -OH, -CN, $-CH_3$, $-CH(CH_3)_2$, $-OCH_3$, $-CF_3$, $-OCF_3$, $-CH_2OH$, -OPh, $-SCH_3$, $-NH_2$, $-NHCOCH_3$, $-NHSO_2CH_3$, $-N(CH_3)_2$, $-SO_2CH_3$, $-CO\mathbf{R^{10}}$,

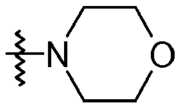 , 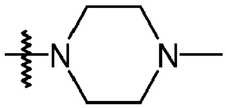 ,

wherein more preferred $\mathbf{R^{10}}$ is -OH, $-CH_3$, $-NH_2$, $-NHCH_3$, $-N(CH_3)_2$, $-NHC_2H_4OH$, $-NHC_2H_4N(CH_3)_2$, $-NH\ (CH_2)_3N(CH_3)_2$,

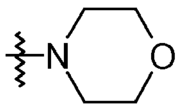 , 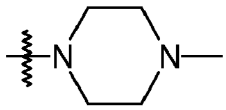 ;

more preferred $\mathbf{R^4}$ is

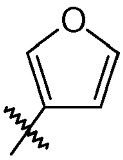 , 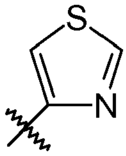 , 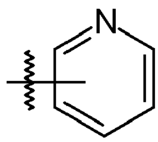 ,

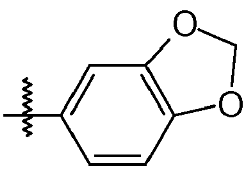 , 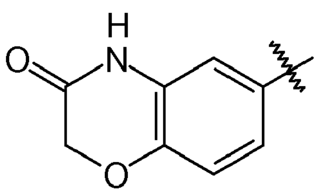 , 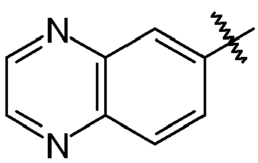 ,

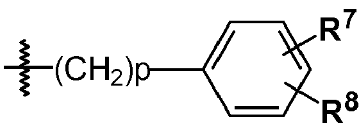 ,

wherein more preferred $\mathbf{R^7}$ and $\mathbf{R^8}$ are independently of each other -H, -F, -OH, -CN, $-CH_3$, $-OCH_3$, $-CF_3$, $-OCF_3$,

$-NHCOCH_3$, $-O-(CH_2)_3N(CH_3)_2$,

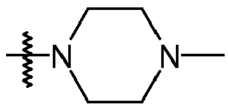

.

Even more preferred are compounds of the general formula (IV)

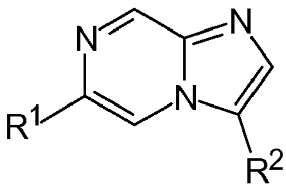

(IV)

wherein
**$R^1$** represents $-(CH_2)_n$-**$R^3$**;
**$R^2$** represents $-(CH_2)_m$-**$R^4$**, or $-NHCO-(CH_2)_m$-**$R^4$**;
**$R^3$** is $-CH=CH-C_4H_9$, $-CH=CH-Ph$,

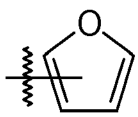
,
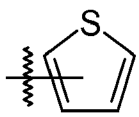
,
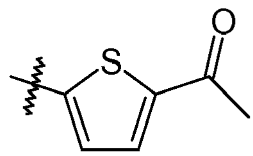
,

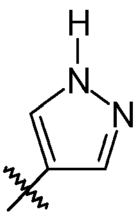
,
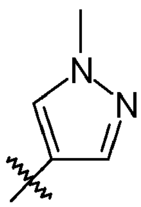
,
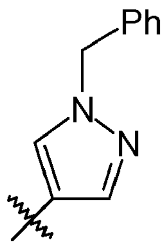
,

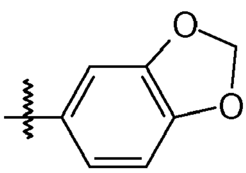
,
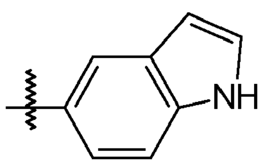
,
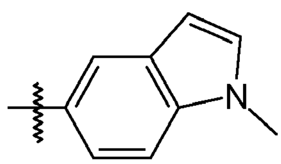
,

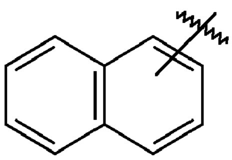
,
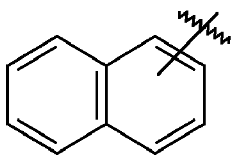
,
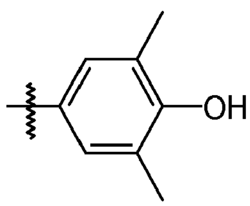
,

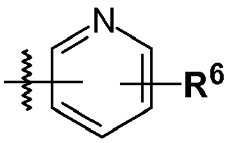 , 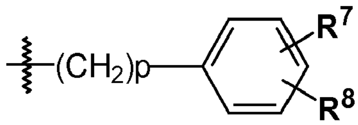 , 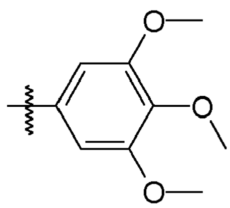 ,

wherein more preferred $R^6$ is -H, $-NH_2$, -OMe, $-O-(CH_2)_3N(CH_3)_2$,

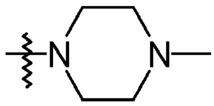 , 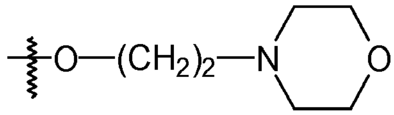 ;

more preferred $\mathbf{R^7}$ and $\mathbf{R^8}$ are independently of each other -H, -F, -Br, -Cl, -OH, $NO_2$, $-CH_3$, $-CH(CH_3)_2$, $-OCH_3$, $-OC_2H_5$, $-OCH(CH_3)_2$, $-CF_3$, $-OCF_3$, $-CH_2OH$, $-CH_2N(CH_3)_2$, $-CH_2NHPh$, -OPh, $-SCH_3$, $-NH_2$, $-NHCOCH_3$, $-NHSO_2CH_3$, $-N(CH_3)_2$, $-CO\mathbf{R^{10}}$,

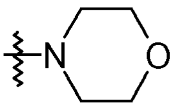 , 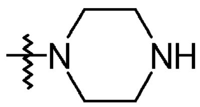 , 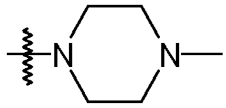 , 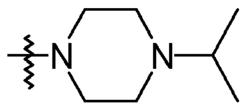 ;

wherein more preferred $\mathbf{R^{10}}$ is $-CH_3$, $-NH_2$, $-N(CH_3)_2$, $-NHC_2H_4OH$, $-NHC_2H_4N(CH_3)_2$, $-NH (CH_2)_3N(CH_3)_2$,

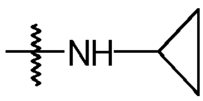 , 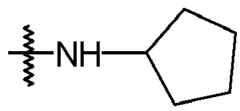 ;

More preferred $\mathbf{R^4}$ is $-CH_3$, cyclo-$C_3H_5$, $-CH(CH_3)_2$, cyclo-$C_4H_7$, $-CH(CH_3)Ph$,

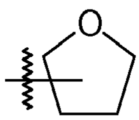 , 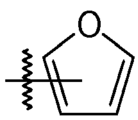 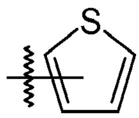 ,

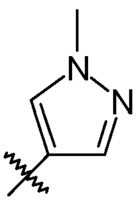 , 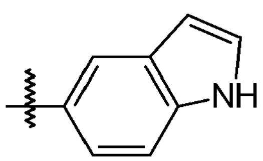 , 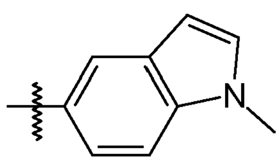 ,

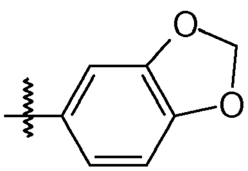 , 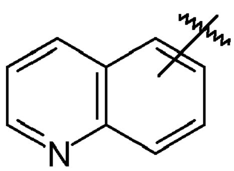 , 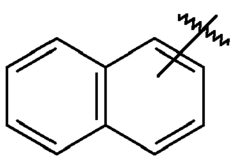 ,

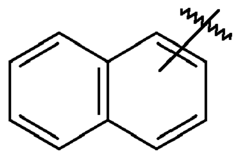 , 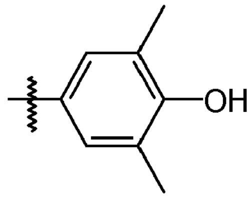 , 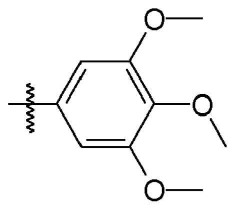 ,

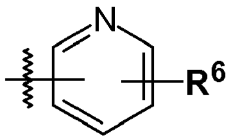 , 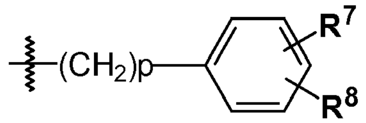 ;

wherein more preferred **$R^6$** is -H, -OMe,
more preferred **$R^7$** and **$R^8$** are independently of each other
-H, -F, -Cl, -OH, -CN, $-CH_3$, $-C(CH_3)_3$, $-OCH_3$, $-OCF_3$, -OPh, $-CH_2OH$, $-N(CH_3)_2$, $-C_2H_4CO_2H$, -CO**$R^{10}$**, wherein more preferred **$R^{10}$** is $-CH_3$, $-N(CH_3)_2$,

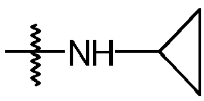 , 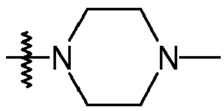 .

**Chemical synthesis**

The inventive compound (I) according to the present invention can be prepared by reactions known to a person skilled in the art. The synthesis is preferably carried out according to the general synthetic approaches shown in schemes 1 - 3.

Synthesis of compound with the general formula (II)

Scheme 1 shows the synthetic approaches of compound (II), Compound (II) has imidazo[1,2-*b*]pyridazine as main structure. As depicted in scheme 1, imidazo[1,2-*b*]pyridazine or 6-chloro-imidazo[1,2-b]pyridazine are used as starting material **1***. After halogenation (bromination or iodation) of compound **1*** intermidate compounds **2*** and **5*** halogenated at C-3 position were obtained. In second step, by Suzuki coupling reaction these intermediate compounds **2*** and **5*** are coupled with any suitable boronic acid $R^2B(OH)_2$ that is commercially available or can be synthesized according to or in analogy to literature procedures in order to give intermediate compound **6*** or to produce directly product (II) if intermediate **2*** is not subsituted at C-6 position (X = H). By further coupling of the intermediate compound **6*** with another suitable boronic acid $R^1B(OH)_2$ that is also commercially available or can be synthesized according to or in analogy to literature procedures, product (II) is obtained.

The intermediate compound **2*** can be subsituted at C-6 position by amination with an amine $R^1H$ that is commercially available or can be synthesized according to or in analogy to literature procedures in order to give intermediate compound **4***. The following Suzuki coupling reaction of compound **4*** with a boronic acid $R^2B(OH)_2$ provides 3,6-disubstituted imidazo[1,2-b]pyridazine product (II).

## Scheme 1

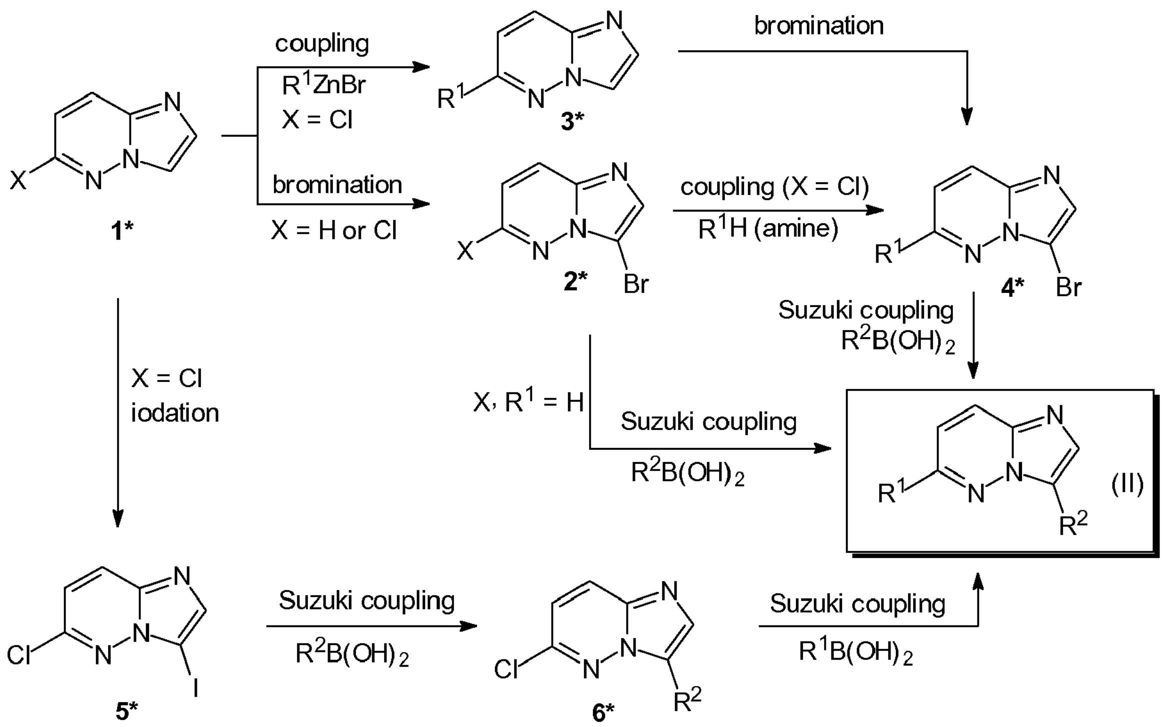


As alternative approach, imidazo[1,2-b]pyridazine derivate **3*** substituted with $R^1$ at C-6 position is firstly obtained by coupling of starting material **1*** with a zinc bromide compound $R^1ZnBr$ that is synthesized *in situ* from Zn and $R^1Br$ which is commercially available or can be synthesized according to or in analogy to literature procedures. The bromination of this intermediate compound **3*** is followed and the resulting brominated intermediate compound **4*** is subsequently coupled with a boronic acid $R^2B(OH)_2$ as above-described to give product (II).

Therefore, the synthetic approaches according to scheme 1 enable the synthesis of any of the imidazo[1,2-b]pyridazine derivatives of the general formula (II) disclosed in the present invention.

Synthetic method of general formula (III)

Scheme 2 shows the synthetic approaches of compound (III) having 1,2,4-triazolo[4,3-a]pyridine as main scaffold. The condensation of pyridinyl hydrazine **7*** with a suitable aldehyde that is commercially available or can be synthesized according to or in analogy to literature procedures and the following cyclization of resulting hydrazone compound provide 3-$R^2$-[1,2,4]triazolo[4,3,-a]pyridine **8*** as intermediate. By C-C coupling reaction of intermediate compound **8*** with $R^1Y$ (Y = $B(OH)_2$, $Sn(Bu)_3$ or H) under suitable conditions 3-$R^2$-6-$R^1$-[1,2,4]triazolo[4,3-a]pyridine (III) is obtained. According to the reagent $R^1Y$, the suitable C-C coupling reaction is selected. If $R^1Y$ is a boronic acid ($R^1B(OH)_2$), Suzuki coupling reaction is applied. Product (III) is also synthesized by Stille coupling reaction of intermediate compound **8*** with a stannane compound ($R^1Sn(Bu)_3$). Further, the intermediate compound **8*** can be couplied with an acetylene derivate ($R^1Y$, Y = H) by Sonogashira coupling reaction to give product (III).

As alternative synthetic approach, 6-iodo-[1,2,4]triazolo[4,3-*a*]pyridine **9*** is synthesized by cyclization reaction of pyridinyl hydrazine **7*** with alkyl orthoformate which has preferably methyl or ethyl as alkyl group. After first Suzuki coupling of intermediate compound **9*** with a suitable boronic acid $R^1B(OH)_2$, resulting monosubstituted [1,2,4]triazolo[4,3-a]pyridine **10*** is brominated. Second Suzuki coupling of brominated intermediate **11*** with another suitable boronic acid $R^2B(OH)_2$ gives the product (III).

Therefore, the synthetic approaches according to scheme 2 enable the synthesis of any of the [1,2,4]triazolo[4,3-a]pyridine derivatives of the general formula (III) disclosed in the present invention.

## Scheme 2

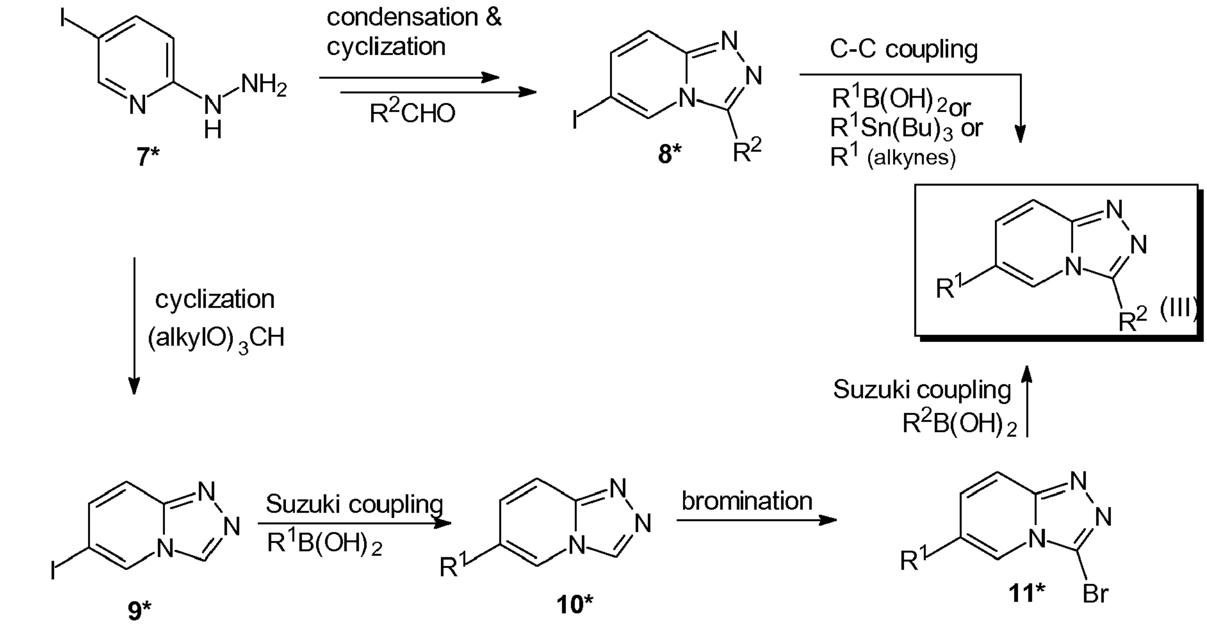


7* 8* (III) 9* 10* 11*

Synthetic method of general formula (IV)

In scheme 3 synthetic approaches of compound (IV) having imidazo[1,2-*a*]pyrazine as main scaffold are shown. An intermediate compound, 6-bromo-imidazo[1,2-a]pyrazine **16*** is synthesized by condensation reaction of compound **12*** with bromoacetaldehyde dialkylacetal which has preferably methyl or ethyl as alkyl group. Another intermediate 6-bromo-3-iodo-imidazo[1,2-a]pyrazine **17*** is produced by iodations of compound **16***. At first by Suzuki coupling reaction of the intermediate **17*** with a suitable boronic acid $R^2B(OH)_2$, or Stille coupling reaction of the intermediate **17*** with a suitable stannane $R^2Sn(nBu)_3$, $R^2$ group is substituted regioselectively at C-3 position, because iodide at C-3 postion is more reactive than bromide at C-6 position. Following second Suzuki coupling reaction of the resulting compound with another suitable boronic acid $R^1B(OH)_2$ gives 6-$R^1$-3-$R^2$-imidazo[1,2-a]pyrazine (IV) as desired product.

As alternative synthetic route, the intermediate **16*** can be firstly coupled with a subitable boronic acid $R^1B(OH)_2$. After iodation of the resulting intermediate 6-$R^1$-imidazo[1,2-a]pyrazine **19***, the second Suzuki coupling reaction of 3-iodo-6-$R^1$-imidazo[1,2-a]pyrazine **20*** with another suitable boronic acid $R^2B(OH)_2$ is followed.

If $R^2$ has an amide bond which is connected directly to imidazo[1,2-a]pyrazine ($R^2$ = $-NHCO-(CH_2)_m-R^4$), another synthetic approach is available. Intermidiate compound **13*** having amino group at C-3 position is obtained by condensation reaction of tosylated starting material with bromoacetonitrile and following cylization reaction. Coupling of compound **13*** and a suitable acyl chloride **14*** that is synthesized *in situ* from corresponding carboxylic acid or is commercially available provides monosubstituted imidazo[1,2-a]pyrazine **15***. In this synthetic step another alternative coupling methods can be used instead of the abovementioned coupling reaction. In alternative coupling methods, carboxylic acid can be activated by bromination or another activating reagents such as carbodiimides and triazoles. Suzuki coupling of intermediate **15*** with a suitable boronic acid $R^1B(OH)_2$ gives product (IV).

Therefore, the synthetic approaches according to scheme 3 enable the synthesis of any of the imidazo[1,2-a]pyrazine derivatives of the general formula (IV) disclosed in the present invention.

## Scheme 3

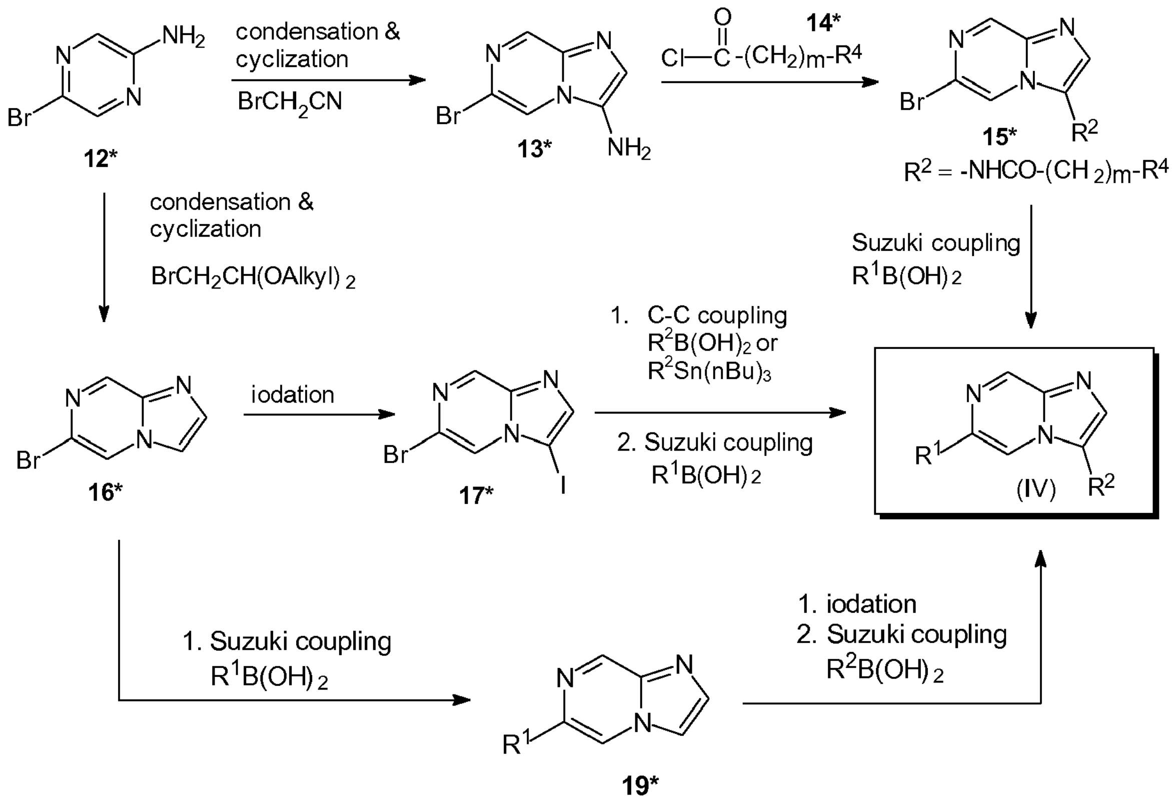


Most preferred compounds are selected from the group consisting of:

| compound | name |
|---|---|
| 1 | 4-[6-[(4-fluorophenyl)methyl]imidazo[1,2-b]pyridazin-3-yl]benzamide |
| 2 | 4-(6-benzylimidazo[1,2-b]pyridazin-3-yl)benzamide |
| 3 | 6-(1-methylpyrazol-4-yl)-3-(2-thienyl)imidazo[1,2-b]pyridazine |
| 4 | N-(2-dimethylaminoethyl)-3-[6-(4-hydroxy-3-methoxy-phenyl)imidazo[1,2-b]pyridazin-3-yl]benzamide |
| 5 | (2S)-2-[[3-(4-aminophenyl)imidazo[1,2-b]pyridazin-6-yl]amino]-3-methyl-butan-1-ol |
| 6 | 3-(2,4-dimethoxyphenyl)-N-(2-thienylmethyl)imidazo[1,2-b]pyridazin-6-amine |
| 7 | 4-[6-(2-methoxyethylamino)imidazo[1,2-b]pyridazin-3-yl]-N-(4-methoxyphenyl)benzamide |
| 8 | 2-[[3-[(E)-hex-1-enyl]imidazo[1,2-b]pyridazin-6-yl]amino]butan-1-ol |
| 9 | 2-[[3-(2-chlorophenyl)imidazo[1,2-b]pyridazin-6-yl]amino]butan-1-ol |
| 10 | 3-(3-pyridyl)-6-(3,4,5-trimethoxyphenyl)imidazo[1,2-b]pyridazine |
| 11 | 6-(3,4-dimethoxyphenyl)-3-(4-pyridyl)imidazo[1,2-b]pyridazine |
| 12 | N-[3-[3-(3-acetamidophenyl)imidazo[1,2-b]pyridazin-6-yl]phenyl]acetamide |
| 13 | 2-methoxy-4-[6-(3,4,5-trimethoxyphenyl)imidazo[1,2-b]pyridazin-3-yl]phenol |
| 14 | N-(2-dimethylaminoethyl)-3-[6-[3-(methanesulfonamido)phenyl]imidazo[1,2-b]pyridazin-3-yl]benzamide |
| 15 | N-[(3-chlorophenyl)methyl]-3-[4-(trifluoromethoxy)phenyl]imidazo[1,2-b]pyridazin-6-amine |

(continued)

| compound | name |
|---|---|
| **16** | 4-[6-[(3-chlorophenyl)methylamino]imidazo[1,2-b]pyridazin-3-yl]-2-methoxy-phenol |
| **17** | methyl 4-[6-[(3-chlorophenyl)methylamino]imidazo[1,2-b]pyridazin-3-yl]benzoate |
| **18** | N-[(4-fluorophenyl)methyl]-3-(3-thienyl)imidazo[1,2-b]pyridazin-6-amine |
| **19** | N-[3-[6-[(4-fluorophenyl)methylamino]imidazo[1,2-b]pyridazin-3-yl]phenyl]acetamide |
| **20** | 4-[6-(propylamino)imidazo[1,2-b]pyridazin-3-yl]benzoic acid |
| **21** | (E)-3-[3-[6-(propylamino)imidazo[1,2-b]pyridazin-3-yl]phenyl]prop-2-enoic acid |
| **22** | 3-(3-aminophenyl)-N-[(3,4-dichlorophenyl)methyl]imidazo[1,2-b]pyridazin-6-amine |
| **23** | 3-(4-fluorophenyl)-N-(2-methoxyethyl)imidazo[1,2-b]pyridazin-6-amine |
| **24** | 3-(4-morpholinophenyl)-N-[2-(3-pyridyl)ethyl]imidazo[1,2-b]pyridazin-6-amine |
| **25** | 3-(2-naphthyl)-N-[2-(3-pyridyl)ethyl]imidazo[1,2-b]pyridazin-6-amine |
| **26** | N-(1,3-benzodioxol-5-ylmethyl)-3-(4-morpholinophenyl)imidazo[1,2-b]pyridazin-6-amine |
| **27** | N-(1,3-benzodioxol-5-ylmethyl)-3-(8-quinolyl)imidazo[1,2-b]pyridazin-6-amine |
| **28** | N-(1,3-benzodioxol-5-ylmethyl)-3-(4-chlorophenyl)imidazo[1,2-b]pyridazin-6-amine |
| **29** | 3-(2-fluorophenyl)-N-(2-methoxyethyl)imidazo[1,2-b]pyridazin-6-amine |
| **30** | (E)-3-[3-[6-(1,3-benzodioxol-5-ylmethylamino)imidazo[1,2-b]pyridazin-3-yl]phenyl]prop-2-enoic acid |
| **31** | 3-(2-phenoxyphenyl)-N-(4-pyridylmethyl)imidazo[1,2-b]pyridazin-6-amine |
| **32** | 4-[(3-bromoimidazo[1,2-b]pyridazin-6-yl)amino]cyclohexanol |
| **33** | 3-(3-aminophenyl)-N-tetrahydropyran-4-yl-imidazo[1,2-b]pyridazin-6-amine |
| **34** | 3-(4-phenoxyphenyl)-N-tetrahydropyran-4-yl-imidazo[1,2-b]pyridazin-6-amine |
| **35** | 3-(benzofuran-2-yl)-N-[(4-methoxyphenyl)methyl]imidazo[1,2-b]pyridazin-6-amine |
| **36** | 4-[6-[(4-methoxyphenyl)methylamino]imidazo[1,2-b]pyridazin-3-yl]phenol |
| **37** | 3-(1H-indol-5-yl)-N-[(4-methoxyphenyl)methyl]imidazo[1,2-b]pyridazin-6-amine |
| **38** | 3-(1-naphthyl)-N-[2-(2-pyridyl)ethyl]imidazo[1,2-b]pyridazin-6-amine |
| **39** | 3-(2,4-dimethoxyphenyl)-N-[(4-methoxyphenyl)methyl]imidazo[1,2-b]pyridazin-6-amine |
| **40** | 3-[[3-(2-furyl)imidazo[1,2-b]pyridazin-6-yl]amino]propan-1-ol |
| **41** | 3-[[3-(4-methoxyphenyl)imidazo[1,2-b]pyridazin-6-yl]amino]propan-1-ol |
| **42** | 3-[[3-(2,4-dimethoxyphenyl)imidazo[1,2-b]pyridazin-6-yl]amino]propan-1-ol |
| **43** | N-(3-morpholinopropyl)-3-(3,4,5-trimethoxyphenyl)imidazo[1,2-b]pyridazin-6-amine |
| **44** | 3-bromo-N-(3-morpholinopropyl)imidazo[1,2-b]pyridazin-6-amine |
| **45** | (2S)-3-methyl-2-[[3-(2-naphthyl)imidazo[1,2-b]pyridazin-6-yl]amino]butan-1-ol |
| **46** | (2S)-2-[[3-(2,4-dimethoxyphenyl)imidazo[1,2-b]pyridazin-6-yl]amino]-3-methyl-butan-1-ol |
| **47** | 3-(3,4-dimethoxyphenyl)-N-(2-pyridylmethyl)imidazo[1,2-b]pyridazin-6-amine |
| **48** | 3-(5-isopropyl-2-methoxy-phenyl)-N-(2-pyridylmethyl)imidazo[1,2-b]pyridazin-6-amine |
| **49** | 3-(4-dimethylaminophenyl)-N-[(3,4,5-trimethoxyphenyl)methyl]imidazo[1,2-b]pyridazin-6-amine |
| **50** | N',N'-dimethyl-N-[3-(p-tolyl)imidazo[1,2-b]pyridazin-6-yl]ethane-1,2-diamine |
| **51** | N-(cyclopropylmethyl)-3-(6-methoxy-3-pyridyl)imidazo[1,2-b]pyridazin-6-amine |
| **52** | 4-[6-[(2,4-dimethylphenyl)methylamino]imidazo[1,2-b]pyridazin-3-yl]phenol |
| **53** | 3-[4-[6-[(2,4-dimethylphenyl)methylamino]imidazo[1,2-b]pyridazin-3-yl]phenyl]propanoic acid |

(continued)

| compound | name |
|---|---|
| 54 | N-(2-dimethylaminoethyl)-4-[6-[(2,4-dimethylphenyl)methylamino]imidazo[1,2-b]pyridazin-3-yl]benzamide |
| 55 | N-[(2,4-dimethylphenyl)methyl]-3-(3-methoxyphenyl)imidazo[1,2-b]pyridazin-6-amine |
| 56 | 4-[[[3-(1,3-benzodioxol-5-yl)imidazo[1,2-b]pyridazin-6-yl]amino]methyl]benzenesulfonamide |
| 57 | 4-[[[3-(1-benzylpyrazol-4-yl)imidazo[1,2-b]pyridazin-6-yl]amino]methyl]benzenesulfonamide |
| 58 | 4-[6-[[4-(4-methylpiperazin-1-yl)phenyl]methylamino]imidazo[1,2-b]pyridazin-3-yl]benzonitrile |
| 59 | (Z)-5-[6-(methylamino)imidazo[1,2-b]pyridazin-3-yl]pent-4-en-1-ol |
| 60 | 2-[6-(methylamino)imidazo[1,2-b]pyridazin-3-yl]phenol |
| 61 | N,N-dimethyl-3-[6-(methylamino)imidazo[1,2-b]pyridazin-3-yl]benzamide |
| 62 | 1-[2-[6-(methylamino)imidazo[1,2-b]pyridazin-3-yl]phenyl]ethanone |
| 63 | 3-[4-(dimethylaminomethyl)phenyl]-N-methyl-imidazo[1,2-b]pyridazin-6-amine |
| 64 | 3-(3,3-dimethylbut-1-ynyl)-N-methyl-imidazo[1,2-b]pyridazin-6-amine |
| 65 | N-[2-[6-(methylamino)imidazo[1,2-b]pyridazin-3-yl]phenyl]acetamide |
| 66 | 3-methyl-4-[6-(methylamino)imidazo[1,2-b]pyridazin-3-yl]phenol |
| 67 | 3-[(5-imidazo[1,2-b]pyridazin-3-yl-2-pyridyl)oxy]-N,N-dimethyl-propan-1-amine |
| 68 | 1-(2-imidazo[1,2-b]pyridazin-3-ylphenyl)-N,N-dimethyl-methanamine |
| 69 | 3-[6-(4-methylpiperazin-1-yl)-3-pyridyl]imidazo[1,2-b]pyridazine |
| 70 | 3-(benzothiophen-2-yl)-N-[(3,4,5-trimethoxyphenyl)methyl]imidazo[1,2-b]pyridazin-6-amine |
| 71 | 3-dibenzofuran-4-ylimidazo[1,2-b]pyridazine |
| 72 | 3-(4-methylsulfanylphenyl)imidazo[1,2-b]pyridazine |
| 73 | 3-(4-chlorophenyl)imidazo[1,2-b]pyridazine |
| 74 | 3-[(E)-styryl]imidazo[1,2-b]pyridazine |
| 75 | 2-imidazo[1,2-b]pyridazin-3-ylbenzoic acid |
| 76 | 3-(3-ethoxyphenyl)imidazo[1,2-b]pyridazine |
| 77 | 4-imidazo[1,2-b]pyridazin-3-yl-2,6-dimethyl-phenol |
| 78 | N-(2-hydroxyethyl)-4-imidazo[1,2-b]pyridazin-3-yl-benzamide |
| 79 | (4-imidazo[1,2-b]pyridazin-3-ylphenyl)-(4-methylpiperazin-1-yl)methanone |
| 80 | 3-(2,3-dihydrobenzofuran-5-yl)imidazo[1,2-b]pyridazine |
| 81 | 3-(3-fluoro-4-methyl-phenyl)imidazo[1,2-b]pyridazine |
| 82 | 3-imidazo[1,2-b]pyridazin-3-ylbenzonitrile |
| 83 | 3-(3,4-difluorophenyl)imidazo[1,2-b]pyridazine |
| 84 | 3-(m-tolyl)imidazo[1,2-b]pyridazine |
| 85 | 3-(4-ethoxyphenyl)imidazo[1,2-b]pyridazine |
| 86 | 3-(2-methylsulfanylphenyl)imidazo[1,2-b]pyridazine |
| 87 | 1-(4-imidazo[1,2-b]pyridazin-3-ylphenyl)ethanone |
| 88 | 5-imidazo[1,2-b]pyridazin-3-ylquinoline |
| 89 | N-cyclopropyl-4-imidazo[1,2-b]pyridazin-3-yl-benzamide |
| 90 | 4-imidazo[1,2-b]pyridazin-3-ylisoquinoline |

(continued)

| compound | name |
|---|---|
| 91 | (2-imidazo[1,2-b]pyridazin-3-ylphenyl)methanol |
| 92 | 3-(2-fluoro-3-methoxy-phenyl)imidazo[1,2-b]pyridazine |
| 93 | (3-imidazo[1,2-b]pyridazin-3-ylphenyl)-morpholino-methanone |
| 94 | 2-(4-imidazo[1,2-b]pyridazin-3-ylphenyl)acetonitrile |
| 95 | N-(2-furylmethyl)-3-imidazo[1,2-b]pyridazin-3-yl-benzamide |
| 96 | N-(4-imidazo[1,2-b]pyridazin-3-ylphenyl)methanesulfonamide |
| 97 | 4-[(4-imidazo[1,2-b]pyridazin-3-ylphenyl)methyl]morpholine |
| 98 | 3-(1-isobutylpyrazol-4-yl)imidazo[1,2-b]pyridazine |
| 99 | N-cyclopropyl-3-imidazo[1,2-b]pyridazin-3-yl-benzamide |
| 100 | 4-(3-phenylimidazo[1,2-b]pyridazin-6-yl)benzamide |
| 101 | 3-(1,3-benzodioxol-5-yl)-6-phenyl-imidazo[1,2-b]pyridazine |
| 102 | 3-(1,3-benzodioxol-5-yl)-6-[3-(trifluoromethyl)phenyl]imidazo[1,2-b]pyridazine |
| 103 | 3-(3,4-dimethylphenyl)-N-[(3,4,5-trimethoxyphenyl)methyl]imidazo[1,2-b]pyridazin-6-amine |
| 104 | 3-(1,3-benzodioxol-5-yl)-6-(3-fluorophenyl)imidazo[1,2-b]pyridazine |
| 105 | N-[3-[3-(4-pyridyl)imidazo[1,2-b]pyridazin-6-yl]phenyl]methanesulfonamide |
| 106 | [4-[3-(3-pyridyl)imidazo[1,2-b]pyridazin-6-yl]phenyl]methanol |
| 107 | 6-(3-furyl)-3-(3-pyridyl)imidazo[1,2-b]pyridazine |
| 108 | 3-(3-pyridyl)-6-(2-thienyl)imidazo[1,2-b]pyridazine |
| 109 | N-[3-[6-(3-pyridyl)imidazo[1,2-b]pyridazin-3-yl]phenyl]acetamide |
| 110 | N-[3-[6-(3-acetylphenyl)imidazo[1,2-b]pyridazin-3-yl]phenyl]acetamide |
| 111 | N-[3-[6-[(3,4-difluorophenyl)methyl]imidazo[1,2-b]pyridazin-3-yl]phenyl]acetamide |
| 112 | 3-[3-(3-acetamidophenyl)imidazo[1,2-b]pyridazin-6-yl]-N-methyl-benzamide |
| 113 | 3-(3-chloro-4-fluoro-phenyl)-6-(2-methoxyphenyl)imidazo[1,2-b]pyridazine |
| 114 | 3-(3-chloro-4-fluoro-phenyl)-6-(3,4,5-trimethoxyphenyl)imidazo[1,2-b]pyridazine |
| 115 | 6-(2-methoxyphenyl)-3-[3-(trifluoromethoxy)phenyl]imidazo[1,2-b]pyridazine |
| 116 | N-(2-dimethylaminoethyl)-3-[3-[3-(trifluoromethoxy)phenyl]imidazo[1,2-b]pyridazin-6-yl]benzamide |
| 117 | 4-[3-[3-(trifluoromethoxy)phenyl]imidazo[1,2-b]pyridazin-6-yl]benzamide |
| 118 | 3-(4-methyl-2-thienyl)-N-[(3,4,5-trimethoxyphenyl)methyl]imidazo[1,2-b]pyridazin-6-amine |
| 119 | 6-benzyl-3-(4-methylsulfonylphenyl)imidazo[1,2-b]pyridazine |
| 120 | 3-[6-(3-chlorophenyl)imidazo[1,2-b]pyridazin-3-yl]-N-(2-dimethylaminoethyl)benzamide |
| 121 | N-(2-dimethylaminoethyl)-3-[6-[3-(hydroxymethyl)phenyl]imidazo[1,2-b]pyridazin-3-yl]benzamide |
| 122 | 6-[(4-fluorophenyl)methyl]-3-(5-methoxy-3-pyridyl)imidazo[1,2-b]pyridazine |
| 123 | 3-[3-(3-chlorophenyl)imidazo[1,2-b]pyridazin-6-yl]an il ine |
| 124 | 3-(3-chlorophenyl)-6-(3,4-dimethoxyphenyl)imidazo[1,2-b]pyridazine |
| 125 | 3-(3-chlorophenyl)-6-(4-methoxy-2-methyl-phenyl)imidazo[1,2-b]pyridazine |
| 126 | 3-(3-chlorophenyl)-6-(3-methoxyphenyl)imidazo[1,2-b]pyridazine |
| 127 | 3-[6-(4-fluorophenyl)imidazo[1,2-b]pyridazin-3-yl]phenol |
| 128 | 3-[6-(5-quinolyl)imidazo[1,2-b]pyridazin-3-yl]phenol |

(continued)

| compound | name |
|---|---|
| 129 | [4-[6-(4-dimethylaminophenyl)imidazo[1,2-b]pyridazin-3-yl]phenyl]methanol |
| 130 | [4-(6-pyrimidin-5-ylimidazo[1,2-b]pyridazin-3-yl)phenyl]methanol |
| 131 | [4-[6-(1-methylpyrazol-4-yl)imidazo[1,2-b]pyridazin-3-yl]phenyl]methanol |
| 132 | N-(2-hydroxyethyl)-3-[3-[3-(hydroxymethyl)phenyl]imidazo[1,2-b]pyridazin-6-yl]benzamide |
| 133 | [3-[6-(3-phenoxyphenyl)imidazo[1,2-b]pyridazin-3-yl]phenyl]methanol |
| 134 | 6-(4-pyridyl)-3-[3-(trifluoromethyl)phenyl]imidazo[1,2-b]pyridazine |
| 135 | 3-[3-[3-(trifluoromethyl)phenyl]imidazo[1,2-b]pyridazin-6-yl]phenol |
| 136 | 6-cyclopropyl-3-[3-(trifluoromethyl)phenyl]imidazo[1,2-b]pyridazine |
| 137 | 3-(3-fluorophenyl)-6-[(4-fluorophenyl)methyl]imidazo[1,2-b]pyridazine |
| 138 | 2-methoxy-4-[6-[3-(trifluoromethoxy)phenyl]imidazo[1,2-b]pyridazin-3-yl]phenol |
| 139 | 3-[3-(dimethylamino)phenyl]-N-(2-furylmethyl)imidazo[1,2-b]pyridazin-6-amine |
| 140 | 4-[6-(2-furylmethylamino)imidazo[1,2-b]pyridazin-3-yl]benzoic acid |
| 141 | N-[3-[3-(3-furyl)imidazo[1,2-b]pyridazin-6-yl]phenyl]acetamide |
| 142 | 3-[3-(3-furyl)imidazo[1,2-b]pyridazin-6-yl]benzoic acid |
| 143 | 3-(3-furyl)-6-(5-methoxy-3-pyridyl)imidazo[1,2-b]pyridazine |
| 144 | N-[4-[3-(3-furyl)imidazo[1,2-b]pyridazin-6-yl]phenyl]acetamide |
| 145 | 4-[6-[(3,4-difluorophenyl)methyl]imidazo[1,2-b]pyridazin-3-yl]benzamide |
| 146 | 4-[6-(m-tolylmethyl)imidazo[1,2-b]pyridazin-3-yl]benzamide |
| 147 | N-[4-[6-(4-morpholinophenyl)imidazo[1,2-b]pyridazin-3-yl]phenyl]acetamide |
| 148 | N-[4-[6-(1,3-benzodioxol-5-yl)imidazo[1,2-b]pyridazin-3-yl]phenyl]acetamide |
| 149 | N-[4-[6-(4-methylsulfonylphenyl)imidazo[1,2-b]pyridazin-3-yl]phenyl]acetamide |
| 150 | 6-benzyl-3-pyrimidin-5-yl-imidazo[1,2-b]pyridazine |
| 151 | 6-[(4-fluorophenyl)methyl]-3-(4-methoxy-2-methyl-phenyl)imidazo[1,2-b]pyridazine |
| 152 | 6-[(4-fluorophenyl)methyl]-3-(3-phenoxyphenyl)imidazo[1,2-b]pyridazine |
| 153 | 1-[3-[6-(6-amino-3-pyridyl)imidazo[1,2-b]pyridazin-3-yl]phenyl]ethanone |
| 154 | 3-[3,5-bis(trifluoromethyl)phenyl]-N-(2-thienylmethyl)imidazo[1,2-b]pyridazin-6-amine |
| 155 | N,N-dimethyl-3-[3-(2-thienyl)imidazo[1,2-b]pyridazin-6-yl]aniline |
| 156 | 6-(3-chloro-4-fluoro-phenyl)-3-(2-thienyl)imidazo[1,2-b]pyridazine |
| 157 | 2-methoxy-4-[3-(2-thienyl)imidazo[1,2-b]pyridazin-6-yl]phenol |
| 158 | 6-(2-chlorophenyl)-3-(2-thienyl)imidazo[1,2-b]pyridazine |
| 159 | N-[3-[6-[(4-fluorophenyl)methyl]imidazo[1,2-b]pyridazin-3-yl]phenyl]methanesulfonamide |
| 160 | 3-(1-methylpyrazol-4-yl)-6-(m-tolylmethyl)imidazo[1,2-b]pyridazine |
| 161 | 5-(6-benzylimidazo[1,2-b]pyridazin-3-yl)pyridin-2-amine |
| 162 | (4Z)-4-[dimethylamino(imidazo[1,2-b]pyridazin-3-yl)methylene]-2-phenyl-oxazol-5-one |
| 163 | 3-(3-bromophenyl)-N-(2-thienylmethyl)imidazo[1,2-b]pyridazin-6-amine |
| 164 | 6-(1,3-benzodioxol-5-yl)-3-phenyl-[1,2,4]triazolo[4,3-a]pyridine |
| 165 | N-[3-(3-phenyl-[1,2,4]triazolo[4,3-a]pyridin-6-yl)phenyl]acetamide |
| 166 | 6-(4-methoxy-2-methyl-phenyl)-3-phenyl-[1,2,4]triazolo[4,3-a]pyridine |

(continued)

| compound | name |
|---|---|
| **167** | 3-phenyl-6-[2-(2-pyridyl)ethynyl]-[1,2,4]triazolo[4,3-a]pyridine |
| **168** | N,N-dimethyl-3-(3-phenyl-[1,2,4]triazolo[4,3-a]pyridin-6-yl)prop-2-yn-1-amine |
| **169** | N-[3-(3-phenyl-[1,2,4]triazolo[4,3-a]pyridin-6-yl)phenyl]methanesulfonamide |
| **170** | 3-(1,3-benzodioxol-5-yl)-6-(3-chlorophenyl)-[1,2,4]triazolo[4,3-a]pyridine |
| **171** | 3-(1,3-benzodioxol-5-yl)-6-(3-furyl)-[1,2,4]triazolo[4,3-a]pyridine |
| **172** | 3-(1,3-benzodioxol-5-yl)-6-[2-(3-methylimidazol-4-yl)ethynyl]-[1,2,4]triazolo[4,3-a]pyridine |
| **173** | 3-[3-(1,3-benzodioxol-5-yl)-[1,2,4]triazolo[4,3-a]pyridin-6-yl]prop-2-yn-1-ol |
| **174** | 3-(1,3-benzodioxol-5-yl)-6-(1-methylpyrazol-4-yl)-[1,2,4]triazolo[4,3-a]pyridine |
| **175** | 3-[3-(1,3-benzodioxol-5-yl)-[1,2,4]triazolo[4,3-a]pyridin-6-yl]-N-methyl-benzam ide |
| **176** | 4-[4-[3-(4-pyridyl)-[1,2,4]triazolo[4,3-a]pyridin-6-yl]phenyl]morpholine |
| **177** | 6-(3,4-dimethoxyphenyl)-3-(4-pyridyl)-[1,2,4]triazolo[4,3-a]pyridine |
| **178** | 3-(4-pyridyl)-6-[3-(trifluoromethoxy)phenyl]-[1,2,4]triazolo[4,3-a]pyridine |
| **179** | [4-[3-(4-pyridyl)-[1,2,4]triazolo[4,3-a]pyridin-6-yl]phenyl]methanol |
| **180** | 3-(4-pyridyl)-6-[2-[3-(trifluoromethyl)phenyl]ethynyl]-[1,2,4]triazolo[4,3-a]pyridine |
| **181** | 6-(3-phenoxyphenyl)-3-(4-pyridyl)-[1,2,4]triazolo[4,3-a]pyridine |
| **182** | N,N-dimethyl-4-[3-(3-pyridyl)-[1,2,4]triazolo[4,3-a]pyridin-6-yl]aniline |
| **183** | 6-(3-isopropylphenyl)-3-(3-pyridyl)-[1,2,4]triazolo[4,3-a]pyridine |
| **184** | 6-(2-chlorophenyl)-3-(3-pyridyl)-[1,2,4]triazolo[4,3-a]pyridine |
| **185** | 3-[3-(3,4-dimethoxyphenyl)-[1,2,4]triazolo[4,3-a]pyridin-6-yl]-N,N-dimethyl-aniline |
| **186** | 3-(3,4-dimethoxyphenyl)-6-(4-methylsulfonylphenyl)-[1,2,4]triazolo[4,3-a]pyridine |
| **187** | 3-(3,4-dimethoxyphenyl)-6-(3-fluorophenyl)-[1,2,4]triazolo[4,3-a]pyridine |
| **188** | 3-[3-(3,4-dimethoxyphenyl)-[1,2,4]triazolo[4,3-a]pyridin-6-yl]-N,N-dimethyl-benzamide |
| **189** | 3-(3,4-dimethoxyphenyl)-6-[2-(3-pyridyl)ethynyl]-[1,2,4]triazolo[4,3-a]pyridine |
| **190** | 3-(3,4-dimethoxyphenyl)-6-[2-(4-pyridyl)ethynyl]-[1,2,4]triazolo[4,3-a]pyridine |
| **191** | 3-(3,4-dimethoxyphenyl)-6-(2-thienyl)-[1,2,4]triazolo[4,3-a]pyridine |
| **192** | 6-(5-methoxy-3-pyridyl)-3-[3-(trifluoromethoxy)phenyl]-[1,2,4]triazolo[4,3-a]pyridine |
| **193** | 6-(3-methylsulfonylphenyl)-3-[3-(trifluoromethoxy)phenyl]-[1,2,4]triazolo[4,3-a]pyridine |
| **194** | 6-[2-(3-methoxyphenyl)ethynyl]-3-[3-(trifluoromethoxy)phenyl]-[1,2,4]triazolo[4,3-a]pyridine |
| **195** | 6-(2-thienyl)-3-[3-(trifluoromethoxy)phenyl]-[1,2,4]triazolo[4,3-a]pyridine |
| **196** | 6-(2-phenylethynyl)-3-[3-(trifluoromethoxy)phenyl]-[1,2,4]triazolo[4,3-a]pyridine |
| **197** | 3-[3-(3-chlorophenyl)-[1,2,4]triazolo[4,3-a]pyridin-6-yl]an il ine |
| **198** | 6-(1,3-benzodioxol-5-yl)-3-(3-chlorophenyl)-[1,2,4]triazolo[4,3-a]pyridine |
| **199** | 3-(3-chlorophenyl)-6-(4-pyridyl)-[1,2,4]triazolo[4,3-a]pyridine |
| **200** | 3-(3-chlorophenyl)-6-(3-pyridyl)-[1,2,4]triazolo[4,3-a]pyridine |
| **201** | 3-(3-chlorophenyl)-6-(3-furyl)-[1,2,4]triazolo[4,3-a]pyridine |
| **202** | N-[4-[3-(3-chlorophenyl)-[1,2,4]triazolo[4,3-a]pyridin-6-yl]phenyl]acetamide |
| **203** | 3-(3-chlorophenyl)-6-pyrimidin-5-yl-[1,2,4]triazolo[4,3-a]pyridine |
| **204** | 3-(3-chlorophenyl)-6-pyrimidin-2-yl-[1,2,4]triazolo[4,3-a]pyridine |

(continued)

| compound | name |
|---|---|
| **205** | 4-[3-(3-chlorophenyl)-[1,2,4]triazolo[4,3-a]pyridin-6-yl]isoquinoline |
| **206** | 4-[3-(3-hydroxyphenyl)-[1,2,4]triazolo[4,3-a]pyridin-6-yl]benzonitrile |
| **207** | 3-[6-(4-isopropylphenyl)-[1,2,4]triazolo[4,3-a]pyridin-3-yl]phenol |
| **208** | 3-[6-(4-fluorophenyl)-[1,2,4]triazolo[4,3-a]pyridin-3-yl]phenol |
| **209** | 3-[6-(5-quinolyl)-[1,2,4]triazolo[4,3-a]pyridin-3-yl]phenol |
| **210** | 3-[3-(trifluoromethyl)phenyl]-6-(3,4,5-trimethoxyphenyl)-[1,2,4]triazolo[4,3-a]pyridine |
| **211** | N-[3-(dimethylamino)propyl]-4-[3-[3-(trifluoromethyl)phenyl]-[1,2,4]triazolo[4,3-a]pyridin-6-yl]benzamide |
| **212** | morpholino-[4-[3-[3-(trifluoromethyl)phenyl]-[1,2,4]triazolo[4,3-a]pyridin-6-yl]phenyl]methanone |
| **213** | N,N-dimethyl-4-[3-[3-(trifluoromethyl)phenyl]-[1,2,4]triazolo[4,3-a]pyridin-6-yl]benzamide |
| **214** | 5-[3-[3-(trifluoromethyl)phenyl]-[1,2,4]triazolo[4,3-a]pyridin-6-yl]pyridin-2-amine |
| **215** | 2-methoxy-4-[6-[4-(4-methylpiperazin-1-yl)phenyl]-[1,2,4]triazolo[4,3-a]pyridin-3-yl]phenol |
| **216** | 2-methoxy-4-[6-(6-methoxy-3-pyridyl)-[1,2,4]triazolo[4,3-a]pyridin-3-yl]phenol |
| **217** | 4-[6-(3-fluorophenyl)-[1,2,4]triazolo[4,3-a]pyridin-3-yl]-2-methoxy-phenol |
| **218** | 2-methoxy-4-[6-(o-tolyl)-[1,2,4]triazolo[4,3-a]pyridin-3-yl]phenol |
| **219** | 3-(3-furyl)-6-(3,4,5-trimethoxyphenyl)-[1,2,4]triazolo[4,3-a]pyridine |
| **220** | 3-(3-furyl)-6-(4-methoxyphenyl)-[1,2,4]triazolo[4,3-a]pyridine |
| **221** | 3-(3-furyl)-6-(2-thienyl)-[1,2,4]triazolo[4,3-a]pyridine |
| **222** | 3-[6-(2-pyridyl)-[1,2,4]triazolo[4,3-a]pyridin-3-yl]benzonitrile |
| **223** | 6-(3,4-dimethoxyphenyl)-3-(o-tolyl)-[1,2,4]triazolo[4,3-a]pyridine |
| **224** | N-(2-hydroxyethyl)-3-[3-(o-tolyl)-[1,2,4]triazolo[4,3-a]pyridin-6-yl]benzamide |
| **225** | 4-[3-(o-tolyl)-[1,2,4]triazolo[4,3-a]pyridin-6-yl]benzamide |
| **226** | 4-[4-(3-thiazol-4-yl-[1,2,4]triazolo[4,3-a]pyridin-6-yl)phenyl]morpholine |
| **227** | N,N-dimethyl-3-(3-thiazol-4-yl-[1,2,4]triazolo[4,3-a]pyridin-6-yl)aniline |
| **228** | 4-[6-(3-chloro-4-fluoro-phenyl)-[1,2,4]triazolo[4,3-a]pyridin-3-yl]thiazole |
| **229** | 4-[6-(1H-indol-5-yl)-[1,2,4]triazolo[4,3-a]pyridin-3-yl]thiazole |
| **230** | 3-(3-thiazol-4-yl-[1,2,4]triazolo[4,3-a]pyridin-6-yl)benzamide |
| **231** | 4-[6-(4-methoxy-2-methyl-phenyl)-[1,2,4]triazolo[4,3-a]pyridin-3-yl]thiazole |
| **232** | 4-[3-(3-thiazol-4-yl-[1,2,4]triazolo[4,3-a]pyridin-6-yl)prop-2-ynyl]-1,4-thiazinane 1,1-dioxide |
| **233** | 4-[6-(2-chlorophenyl)-[1,2,4]triazolo[4,3-a]pyridin-3-yl]thiazole |
| **234** | 4-[6-(3-methoxyphenyl)-[1,2,4]triazolo[4,3-a]pyridin-3-yl]thiazole |
| **235** | 4-[6-(6-quinolyl)-[1,2,4]triazolo[4,3-a]pyridin-3-yl]thiazole |
| **236** | 3-[4-[6-(3,4-dimethoxyphenyl)-[1,2,4]triazolo[4,3-a]pyridin-3-yl]phenoxy]-N,N-dimethyl-propan-1-amine |
| **237** | 3-[4-[6-(4-chlorophenyl)-[1,2,4]triazolo[4,3-a]pyridin-3-yl]phenoxy]-N,N-dimethyl-propan-1-amine |
| **238** | 4-[2-[3-[4-[3-(dimethylamino)propoxy]phenyl]-[1,2,4]triazolo[4,3-a]pyridin-6-yl]ethynyl]-N,N-dimethyl-benzamide |
| **239** | 3-[4-[6-[2-(3,5-dimethoxyphenyl)ethynyl]-[1,2,4]triazolo[4,3-a]pyridin-3-yl]phenoxy]-N,N-dimethyl-propan-1-amine |

(continued)

| compound | name |
|---|---|
| **240** | 4-[3-[4-[3-(dimethylamino)propoxy]phenyl]-[1,2,4]triazolo[4,3-a]pyridin-6-yl]-N-methyl-benzamide |
| **241** | N-[3-(3-quinoxalin-6-yl-[1,2,4]triazolo[4,3-a]pyridin-6-yl)phenyl]acetamide |
| **242** | N-(2-dimethylaminoethyl)-3-(3-quinoxalin-6-yl-[1,2,4]triazolo[4,3-a]pyridin-6-yl)benzamide |
| **243** | (4-methylpiperazin-1-yl)-[3-(3-quinoxalin-6-yl-[1,2,4]triazolo[4,3-a]pyridin-6-yl)phenyl]methanone |
| **244** | 2-methoxy-4-(3-quinoxalin-6-yl-[1,2,4]triazolo[4,3-a]pyridin-6-yl)phenol |
| **245** | 6-[6-(3-furyl)-[1,2,4]triazolo[4,3-a]pyridin-3-yl]quinoxaline |
| **246** | N,N-dimethyl-3-(3-quinoxalin-6-yl-[1,2,4]triazolo[4,3-a]pyridin-6-yl)benzamide |
| **247** | 3-[3-(2-pyridyl)-[1,2,4]triazolo[4,3-a]pyridin-6-yl]phenol |
| **248** | [3-[3-(2-pyridyl)-[1,2,4]triazolo[4,3-a]pyridin-6-yl]phenyl]methanol |
| **249** | 6-(3-methylsulfonylphenyl)-3-(2-pyridyl)-[1,2,4]triazolo[4,3-a]pyridine |
| **250** | N-[6-(2,3-dichlorophenyl)imidazo[1,2-a]pyrazin-3-yl]-2-(2-methoxyphenyl)acetamide |
| **251** | N-[6-(4-isopropylphenyl)imidazo[1,2-a]pyrazin-3-yl]-2-(2-methoxyphenyl)acetam ide |
| **252** | N-[6-[2-(dimethylaminomethyl)phenyl]imidazo[1,2-a]pyrazin-3-yl]-2-(3-methoxyphenyl)acetamide |
| **253** | N-[6-[(E)-hex-1-enyl]imidazo[1,2-a]pyrazin-3-yl]-2-(3-methoxyphenyl)acetamide |
| **254** | N-[6-(1H-indol-5-yl)imidazo[1,2-a]pyrazin-3-yl]-2-(3-methoxyphenyl)acetamide |
| **255** | N-[6-[3-(hydroxymethyl)phenyl]imidazo[1,2-a]pyrazin-3-yl]-2-(3-methoxyphenyl)acetamide |
| **256** | 2-(3-methoxyphenyl)-N-[6-[3-(trifluoromethyl)phenyl]imidazo[1,2-a]pyrazin-3-yl]acetamide |
| **257** | N-[6-(4-fluorophenyl)imidazo[1,2-a]pyrazin-3-yl]-2-(3-methoxyphenyl)acetamide |
| **258** | N-[6-(3-furyl)imidazo[1,2-a]pyrazin-3-yl]-2-(3-methoxyphenyl)acetamide |
| **259** | 2-(3-methoxyphenyl)-N-[6-(4-phenoxyphenyl)imidazo[1,2-a]pyrazin-3-yl]acetamide |
| **260** | 2-(3-methoxyphenyl)-N-[6-(2-thienyl)imidazo[1,2-a]pyrazin-3-yl]acetamide |
| **261** | 2-(3-methoxyphenyl)-N-[6-(1-methylpyrazol-4-yl)imidazo[1,2-a]pyrazin-3-yl]acetamide |
| **262** | N-[6-[(E)-hex-1-enyl]imidazo[1,2-a]pyrazin-3-yl]pyridine-4-carboxamide |
| **263** | N-[6-(3-thienyl)imidazo[1,2-a]pyrazin-3-yl]thiophene-2-carboxamide |
| **264** | N-[6-(4-isopropylphenyl)imidazo[1,2-a]pyrazin-3-yl]acetamide |
| **265** | N-[6-[3-(2-dimethylaminoethylcarbamoyl)phenyl]imidazo[1,2-a]pyrazin-3-yl]furan-2-carboxamide |
| **266** | N-[6-(1-benzylpyrazol-4-yl)imidazo[1,2-a]pyrazin-3-yl]-2-methyl-propanamide |
| **267** | N-[6-(2-methoxyphenyl)imidazo[1,2-a]pyrazin-3-yl]benzamide |
| **268** | N-[6-[3-(dimethylamino)phenyl]imidazo[1,2-a]pyrazin-3-yl]benzamide |
| **269** | N-[6-(4-aminophenyl)imidazo[1,2-a]pyrazin-3-yl]benzamide |
| **270** | N-[6-(1,3-benzodioxol-5-yl)imidazo[1,2-a]pyrazin-3-yl]-2-phenyl-propanamide |
| **271** | N-[6-(3-nitrophenyl)imidazo[1,2-a]pyrazin-3-yl]-2-phenyl-propanamide |
| **272** | 2-(o-tolyl)-N-[6-(1H-pyrazol-4-yl)imidazo[1,2-a]pyrazin-3-yl]acetam ide |
| **273** | N-[6-(1-benzylpyrazol-4-yl)imidazo[1,2-a]pyrazin-3-yl]cyclobutanecarboxam ide |
| **274** | N-[6-(3-acetylphenyl)imidazo[1,2-a]pyrazin-3-yl]-2-cyclopropyl-acetamide |
| **275** | N-[6-(2-naphthyl)imidazo[1,2-a]pyrazin-3-yl]tetrahydrofuran-3-carboxamide |
| **276** | N-[6-(4-methoxyphenyl)imidazo[1,2-a]pyrazin-3-yl]tetrahydrofuran-3-carboxamide |
| **277** | N-[6-(3-aminophenyl)imidazo[1,2-a]pyrazin-3-yl]-2-(3,4-difluorophenyl)acetamide |

(continued)

| compound | name |
|---|---|
| **278** | N-[6-(2-fluorophenyl)imidazo[1,2-a]pyrazin-3-yl]-2-(3-thienyl)acetamide |
| **279** | N-[6-(3-methoxyphenyl)imidazo[1,2-a]pyrazin-3-yl]-2-(3-thienyl)acetamide |
| **280** | N-[6-(2,4-dimethoxyphenyl)imidazo[1,2-a]pyrazin-3-yl]-2-(3-thienyl)acetamide |
| **281** | 4-[6-[6-[3-(dimethylamino)propoxy]-3-pyridyl]imidazo[1,2-a]pyrazin-3-yl]-N,N-dimethyl-aniline |
| **282** | 5-[3-(4-dimethylaminophenyl)imidazo[1,2-a]pyrazin-6-yl]-N-(2-morpholinoethyl)pyridin-2-amine |
| **283** | 2-[3-(4-dimethylaminophenyl)imidazo[1,2-a]pyrazin-6-yl]-N,N-dimethyl-aniline |
| **284** | 4-[3-(4-dimethylaminophenyl)imidazo[1,2-a]pyrazin-6-yl]phenol |
| **285** | 4-[3-(4-dimethylaminophenyl)imidazo[1,2-a]pyrazin-6-yl]-N-[3-(dimethylamino)propyl]benzamide |
| **286** | 3-[3-(4-dimethylaminophenyl)imidazo[1,2-a]pyrazin-6-yl]-N-(2-hydroxyethyl)benzam ide |
| **287** | [4-[3-(4-dimethylaminophenyl)imidazo[1,2-a]pyrazin-6-yl]phenyl]methanol |
| **288** | 4-[6-(6-methoxy-3-pyridyl)imidazo[1,2-a]pyrazin-3-yl]-N,N-dimethyl-aniline |
| **289** | N-(2-dimethylaminoethyl)-4-[3-(4-dimethylaminophenyl)imidazo[1,2-a]pyrazin-6-yl]benzamide |
| **290** | 4-[3-(4-dimethylaminophenyl)imidazo[1,2-a]pyrazin-6-yl]-N-(2-hydroxyethyl)benzamide |
| **291** | [4-[3-(4-dimethylaminophenyl)imidazo[1,2-a]pyrazin-6-yl]phenyl]-morpholino-methanone |
| **292** | 3-[3-(4-dimethylaminophenyl)imidazo[1,2-a]pyrazin-6-yl]benzamide |
| **293** | 3-[3-(4-dimethylaminophenyl)imidazo[1,2-a]pyrazin-6-yl]-N,N-dimethyl-benzamide |
| **294** | 4-[6-(2-chlorophenyl)imidazo[1,2-a]pyrazin-3-yl]-N,N-dimethyl-aniline |
| **295** | N-[3-[3-(4-dimethylaminophenyl)imidazo[1,2-a]pyrazin-6-yl]phenyl]methanesulfonamide |
| **296** | 3-phenyl-6-(3-thienyl)imidazo[1,2-a]pyrazine |
| **297** | 6-(3-fluorophenyl)-3-phenyl-imidazo[1,2-a]pyrazine |
| **298** | 3-phenyl-6-(2-thienyl)imidazo[1,2-a]pyrazine |
| **299** | N-cyclopropyl-4-(3-phenylimidazo[1,2-a]pyrazin-6-yl)benzamide |
| **300** | 3-(1,3-benzodioxol-5-yl)-6-phenyl-imidazo[1,2-a]pyrazine |
| **301** | 3-(1,3-benzodioxol-5-yl)-6-(3-thienyl)imidazo[1,2-a]pyrazine |
| **302** | 3-(1,3-benzodioxol-5-yl)-6-(3-ethoxyphenyl)imidazo[1,2-a]pyrazine |
| **303** | 3-(1,3-benzodioxol-5-yl)-6-(3-chlorophenyl)imidazo[1,2-a]pyrazine |
| **304** | 3-(1,3-benzodioxol-5-yl)-6-(3-fluorophenyl)imidazo[1,2-a]pyrazine |
| **305** | 3-(1,3-benzodioxol-5-yl)-6-(o-tolyl)imidazo[1,2-a]pyrazine |
| **306** | 3-(1,3-benzodioxol-5-yl)-6-(2-thienyl)imidazo[1,2-a]pyrazine |
| **307** | 3-(1,3-benzodioxol-5-yl)-6-(1-benzylpyrazol-4-yl)imidazo[1,2-a]pyrazine |
| **308** | 3-(1,3-benzodioxol-5-yl)-6-(3,5-dimethoxyphenyl)imidazo[1,2-a]pyrazine |
| **309** | 5-[3-(1,3-benzodioxol-5-yl)imidazo[1,2-a]pyrazin-6-yl]pyridin-2-amine |
| **310** | 3-(1,3-benzodioxol-5-yl)-6-(3-isopropoxyphenyl)imidazo[1,2-a]pyrazine |
| **311** | 6-(2-phenoxyphenyl)-3-(4-pyridyl)imidazo[1,2-a]pyrazine |
| **312** | 2,6-dimethyl-4-[3-(4-pyridyl)imidazo[1,2-a]pyrazin-6-yl]phenol |
| **313** | morpholino-[4-[3-(4-pyridyl)imidazo[1,2-a]pyrazin-6-yl]phenyl]methanone |
| **314** | 3-(4-pyridyl)-6-(2-thienyl)imidazo[1,2-a]pyrazine |
| **315** | 4-[4-[3-(3-pyridyl)imidazo[1,2-a]pyrazin-6-yl]phenyl]morpholine |

(continued)

| compound | name |
|---|---|
| **316** | 6-(benzothiophen-2-yl)-3-(3-pyridyl)imidazo[1,2-a]pyrazine |
| **317** | 6-(4-methylsulfanylphenyl)-3-(3-pyridyl)imidazo[1,2-a]pyrazine |
| **318** | N-[3-[3-(3-pyridyl)imidazo[1,2-a]pyrazin-6-yl]phenyl]methanesulfonamide |
| **319** | 6-(2-furyl)-3-(3-thienyl)imidazo[1,2-a]pyrazine |
| **320** | 6-(3-chloro-4-fluoro-phenyl)-3-(3-thienyl)imidazo[1,2-a]pyrazine |
| **321** | N-(2-hydroxyethyl)-4-[3-(3-thienyl)imidazo[1,2-a]pyrazin-6-yl]benzamide |
| **322** | 6-(2-thienyl)-3-(3-thienyl)imidazo[1,2-a]pyrazine |
| **323** | 6-(3,5-dimethoxyphenyl)-3-(3-thienyl)imidazo[1,2-a]pyrazine |
| 324 | 4-[6-(4-chlorophenyl)imidazo[1,2-a]pyrazin-3-yl]phenol |
| **325** | 4-[6-[4-(4-isopropylpiperazin-1-yl)phenyl]imidazo[1,2-a]pyrazin-3-yl]phenol |
| **326** | N-cyclopropyl-4-[3-(4-hydroxyphenyl)imidazo[1,2-a]pyrazin-6-yl]benzamide |
| **327** | 4-[6-(1-methylpyrazol-4-yl)imidazo[1,2-a]pyrazin-3-yl]phenol |
| **328** | 3-[4-[6-(3-ethoxyphenyl)imidazo[1,2-a]pyrazin-3-yl]phenyl]propanoic acid |
| **329** | 3-[4-[6-(3-chlorophenyl)imidazo[1,2-a]pyrazin-3-yl]phenyl]propanoic acid |
| **330** | 3-[4-[6-(o-tolyl)imidazo[1,2-a]pyrazin-3-yl]phenyl]propanoic acid |
| **331** | 3-[4-[6-(2-chlorophenyl)imidazo[1,2-a]pyrazin-3-yl]phenyl]propanoic acid |
| **332** | 3-[4-[6-(2-thienyl)imidazo[1,2-a]pyrazin-3-yl]phenyl]propanoic acid |
| **333** | 3-[4-[6-[2-(hydroxymethyl)phenyl]imidazo[1,2-a]pyrazin-3-yl]phenyl]propanoic acid |
| **334** | 3-[4-[6-(2,3-dimethylphenyl)imidazo[1,2-a]pyrazin-3-yl]phenyl]propanoic acid |
| **335** | 4-(6-phenylimidazo[1,2-a]pyrazin-3-yl)benzonitrile |
| **336** | 4-[6-(3-thienyl)imidazo[1,2-a]pyrazin-3-yl]benzonitrile |
| **337** | 4-[6-(4-chlorophenyl)imidazo[1,2-a]pyrazin-3-yl]benzonitrile |
| **338** | 4-[6-[4-(hydroxymethyl)phenyl]imidazo[1,2-a]pyrazin-3-yl]benzonitrile |
| **339** | 4-[6-(2-thienyl)imidazo[1,2-a]pyrazin-3-yl]benzonitrile |
| **340** | 6-[6-(4-methylpiperazin-1-yl)-3-pyridyl]-3-(3,4,5-trimethoxyphenyl)imidazo[1,2-a]pyrazine |
| **341** | 3-[6-[6-[3-(dimethylamino)propoxy]-3-pyridyl]imidazo[1,2-a]pyrazin-3-yl]-N,N-dimethyl-aniline |
| **342** | N,N-dimethyl-3-[6-(4-pyridyl)imidazo[1,2-a]pyrazin-3-yl]aniline |
| **343** | N,N-dimethyl-3-[6-(3-thienyl)imidazo[1,2-a]pyrazin-3-yl]aniline |
| **344** | [4-[3-[3-(dimethylamino)phenyl]imidazo[1,2-a]pyrazin-6-yl]phenyl]methanol |
| **345** | N,N-dimethyl-3-[6-(2-thienyl)imidazo[1,2-a]pyrazin-3-yl]aniline |
| **346** | 3-[6-(3,5-dimethoxyphenyl)imidazo[1,2-a]pyrazin-3-yl]-N,N-dimethyl-aniline |
| **347** | 3-(4-chlorophenyl)-6-(4-pyridyl)imidazo[1,2-a]pyrazine |
| **348** | 3-(4-chlorophenyl)-6-(3-thienyl)imidazo[1,2-a]pyrazine |
| **349** | 3-(4-chlorophenyl)-6-(2-thienyl)imidazo[1,2-a]pyrazine |
| **350** | N-[3-[3-(3-chloro-4-fluoro-phenyl)imidazo[1,2-a]pyrazin-6-yl]phenyl]acetamide |
| **351** | 3-(3-chloro-4-fluoro-phenyl)-6-(2-furyl)imidazo[1,2-a]pyrazine |
| **352** | 6-(3-pyridyl)-3-[3-(trifluoromethoxy)phenyl]imidazo[1,2-a]pyrazine |
| **353** | N-(2-hydroxyethyl)-3-[3-[3-(trifluoromethoxy)phenyl]imidazo[1,2-a]pyrazin-6-yl]benzamide |

(continued)

| compound | name |
|---|---|
| **354** | (4-methylpiperazin-1-yl)-[3-[6-(2-phenoxyphenyl)imidazo[1,2-a]pyrazin-3-yl]phenyl]methanone |
| **355** | [3-[6-(1,3-benzodioxol-5-yl)imidazo[1,2-a]pyrazin-3-yl]phenyl]-(4-methylpiperazin-1-yl)methanone |
| **356** | (4-methylpiperazin-1-yl)-[3-[6-[4-(trifluoromethoxy)phenyl]imidazo[1,2-a]pyrazin-3-yl]phenyl]methanone |
| **357** | [3-[6-(3-chlorophenyl)imidazo[1,2-a]pyrazin-3-yl]phenyl]-(4-methylpiperazin-1-yl)methanone |
| **358** | [3-[6-(1-methylindol-5-yl)imidazo[1,2-a]pyrazin-3-yl]phenyl]-(4-methylpiperazin-1-yl)methanone |
| **359** | [3-[6-[4-(anilinomethyl)phenyl]imidazo[1,2-a]pyrazin-3-yl]phenyl]-(4-methylpiperazin-1-yl)methanone |
| **360** | [4-[3-(3-chlorophenyl)imidazo[1,2-a]pyrazin-6-yl]phenyl]methanol |
| **361** | [4-[3-(4-methoxyphenyl)imidazo[1,2-a]pyrazin-6-yl]phenyl]methanol |
| **362** | 3-(4-methoxyphenyl)-6-(2-thienyl)imidazo[1,2-a]pyrazine |
| **363** | 5-[3-(4-methoxyphenyl)imidazo[1,2-a]pyrazin-6-yl]pyridin-2-amine |
| **364** | 3-[6-(benzothiophen-2-yl)imidazo[1,2-a]pyrazin-3-yl]phenol |
| **365** | 3-[6-(4-chlorophenyl)imidazo[1,2-a]pyrazin-3-yl]phenol |
| **366** | 3-[6-[(E)-styryl]imidazo[1,2-a]pyrazin-3-yl]phenol |
| **367** | 3-[6-[4-(4-methylpiperazin-1-yl)phenyl]imidazo[1,2-a]pyrazin-3-yl]phenol |
| **368** | 3-[6-(3-chlorophenyl)imidazo[1,2-a]pyrazin-3-yl]phenol |
| **369** | 3-[6-(6-methoxy-3-pyridyl)imidazo[1,2-a]pyrazin-3-yl]phenol |
| **370** | 3-[6-(2-thienyl)imidazo[1,2-a]pyrazin-3-yl]phenol |
| **371** | 3-[6-(3,5-dimethoxyphenyl)imidazo[1,2-a]pyrazin-3-yl]phenol |
| **372** | 3-[6-(2-fluoro-3-methoxy-phenyl)imidazo[1,2-a]pyrazin-3-yl]phenol |
| **373** | [4-[6-(2-furyl)imidazo[1,2-a]pyrazin-3-yl]phenyl]methanol |
| **374** | [4-[6-(2,4-dichlorophenyl)imidazo[1,2-a]pyrazin-3-yl]phenyl]methanol |
| **375** | N-cyclopentyl-4-[3-[4-(hydroxymethyl)phenyl]imidazo[1,2-a]pyrazin-6-yl]benzamide |
| **376** | [3-(6-phenylimidazo[1,2-a]pyrazin-3-yl)phenyl]methanol |
| **377** | [3-[6-(4-methylsulfanylphenyl)imidazo[1,2-a]pyrazin-3-yl]phenyl]methanol |
| **378** | [3-[6-(3-ethoxyphenyl)imidazo[1,2-a]pyrazin-3-yl]phenyl]methanol |
| **379** | [3-[6-(2-thienyl)imidazo[1,2-a]pyrazin-3-yl]phenyl]methanol |
| **380** | [3-[6-(3-isopropoxyphenyl)imidazo[1,2-a]pyrazin-3-yl]phenyl]methanol |
| **381** | 3-(6-methoxy-3-pyridyl)-6-[4-(4-methylpiperazin-1-yl)phenyl]imidazo[1,2-a]pyrazine |
| **382** | 3,6-bis(6-methoxy-3-pyridyl)imidazo[1,2-a]pyrazine |
| **383** | 3-[3-(6-methoxy-3-pyridyl)imidazo[1,2-a]pyrazin-6-yl]benzamide |
| **384** | 3-[3-(3-fluorophenyl)imidazo[1,2-a]pyrazin-6-yl]aniline |
| **385** | 3-(3-fluorophenyl)-6-phenyl-imidazo[1,2-a]pyrazine |
| **386** | 6-(1,3-benzodioxol-5-yl)-3-(3-fluorophenyl)imidazo[1,2-a]pyrazine |
| **387** | 3-(3-fluorophenyl)-6-(4-piperazin-1-ylphenyl)imidazo[1,2-a]pyrazine |
| **388** | 6-(4-chlorophenyl)-3-(3-fluorophenyl)imidazo[1,2-a]pyrazine |
| **389** | [4-[3-(3-fluorophenyl)imidazo[1,2-a]pyrazin-6-yl]phenyl]methanol |

(continued)

| compound | name |
|---|---|
| **390** | 3-(3-fluorophenyl)-6-(2-thienyl)imidazo[1,2-a]pyrazine |
| **391** | 4-[6-[6-[3-(dimethylamino)propoxy]-3-pyridyl]imidazo[1,2-a]pyrazin-3-yl]-2-methoxy-phenol |
| **392** | 2-methoxy-4-[6-[6-(2-morpholinoethylamino)-3-pyridyl]imidazo[1,2-a]pyrazin-3-yl]phenol |
| **393** | 2-methoxy-4-[6-[6-(4-methylpiperazin-1-yl)-3-pyridyl]imidazo[1,2-a]pyrazin-3-yl]phenol |
| **394** | 4-[6-(1,3-benzodioxol-5-yl)imidazo[1,2-a]pyrazin-3-yl]-2-methoxy-phenol |
| **395** | 4-[6-(3-ethoxyphenyl)imidazo[1,2-a]pyrazin-3-yl]-2-methoxy-phenol |
| **396** | 4-[6-(6-amino-3-pyridyl)imidazo[1,2-a]pyrazin-3-yl]-2-methoxy-phenol |
| **397** | 2-methoxy-4-[6-(1-methylpyrazol-4-yl)imidazo[1,2-a]pyrazin-3-yl]phenol |
| **398** | N-[3-(dimethylamino)propyl]-4-[3-(1-methylindol-5-yl)imidazo[1,2-a]pyrazin-6-yl]benzamide |
| **399** | N-(2-hydroxyethyl)-3-[3-(1-methylindol-5-yl)imidazo[1,2-a]pyrazin-6-yl]benzamide |
| **400** | 2-methoxy-4-[3-(1-methylindol-5-yl)imidazo[1,2-a]pyrazin-6-yl]phenol |
| **401** | 3-[3-(1-methylindol-5-yl)imidazo[1,2-a]pyrazin-6-yl]benzamide |
| **402** | (4-methylpiperazin-1-yl)-[4-[6-[(E)-styryl]imidazo[1,2-a]pyrazin-3-yl]phenyl]methanone |
| **403** | N-[3-[3-(4-phenoxyphenyl)imidazo[1,2-a]pyrazin-6-yl]phenyl]methanesulfonamide |
| **404** | 6-(3-fluorophenyl)-3-(o-tolyl)imidazo[1,2-a]pyrazine |
| **405** | 3-(o-tolyl)-6-(2-thienyl)imidazo[1,2-a]pyrazine |
| **406** | 4-[3-(2-chlorophenyl)imidazo[1,2-a]pyrazin-6-yl]-2-methoxy-phenol |
| **407** | 3-[3-(4-tert-butylphenyl)imidazo[1,2-a]pyrazin-6-yl]-N,N-dimethyl-benzamide |
| **408** | 5-[3-(4-tert-butylphenyl)imidazo[1,2-a]pyrazin-6-yl]pyridin-2-amine |
| **409** | 1-[3-[6-(3-pyridyl)imidazo[1,2-a]pyrazin-3-yl]phenyl]ethanone |
| **410** | 1-[3-[6-(3-fluorophenyl)imidazo[1,2-a]pyrazin-3-yl]phenyl]ethanone |
| **411** | 4-[3-(3-acetylphenyl)imidazo[1,2-a]pyrazin-6-yl]-N-(2-dimethylaminoethyl)benzamide |
| **412** | 1-[3-[6-(2-thienyl)imidazo[1,2-a]pyrazin-3-yl]phenyl]ethanone |
| **413** | N,N-dimethyl-2-[3-(2-thienyl)imidazo[1,2-a]pyrazin-6-yl]aniline |
| **414** | 6-(4-pyridyl)-3-(2-thienyl)imidazo[1,2-a]pyrazine |
| **415** | 3-(2-thienyl)-6-(3-thienyl)imidazo[1,2-a]pyrazine |
| **416** | 6-(benzothiophen-2-yl)-3-(2-thienyl)imidazo[1,2-a]pyrazine |
| **417** | 4-[3-(2-thienyl)imidazo[1,2-a]pyrazin-6-yl]phenol |
| **418** | 3-(2-thienyl)-6-[4-(trifluoromethoxy)phenyl]imidazo[1,2-a]pyrazine |
| **419** | 6-[(E)-styryl]-3-(2-thienyl)imidazo[1,2-a]pyrazine |
| **420** | N-(2-hydroxyethyl)-3-[3-(2-thienyl)imidazo[1,2-a]pyrazin-6-yl]benzamide |
| **421** | 6-(3-chlorophenyl)-3-(2-thienyl)imidazo[1,2-a]pyrazine |
| **422** | [4-[3-(2-thienyl)imidazo[1,2-a]pyrazin-6-yl]phenyl]methanol |
| **423** | N-(2-hydroxyethyl)-4-[3-(2-thienyl)imidazo[1,2-a]pyrazin-6-yl]benzamide |
| **424** | 3,6-bis(2-thienyl)imidazo[1,2-a]pyrazine |
| **425** | N-[3-[3-(2-thienyl)imidazo[1,2-a]pyrazin-6-yl]phenyl]methanesulfonamide |
| **426** | 6-(3,5-dimethoxyphenyl)-3-(2-thienyl)imidazo[1,2-a]pyrazine |
| **427** | 5-[3-(2-thienyl)imidazo[1,2-a]pyrazin-6-yl]pyridin-2-amine |

(continued)

| compound | name |
|---|---|
| **428** | 6-(3-isopropoxyphenyl)-3-(2-thienyl)imidazo[1,2-a]pyrazine |
| **429** | N,N-dimethyl-4-[6-[6-(4-methylpiperazin-1-yl)-3-pyridyl]imidazo[1,2-a]pyrazin-3-yl]benzamide |
| **430** | N,N-dimethyl-4-[6-(2-phenoxyphenyl)imidazo[1,2-a]pyrazin-3-yl]benzamide |
| **431** | N,N-dimethyl-4-[6-(3-thienyl)imidazo[1,2-a]pyrazin-3-yl]benzamide |
| **432** | 4-[6-(3-acetamidophenyl)imidazo[1,2-a]pyrazin-3-yl]-N,N-dimethyl-benzamide |
| **433** | N,N-dimethyl-4-[6-(1-methylindol-5-yl)imidazo[1,2-a]pyrazin-3-yl]benzamide |
| **434** | 4-[6-(5-acetyl-2-thienyl)imidazo[1,2-a]pyrazin-3-yl]-N-cyclopropyl-benzamide |
| **435** | N-cyclopropyl-4-[6-[4-(4-isopropylpiperazin-1-yl)phenyl]imidazo[1,2-a]pyrazin-3-yl]benzamide |
| **436** | 2-[3-(3,5-dimethoxyphenyl)imidazo[1,2-a]pyrazin-6-yl]-N,N-dimethylaniline |
| **437** | 3-(3,5-dimethoxyphenyl)-6-(3-isopropoxyphenyl)imidazo[1,2-a]pyrazine |
| **438** | 3-(1-methylpyrazol-4-yl)-6-(4-pyridyl)imidazo[1,2-a]pyrazine |
| **439** | 6-(benzothiophen-2-yl)-3-(1-methylpyrazol-4-yl)imidazo[1,2-a]pyrazine |
| **440** | 6-[6-(3,4,5-trimethoxyphenyl)imidazo[1,2-a]pyrazin-3-yl]quinoline |
| **441** | 2-methoxy-4-[3-(6-quinolyl)imidazo[1,2-a]pyrazin-6-yl]phenol |
| **442** | 2,6-dimethyl-4-[3-(6-quinolyl)imidazo[1,2-a]pyrazin-6-yl]phenol |
| **443** | 6-[6-(1-methylindol-5-yl)imidazo[1,2-a]pyrazin-3-yl]quinoline |
| **444** | N-[3-[3-(6-quinolyl)imidazo[1,2-a]pyrazin-6-yl]phenyl]methanesulfonamide |
| **445** | 4-[3-[6-(2-thienyl)imidazo[1,2-a]pyrazin-3-yl]phenyl]morpholine |
| **446** | N-[3-[6-(2-thienyl)imidazo[1,2-a]pyrazin-3-yl]phenyl]methanesulfonamide |
| **447** | 3-(benzothiophen-2-yl)-6-(2-thienyl)imidazo[1,2-a]pyrazine |
| **448** | 3-[6-(2-thienyl)imidazo[1,2-a]pyrazin-3-yl]aniline |
| **449** | 5-[6-(2-thienyl)imidazo[1,2-a]pyrazin-3-yl]pyridin-2-amine |
| **450** | 3-(3-isopropoxyphenyl)-6-(2-thienyl)imidazo[1,2-a]pyrazine |
| **451** | 3-[4-(1-piperidyl)phenyl]-6-(2-thienyl)imidazo[1,2-a]pyrazine |
| **452** | (2S)-3-methyl-2-[[3-[4-(1-piperidyl)phenyl]imidazo[1,2-b]pyridazin-6-yl]amino]butan-1-ol |
| **453** | (2S)-2-[[3-[3-(dimethylamino)phenyl]imidazo[1,2-b]pyridazin-6-yl]amino]-3-methyl-butan-1-ol |
| **454** | (2S)-3-methyl-2-[[3-(3-morpholinophenyl)imidazo[1,2-b]pyridazin-6-yl]amino]butan-1-ol |
| **455** | (2S)-2-[[3-(6-amino-3-pyridyl)imidazo[1,2-b]pyridazin-6-yl]amino]-3-methyl-butan-1-ol |
| **456** | (2S)-2-[[3-(3-isopropoxyphenyl)imidazo[1,2-b]pyridazin-6-yl]amino]-3-methyl-butan-1-ol |
| **457** | N,N-dimethyl-3-(3-thiazol-4-ylimidazo[1,2-a]pyrazin-6-yl)aniline |
| **458** | 4-[3-(3-thiazol-4-ylimidazo[1,2-a]pyrazin-6-yl)phenyl]morpholine |
| **459** | 5-(3-thiazol-4-ylimidazo[1,2-a]pyrazin-6-yl)pyridin-2-amine |
| **460** | 4-[6-(3-isopropoxyphenyl)imidazo[1,2-a]pyrazin-3-yl]thiazole |
| **461** | N-[3-(3-thiazol-4-ylimidazo[1,2-a]pyrazin-6-yl)phenyl]methanesulfonamide |
| **462** | 3-(3-thiazol-4-ylimidazo[1,2-a]pyrazin-6-yl)aniline |

Surprisingly it was found that the above-mentioned compounds of general formulae (I) - (IV) as well as pharmaceutical compositions comprising said compounds are most probably useful for the inhibition of GRK5 and/or Scyl1.

Therefore one aspect of the present invention is that the compounds according to the general formulae (I) -

(IV) are suitable for use as inhibitor of GRK5 and/or Scyl1. Inhibitors of GRK5 as used herein are defined as chemical compounds that inhibit the kinase activity of GRK5. Inhibitors of Scyl1 as used herein are defined as chemical compounds that bind to the ATP binding site of Scy1.

Scyl1 as used herein refers to the protein having accession number Q96KG9 with UniProtKB/Swiss-Prot. Alternatives names of this protein are: N-terminal kinase-like protein, coated vesicle-associated kinase of 90 kDa, SCY1-like protein 1, telomerase regulation-associated protein and telomerase transcriptional element-interacting factor. GRK5 as used herein refers to the protein having accession number P34947 with UniProtKB/Swiss-Prot. Alternatives names of this protein are: G protein-coupled receptor kinase 5, EC = 2.7.11.16 and G protein-coupled receptor kinase GRK5.G protein-coupled receptor kinase 5.

The inventors used three different screening assays to search for new compounds being inhibitors of GRK5 and/or Scyl 1 and show an effect in glucose dependent regulation of insulin production or release and/or for increasing glucose uptake. It could be determined that compounds of general formulae (I) - (IV) are suitable as inhibitors of GRK5 and/or Scyl 1 and show an effect in glucose dependent regulation of insulin production or release and/or for increasing glucose uptake.

Different compound libraries with overall around 120,000 compounds were tested using a Sycl 1 based assay (example 2), a GRK5 based assay (example 7) and an assay screening for effect to insulin production or release (example 14). The hits of all three assays which are covered by the general formula (I) of the present invention were subject of further verification by investigation their effects on insulin release and glucose uptake after compound treatment in C2C12 and Beta-TC6 cells.

Thus, the compounds of the present invention can be used for glucose dependent regulation of insulin production and/or release of insulin and/or for increasing glucose uptake. The compounds of general formulae (I) - (IV) are suitable for the treatment or prophylaxis of a metabolic disease. The present invention refers further to the use of at least one compound of general formulae (I) - (IV) for the manufacture of a pharmaceutical formulation for the prophylaxis and treatment of a metabolic disease.

The results of previous experiments of the inventors (cf. WO 2012/028335 A1) as well as prior art (see above) suggest that GRK5 and Scyl1 are suitable targets for development of anti-diabetic drugs and active agents against obesity. According to the invention, the inventive compounds or the pharmaceutical composition of the invention can be used in treatment or prophylaxis of a metabolic disease. It is preferred that the metabolic disease according to the invention is selected from the group comprising or consisting of diabetes, obesity and impaired adipogenesis. Especially preferred the disease to be treated is diabetes mellitus type 2.

The compounds of the present invention are suitable for use as pharmaceutically active agent in medicine, particularly in human medicine, but also in veterinary medicine. The compounds of the present invention may be administered in a pharmaceutically effective amount by any suitable route to subjects in need thereof, e.g. parenterally, topically, rectally, nasally, buccally, vaginally, transdermally, by inhalation, by injection or infusion, by spray or via implanted reservoirs. Preferably, the compounds are administered orally or by injection or infusion, e.g. intravenously. For medical purposes, the compounds are preferably formulated as a pharmaceutical composition, which comprises at least one compound as described above, and pharmaceutically acceptable carriers, solvent or excipient. The present invention therefore refers also to pharmaceutical composition at least one compound of the present invention and further comprising at least a second active agent being an anti-diabetic drug. It is thereby preferred that the anti-diabetic drug is selected from the group comprising or consisting of thiazolidinediones, metformin, sulfonylurea and further oral anti-diabetic drugs.

Sulfonylurea is preferred selected from the group comprising or consisting of carbutamide, acetohexamide, chlorpropamide, tolbutamide, tolazamide, glipizide, gliclazide, glibenclamide (glyburide), glibornuride, gliquidone, glisoxepide, glyclopyramide and glimepiride. Thiazolidinedione is preferred selected from the group comprising or consisting of rosiglitazone, pioglitazone and troglitazone. The compounds of the general formulae (I) - (IV) can also be administered in form of their pharmaceutically active salts optionally using substantially nontoxic pharmaceutically acceptable carrier, excipients, adjuvants or diluents. The medications of the present invention are prepared in a conventional solid or liquid carrier or diluents and a conventional pharmaceutically-made adjuvant at suitable dosage level in a known way. These administratable forms, for example, include pills, tablets, film tablets, coated tablets, capsules, powders and deposits.

Within the disclosed methods the pharmaceutical compositions of the present invention, containing at least one compound of the general formulae (I) - (IV) or pharmaceutically acceptable salts thereof as an active ingredient will typically be administered in admixture with suitable carrier materials suitably selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active ingredient may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions of the present invention may be formulated in sustained release form to provide the rate controlled release of any one or more of the components or active ingredients to optimize the therapeutic effects, i.e. antihistaminic activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are solid form preparations which are intended to be converted, shortly before use, to liquid form preparations for either oral or parenteral administration. Such liquid forms include solutions, suspensions and emulsions.

The compounds of the present invention may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refer to the active ingredients dispersed or solubilized in a hydrophilic semi-solid matrix. "Powders for constitution" refers to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates

such as magnesium stearate, calcium stearate or potassium stearate; stearic acid; high melting point waxes; and water soluble lubricants such as sodium chloride, sodium benzoate, sodium acetate, sodium oleate, polyethylene glycols and d'l-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1 % to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1%.

Techniques for the formulation and administration of the compounds of the present invention may be found in "Remington's Pharmaceutical Sciences" Mack Publishing Co., Easton PA. A suitable composition comprising at least one compound of the invention and/or pharmaceutically acceptable salts thereof may be a solution of the compound in a suitable liquid pharmaceutical carrier or any other formulation such as tablets, pills, film tablets, coated tablets, dragees, capsules, powders and deposits, gels, syrups, slurries, suspensions, emulsions, and the like.

A therapeutically effective dosage of a compound of the general formula (I) refers to that amount of the compound that results in an at least partial inhibition of GRK5 and/or Scyl1. Toxicity and therapeutic efficacy of such compounds can be determined by standard pharmaceutical, pharmacological, and toxicological procedures in cell cultures or experimental animals for determining the LD50 (the dose lethal to 50% of the population) and the ED50 (the dose therapeutically effective in 50% of the population). The dose ratio between toxic and therapeutic effect is the therapeutic index and can be expressed as the ratio between LD50 and ED50. The dosage of the compound lies preferably within a range of circulating concentrations that include the ED50 with little or no toxicity. The actual amount of the composition administered will be dependent on the subject being treated, on the subject's weight, the severity of the affliction, the manner of administration and the judgement of the prescribing physician.

**Description of the figures:**

Figure 1 shows proliferation of C2C12 myoblasts (A and C) and beta-TC6 (B and D) after treatment with one compound according to the invention for 72 h measured by MTT assay.

Figure 2 shows 5 μM compound treatment increased insulin release in beta-TC6 cells. Scyl1 inhibition by compound 10 to 14 led to a remarkable effect on insulin release, ranging from 60.2 ± 8.9 % (Cpd **10**) to 30.5 ± 4.5 % (Cpd **12**). The negative controls - Cpds without inhibition efficacy - displayed no effect -11.7 ± 0.35 (Cpd **c3**) and 3.69 ± 0.43 (Cpd **c4**) while the sunitinib control did (Sut: 58.2 ± 2.2 %).

Figure 3 shows insulin released by beta-TC6 cells in response to various concentrations ranging from 2 μM to 10 μM for each compound in high glucose media (4.5 g/L).

Figure 4A shows illustration for 2-NBDG uptake after treatment with inventive compounds in beta-TC6 cells.: Upper panel left to right compounds 10 - 12, middle panel left to right compounds **13 - 14**, lower panel left to right control compounds **c3 - c4**; black = glucose free medium; red = glucose free medium + compound; blue = 100μM 2-NBDG, yellow = 100μM 2-NBDG + compound.

Figure 4B shows illustration for 2-NBDG uptake after treatment with inventive compounds in C2C12 cells. Upper panel left to right compounds **10 - 12**, middle panel left to right compounds **13 - 14**, lower panel left to right control compounds **c3 -c4*;*** black = glucose free medium; red = glucose free medium + compound; blue = 100μM 2-NBDG, yellow = 100μM 2-NBDG + compound.

Figure 5 shows inhibition of that treatment beta-TC6 cells with inventive compounds in a low glucose environment displayed no increased insulin secretion.

Figure 6 shows 5 μM treatment with inventive compounds inhibiting GRK5 increases insulin release in beta-TC6 cells. Compounds **1** to **4** led to a remarkable effect on insulin release in our system (increase in percent ± SEM: Cpd **1**: 42.8 ± 6.8; Cpd **2**: 35.9 ± 9.0; Cpd **3**: 65.7 ± 78.3; Cpd **4**: 50.2 ± 41.2 at 5μM). The negative control - compounds without inhibition efficacy - displayed no effect (Cpd **c1**: 1.3 ± 0.3 %; Cpd **c2**: 2.0 ± 10.0 %) while the sunitinib control did (Sut: 53.2 ± 7.5 %).

Figure 7 shows GRK5 inhibitor treatment with various concentrations for each compound in high glucose media (4.5 g/L) ranging from 2 μM to 10 μM.

Figure 8 shows example illustration for 2-NBDG uptake after treatment with inventive compounds of beta-TC6 cells. Upper panel left to right compounds **1 - 3,** lower panel left to right compound **4** and control compound **c2;** black = glucose free medium; red = glucose free medium + compound; blue = 100 μM 2-NBDG, yellow = 100μM 2-NBDG + compound.

Figure 9 shows example illustration for 2-NBDG uptake after treatment with inventive compounds of C2C12 myoblast cells. Upper panel left to right compounds **1 - 3,** lower panel left to right compound **4** and control compound **c2;** black = glucose free medium; red = glucose free medium + compound; blue = 100μM 2-NBDG, yellow = 100μM 2-NBDG + compound.

Figure 10 shows comparison of glucose uptake via 2-NBDG after treatment with inventive compounds in C2C12 mouse myoblasts. Despite a background of 1.7 ± 0.11 (ratio ± SEM) the compounds **1 - 3** displayed a significant higher increase of 2-NBDG uptake upon a 1 h compound treatment.

Figure 11 shows inhibition of GRK5 in a low glucose environment led to a significant decrease of insulin secretion.

Figure 12 shows insulin dependency of compound structures Cpd **10** (Scly1) and Cpd **1** (Grk5). Beside the glucose uptake mediated by insulin (average = 11.78 %), the compound structures Cpd **10** and Cpd **1** led to an insulin independent increase of 18.2 ± 3.5 %, respectively 48.9 ± 7.2 %.

Figure 13 shows insulin release in beta-TC6 cells upon various treatments. Cpd **10** (14; 5 μM) as well as Cpd **1** (4; 5 μM) and their negative controls (Cpd **c2**, Cpd **c3**; 5 μM) were compared to sunitinib as well as AKT1-, MEKII inhibitors (10 μM each) and GLP-1 and Exendin-4 (20 μg/ml each).

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

Abbreviations used in the present description have the following meanings: ACN (acetonitrile), ADP (Adenosine diphosphate), AMP (Adenosine monophosphate), ATP (Adenosine triphosphate), Bu (butyl), Cpd (compound), DCM (dichloromethane), $CDCl_3$ (deuterated chloroform), dba(dibenzylideneacetone), DIPEA (N-ethyl-N,N-diisopropylamine), DMEM (Dulbecco's Modified Eagle Medium), DMF (dimethylformamide), DMSO-$d_6$ (deuterated dimethylsulfoxide), dppp (1,3-Bis(diphenylphosphino)propane), dppf (1,1'-Bis(diphenylphosphino)ferrocene), DTT (Dithiothreitol), EtOAc (ethyl acetate), EtOH (ethanol), IPA (isopropyl alcohol), MeOH (methanol), MeCN (acetonitrile), MOPS (3-(N-morpholino)propanesulfonic acid), MTT (3-(4,5-dimethylthiazol-2-yl)-2,5-diphenyl tetrazolium bromide), 2-NBDG (2-[*N*-(7-nitrobenz-2-oxa-1,3-diazol-4-yl)-amino]-2-deoxy-d-glucose), NBS (N-Bromosuccinimide), NIS (N-Iodosuccinimide), OD (optical density), PBS (phosphate buffered saline), Ph (phenyl), $Pd(PPh_3)_4$ (tetrakis(triphenylphosphine)palladium(0)), PP (Polypropylene), Pyr (pyridine), TFA (trifluoroacetic acid), THF (tetrahydrofurane), TKI (tyrosine-kinase inhibitor), XPhos (2-Dicyclohexylphosphino-2',4',6'-triisopropylbiphenyl), Pen (Penicilin), Strep (Streptomycin), MES (2-(*N*-morpholino)ethanesulfonic acid), RFU (relative fluorescence units).

The following abbreviations are used for the common amino acids referred to herein.

| Abbreviation | Amino acid |
|---|---|
| Ala | Alanine |
| Arg | Arginine |
| Asn | Asparagine |
| Asp | Aspartic acid (Aspartate) |
| Cys | Cysteine |
| Gln | Glutamine |
| Glu | Glutamic acid (Glutamate) |
| Gly | Glycine |
| His | Histidine |
| Ile | Isoleucine |
| Leu | Leucine |
| Lys | Lysine |
| Met | Methionine |
| Phe | Phenylalanine |
| Pro | Proline |
| Ser | Serine |
| Thr | Threonine |
| Trp | Tryptophan |
| Tyr | Tyrosine |
| Val | Valine |

## EXAMPLES

### General cell culture techniques:

Cell lines were routinely assayed for mycoplasma contamination and cultured at 95 % air, 5 % $CO_2$ and 37 °C in a Hera-Cell-150 incubator. Before seeding, the cell amount was determined using a Coulter Counter system (Coulter Electronics) and the corresponding cell amount for seeding was calculated. All cells were cultured according to the appropriate recommendations of ATCC protocols.

For analysis using the following assays, cells were seeded at different plate size and suitable cell-density. After 24 h the cells were washed with PBS and fresh media containing DMSO respectively the appropriate treatment with TKIs, peptides or chemicals, was replaced. Starving for insulin dependent examinations was performed in glucose- and FCS-free DMEM media for 4 h.

**Table 1**: Condition for the used cell lines:

| Cell line | Culture media | Initial cell amount 96-/ 12- / 6- well |
|---|---|---|
| 3T3-L1 | DMEM 1.0 g/L glucose, 10 % (v/v) NCS, 2 mM L-glutamine, 1x Pen/Strep | 5 x103 / 5 x104 / 1 x105 |
| Beta-TC6 | DMEM 4.5 g/L glucose, 15 % (v/v) FCS, 2 mM L-glutamine, 1x Pen/Strep | 3 x104 / 3 x105 / 6 x105 |

(continued)

| Cell line | Culture media | Initial cell amount 96-/ 12- / 6- well |
|---|---|---|
| C2C12 | DMEM 4.5 g/L glucose, 10 % (v/v) FCS, 2 mM L-glutamine, 1x Pen/Strep | 3 x103 / 3x104 / 6 x104 |
| RIN-5AH-T2B | RPMI-1640 4.5 g/L glucose, 10 % (v/v) FCS, 2 mM L-glutamine, 1x Pen/Strep | 3x104 / 3 x105 / 6 x105 |

**Table 2**: control compounds

| Compound | Structure | Compound | Structure |
|---|---|---|---|
| **c1** | 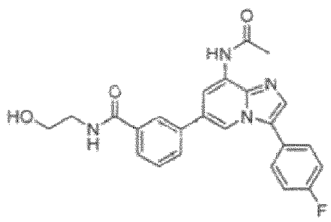 | **c3** | 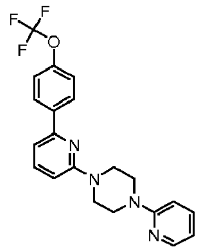 |
| **c2** | 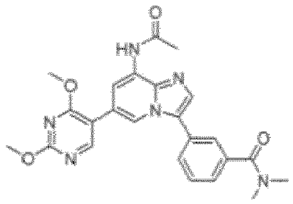 | **c4** | 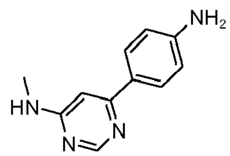 |
| Sunitinib (Sut, Sutent) | 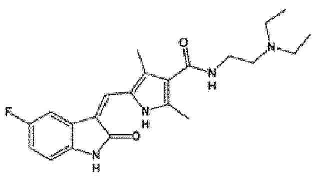 | Akt1 | 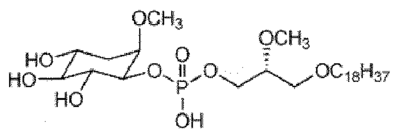 |
| Exendin-4 | His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-$NH_2$ | GLP-1 | His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-$NH_2$ |
| MEK | 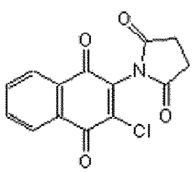 | | |

**Example 1. Cytotoxicity Assays**

Using a MTT approach, we determined the effect on cell viability and proliferation of beta-TC6 and C2C12 cells to display potential cytotoxicity of the compounds according to the invention *in vitro* after a 72 h treatment.

For MTT (3-(4,5-dimethylthiazol-2-yl)-2,5-diphenyl tetrazolium bromide) transformation, 1/5 of total volume of a 5 mg/mL MTT stock solution was added to the cells as well as control wells and incubated at 37 °C, 5 % $CO_2$ (v/v) for 1 h. Then 1/2 of total volume of MTT-stop solution was added and plates were incubated overnight in the dark at 25 °C. The optical density (OD) was measured using a multiwell spectrophotometer at a wavelength of 570 nm. Results are shown in Figure 1.

Interestingly, beta-TC6 cells seemed to be more robust against the used Scyl1 inhibitors than the C2C12 cell line (Figure 1), suggesting that the metabolic turnover of the compounds inclines with the slow doubling time of approx. 36 - 48 h. Compounds **10** and **12** seemed to have no toxic effect at all, even at concentrations up to 10 $\mu$M. Focusing on compounds **11** and **14**, we detected only a slight decreased proliferation after a 10 $\mu$M treatment; compound **14** with 1.35 $\pm$ 0.038 raw data intensity (RDI) and compound **11** with 1.17 $\pm$ 0.096 RDI. The positive control sunitinib appeared to be toxic in lower concentrations and decreased the proliferation to 0.638 $\pm$ 0.167 (sunitinib, 10$\mu$M). In contrast, C2C12 cells, which had a doubling time of 13 h, displayed impaired proliferation and viability for all used compounds. Just compounds **10 - 12** revealed less toxicity at a 2 $\mu$M treatment (Figure 1). The compounds **1 - 4** led to a decreased proliferation over a 72 h treatment.

**Example 2: High throughput screening (HTS) based on SCYL1 probe displacement assay**

The reporter displacement assay is based on reporter probe (PRO16, Proteros GmbH) that is designed to bind to ATP-binding site of SCYL1 (Methods in Enzymology, 2011, vol. 493, p. 300-319). The proximity between reporter probe and target protein (SCYL1) results in the emission of an optical signal. Test compounds that bind to the same site as the reporter probe displace the probe, causing signal diminution. The rate of reporter probe displacement is measured over time after addition of test compounds at various concentrations.

For $K_d$ determination, percent reporter probe displacement values are calculated for the last time point, at which the system has reached equilibrium. For each compound concentration percent probe displacement values are calculated and plotted against the compound concentration. $IC_{50}$-like values (corresponding to 50% probe displacement) are calculated using standard fitting algorithms. Knowledge of the reporter probe affinity for Scyl1 is essential for the development of the reporter-based binding assay. Therefore, 20nM Scyl1 (1-808) were incubated with increasing concentrations of reporter probe, probe binding was measured, and the $K_d$ value of the probe was quantified to be 607nM. In order to ensure sensitivity for detecting compounds that bind to Scyl1 and thereby compete with the reporter probe, the reporter probe is used at $K_{d(probe)}$ = 607nM. Thus, according to the Cheng Prusoff equation, the $K_d$ value of test compounds can be calculated with $K_d = ½\ IC_{50}$.

$$Kd = \frac{IC50}{1 + \frac{[probe]}{Kd_{probe}}}$$

Cheng Prusoff Equation

General assay protocol was used to measure compound binding to Scyl1. The target enzyme used was the full length human Scyl1 (1-808) protein.

The assay was performed in: 384 well U bottom, PP, black, low volume (Corning, 3676) assay plates at reaction temperature of 25°C. The reaction buffer used was composed of: 20mM Mops, pH 7.5, 1 mM DTT, 0.01 % Tween20.

Firstly to 10 $\mu$l 15/10 fold concentrated Scyl1 in reaction buffer 5 $\mu$l 15/5 fold concentrated reporter probe (PRO16) in reaction buffer were added. After incubation for 30 min 62.4 nl test compound in 100 % DMSO were added using pin tool. The final assay concentrations are 20 nM Scyl1 and 607 nM of the reporter probe PRO16. Subsequently measurement of emission was carried out.

Probe displacement was calculated in relation to the C+(positive) and C-(negative) controls. The signal reflecting 0% probe displacement (C+) was measured in the absence of test compound, while the signal representing 100% probe displacement (C-) was measured in the absence of Scyl1.

**Example 3: $IC_{50}$ determination for compounds binding to Scyl 1**

To determine the $IC_{50}$ values for all compounds $\geq$ 75 % inhibition, triplicates at 12 different concentrations with a dilution factor of three were used. The maximal compound assay concentration was set to 58 $\mu$M, exceeding the compound assay concentration used in the primary screen. For all assay plates, z-factor values were found to be significantly larger than 0.5, proving statistical relevance of the data.

**Example 4: Release of Insulin after treatment with the compounds of example 2**

After having rendered the most promising compounds by Scyl1 probe displacement assay, the impact on insulin released by beta-TC6 cells is determined. Cells were cultured overnight, washed with PBS and then treated with 5 $\mu$M of test compounds as well as non-inhibitor controls for 2 h in a high glucose (4.5 g/L) DMEM at 37°C and 5 % (v/v) $CO_2$.

Release of insulin was detected using a rat/mouse insulin ELISA.

In order to measure the insulin released upon compound treatment beta-TC6 insulinoma (mouse) and RIN-T2B-5AH (rat islet tumor subclone) cells were washed and incubated for 2 h in high (4.5 g/L) or low (1.125 g/L) glucose media at 37 °C. After incubation, the supernatant of the beta-TC6 insulinoma cells was diluted 1:20, whereas the supernatants of the RIN-T2B-5AH were diluted 1:10. The insulin release was measured with rat/mouse insulin ELISA (Merck Millipore Darmstadt, Cat. # EZRMI-13K) by following the manufacture's protocol. The enzyme activity of the horseradish peroxidase of the immobilized biotinylated antibodies was monitored spectrophotometrically by the increased absorbency at 490 nm, and corrected by the absorbency at 610 nm.

Inhibition of Scyl1 by the tested compounds as well as sunitinib treatment displayed a significant increase of insulin release (Figure 2). Here, compound 10 (Inh. 100 % / $IC_{50}$ = 0.5 μM) depicted a very strong effect with an increase of 60.2 ± 8.9 %. The other compounds **11 - 14** also displayed a markedly increased level of insulin release, ranging from 30.5 ± 4.5 % (Cpd **12**) to 42.7 ± 10.45 % (Cpd **11**). The negative controls - compounds **c3** and **c4 -** displayed no effect on insulin release (Cpd **c3**: -11.7 ± 0.35 %; Cpd **c4**: 3.69 ± 0.43 %) while the sunitinib control did (58.2 ± 2.2 %). Note, that insulin release correlated with the previously monitored percentage of probe displacement as well as the detected $IC_{50}$ values.

To expose if the compounds also have a concentration dependent impact on insulin released by beta-TC6 cells, we treated the cells with compound concentrations ranging from 2 μM to 10 μM for 2 h in a high glucose (4.5 g/L) DMEM. Results are shown in Figure 3 and by Table 3.

Inhibition of Scyl1 by test compounds of the invention displayed a concentration dependent increase of insulin release for all compounds but Cpd **13** (Figure 3). Highlighting the 5 μM values, which were matching with the previous performed approach for insulin release match for all compounds but Cpd **10**, where the induction seemed to be slightly higher as before.

**Table 3**: Release of insulin after inhibition of Scyl1 by compounds **10 - 14** with various concentrations each in a beta-TC6-cell system

| Scyl1 Inhibitor | 0 μM | 0 μM SD | 2 μM | 2 μM SD | 5 μM | 5 μM SD | 10 μM | 10μM SD |
|---|---|---|---|---|---|---|---|---|
| Cpd **10** | 1 | 0 | 1.66 | 0.41 | 3.04 | 0.47 | 2.68 | 0.29 |
| Cpd **11** | 1 | 0 | 1.33 | 0.35 | 1.91 | 0.10 | 2.31 | 0.13 |
| Cpd **12** | 1 | 0 | 1.43 | 0.29 | 1.34 | 0.17 | 2.16 | 0.21 |
| Cpd **13** | 1 | 0 | 1.00 | 0.10 | 1.39 | 0.17 | 1.37 | 0.33 |
| Cpd **14** | 1 | 0 | 0.97 | 0.26 | 1.29 | 0.25 | 1.82 | 0.29 |

**Example 5: Glucose uptake after treatment of C2C12 cells and Beta-T6 cells with compounds of example 2**

To investigate the impact of the compounds according to the invention on glucose uptake, we used the fluorescent D-glucose analog 2-[*N*-(7-nitrobenz-2-oxa-1,3-diazol-4-yl)-amino]-2-deoxy-d-glucose (2-NBDG). 2-NBDG is a fluorescently-labeled deoxyglucose analog that is commonly used to directly monitor glucose uptake by living cells. For 2-NBDG detection, cells were cultured together with 5 μM of an inventive compound 1 h in glucose free media supplied with 100 μM 2-NBDG at 37 °C before collecting. Furthermore, as internal controls, samples of glucose free media, respectively glucose free media with test compound, were tested. Subsequently, cells were washed, trypsinized and harvested in ice-cold PBS before centrifuged at 1.6 x$10^3$ rpm at 4°C. Cells were analyzed by flow cytometry (FACS Calibur, BD Bioscience, respectively a BD Accuri® C6 Flow Cytometer). For evaluation, cells were gated using the side- and forward scatter SSC/FSC and quantified by excited at 488 nm and collected at 533 nM (FL1).

The usage of the compounds according to the invention displayed a significant increase of 2-NBDG up to 1.55 to 2.4 fold in C2C12 cells as representative for peripheral tissue and 1.14 to 2.6 fold in beta-TC6 cells. Both negative control compounds did not lead to a 2-NBDG uptake (Figure 4). **Table 4** Ratio of 2-NBDG uptake after treatment of C2C12 or beta-TC6 cells with the inventive compounds as well as reference compounds (cpd **c3** and **c4**).

| Scyl1 Inhibitor | C2C12 | | beta-TC6 | |
|---|---|---|---|---|
| | Ratio | SE | Ratio | SE |
| DMSO | 1 | 0 | 1 | n.a. |
| Cpd **10** | 1.551 | 0.024 | 1.14 | n.a. |

(continued)

| Scyl1 Inhibitor | C2C12 | | beta-TC6 | |
|---|---|---|---|---|
| | Ratio | SE | Ratio | SE |
| Cpd **11** | 2.052 | 0.039 | 2.17 | n.a. |
| Cpd **12** | 2.099 | 0.099 | 2.17 | n.a. |
| Cpd **13** | 2.404 | 0.150 | 2.61 | n.a. |
| Cpd **14** | 2.090 | 0.099 | 1.83 | n.a. |
| Cpd **c3** | 0.914 | 0.040 | 1.26 | n.a. |
| Cpd **c4** | 0.876 | 0.049 | 1.38 | n.a. |

**Example 6: Glucose mediated effect on insulin release by treatment of Beta-T6 cells with compounds of example 2**

Hypoglycemia can cause impairment of cognitive function, motoric control or even consciousness. For safety reasons, it is important that the inventive compounds are glucose dependent and do not enhance the insulin release in low glucose environment. Therefore, we verified the glucose dependent insulin release in beta-TC6 cells using media with 1.125 mM (20 mg/dL) glucose. Cells were cultured for 24 h in a 96-well plate, washed with PBS and then appropriate media was replaced for 2 h at 37°C and 5 % (v/v) $CO_2$. Afterwards, the supernatant was used to detect the amount of insulin released in a rat/mouse insulin ELISA (Merck Millipore Darmstadt, Cat. # EZRMI-13K) by following the manufacture's protocol. The enzyme activity of the horseradish peroxidase of the immobilized biotinylated antibodies was monitored spectrophotometrically by the increased absorbency at 490 nm, and corrected by the absorbency at 610 nm. Results are shown by Figure 5.

Treatment of beta-TC6 cells with compounds of the invention did not lead to increased insulin release like observed in high glucose media. Sunitinib seemed to decrease the insulin release (0.77 $\pm$ 0.03), whereas all the inventive compounds ranged close to the DMSO control. Thus, we rendered Scyl1 as a negatively regulating kinase which has no effect in low glucose media but increases insulin release in a high glucose environment.

**Example 7: High throughput screening based on GRK5 kinase activity assay**

Screening for possible inhibitory compounds of GRK5 was performed using the *ADP Glo™ Kinase Assay technology* (Promega) according to the protocol as describe below. As suitable substrate for full length GRK5 casein was identified. Increasing concentrations of GRK5 are incubated together with 100 μM ATP in the absence and presence of 10 μM casein in a reaction buffer. Within this incubation non-phosphorylated casein is converted into phosphorylated casein and ATP is converted into ADP. Thereafter nonconverted ATP is digested into AMP by addition of the ADP-Glo™Reagent. Subsequently the ADP that is produced by GRK5 activity is phosphorylated back to ATP by addition of Promega Kinase Detection Reagent. These ATP levels serve as measure for the GRK5 activity and are quantified by a luciferase/luciferin reaction. A linear dependence between GRK5 concentration and luminescence signal can be observed. In the absence of casein increasing GRK5 concentration result only in a minor increase of luminescence demonstrating the specificity of the luminescence signal for GRK5 activity.

Km(ATP) was determined by measuring the GRK5 activity at increasing ATP concentrations. Km(ATP) was quantified to be 18.7 μM by fitting the GRK5 activity to the Michaelis Menten equation :

$$v\text{ (GRK5 activity)} = (V_{max} \times [ATP]) / (K_m(ATP) + [ATP])$$

For Grk5 kinase activity assay, the following protocol was used:

The assay was performed in: 384 well U bottom, PP, black, low volume (Corning, 3676) assay plates at reaction temperature of 25°C. The reaction buffer used was composed of: 20 mM MES pH 6.0, 1 mM DTT, 10 mM $MgCl_2$, 0.01 % Tween20 and the reaction volume was 6μl.

Firstly 4 μl 6/4 fold concentrated substrate and 6/4 fold concentrated ATP in 1 fold concentrated reaction buffer are added to each well of the assay plate. Subsequently 67 nl 1000 fold concentrated test compound in 100 % DMSO are added to each well except to C- and C+ wells using pin tool. Then 67 nl 100 % DMSO are pipetted to C- (no kinase,

no compound) and C+ (no compound) wells using pin-tool and 2 μl reaction buffer are added to C- wells. 2 μl 6/2 fold concentrated full length Grk5 (Millipore, #14-714) in reaction buffer is added to each well except C- wells. After incubation for 120 min. at room temperature 6 μl ADP-Glo™ Reagent (ADP Glo™ Kinase Assay Kit: Promega, V9101) are added to each well to stop the kinase reaction and deplete the unconsumed ATP. After a second incubation for 40 min. at room temperature 12 μl of Kinase Detection Reagent are added to convert ADP to ATP and start luciferase / luciferin reaction for detection of ATP. The final assay concentrations are 20 nM GRK5, 18.7 μM ATP and 10 μM of the substrate casein. Finally, after incubation for 40 min at room temperature the luminescence intensity was measured.

**Example 8-1: $IC_{50}$ determination of compounds inhibiting GRK5 by ADP Glo™ Kinase Assay technology (Promega)**

In order to determine the $IC_{50}$ values for all compounds ≥ 35 % inhibition, triplicates at 12 different concentrations with a dilution factor of three were used. The maximal compound assay concentration was set to 100 μM, representing the compound assay concentration used in the primary screen. For all assay plates, z-factor values were found to be significantly larger than 0.5, proving statistical relevance of the data.

The activity of the compounds was classified according to $IC_{50}$ for binding sites of Grk5 into the following ranges:

| | | |
|---|---|---|
| $IC_{50} \leq$ | 5 μM | ++++ |
| 5 μM < $IC_{50} \leq$ | 20 μM | +++ |
| 20 μM < $IC_{50} \leq$ | 80 μM | ++ |
| 80 μM < $IC_{50} \leq$ | 120 μM | + |

**Table 5. $IC_{50}$ values of inventive compounds against GRK5 by ADP Glo™ Kinase assay.**

| compound | $IC_{50}$ |
|---|---|
| **1** | ++ |
| **2** | + |
| **3** | + |
| **4** | ++ |
| **182** | ++ |
| **201** | ++++ |
| **227** | ++ |
| **235** | +++ |
| **412** | +++ |
| **443** | ++ |

**Example 8-2: $IC_{50}$ determination of compounds inhibiting GRK5 by Millipore KinaseProfiler™**

As alternative, $IC_{50}$ values are determined by a radiometric based filtration binding assay, namely Millipore KinaseProfiler™. GRK5 is incubated with 8 mM MOPS pH7.0, 0.2 mM EDTA, 2 mg/mL casein, 10 mM MgAcetate and [$\gamma$-$^{33}$P-APT] (specific activity approx. 500 cpm/pmol, concentration as required). The reaction is initiated by the addition of the MgATP mix. After incubation for 40 minutes at room temperature, the reaction is stopped by the addition of 3 % phosphoric acid solution. 10 μL of the reaction is then spotted onto a P30 filtermat and washed three times for 5 minutes in 75 mM phosphoric acid and once in methanol prior to drying and scintillation counting.

The activity of the compounds was classified according to $IC_{50}$ for binding sites of Grk5 into the following ranges:

| | | |
|---|---|---|
| $IC_{50} \leq$ | 5 μM | ++++ |
| 5 μM < $IC_{50} \leq$ | 20 μM | +++ |
| 20 μM < $IC_{50} \leq$ | 80 μM | ++ |
| 80 μM < $IC_{50} \leq$ | 120 μM | + |

**Table 6. $IC_{50}$ values of inventive compounds against GRK5 by .Millipore KinaseProfiler™ assay**

| compound | $IC_{50}$ |
|---|---|
| **1** | +++ |
| **2** | ++ |
| **3** | ++ |
| **4** | +++ |

**Example 9: Release of Insulin after treatment with the compounds of example 7**

After having identified the most promising compounds acting as Grk5 inhibitors and determined the $IC_{50}$ values, we investigated the impact on insulin release by beta-TC6 cells using the insulin detection assay as described in Example 4, treating the beta-TC6 cells with 5 μM of candidate compounds as well as non-inhibitor controls for 2 h in a high glucose (4.5 g/L) DMEM media at 37 °C and 5 % (v/v) $CO_2$. Afterwards, the supernatant was analyzed for insulin using the rat/mouse insulin ELISA (Merck Millipore Darmstadt, Cat. # EZRMI-13K) by following the manufacture's protocol.. Results are shown in Figure 6.

In spite of the relatively high $IC_{50}$ values, inhibition of Grk5 by the inventive compounds as well as sunitinib treatment resulted in a significant increase of insulin release (Figure 6). Here, Cpd **3** (Inh. 56 % / $IC_{50}$ = 111 μM) depicted the strongest effect with an increase of 65.7 ± 78.3 %, whereas Cpds **1, 2** and **4** also had a significant effect on the increase of insulin release ranging from 35.9 ± 9.0 % (Cpd **2**) to $C_4$: 50.2 ± 41.2 % (Cpd **11**) with lower standard deviations. The negative controls - Cpds without inhibition efficacy - displayed no effect on insulin release (Cpd **c1**: 1.3 ± 0.3 %; Cpd **c2**: 2.0 ± 10.0 %) while the sunitinib control did (53.2 ± 7.5 %). To determine if the identified compounds have also a concentration dependent impact on insulin released by beta-TC6 cells, cells were treated with compound concentrations ranging from 2 μM to 10 μM. Cells were treated for 2 h in a high glucose (4.5 g/L) DMEM media at 37°C and 5 % (v/v) $CO_2$. Insulin release was analyzed using the rat/mouse insulin ELISA. Results are shown in Figure 7 and by Table 7.

All compounds (Cpds) led to a concentration dependent increase of insulin (Figure R41). Interestingly, the insulin release efficiency inclined with the previous inhibitory effect of the compound. Furthermore, the 5 μM values were matching the former insulin release values for all compounds but Cpd **3,** where the induction seemed to be lower than before.

**Table 7.** Release of insulin after inhibition of Grk5 by compounds **1 - 4** with various concentrations in a Beta-TC6-Cell System

| GRK5 Inhibitor | 0μM | 0 μM SD | 2μM | 2 μM SD | 5μM | 5 μM SD | 10μM | 10μM SD |
|---|---|---|---|---|---|---|---|---|
| Cpd **1** | 1 | 0 | 1.24 | 0.17 | 1.81 | 0.31 | 2.03 | 0.37 |
| Cpd **2** | 1 | 0 | 1.13 | 0.21 | 1.70 | 0.32 | 2.00 | 0.35 |
| Cpd **3** | 1 | 0 | 1.12 | 0.09 | 1.38 | 0.06 | 1.44 | 0.08 |
| Cpd **4** | 1 | 0 | 1.11 | 0.19 | 1.56 | 0.09 | 1.58 | 0.31 |

**Example 10: Glucose uptake after treatment of C2C12 and Beta-T6 cells with the compounds of example 7**

To investigate the impact of the Grk5 inhibitors on Glucose uptake, cells were cultured 1 h in glucose free media supplied with 100 μM 2-NBDG at 37 °C and 5 % (v/v) $CO_2$. Furthermore, as internal controls, samples of glucose free media, respectively glucose free media with test compounds, were measured. Fluorescence was analyzed by flow cytometer (FACS Calibur, BD Bioscience, respectively a BD Accuri® C6 Flow Cytometer). For evaluation, cells were gated using the side- and forward scatter SSC/FSC and quantified by excited at 488 and detected at 533 nm (FL1). Exemplary results are shown by Figures 8 and 9 as well as in Table 8.

**Table 8.** Ratio of 2-NBDG uptake after inhibition of Grk5 by revealed inhibitor compounds.

| Grk5 Inhibitor | C2C12 | | beta-TC6 | |
|---|---|---|---|---|
| | Ratio | SE | Ratio | SE |
| DMSO | 1 | 0 | 1 | 0 |
| Cpd **1** | 2.45 | 0.53 | 1.57 | 0.12 |
| Cpd **2** | 2.12 | 0.44 | 1.53 | 0.07 |
| Cpd **3** | 2.34 | 0.46 | 1.68 | 0.21 |
| Cpd **4** | 1.77 | 0.43 | 1.29 | 0.09 |
| Cpd **c2** | 1.73 | 0.11 | | |

As monitored after reduction of target gene expression, inhibition of Grk5 led to an increased uptake of 2-NBDG ranging from 1.77 to 2.45 fold in C2C12 cells (Figure 9), as representatives for peripheral tissue and from 1.29 to 1.68 fold in beta-TC6 cells (Figure 8). Interestingly, the negative control compound displayed a rather high background of 1.74. However, in spite of this high background, the compounds **1 - 3** displayed a significantly higher increase of 2-NBDG uptake upon a 1 h compound treatment.

Inhibition of Grk5 displayed a significantly increased uptake of 2-NBDG in C2C12 cells for three of the five compounds, namely Cpd **1**, Cpd **2** and Cpd **3** (Figure 10).

**Example 11: Glucose mediated effect on insulin release by treatment of Beta-T6 cells with the compounds of example 7**

We investigated the glucose dependent insulin release in beta-TC6 cells using media with 1.125 mM (20 mg/dL) glucose as described in example 6. Cells were cultured for 24 h in a 96-well plate, washed with PBS and fresh media was added for 2 h. Afterwards, the supernatant was taken to detect the amount of insulin released with rat/mouse insulin ELISA. Treatment of beta-TC6 cells with the inventive compounds led to a significantly decreased insulin secretion ranging between 0.14 ± 0.06 (Cpd **2)** and 0.094 ± 0.04 (Cpd **4)** fold to DMSO control (Figure 11). Thus, we rend**e**red GRK5 as glucose dep**e**ndent kinase which decreases insulin release at low glucose and increases insulin secretion at high glucose concentrations.

**Example 12: Evaluation of Insulin Dependence of the inventive compounds**

To investigate if the 2-NBDG uptake of the inventive compound inhibitors of Scyl1 and GRK5 are insulin dependent, 3T3-L pre-adipocytes were differentiated to matured adipocytes. Afterwards, cells were starved for 4 h before they were washed and glucose free media supplied with 100 μM 2-NBDG, in the presence and absence of 10 μg/ml insulin and either Cpd **10** (Scyl1) of Cpd **1** (Grk5) was added for 1 h at 37 °C. Furthermore, as internal controls, samples of glucose free media or glucose free media with compound were measured (data not shown). Fluorescence was analyzed by flow cytometer (FACS Calibur, BD Bioscience, respectively a BD Accuri® C6 Flow Cytometer). For evaluation, cells were gated using the side- and forward scatter SSC/FSC and quantified by excited at 488 and detected at 533 nm (FL1).

The compounds Cpd **10** (Scyl1 inhibitor) and Cpd **1** (Grk5 inhibitor) led to an insulin independent increase of insulin. Whereas insulin alone led to an increase of 2-NBDG uptake of 11.78 ± 1.22 %, Cpd **10** displayed an additional increase of 18.2 ± 3.5 %, respectively Cpd 1 an additional increase 48.9 ± 7.2 %. Interestingly, Cpd **10** did not show the full additional amount of insulin released as without insulin, here an increase of 60.2 ± 8.9 % was determined. For Cpd **1**, in contrast, nearly the same amount of additional insulin release as before (42.8 ± 6.8 %) was monitored.

**Example 13: Comparison of the influence on insulin release of the inventive compounds and commercially available kinase inhibitors**

Based on literature references, several commercially available inhibitors were compared with compounds according to the invention. For comparison we chose the Akt1-Inhibitor II (Calbiochem #124008), which should lead to decreased insulin release, as well as the Map2K3-Inhibitor II (Calbiochem #444938), Exendin-4 (Sigma #E7144) and GLP-1 (Sigma #G8147), which should lead to an increased insulin release. Cells were cultured for 24 h before treated with the inventive compounds and control inhibitors for 2 h at 37 °C and 5 % (v/v) $CO_2$. Insulin in the supernatant was detected by mouse/rat insulin ELISA.

The revealed inhibitors for Grk5 and Scyl1 stimulated insulin released in the beta-TC6 cell system but their non-inhibiting controls did not. All used internal controls like MEK II, GLP-1 and Exendin-4 that should have displayed an increased insulin release, led to the expected effect while the AKT II inhibitor, which was expected to lead to a decrease of insulin, did not. The Grk5 inhibitors (Cpd **1**, Cpd **2**), the Scyl1 inhibitors (Cpd **10**, Cpd **11**, Cpd **13**) as well as sunitinib displayed the highest impact on insulin release, which ranged between 93.35 $\pm$ 14.42 % (Sunitinib) and 53.86 $\pm$ 17.04 % (Cpd **11**). Unfortunatly, Tolbutamide and Glibenclamide displayed no effect.

**Table 9:** Insulin release after target inhibition of Scyl1, Grk5, Akt1, Map2K3 as well as Exendin-4 and GLP-1.

| Name | Inhibitor/Agonist | Conc. | Insulin Release [%] | SE [%] |
|---|---|---|---|---|
| Cpd **1** | Grk5 Inh. | 5μM | 70.74 | 9.94 |
| Cpd **2** | Grk5 Inh. | 5μM | 65.67 | 22.92 |
| Cpd **3** | Grk5 Inh. | 5μM | 14.44 | 2.85 |
| Cpd **4** | Grk5 Inh. | 5μM | 30.13 | 9.49 |
| Cpd **c2** | Grk5 non-Inh. | 5μM | -16.75 | 2.03 |
| Sutent | Pos.-control | 5μM | 93.35 | 14.42 |
| Akt1 | Akt1 Inh. II | 10μM | 0.75 | 7.15 |
| Exendin-4 | Glp-1 Agonist | 20μg/ml | 27.14 | 4.57 |
| GLP-1 | Glp-1 Agonist | 20μg/ml | 16.46 | 5.04 |
| MEK | MAP2K3 Inh. | 10μM | 67.35 | 51.9 |
| Cpd **10** | Scyl1 Inh. | 5μM | 89.8 | 36.07 |
| Cpd **11** | Scyl1 Inh. | 5μM | 53.86 | 17.04 |
| Cpd **12** | Scyl1 Inh. | 5μM | 24.35 | 15.74 |
| Cpd **13** | Scyl1 Inh. | 5μM | 91.62 | 20.61 |
| Cpd **c3** | Scyl1 non-Inh. | 5μM | -8.54 | 13.98 |
| DMSO | Control | 5μM | 0 | 0 |

**Example 14: Insulin HTRF assay principle**

For further screening of compounds derived from compounds described above for their ability to regulate insulin release an insulin HTRF® (Homogeneous Time-Resolved Fluorescence; Cisbio International, France) was used. This assay is based on two antibodies against insulin binding to different epitopes of insulin. One of these antibodies is coupled to Europium (Ab-Eu-Cryptate) and the other one to the fluorophore XL665 (Ab-XL665). Is insulin secreted into the supernatant of cell culture both antibodies can bind to insulin and come therefore into close proximity which allows upon excitation that energy transfer between (FRET) the long-life fluorescent donor Europium (cryptate) and the acceptors XL665. FRET increases proportionally with insulin concentration.

The automated assay protocol is as follows:

> On day 1 15k beta-TC6 cells per well were seeded for 24 or 48 h in 50 μl complete medium. On day 2 or respective on day 3 the medium is removed and cells are washed with 60 μl 1xPBS and 20 μl induction buffer is added using BioTek washer. Subsequently, 10 μl Sunitininb (5 μM f.c.) or the test compound in induction buffer; 0.5% f.c. DMSO (CyBi-well) is transferred to the cells. The cells are then incubated at 37°C for 2, 3, 4h.

The HTRF assay is carried out using Greiner 384-well 784075 assay plates. First 10 μl supernatant is transferred from the cell plates in HTRF plates (CyBi-well). 10 μl of 0, 1, 2, and 4 ng/ml Insulin standard controls in assay medium is used as a control. To each well 10 μl of combined 1:25 Ab-XL665 (Acc) and 1:20 Ab-Eu-Cryptate (WellMate) are added and incubated for 120 min at RT in the dark. Finally HTRF is measured on ViewLux ultra high throughput microplate imager (PerkinElmer).

The evaluation software DataFactoty derives relative activities Ar according to the following equation:

$$Ar = \frac{V - R_{(0\%\,control)}}{R_{(100\%control)} - R_{(0\%\,control)}} \times 100$$

| | |
|---|---|
| Ar | relative activity in % |
| V | raw data value (RFU) of test well (with 10 μM test compound treatment) |
| $R_{(0\%\ control)}$ | median raw data value (RFU) of 0% control wells (without sunitinib treatement) |
| $R_{(100\%\ control)}$ | median raw data value (RFU) of 100% control wells (with 5 μM sunitinib treatement) |

A total of 1,161 compounds were selected and retested in duplicates at 10 μM compound concentration. All 1,161 compounds were active while out of these, 507 compounds confirmed a markedly high relative activity ≥ 70%.

A set of 105 compounds were pursued in dose response experiments and purity determination by LC-MS. After analysis of the compound activities, purity and structural cluster, a set of 64 compounds were further evaluated by ELISA quantification of insulin levels in beta-TC6 cells. These data normalized to the positive control sunitinib are summarized in Table 10.

The activity of the compounds was classified according to their $A_r$ (relative activity) into the following ranges:

| | |
|---|---|
| $A_r \geq 100\ \%$ | ++++ |
| $50\ \% \leq A_r < 100\ \%$ | +++ |
| $20\ \% \leq A_r < 50\ \%$ | ++ |
| $10\ \% < A_r < 20\ \%$ | + |

**Table 10. $A_r$ (relative activity) values of inventive compounds**

| compound | $A_r$ | compound | $A_r$ | compound | $A_r$ |
|---|---|---|---|---|---|
| **1** | ++ | **194** | ++ | **327** | ++ |
| **2** | ++ | **195** | +++ | **328** | ++++ |
| **3** | ++ | **196** | ++ | **329** | ++ |
| **4** | + | **197** | +++ | **330** | ++ |
| **5** | +++ | **198** | +++ | **331** | +++ |
| **6** | ++ | **199** | +++ | **332** | ++ |
| **7** | ++ | **200** | +++ | **333** | ++ |
| **9** | + | **201** | +++ | **334** | +++ |
| **10** | ++ | **202** | ++ | **335** | ++ |
| **11** | ++ | **203** | ++ | **336** | ++ |
| **12** | ++ | **204** | + | **337** | +++ |
| **13** | ++ | **205** | +++ | **338** | ++ |
| **14** | ++ | **206** | +++ | **339** | ++ |
| **16** | ++ | **208** | +++ | **340** | ++ |
| **19** | + | **209** | ++ | **341** | ++ |
| **20** | + | **210** | +++ | **342** | ++ |
| **21** | + | **211** | ++ | **343** | +++ |

(continued)

| compound | $A_r$ | compound | $A_r$ | compound | $A_r$ |
|---|---|---|---|---|---|
| **24** | +++ | **212** | ++ | **344** | ++ |
| **25** | + | **213** | ++ | **345** | ++ |
| **26** | ++ | **214** | ++ | **346** | ++ |
| **32** | + | **215** | ++ | **347** | ++ |
| **33** | ++ | **216** | +++ | **348** | +++ |
| **36** | ++ | **217** | +++ | **349** | ++ |
| **37** | ++ | **218** | +++ | **350** | ++ |
| **39** | + | **219** | +++ | **351** | ++ |
| **41** | + | **220** | +++ | **352** | ++ |
| **42** | + | **221** | +++ | **353** | ++ |
| **44** | + | **222** | ++ | **354** | ++ |
| **46** | ++ | **223** | +++ | **355** | ++ |
| **47** | ++ | **224** | + | **356** | ++ |
| **49** | + | **225** | ++ | **357** | ++ |
| **51** | + | **226** | +++ | **358** | ++ |
| **52** | ++ | **227** | +++ | **359** | + |
| **54** | ++ | **228** | ++ | **360** | ++ |
| **59** | + | **229** | +++ | **361** | +++ |
| **60** | ++ | **230** | ++ | **362** | ++ |
| **61** | ++ | **231** | +++ | **363** | ++ |
| **69** | + | **232** | + | **364** | +++ |
| **72** | + | **233** | +++ | **365** | ++ |
| **74** | ++ | **234** | +++ | **366** | ++ |
| **76** | + | **235** | +++ | **367** | ++ |
| **77** | + | **236** | +++ | **368** | ++ |
| **78** | + | **237** | ++ | **369** | ++ |
| **80** | + | **238** | ++ | **370** | ++ |
| **82** | ++ | **239** | ++ | **371** | ++ |
| **84** | + | **240** | ++ | **372** | ++ |
| **85** | + | **241** | +++ | **373** | ++ |
| **86** | +++ | **242** | ++++ | **374** | ++ |
| **87** | + | **243** | ++ | **375** | ++ |
| **89** | + | **244** | +++ | **376** | ++ |
| **96** | + | **245** | +++ | **377** | ++ |
| **100** | ++ | **246** | +++ | **378** | ++ |
| **101** | ++ | **247** | +++ | **379** | ++ |
| **104** | ++ | **248** | ++ | **380** | ++ |
| **105** | ++ | **249** | ++ | **381** | ++ |

(continued)

| compound | $A_r$ | compound | $A_r$ | compound | $A_r$ |
|---|---|---|---|---|---|
| **106** | ++ | **250** | ++ | **382** | ++ |
| **107** | ++ | **252** | ++ | **383** | ++ |
| **108** | ++ | **253** | + | **384** | ++ |
| **109** | + | **254** | + | **385** | ++ |
| **113** | ++ | **259** | + | **386** | ++ |
| **114** | + | **260** | ++ | **387** | + |
| **115** | + | **261** | + | **388** | ++ |
| **117** | ++ | **263** | ++ | **389** | ++ |
| **119** | + | **267** | ++ | **390** | ++ |
| **122** | ++ | **268** | +++ | **391** | ++ |
| **123** | ++ | **269** | ++ | **392** | ++ |
| **124** | ++ | **270** | ++ | **393** | ++ |
| **125** | + | **271** | ++ | **394** | ++ |
| **126** | + | **273** | ++ | **395** | +++ |
| **127** | ++ | **274** | ++ | **396** | ++ |
| **128** | + | **275** | + | **397** | +++ |
| **129** | +++ | **277** | + | **398** | ++ |
| **130** | + | **278** | + | **399** | ++ |
| **133** | ++ | **279** | ++ | **400** | ++ |
| **134** | ++ | **281** | +++ | **401** | ++ |
| **135** | ++ | **282** | +++ | **402** | + |
| **136** | + | **283** | ++ | **403** | ++ |
| **138** | + | **284** | +++ | **404** | + |
| **145** | ++ | **285** | +++ | **405** | + |
| **147** | ++++ | **286** | ++ | **406** | ++ |
| **148** | ++++ | **287** | +++ | **408** | + |
| **149** | + | **288** | +++ | **409** | ++ |
| **153** | ++ | **289** | +++ | **410** | ++ |
| **155** | ++ | **290** | +++ | **411** | ++ |
| **156** | ++ | **291** | ++ | **412** | +++ |
| **157** | ++ | **292** | ++ | **413** | ++ |
| **158** | + | **293** | ++ | **414** | ++ |
| **159** | + | **294** | ++ | **415** | +++ |
| **161** | ++ | **295** | ++ | **416** | +++ |
| **162** | + | **296** | ++ | **417** | +++ |
| **164** | +++ | **297** | ++ | **418** | ++ |
| **165** | +++ | **298** | ++ | **419** | ++ |
| **166** | ++++ | **299** | ++ | **420** | ++ |

(continued)

| compound | $A_r$ | compound | $A_r$ | compound | $A_r$ |
|---|---|---|---|---|---|
| **167** | +++ | **300** | +++ | **421** | ++ |
| **168** | + | **301** | +++ | **422** | ++ |
| **169** | +++ | **302** | +++ | **423** | + |
| **170** | ++ | **303** | +++ | **424** | +++ |
| **171** | +++ | **304** | +++ | **425** | ++ |
| **172** | ++ | **305** | ++ | **426** | ++ |
| **173** | ++ | **306** | +++ | **427** | ++ |
| **174** | +++ | **307** | +++ | **428** | ++ |
| **175** | ++ | **308** | +++ | **429** | ++ |
| **176** | ++ | **309** | + | **430** | ++ |
| **177** | ++ | **310** | +++ | **431** | ++ |
| **178** | ++ | **311** | ++ | **432** | ++ |
| **179** | ++ | **312** | ++ | **433** | ++ |
| **180** | + | **314** | ++ | **434** | ++ |
| **181** | ++ | **315** | ++ | **435** | ++ |
| **182** | +++ | **316** | ++ | **436** | ++ |
| **183** | +++ | **317** | ++ | **437** | ++ |
| **184** | +++ | **318** | ++ | **438** | ++ |
| **185** | +++ | **319** | ++ | **439** | ++ |
| **186** | + | **320** | ++ | **440** | ++ |
| **187** | ++++ | **321** | + | **441** | ++ |
| **188** | +++ | **322** | +++ | **442** | ++ |
| **190** | +++ | **323** | ++ | **443** | ++++ |
| **191** | +++ | **324** | ++ | **444** | ++ |
| **192** | +++ | **325** | ++ | | |
| **193** | +++ | **326** | ++ | | |

**General chemical procedures**

All reagents were commercial grade and were used as received without further purification, unless otherwise specified. Commercially available anhydrous solvents were used for reactions conducted under inert atmosphere. Reagent grade solvents were used in all other cases, unless otherwise specified. Column chromatography was performed on silica gel 60 (35-70 micron). Thin layer chromatography was carried out using pre-coated silica gel F-254 plates (thickness 0.25 mm). $^1H$ NMR spectra were recorded on a Jeol GSX270 at 270 MHz or a Bruker AM400 (400 MHz) or a Bruker DPX 400 (400 MHz). Chemical shifts for 1 H NMR spectra are given in part per million and either tetramethylsilane (0.00 ppm) or residual solvent peaks were used as internal reference. $^{13}C$-NMR spectra were recorded with a Bruker AM400 (100 MHz) spectrometer using $CDCl_3$ as an internal standard (77.0 ppm). Splitting patterns are designated as follows: s, singlet; d, doublet; t, triplet; q, quartet; p, pentet; m, multiplet; br, broad. Coupling constants are given in Hertz (Hz). Only selected data are reported. Electrospray MS spectra were obtained on a Micromass platform LCMS spectrometer. Compounds were named using Autoname® (MDL). Microwave reactions were performed using a Personal Chemistry Smith's Creator or a CEM microwave.

**General synthetic procedure of compound (II)**

**Example 15 -1 Synthesis of 6-chloro-imidazo[1,2-*b*]pyridazine**

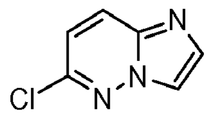

**1*a**

A Solution of 48% HBr (25.2ml) was added to bromoacetaldehyde diethyl acetal (104 g, 0.618 mol) and the resultant mixture was heated to reflux for 1.5 hrs. The reaction mixture was then poured into a suspension of $NaHCO_3$ (20.0 g) in isopropyl alcohol (800 ml). The solution was filtered and 3-amino-6-chloropyridazine (40.0 g, 0.309 mol) was added to the filtrate and the mixture heated at reflux for 2 hours. The mixture was cooled, evaporated to dryness, dissolved in ethyl acetate (1 L), washed with a saturated aqueous solution of $NaHCO_3$ (500 ml), followed by brine (500 ml). The organic layer was then dried ($Na_2SO_4$) and concentrated under reduced pressure. The residue was purified by column chromatography on silica gel (gradient elution with hexane and ethyl acetate 0 to 100%) to give 28.0 g (59%) of 6-chloro-imidazo[1,2-b]pyridazine **1*a** as a brown solid. $^1$H-NMR (400 MHz, DMSO-$d_6$): 8.35 (1H, d, *J* = 0.9 Hz), 8.23 (1 H, d, *J* = 9.5 Hz), 7.86 (1 H, d, *J* = 1.1 Hz), 7.36 (1 H, d, *J* = 9.5 Hz). LCMS-ESI (*m*/*z*); found 154.0 $[M+H]^+$.

**Example 15 -2 Synthesis of 3-bromo-6-chloroimadazo[1,2-b]pyridazine**

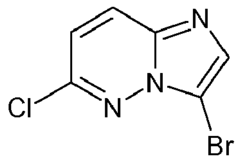

**2*a**

6-Chloroimadazo[1,2-b]pyridazine **1*a** (193.5mmol) was dissolved in chloroform (600 ml) and cooled in an ice bath. NBS (1.05 eq) was then added and the reaction mixture warmed to room temperature, then heated to reflux for 40 minutes. The reaction was cooled, concentrated *in vacuo* and the solid residue dissolved in EtOAc. The organic layer was washed with aq. $K_2CO_3$ (x 3), brine, and then dried with $MgSO_4$, filtered and concentrated *in vacuo.* The product was recrystallised with EtOAc/hexane and triturated with hexane and IPA. Yield (20.4 g) $^1$H-NMR (250MHz,$CDCl_3$) : $\delta$ = 7.11 (1H, d, *J* 7 Hz, Ar), 7.79 (1H, s, Ar), 7.9 (1H, d, *J* 8.6Hz, Ar); HPLC: HPLC Rt 1.86 (100%); m/z (ES+): 233.95 (100%).

**Example 15 -3 Synthesis of 6-chloro-3-iodo-imidazo[1,2-*b*]pyridazine**

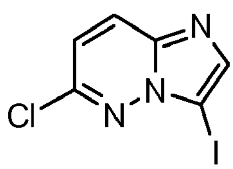

**5***

6-chloroimidazo[1,2-b]pyridazine **1*a** (28.0 g, 0.182 mol) was dissolved in DMSO (196 ml). Iodine (55.0 g, 0.219 mol) was then added and the reaction mixture was stirred at room temperature for 36 hours. The mixture was poured onto a saturated aqueous sodium bicarbonate solution (250 ml) and extracted with dichloromethane (2 x 250ml). The organic layer was washed with an aqueous solution of sodium meta bisulfite (250 ml) followed by brine (250 ml). It was then dried ($Na_2SO_4$) and evaporated under reduced pressure. The residue was recrystallised from ethyl acetate to give 23 g (75 %) of 6-chloro-3-iodo-imidazo[1,2-b]pyridazine 5* as an off white solid. $^1$H-NMR (400 MHz, DMSO-$d_6$) : $\delta$ = 8.27 (1 H, d, *J* = 9.5 Hz), 8.02 (1 H, s), 7.46 (1 H, d, *J* = 9.5 Hz). LCMS-ESI (*m*/*z*); found 280.0 $[M+H]^+$.

**Example 15 -4 Synthesis of 3-bromo-imidazo[1,2-*b*]pyridazine**

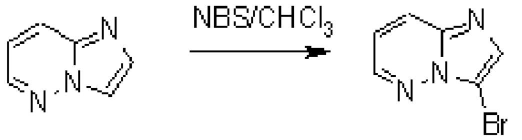


2*b

To imidazo[1,2-b]pyridazine **1*b** (10 g) stirring in chloroform (250 ml) was added *N*-bromosuccinimide (15.7 g) at 5 °C. The cooling bath was removed and the reaction was heated to reflux which was maintained for 30 minutes. After leaving to cool overnight the reaction mixture was concentrated under reduced pressure. The residue was redissolved in ethyl acetate (500 ml), washed with potassium carbonate solution (3 x 200 ml) then brine (100 ml). The organic extract was dried with magnesium sulphate and concentrated *in vacuo* to give 3-bromo-imidazo[1,2-b]pyridazine **2*b,** 12.3 g (74%). $^1$H-NMR (400MHz, DMSO-$D_6$) : $\delta$ = 8.68 (1 H, d, 4.4 Hz, ArH), 8.20 (1 H, d, 9.2 Hz, ArH), 7.95 (1 H, s, ArH), 7.33 (1 H, q, 3.4 Hz, ArH).

**Example 15-5 General procedure for the synthesis of 3-bromo-6-$R^1$-imidazo[1,2-*b*]pyridazine 4*. ($R^1$= aminyl) by amine ($R^1H$) displacement**

A mixture of 3-bromo-6-chloroimadazo[1,2-b]pyridazine **2*a** (15 g, 64.52 mmol, 1 eq.), the appropriate amine ($R^1H$, 96.78 mmol, 1.5 eq.) and DIPEA (16.90 ml, 96.78 mmol, 1.5eq.) in ethanol (15 ml) was placed in the CEM Microwave and stirred and heated at 160 °C for 10 minutes and 180 °C for 2 hours. The solvent was removed under reduced pressure. The residue was dissolved in ethyl acetate, washed with water (twice) and brine and then filtered through a pad of silica and washed with ethyl acetate. The organic solvent was evaporated under reduced pressure and the crude residue was slurried in hexane/10% ether. The crystalline product was filtered and washed with hexane/ 10% ether and dried to give the intermediate 3-bromo-6-$R^1$-imidazo[1,2-*b*]pyridazine **4***.

Typical example: 3-bromo-*N*-(2-methoxyethyl)imidazo[1,2-b]pyridazin-6-amine

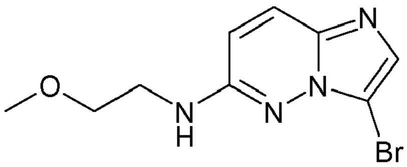

4*a

Yield 3.58 g, (68 %). $^1$H-NMR (270MHz, $CDCl_3$): $\delta$ = 2.96(s, 6H, $CH_3$ x 2), 6.28 (brs, 2H, $NH_2$), 6.78 (d, *J* 9 Hz, 2H, Ar), 7.56 (d, *J* 9 Hz, 2H, Ar), 7.92 (s, 1 H, H-6). HPLC: Rt (min): 1.46 (99%); m/z; (AP+): 293 & 295 (isotopes|, M+1, 100%).

Typical example: (3-bromoimidazo[1,2-*b*]pyrazin-6-yl)-methylamine

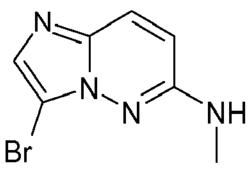

4b*

3-Bromo-6-chloroimidazo[1,2-b]pyridazine **2*a** (20.32 g, 87.6 mmol) was suspended in 33% $MeNH_2$ in EtOH (80 ml) and heated to 150 °C in a CEM MARS microwave reactor for 45 minutes. The reaction was cooled to room

temperature and concentrated under reduced pressure. The crude product was purified by trituration with boiling water to give (3-bromoimidazo[1,2-a]pyrazin-6-yl)-methylamine (19.8 g, 87 mmol, 99%). $^1$H-NMR (400MHz, DMSO-$d_6$) : δ = 7.69 (1 H, d, *J* 9.6 Hz, ArH), 7.48 (1 H, s, ArH), 7.16 (1 H, d, *J* 4.8 Hz, ArH), 6.68 (1 H, d, *J* 9.6 Hz, ArH) and 2.83 (1 H, d, *J* 5.2 Hz, Me); LCMS DAD:TIC 100%, *m/z* (ES): 227 (M+H).

**Example 15-6 General procedure for the synthesis of 6-R$^1$-imidazo[1,2-b]pyridazines 3* (R$^1$= alkyl)**

To a suspension of Zn powder (2.5 g, 38 mmol) in 1,4-dioxane (40 ml) was added iodine (0.9 mmol) and the mixture was placed under an atmosphere of nitrogen. The benzyl bromide (19 mmol) was added in portions (after the first portion the mixture was gently heated to initiate reaction). Subsequent portions did not require heating and once all the portions were added the mixture was allowed to stir at room temperature for 4 hours. After this time the mixture was allowed to settle and the liquid was decanted from the excess Zinc and the resulting solution could be used immediately in the next reaction.

A solution of the alkyl Zinc reagent (see note below) (2 eq.) was added to $NiCl_2$dppp (10 mol %) under an atmosphere of nitrogen and the mixture was stirred at room temperature for 5 minutes. After this time the 6-chloro-imidazo[1,2-b]pyridazine (1 eq.) was added as a solid in one portion and the mixture was heated at 50 °C for 18 hours. The mixture was cooled to room temperature and a saturated aqueous solution of $NH_4Cl$ was added followed by EtOAc. The mixture was filtered through celite and the phases were separated. The organic phase was washed with brine, dried ($MgSO_4$) and concentrated under reduced pressure and the residue was purified by column chromatography on silica gel (mixtures of Ethyl Acetate and Petroleum Ether).

Note: The alkyl Zinc reagents could be purchased as 0.5 M solutions in THF or they could be synthesized from the corresponding alkyl halides as solutions in 1,4-dioxane. A general procedure for this is shown below.

Typical example: 6-benzyl-imidazo[1,2-b]pyridazine

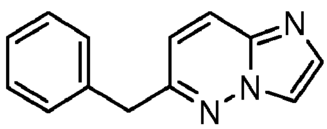

**3*a**

The benzyl zinc bromide used for this reaction was obtained from a commercial supplier (Aldrich). Yield 97 %. $^1$H-NMR (400 MHz, DMSO-$d_6$) : δ = 8.31-8.31 (1H, m), 8.11 (1 H, d, *J* = 9.6 Hz), 7.75 (1 H, d, *J* = 1.3 Hz), 7.33-7.32 (4H, m), 7.27-7.23 (1 H, m), 7.21 (1 H, d, *J* = 9.4 Hz), 4.18 (2H, s).

Typical example: 6-(3,4difluoro-benzyl-imidazo[1,2-b]pyridizine

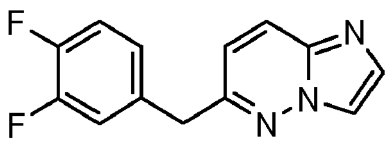

**3*b**

The benzyl zinc bromide used for this reaction was generated. Yield 100 %. $^1$H-NMR (400 MHz, DMSO-$d_6$) δ = 8.26 (1 H, m), 8.08 (1 H, d, *J* = 9.4 Hz), 7.74 (1 H, d, *J* = 1.2 Hz), 7.47-7.34 (2H, m), 7.20-7.18 (2H, m), 4.19 (2H, s).

**Example 15-7 General procedure for the synthesis of 6-R$^1$-3-bromo-imidazo[1,2-*b*]pyridazines 4* (R$^1$=alkyl)**

To a stirred solution of the 6-alkyl-imidazo[1,2-*b*]pyridazines (9.6 mmol) in acetonitrile (16 ml) was added NBS (9.6 mmol) in one portion and the mixture was stirred at room temperature for 1 hour. The solvent was removed under reduced pressure and the residue was taken up in ethyl acetate (100 ml) then washed with 2N NaOH (50 ml), an aqueous solution of sodium metabisulfite solution, dried ($MgSO_4$) and concentrated under reduced pressure to give the desired compound.

Typical example: 3-bromo-6-(3-methyl-benzyl)imidazo[1,2-*b*]pyridazine

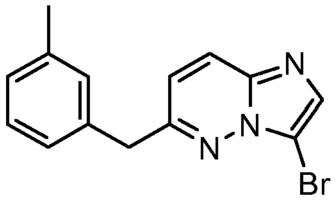

**4*c**

Yield 86 %. $^1$H-NMR (400 MHz, DMSO-$d_6$) δ = 8.07 (1 H, d, *J* = 9.4 Hz), 7.88 (1 H, s), 7.24-7.06 (5H, m), 4.18 (2H, s).

Typical example: 3-bromo-6-(3,4-difluoro-benzyl)imidazo[1,2-*b*]pyridazine

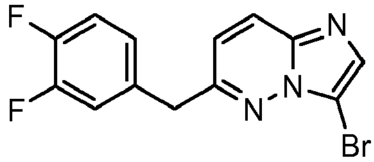

**4*d**

Yield 83 %. $^1$H-NMR (400 MHz, DMSO-$d_6$) δ = 8.10 (1 H, d, *J* = 9.4 Hz), 7.88 (1 H, s), 7.48-7.42 (2H, m), 7.22 (1 H, d, *J* = 9.4 Hz), 7.20-7.17 (1 H, m), 4.24 (2H, s).

**Example 15 -8 Library production of compound (II) by Suzuki Reactions of intermediate 4***

To a solution of intermediate 6-$R^1$-3-bromo-imidazo[1,2-*b*]pyridazines **4*** (0.3 mmol) in DMF (0.5 ml) was added a solution of boronic acid ($R^2B(OH)_2$) (0.36 mmol) in DMF (0.6 ml) and degassed aqueous sodium carbonate solution (1.5 M, 0.5 ml). The reaction vessels were then flushed with nitrogen in a glove box. A solution of palladium acetate (95 mg) and triphenylphosphine (335 mg) in 1,4-dioxane (15 ml) was prepared and 0.3 ml was added to each reaction vessel under nitrogen. The vessels were then heated at 80 °C with agitation for 16 hours. The reaction mixtures were filtered and purified by preparative HPLC.

**Example 15-9 Library production of compound (II) by Suzuki reactions of compound 4*b**

All solvents used were degassed by bubbling nitrogen through them for 1h before use. To a solution of (3-bromoimidazo[1,2-*b*]pyrazin-6-yl)-methylamine **4*b** (1.0 mmol) in DMF (1 ml) was added a solution of boronic acid (1.2 mmol) in DMF (1.2 ml), 4.0 M aq. CsF (4.0 mmol, 1 ml) and palladium acetate (0.05 mmol) in DMF (0.2 ml). Triphenylphosphine (0.15 mmol) in DMF (0.2 ml) was added then the reaction tubes flushed with nitrogen and capped. The reactions were heated at 90 °C with agitation for 16 hours. The reaction mixtures were filtered and purified by preparative reverse phase HPLC.

**Example 15-10 General procedure for the synthesis of 6-chloro-3-$R^2$-imidazo[1,2-*b*]pyridazines 6* ($R^2$ = Aryl)**

To a solution of 3-iodo-6-chloroimidazo [1,2-*b*] pyridazine **5*** (14.0 g, 0.0500 mol) in deoxygenated dimethylformamide (168 ml) and water (56 ml) was added $Na_2CO_3$ (13.3 g, 0.125 mol), $PPh_3$ (1.96 g, 0.007 mol) and $Pd(OAc)_2$ (0.560 g, 0.003 mol), followed by the boronic acid ($R^2B(OH)_2$) (0.0500 mol) under a nitrogen atmosphere. The reaction mixture was heated at 80°C for 18 hours, then cooled to room temperature, evaporated to dryness, dissolved in ethyl acetate, 500 ml and washed with brine solution (250 ml). The organic layer was dried ($Na_2SO_4$) and concentrated in under reduced pressure. The crude product was purified by column chromatography on silica gel using hexane and ethyl acetate mixtures.

Typical example: *N*-[4-(6-chloro-imadazo[1,2-b]pyridazin-3-yl)-phenyl]-acetamide

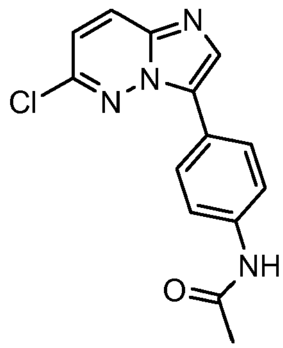

**6*a**

Yield 47%. $^1$H-NMR (400 MHz, DMSO-$d_6$) δ = 10.15 (1H, s), 8.29 (1H, d, *J* = 9.4 Hz), 8.26 (1 H, s), 8.03 (2H, d, *J* = 8.8 Hz), 7.75 (2H, d, *J* = 8.8 Hz), 7.40 (1 H, d, *J* = 9.4 Hz), 2.09 (3H, s). LCMS-ESI (m/z); found 287.1 $[M+H]^+$

Typical example: 1-[3-(6-chloro-imidazo[1,2-b]pyridazin-3-yl)-phenyl]-ethanone

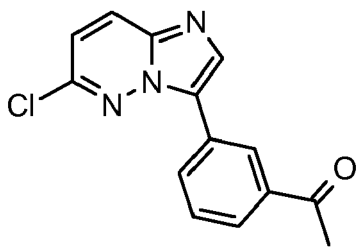

**6*b**

Yield 54 %. $^1$H-NMR (400 MHz, DMSO-$d_6$) : δ = 8.72-8.71 (1 H, m), 8.48 (1 H, s), 8.40-8.37 (2H, m), 8.06-8.03 (1 H, m), 7.77-7.73 (1 H, m), 7.51 (1 H, d, *J* = 9.5 Hz), 2.71 (3H, s). LCMS-ESI (*m/z*); found 272.1 $[M+H]^+$

**General synthetic procedure of compound (III)**

**Example 16-1 Synthesis of 3-hydrazino-5-iodo-pyridine**

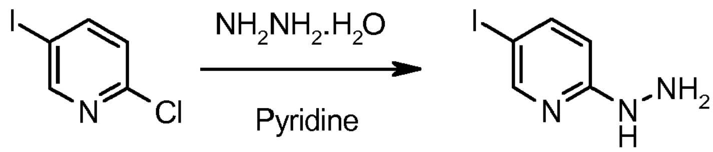


**7***

To a solution of 2-chloro-5-iodopyridine (25 g, 0.104 mol) in pyridine (250 mL), was added hydrazine hydrate (24.95 mL, 0.522 mol) and the reaction heated to 100-110 °C for 18 hours. The mixture was poured onto cracked ice, extracted with dichloromethane, washed with 2 M sodium hydroxide solution and dried over sodium sulfate. The organic layer was evaporated under reduced pressure to afford pure product as a yellow solid, 24.0 g (89%).
$^1$H-NMR (400 MHz, $CDCl_3$): δ = 8.28 (d, 1 H, J=1.2 Hz), 7.69 (dd, 1 H, $J_1$=2.0 Hz, $J_2$=6.8 Hz), 6.60 (d, 1 H, J=4.8 Hz), 5.81 (br, 1 H), 3.79 (br, 2H). LC-MS: 235 (M) HPLC: 87.69%.

**Example 16-2 Synthesis of N-R$^2$-N'-(5-iodo-pyridin-2-yl)-hydrazone**

Typical example: N-benzylidene-N'-(5-iodo-pyridin-2-yl)-hydrazone

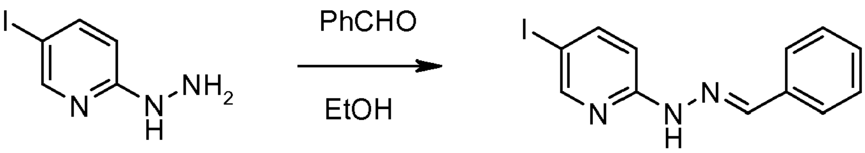


**7*a**

To a solution of (5-iodo-pyridin-2-yl)hydrazine **7*** (5 g, 0.22 mol) in ethanol (50 mL), was added the benzaldehyde and the reaction mixture was stirred at 50 °C for 30 minutes. It was then cooled, filtered and washed with cold ethanol to afford the title compound as pure solid.
$^1$H-NMR (400 MHz, DMSO): δ = 11.03 (1 H, s), 8.29 (1 H, d,J=2.26), 8.03 (1 H, s), 7.91 (1 H, dd, J= 8.79, 2.31), 7.67 (2 H, m), 7.37 (3H, m), 7.14 (1 H, d, J=8.8). LC-MS: 323 (M). Yield 72%.

**Example 16-3 Synthesis of 6-Iodo-3-R$^2$-[1,2,4]triazolo[4,3-a]pyridine 8* :**

Typical example: 6-Iodo-3-phenyl-[1,2,4]triazolo[4,3-a]pyridine

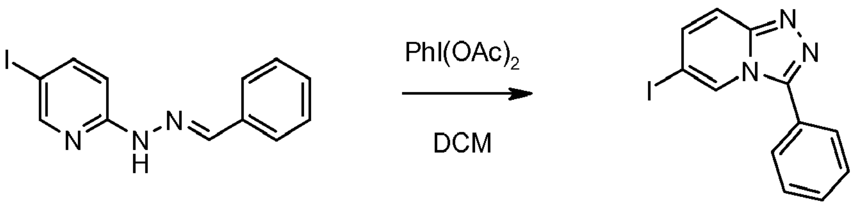


**8*a**

*N*-benzylidene-*N*'-(5-iodo-pyridin-2-yl)-hydrazone **7*a** (20 mmol) and iodobenzene diacetate (20 mmol) were dissolved in dichloromethane (10 vol) and stirred at room temperature for 16 hours. The solvent was evaporated at reduced pressure and the residue obtained was purified by silica gel chromatography (60-120 mesh) using 0-5% MeOH / $CHCl_3$ as eluent.
$^1$H-NMR (400 MHz, $CDCl_3$): δ = 8.52 (1 H, t, J = 1.22 Hz), 7.83-7.79 (2 H, m), 7.65-7.56 (4 H, m), 7.43 (1 H, dd, J = 9.57, 1.49 Hz). LC-MS: 321 (M). Yield 71 %

**Example 16-4 Synthesis of 6-iodo-1,2,4-triazolo[4,3-*a*]pyridine**

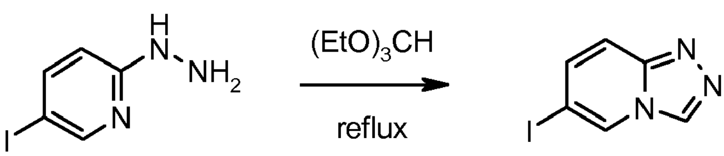


**9***

A mixture of 3-hydrazino-5-iodo-pyridine (2.0 g) and triethyl orthoformate (25 mL) were heated together under reflux for 18 hours. The reaction was cooled to 0 °C and the solid filtered off, washed with cold diethyl ether and dried to afford the desired compound (1.3 g, 65%).
$^1$H-NMR (400 MHz, $CDCl_3$): δ = 8.75 (1 H, s), 8.43 (1 H, t, J = 1.23 Hz), 7.61 (1 H, d, J = 9.59 Hz), 7.42 (1 H, dd, J = 9.59, 1.51 Hz). $^1$H-NMR (400 MHz, DMSO): 9.14 (1 H, d, J = 0.85 Hz), 8.96 (1 H, t, J = 1.28 Hz), 7.64 (1 H, dt, J = 9.54, 0.95 Hz), 7.53 (1 H, dd, J = 9.54, 1.56 Hz). LC-MS: 245 (M).

**Example 16-5 Synthesis of 6-$R^1$-1,2,4-triazolo[4,3-*a*]pyridine 10* ($R^1$ =** aryl)

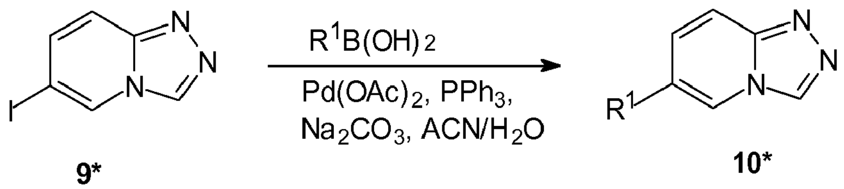


**9*** **10***

Palladium acetate (70 mg) was added to a degassed mixture of 6-iodo-1,2,4-triazolo[4,3-a]pyridine **9*** (1.5 g), arylboronic acid ($R^1B(OH)_2$, 1.2 equiv), sodium carbonate (2.0 g) and triphenyl phosphine (320 mg) in acetonitrile/water (3:1, 40 mL). The mixture was then heated at 85 °C under nitrogen for 18 hours, and then cooled. The organic phase was separated, washed with water, dried and evaporated to give the crude product (contaminated with some triphenylphosphine and triphenylphosphine oxide) which was sufficiently pure to use in the following step.

Typical example: 6-Phenyl-1,2,4-triazolo[4,3-a]pyridine

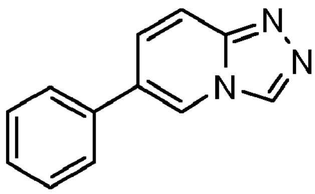

**10*a**

Yield: 800 mg. $^1$H-NMR (400 MHz, DMSO): $\delta$ = 9.26 (1 H, s), 8.92 (1 H, t, J = 1.34 Hz), 8.01 (2 H, s), 7.88 (1 H, d, J = 9.56 Hz), 7.83-7.71 (5 H, m). LC-MS (M) 195

**Example 16-6 Synthesis of 6-$R^1$-3-bromo-1,2,4-triazolo[4,3-a]pyridines**

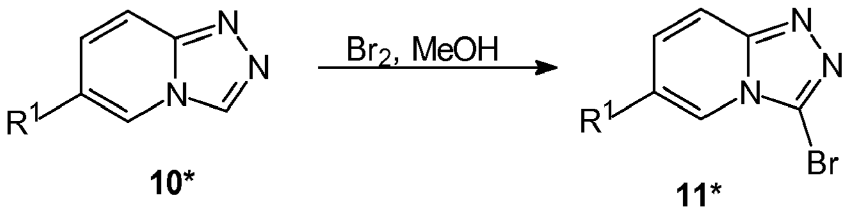


**10*** **11***

Bromine (0.8 mL, 0.0155 mol) was added dropwise to a solution of the 6-$R^1$-1,2,4-triazolo[4,3-a]pyridines **10*** (0.004 mol) in methanol (20 mL) at room temperature. The mixture was stirred for 1 hour and then evaporated. Ethyl acetate and sodium hydrogen carbonate solution were then added and the organic phase separated, washed with brine, dried with magnesium sulphate and evaporated. The product was purified by column chromatography over silica gel using petroleum ether and ethyl acetate as eluent (gradient 75:25 to 0:100) to give the product **11***.

Typical example: 6-Phenyl-3-bromo-1,2,4-triazolo[4,3-a]pyridine

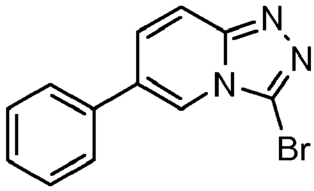

11*a

Yield: 0.75 g (67%). $^1$H-NMR (400 MHz, $CDCl_3$): δ = 8.14 (1 H, s), 7.84 (1 H, dd, J = 9.54, 1.05 Hz), 7.63-7.45 (6 H, m). LC-MS (M) 273/275

**Example 16-7 Library production of compound (III) via Suzuki coupling**

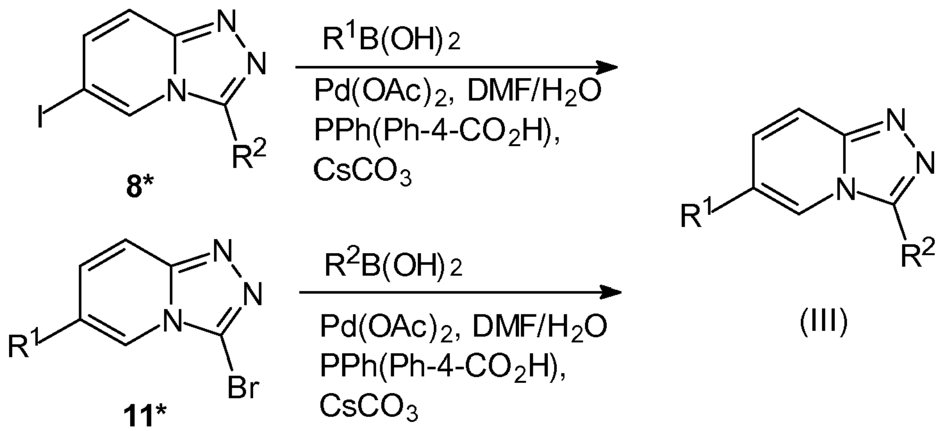


8*

11*

(III)

All reactions were carried out in parallel and stock solutions of the following reagents were prepared; 6-iodo-3-$R^2$-1,2,4-triazolo[4,3-a]pyridines **8*** or 6-$R^1$-3-bromo-1,2,4-triazolo[4,3-a]pyridines **11*** (0.2 M in DMF), boronic acid ($R^1B(OH)_2$ or $R^2$B(OH)2, 0.36 M in DMF), 4-($CO_2$H)($C_6H_4$)$PPh_2$ (0.05 M in DCM), $Pd(OAc)_2$ (0.03 M in DMF) and $CsCO_3$ (4.6 M in $H_2O$). The reactions were performed in 17 x 125 mm sealable tubes.

The reaction vessels were charged with 4-($CO_2$H)($C_6H_4$)$PPh_2$ (1.0 mL) and the solvent evaporated off. Then the vessels were charged with 6-iodo-3-$R^2$-1,2,4-triazolo[4,3-a]pyridines **8*** or 6-$R^1$-3-bromo-1,2,4-triazolo[4,3-a]pyridines **11*** (1.5 mL, 0.3 mmol) followed by the requisite boronic acid ($R^1B(OH)_2$ or $R^2B(OH)_2$, 1 mL, 0.36 mmol), $CsCO_3$ (1.0 mL, 1.15 mmol) and $Pd(OAc)_2$ (0.5 mL, 0.025 mmol). The reactions vessels were flushed with nitrogen, sealed and heated at 100 °C for 18 hours. After this time, the mixtures were allowed to cool to room temperature. Centrifuged for 20 - 30 minutes then the supernatant solvent was removed, filtered through cotton wool and purified by reverse phase preparative HPLC.

Typical example: 3,6-diphenyl-1,2,4-triazolo[4,3-*a*]pyridine

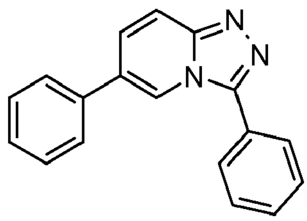

16-7

Yield 56 mg. $^1$H-NMR (400 MHz, $CDCl_3$): δ = 8.39 (1 H, t, J = 1.32 Hz), 7.91-7.84 (3 H, m), 7.64-7.52 (6 H, m), 7.51-7.41 (3 H, m).

**Example 16-8 Library production of compound (III) *via* Stille coupling**

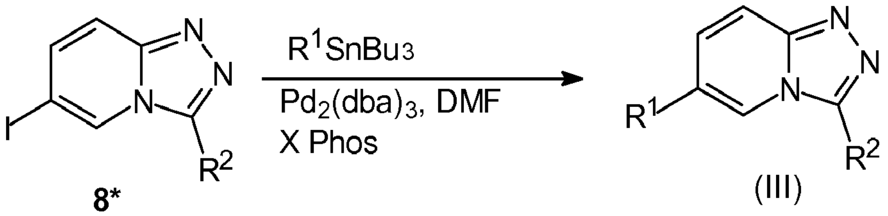


8*

(III)

All reactions were carried out in parallel in 17 x 125 mm sealable tubes. The reaction vessels were charged with 6-iodo-3-$R^2$-1,2,4-triazolo[4,3-a]pyridines 8* (1.5 mL, 0.3 mmol), $Pd_2(dba)_3$ (2 mg), X-phos (4 mg), tributyl-2-pyridylstannane and dry dimethylformamide (3 mL). The tubes were then flushed with nitrogen and heated at 70 °C for 3 hours. The cooled solutions were then evaporated, dissolved in a minimum of methanol and loaded onto a SPE extraction column (5 g, sulfonic acid) eluted initially with methanol (10 mL) and then the crude product eluted with a 1 M solution of ammonia in methanol (10 mL). The solution was then evaporated, dissolved in dimethyl formamide and purified by reverse phase preparative HPLC.

Typical example: 6-(2'-pyrimidinyl)-3-phenyl-1,2,4-triazolo[4,3-a]pyridine

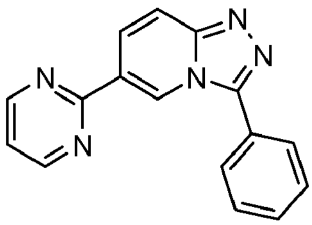

**16-8**

Yield 30 mg. $^1$H-NMR (400 MHz, $CDCl_3$): $\delta$ = 9.44 (1 H, d, J = 1.56 Hz), 8.81 (2 H, d, J = 4.83 Hz), 8.38 (1 H, dd, J = 9.71, 1.51 Hz), 7.95-7.88 (3 H, m), 7.68-7.60 (3 H, m).

**Example 16-9 Library production of compound (III) *via* Sonogashira coupling**

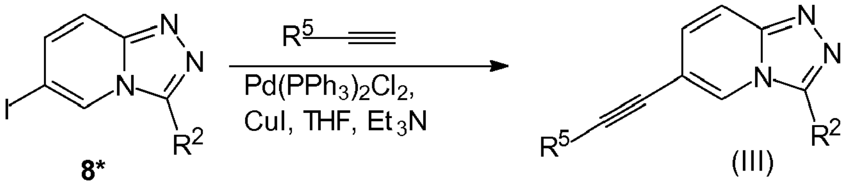


8*

(III)

All reactions were carried out in parallel in 17 x 125 mm sealable tubes. Stock solutions or fine suspensions of the following reagents were prepared; 6-iodo-3-$R^2$-1,2,4-triazolo[4,3-a]pyridines **8*** (0.15 M in THF) and copper iodide (0.042 M in $Et_3N$)
The reaction vessels were charged with 6-iodo-3-$R^2$-1,2,4-triazolo[4,3-a]pyridines **8*** (2 mL), copper iodide in triethylamine (0.042 M, 0.25 mL), the requisite acetylene (0.036 M) and $(Ph_3)_2PdCl_2$ (2 mg). The tubes were then flushed with nitrogen, sealed then heated at 50 °C for two hours, then cooled and evaporated. The residues were then suspended in dry dimethylformamide (3.5 mL), filtered and purified by reverse phase preparative HPLC.

Typical example: 6-(3'-dimethylaminoprop-1-ynyl)-3-phenyl-1,2,4-triazolo[4,3-a]pyridine

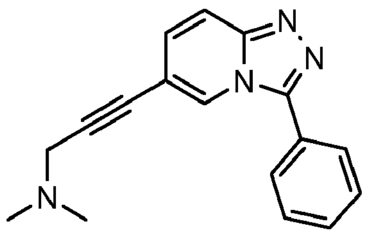

**16-9**

Yield 64 mg. $^1$H-NMR (400 MHz, $CDCl_3$): δ = 8.37 (1 H, s), 7.87-7.75 (3 H, m), 7.64-7.55 (3 H, m 3.45 (2 H, s), 2.37 (6H, s). LC-MS (M) 276

**General synthetic procedure of compound (IV)**

**Example 17-1 Synthesis of 2-amino-5-bromopyrazine**

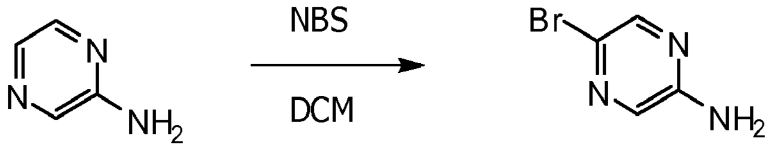


**12***

To a solution of 2-aminopyrazine (200 g, 2.1 mol) in dichloromethane (2.5 L) cooled to between 0 °C and -5 °C, was added *N*-bromosuccimide (375 g, 2.1 mol) over a 4 hour period, during which time the temperature was kept below 0 °C. The reaction mixture was kept below 0 °C overnight, stirred vigorously and quenched with water (1 L). The mixture was filtered and washed with 10% aqueous $K_2CO_3$. The organic phase was dried over $MgSO_4$ and concentrated *in vacuo.* The resultant solid was triturated with hexane and ethyl acetate. The yellow/brown solid was filtered and dried to give 2-amino-5-bromopyrazine **12*** (142 g, 39 %). $^1$H-NMR (270MHz, $CDCl_3$): δ = 4.79 (2H, br.s, $NH_2$), 6.98-7.77 (1H, s, Ar), 8.08(1 H, s, Ar).

**Example 17-2 Synthesis of compound *N*-(5-bromo-pyrazine-2-yl)-4-methyl-benzenesulfonamide**

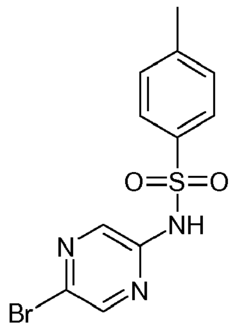

**12*a**

To a stirred solution of 2-amino-5-bromopyrazine **12*** (60 g, 0.34 mol) in pyridine (600 ml) was added tosyl chloride (73.2 g, 0.38 mol). The reaction was stirred at room temperature for 18 hours then concentrated *in vacuo.* The residue was triturated (50% methanol in dichloromethane) to yield the compound **12*a** (87.6 g, 79%). $^1$H-NMR (270 MHz, $CDCl_3$): δ = 2.49 (3H, s), 7.54 (2H, d, *J* 8.1 Hz), 8.01 (2H, d, *J* 8.3 Hz), 8.32 (1 H, d, *J* 1.5 Hz), 8.57 (1 H, d, *J* 1.5 Hz).

**Example 17-3 Synthesis of *N*-(5-bromo-pyrazin-2-yl)-N-cyanomethyl-4-methyl-benzenesufonamide**

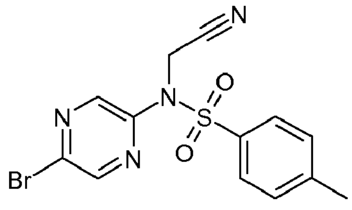

**12*b**

*N*-(5-bromo-pyrazine-2-yl)-4-methyl-benzenesulfonamide **12*a** (60 g, 0.19 mol) was added in portions to a suspension of sodium hydride (60% in mineral oil, 8.7 g, 0.22 mol), in DMF (500 ml) over a period of 30 minutes. The mixture was stirred at room temperature for 30 minutes before the addition of bromoacetonitrile (15 ml, 0.23 mol) over a further 30 minutes. The reaction was heated at 60 °C for three hours then stirred for 18 hours at room temperature. The reaction was concentrated in *vacuo* and the residue was purified by trituration with methanol to yield the compound **12*b** (49 g, 68%). $^1$H-NMR (270 MHz, $CDCl_3$): $\delta$ = 2.41 (3H, s), 4.68 (2H, s), 7.42 (2H, d, *J* 8.1 Hz), 7.71 (2H, d, *J* 8.1 Hz), 8.41 (1 H, d, *J* 1.2 Hz), 8.92 (1 H, d, *J* 1.2 Hz).

**Example 17-4 Synthesis of 6-bromo-imidazo[1,2-a]pyrazin-3-ylamine**

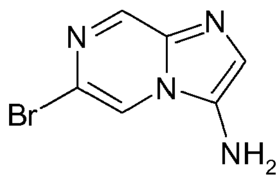

**13***

A solution of *N*-(5-bromo-pyrazin-2-yl)-*N*-cyanomethyl-4-methyl-benzenesufonamide **12*b** (49 g, 0.13 mol) in a mixture of trifluoroacetic acid (360 ml) and water (40 ml) was heated at 40 °C for 1 hour. Toluene (2 x 250 ml) was then added and the mixture concentrated *in vacuo.* The residue was treated with an aqueous solution of sodium acetate (40 g, 150 ml) and stirred for 1 hour at 0 °C. The product was collected by filtration and washed with cold water to yield the compound **13*** (25 g, 85%). $^1$H-NMR (270 MHz, $CDCl_3$): $\delta$ = 6.03 (2H, br s), 7.27 (1 H, s), 8.52 (d, *J* 1.2 Hz), 8.72 (1 H, d, *J* 1.2 Hz); m/z (APCI) 213 (M+); HPLC 91 %

**Example 17-5 Synthesis of intermediate (15*) by amide coupling with carboxyl chloride(14*): typical example; *N*-(6-bromo-imidazo[1,2-*a*]pyrazin-3-yl)-2-methyl-benzamide.**

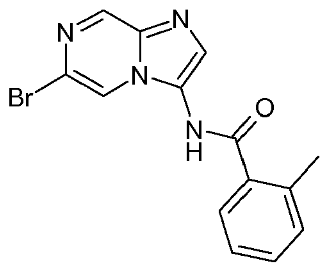

**15*a**

To a solution of o-tolylacetic acid (18.02 g, 120 mmol) in $CH_2Cl_2$ (200 ml) was added oxalyl chloride (32 ml, 360 mmol) followed by DMF (3 drops). The reaction was stirred at room temperature for 3 hours then concentrated *in vacuo.* The residue was dissolved in THF (50 ml) then added to a suspension of 6-bromo-imidazo[1,2-a]pyrazin-3-ylmine **13*** in THF (150 ml) and pyridine (9.7 ml, 120 mmol). The mixture was heated at 50 °C for 30 minutes. After this time the reaction was concentrated *in vacuo* and a mixture of ethyl acetate (250 ml) and a saturated solution of $NaHCO_3$ (250 ml) was added. The resultant brown solid was collected by filtration to give the desired compound **15*a** (16.4 g, 79%). $^1$H-NMR (250 MHz, DMSO): $\delta$ = 2.3 (3H, s), 3.86 (2H, s), 7.12-7.16 (3H, m), 7.27-7.30 (1 H, m), 7.87 (1 H, s), 8.76 (1 H, d, *J* 1.2 Hz), 8.87 (1 H, d, *J* 1.2 Hz); m/z (APCI) 345 (M+); HPLC 100%.

**Example 17-6 Library Production of compound (IV) by Suzuki reactions**

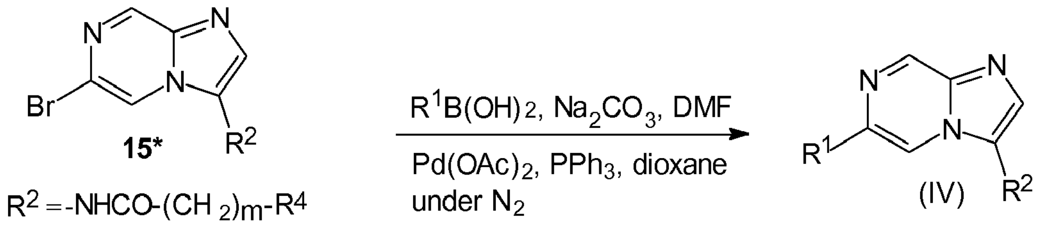


15*

$R^2$ = -NHCO-$(CH_2)_m$-$R^4$

(IV)

To a solution of intermediate **15*** in DMF (0.3 mmol, 0.5 ml) was added a solution of boronic acid ($R_1B(OH)_2$) in DMF (0.36 mmol in 0.6 ml) and 1.5 M aq. $Na_2CO_3$ (0.75mmol, 0.5ml). The reaction vessels were then placed in a nitrogen filled glovebox for 30 min. A solution of palladium acetate and triphenylphosphine in dioxane (0.3 ml of a stock solution of 95 mg palladium acetate 335 mg triphenylphosphine in 15 ml 1,4-dioxane) was added to each reaction vessel inside the glovebox. The vessels were capped and then heated at 80 °C with agitation for 16 hours. The reaction mixtures were filtered and purified by automated preparative HPLC.

**Example 17-7 Synthesis of 6-bromoimidazo[1,2-*a*]pyrazine**

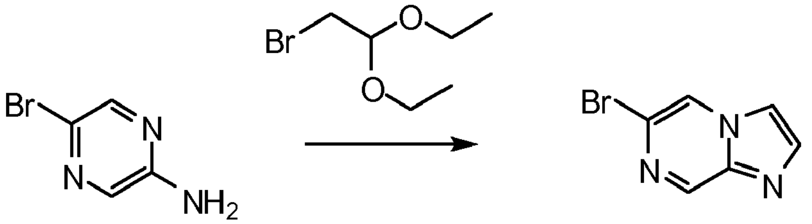

**16***

A mixture of bromoacetaldehyde diethylacetal (19.7 g, 0.1 M) and 48% hydrobromic acid (4 ml) was heated to 140 °C under nitrogen for 1.5 hours. The resulting mixture was poured onto a stirred suspension of sodium bicarbonate (40 g) in propan-2-ol (200 ml) and the resulting suspension was stirred for 10 minutes and then filtered. 5-Bromo-pyrazine-2-ylamine **12*** (8.65 g, 0.05 M) was added to the filtrate and the resulting solution was heated at reflux for two hours. The solvent was evaporated *in vacuo* to give dark brown viscous material. The residue was treated with saturated sodium bicarbonate (150 ml) and extracted twice with dichloromethane (500 ml). The combined extracts were dried and evaporated *in vacuo* to give a brown oil, which was purified by flash chromatography with ethyl acetate as eluant to give 7.0 g of the desired product **16*** as a light brown solid (Yield 71%). $^1$H-NMR (400 MHz, $CDCl_3$): $\delta$ = 7.71 (s, 1 H, Ar), 7.85 (s, 1 H, ArH), 8.30 (s, 1 H, ArH), 8.92 (s, 1 H, ArH).

**Example 17-8 Synthesis of 6-bromo-3-iodo-imidazo[1,2-*a*]pyrazine**

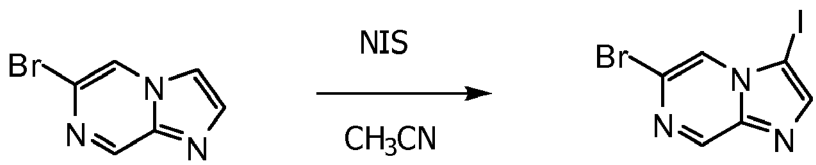


**17***

6-Bromoimidazo[1,2-*a*]pyrazine **16*** (1.98 g, 0.01 M) was added to N-iodosuccinimide (2.46 g, 0.011 M) in acetonitrile (50 ml). The resulting mixture was heated at reflux overnight. The solvent was evaporated *in vacuo* and the residue was treated with sodium carbonate solution and extracted with ethyl acetate (3 x 200 ml). The combined extracts were dried over magnesium sulphate and evaporated *in vacuo* to give a pale brown solid. The solid was washed with

ethyl acetate (10 ml) to give 2.23 g of the desired product. $^1$H-NMR (400 MHz, $CDCl_3$): δ = 7.88 (1 H, s, ArH), 8.25 (1 H, s, ArH), 8.81 (1 H, s, ArH).

**Example 17-9 General Procedure for Suzuki reactions**

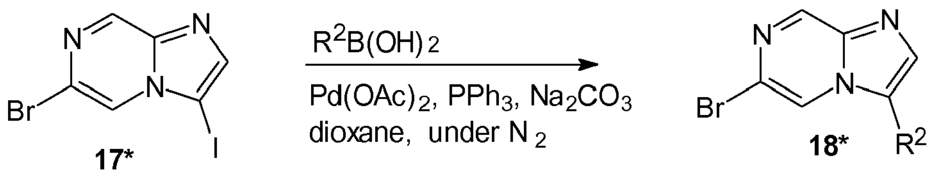


17* 18*

6-bromo-3-iodo-imidazo[1,2-*a*]pyrazine **17*** (0.648 g, 2 mmol) was added to a degassed mixture of dioxane (10 ml) and water (1.5 ml). The aromatic boronic acid ($R^2B(OH)_2$) (2.2 mmol) was added followed by palladium acetate (0.022 g, 5 mol%), triphenyl phosphine (0.025 g, 5 mol%) and sodium carbonate (0.32 g, 3 mmol). The reaction mixture was stirred at 80 °C under nitrogen for 18 hours. The solvent was evaporated *in vacuo* and the residue was partitioned between water (20 ml) and dichloromethane (50 ml). The dichloromethane was separated, dried over magnesium sulphate and evaporated *in vacuo.* The residue was purified by flash chromatography.

Typical examples:

6-Bromo-3-(2-chloro-phenyl)-imidazo[1,2-a]]pyrazine **18*a**

$^1$H-NMR (400 MHz, $CDCl_3$): δ = 8.98 (s, 1 H), 7.96 (S, 1 H), 7.91 (S, 1 H), 7.63 (d, *J* =7.6Hz, 1 H), 7.53-7.46 (m,3H). Yield: 33.7%

6-Bromo-3-pyridin-4-yl-imidazo[1,2- a]pyrazine **18*b**

$^1$H-NMR (400 MHz, $CDCl_3$): δ = 9.01 (d, *J* =4.8Hz, 1 H), 8.84 (dd, *J* =4.8Hz, *J* =1.6Hz, 2H), 8.49 (d, *J* =1.2Hz, 1H), 8.04 (s, 1H), 7.55-7.51 (m, 2H). Yield: 41.1%

6-Bromo-3-thiophen-2-yl-imidazo[1,2- a]pyrazine **18*c**

Note: An additional 1 mol% of Palladium Acetate and triphenylphosphine was added after 12 hours to increase the yield of the reaction.
$^1$H-NMR (400 MHz, $CDCl_3$): δ = 8.94 (s, 1 H), 8.49 (s, 1 H), 7.95 (s, 1 H), 7.55 (d, *J* =5.2Hz, 1 H), 7.38 (d, *J* =3.2Hz, 1 H), 7.27 (s, 1H). Yield: 42.3%.

**Example 17-10 General Procedure for Stille reactions**

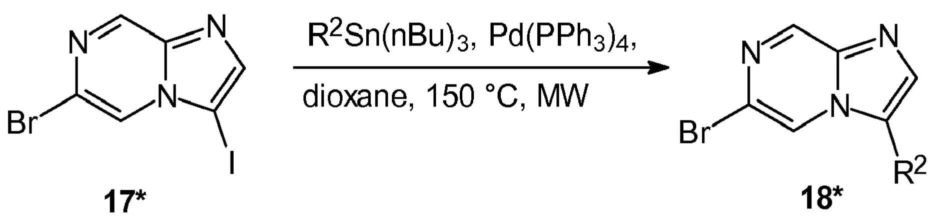


17* 18*

A suspension of tributyl($R^2$)stanne (3.11 mmol), 6-bromo-3-iodo-imidazo[1,2-*a*]pyrazine **17*** (3.11 mmol) and $Pd(PPh_3)_4$ (0.93 mmol) in dioxane (20 mL) was heated to 150 °C in a microwave tube for 20 minutes. The resulting mixture was filtered through celite and evaporated under reduced pressure. If necessary, the reaction was then carried out more times on the same scale. The crude products were combined and purified by flash chromatography to give the intermediate **18*.**

Typical examples:

4-(6-Bromoimidazo[1,2-a]pyrazin-3-yl)thiazole **18*d**

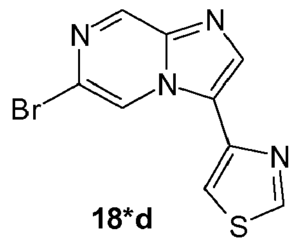

**18*d**

$^1$H-NMR ($CDCl_3$): $\delta$ = 9.51 (1 H, d, ArH), 9.04 (1 H, d, ArH), 8.95 (1 H, d, ArH), 8.16 (1 H, s, ArH), 7.71 (1 H, d, ArH).

**Example 17-11 <u>Library Production of compound (IV) by Suzuki reactions</u>**

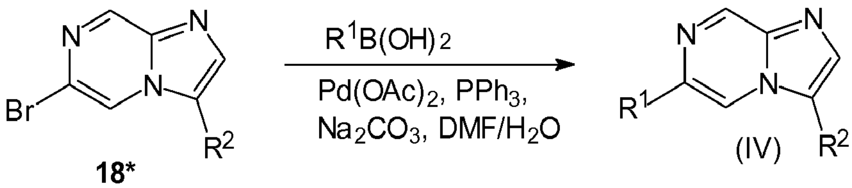


**18*** (IV)

Note: All solvents de-gassed prior to use.

The 6-bromo-3-$R^2$-2-yl-imidazo[1,2-*a*]]pyrazine **18*** (0.3 mmol) produced by synthetic procedure in example 17-9 or 17-10 was dissolved (or suspended if not soluble) in dimethylformamide (0.5 ml) in a 16 mm reaction tube and the boronic acid (0.33 mmol) in dimethylformamide (0.25 ml) was added followed by sodium carbonate (0.45 mmol) in water (0.25 ml). Palladium acetate (3 mg) in dimethylformamide (0.2 ml) and triphenylphosphine (4 mg) in dimethylformamide (0.2 ml) were then added and the tube was flushed with nitrogen and capped. The reaction was then shaken at 80 °C for 16 hours. After cooling the reaction was filtered through Celite and the Celite was washed with a further 0.5 ml of dimethylformamide. The filtrate was then purified by preparative HPLC.

**Example 17-12 General Synthetic procedure of intermediate 19* by Suzuki reactions**

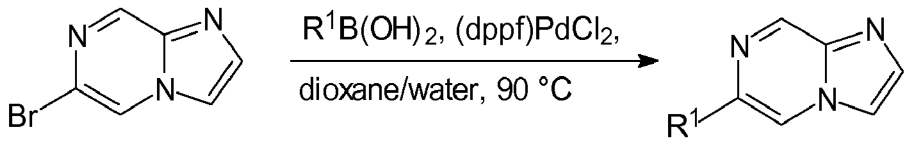


**16*** **19***

A suspension of 6-bromoimidazo[1,2-*a*]pyrazine **16*** (13 mmol), a boronic acid (14 mmol) and potassium carbonate (42 mmol) in dioxane (40 mL) plus water (5 mL) was stirred under nitrogen for 10 minutes. (dppf)$PdCl_2$ (1.3 mmol) was added and the resulting suspension was stirred at 90 °C for 2.5 hours. The reaction mixture was evaporated under reduced pressure and the residue was partitioned between dichloromethane and water, the organic phase was separated and evaporated under reduced pressure. The crude product was purified by flash chromatography to give the intermediate **19*.**

Typical examples:

6-(2-thienyl)imidazo[1,2-*a*]pyrazine **19*a**

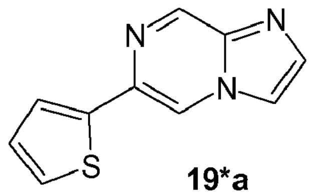

**19*a**

$^1$H-NMR ($CDCl_3$): δ = 9.11 (1 H, d, ArH), 8.42 (1 H, d, ArH), 7.89-7.76 (1 H, m, ArH), 7.74-7.68 (1 H, m, ArH), 7.52 (1 H, dd, ArH), 7.39 (1 H, dd, ArH), 7.13 (1 H, dd, ArH).

**Example 17-13 Iodation of intermediate 19***

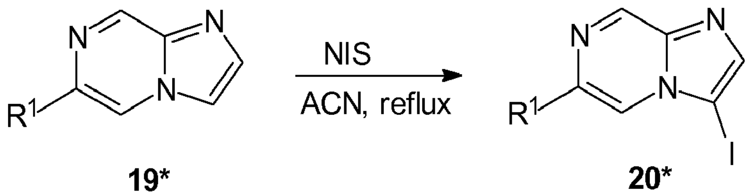


**19*** **20***

The intermediate **19*** (1 mmol) was stirred under reflux with NIS (1.1 mmol) in acetonitrile (40 mL) for 20 hours, more NIS (0.2 mmol) was added and reflux was continued for 8 hours. The reaction mixture was evaporated under reduced pressure and the residue was purified by flash chromatogrphay to give 3-iodo-6-R$^1$-imidazo[1,2-*a*]pyrazine **20*.**

Typical examples:

3-iodo-6-(2-thienyl)imidazo[1,2-*a*]pyrazine **20*a**

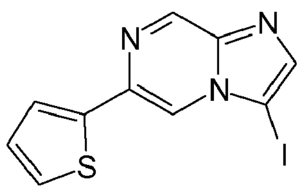

**20*a**

$^1$H-NMR ($CDCl_3$): δ = 9.02 (1 H, dd, ArH), 8.72 (1 H, d, ArH), 7.99-7.90 (2 H, m, ArH), 7.66 (1 H, dd, ArH), 7.20 (1 H, dd, ArH).

**Example 17-14** **<u>Library Production of compound (IV) by Suzuki reactions</u>**

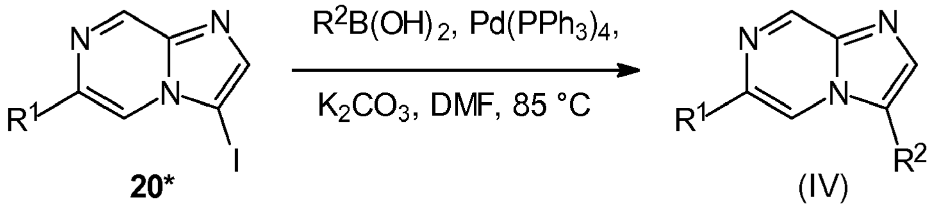


**20*** (IV)

A suspension of 3-iodo-6-$R^1$-imidazo[1,2-*a*]pyrazine **20***(0.2 mmol), a boronic acid (0.25 mmol) and potassium carbonate (0.5 mmol) in DMF (1 mL) plus water (0.2 mL) in a 16 mm reaction tube ws flushed with nitrogen. To this suspension $Pd(PPh_3)_4$ (0.01 mmol) was added and the sealed tube was warmed to 85 °C for 20 hours. The mixture was filtered and purified by HPLC to give the product (IV).

Typical example: compound **450.**

**Example 18-1 compound 1**

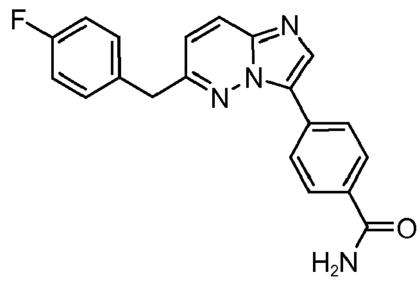

Mw.: 346.12, LCMS-ESI (*m/z*) - found 347.12 $[M+H]^+$, 345.12 $[M-H]^-$

**Example 18-2 compound 2**

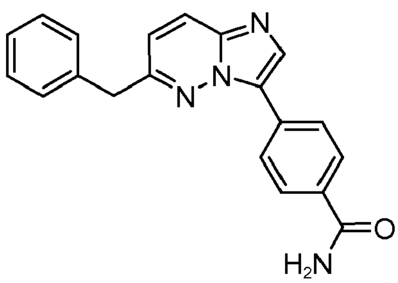

Mw.: 328.13, LCMS-ESI (*m/z*) - found 329.13 $[M+H]^+$, 327.13 $[M-H]^-$.

**Example 18-3 compound 3**

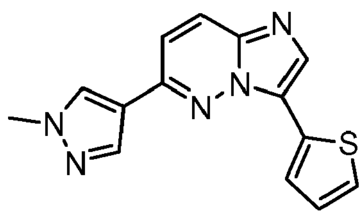

Mw.: 281.07, LCMS-ESI (*m/z*) - found 282.07 $[M+H]^+$, 280.07 $[M-H]^-$.

**Example 18-4 compound 4**

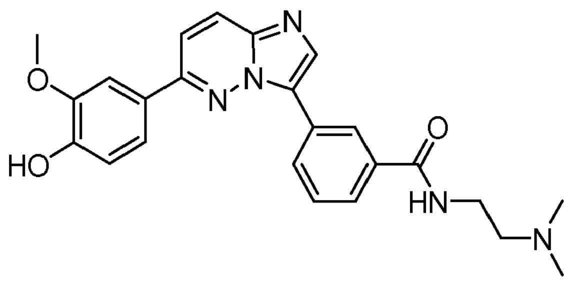

Mw.: 431.20, LCMS-ESI (*m/z*) - found 432.19 $[M+H]^+$, 430.19 $[M-H]^-$.

**Example 18-5 compound 5**

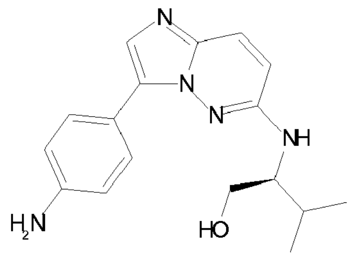

Mw.: 311.17, LCMS-ESI (*m/z*) - found 312.17 $[M+H]^+$, 310.17 $[M-H]^-$.

**Example 18-6 compound 6**

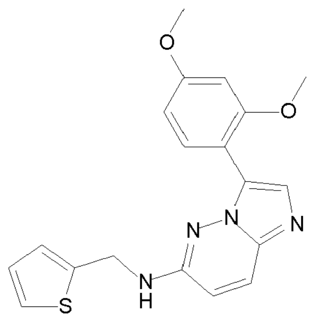

Mw.: 366.12, LCMS-ESI (*m/z*) - found 367.11 $[M+H]^+$, 365.11 $[M-H]^-$.

**Example 18-7 compound 7**

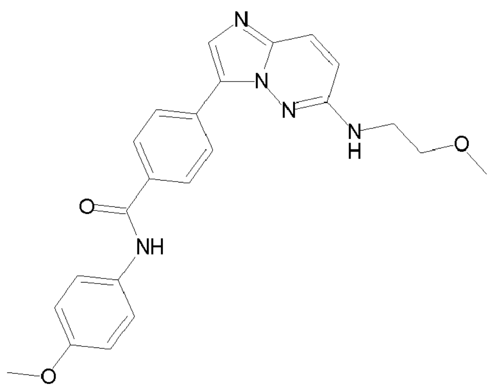

Mw.: 417.18, LCMS-ESI (*m*/*z*) - found 418.18 [M+H]$^+$, 416.18 [M-H]$^-$.

**Example 18-8 compound 8**

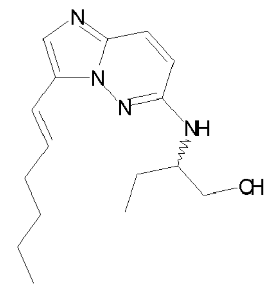

Mw.: 288.19, LCMS-ESI (*m*/*z*) - found 289.19 [M+H]$^+$, 287.19 [M-H]$^-$.

**Example 18-9 compound 9**

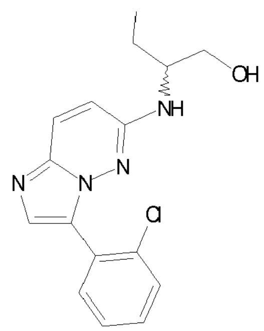

Mw.: 316.11, LCMS-ESI (*m*/*z*) - found 317.10 [M+H]$^+$, 315.10 [M-H]$^-$.

**Example 18-10 compound 10**

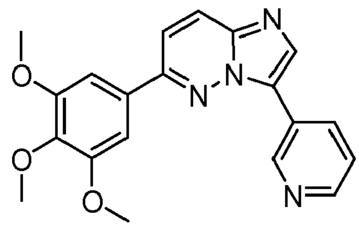

Mw.: 362.14, LCMS-ESI (*m*/*z*) - found 363.13 $[M+H]^+$, 361.13 $[M-H]^-$.

**Example 18-11 compound 11**

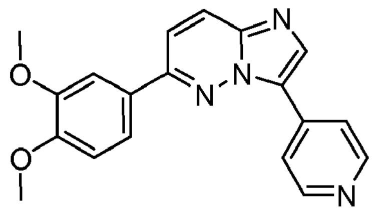

Mw.: 332.13, LCMS-ESI (*m*/*z*) - found 333.12 $[M+H]^+$, 331.12 $[M-H]^-$.

**Example 18-12 compound 12**

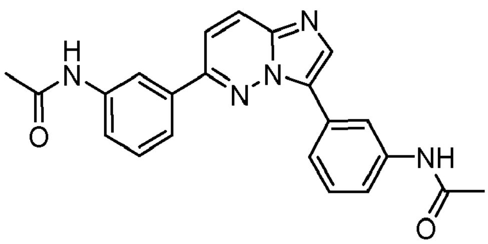

Mw.: 385.15, LCMS-ESI (*m*/*z*) - found 386.15 $[M+H]^+$, 384.15 $[M-H]^-$.

**Example 18-13 compound 13**

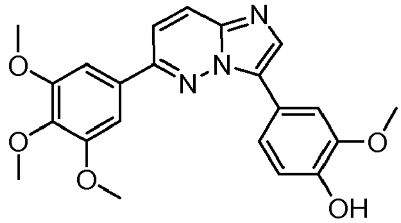

Mw.: 407.14, LCMS-ESI (*m*/*z*) - found 408.14 $[M+H]^+$, 406.14 $[M-H]^-$.

**Example 18-14 compound 14**

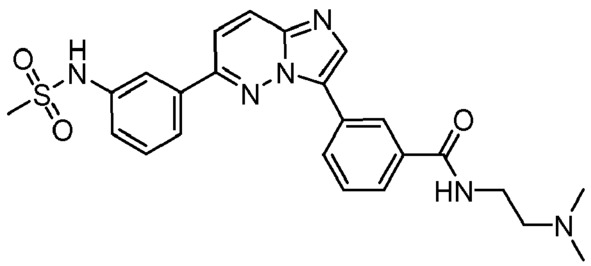

Mw.: 478.18, LCMS-ESI (*m/z*) - found 479.17 $[M+H]^+$, 477.17 $[M-H]^-$.

**Example 18-15 compound 15**

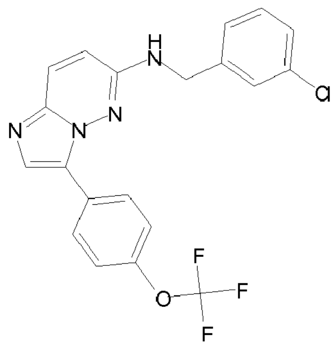

Mw.: 418.08, LCMS-ESI (*m/z*) - found 419.08 $[M+H]^+$, 417.08 $[M-H]^-$.

**Example 18-16 compound 16**

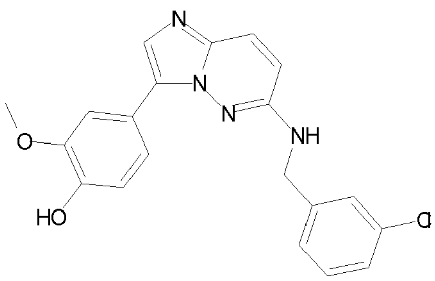

Mw.: 380.10, LCMS-ESI (*m/z*) - found 381.10 $[M+H]^+$, 379.10 $[M-H]^-$.

**Example 18-17 compound 17**

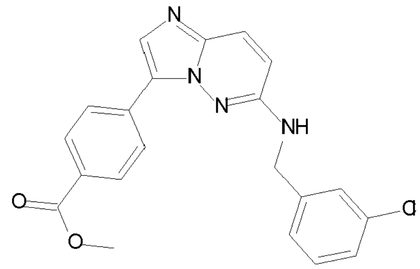

Mw.: 392.10, LCMS-ESI (*m/z*) - found 393.10 $[M+H]^+$, 391.10 $[M-H]^-$.

**Example 18-18 compound 18**

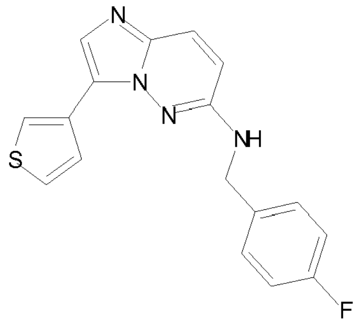

Mw.: 324.08, LCMS-ESI (*m/z*) - found 325.08 $[M+H]^+$, 323.08 $[M-H]^-$.

**Example 18-19 compound 19**

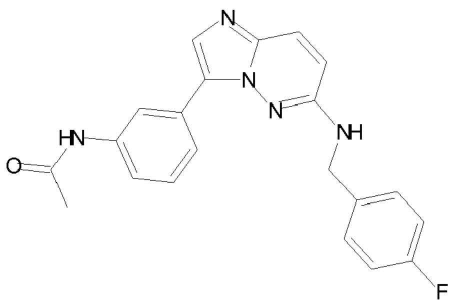

Mw.: 375.15, LCMS-ESI (*m/z*) - found 376.15 $[M+H]^+$, 374.14 $[M-H]^-$.

**Example 18-20 compound 20**

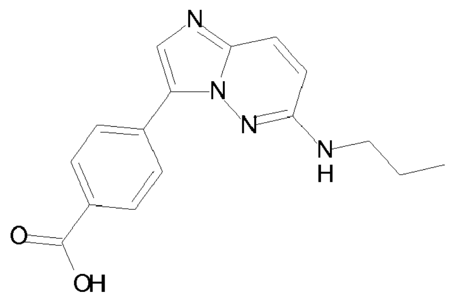

Mw.: 296.13, LCMS-ESI (*m*/*z*) - found 297.12 $[M+H]^+$, 295.12 $[M-H]^-$.

**Example 18-21 compound 21**

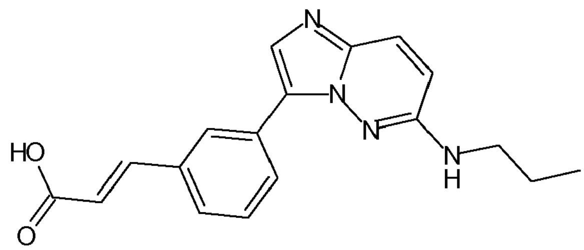

Mw.: 322.14, LCMS-ESI (*m*/*z*) - found 323.14 $[M+H]^+$, 321.14 $[M-H]^-$.

**Example 18-22 compound 22**

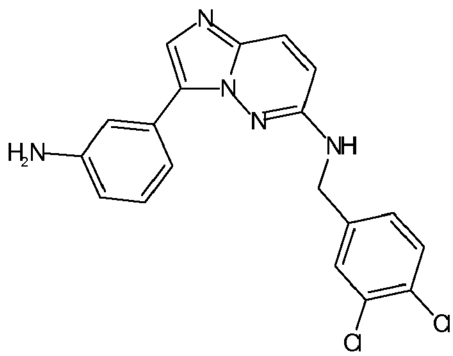

Mw.: 383.07, LCMS-ESI (*m*/*z*) - found 384.07 $[M+H]^+$, 382.07 $[M-H]^-$.

**Example 18-23 compound 23**

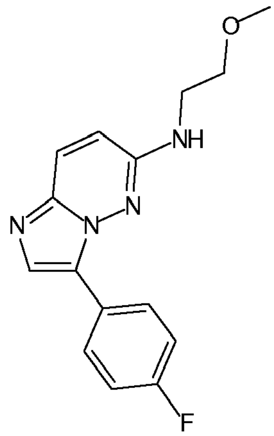

Mw.: 286.12, LCMS-ESI (*m/z*) - found 287.12 $[M+H]^+$, 285.12 $[M-H]^-$.

**Example 18-24 compound 24**

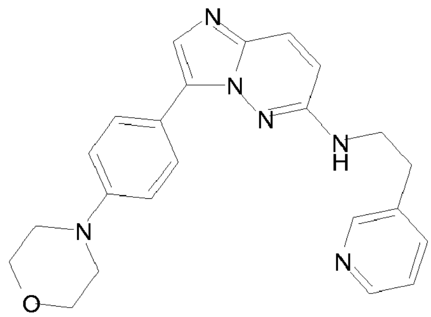

Mw.: 400.20, LCMS-ESI (*m/z*) - found 401.20 $[M+H]^+$, 399.20 $[M-H]^-$.

**Example 18-25 compound 25**

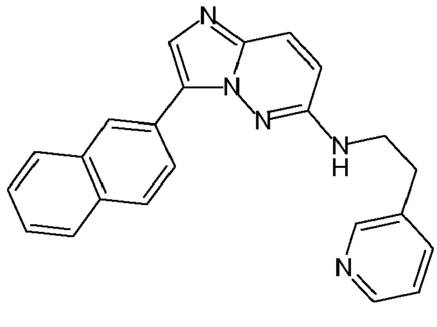

Mw.: 365.16, LCMS-ESI (*m/z*) - found 366.16 $[M+H]^+$, 364.16 $[M-H]^-$.

**Example 18-26 compound 26**

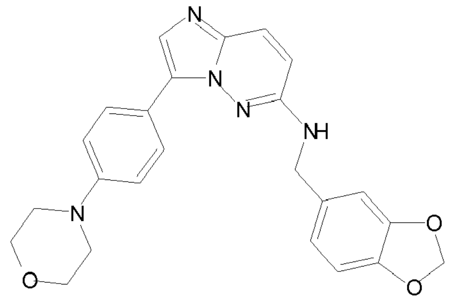

Mw.: 429.18, LCMS-ESI (*m*/*z*) - found 430.18 $[M+H]^+$, 428.18 $[M-H]^-$.

**Example 18-27 compound 27**

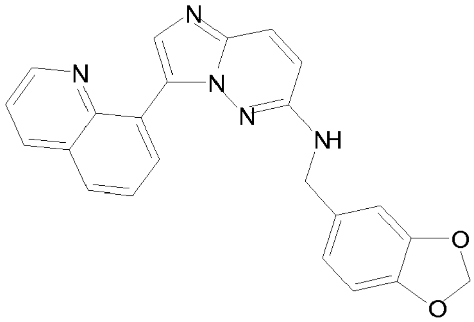

Mw.: 395.14, LCMS-ESI (*m*/*z*) - found 396.13 $[M+H]^+$, 394.13 $[M-H]^-$.

**Example 18-28 compound 28**

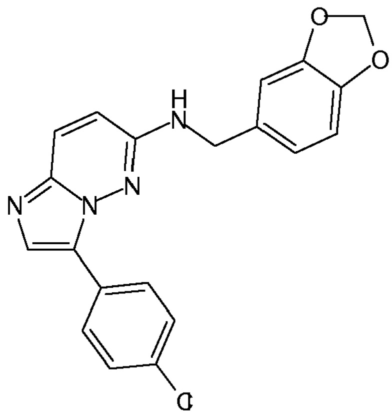

Mw.: 378.09, LCMS-ESI (*m*/*z*) - found 379.08 $[M+H]^+$, 377.08 $[M-H]^-$.

**Example 18-29 compound 29**

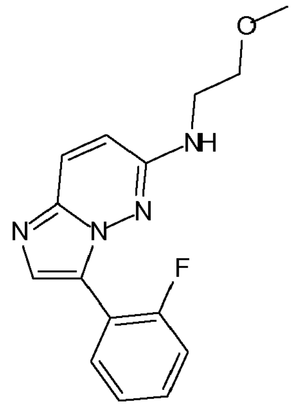

Mw.: 286.12, LCMS-ESI (*m/z*) - found 287.12 $[M+H]^+$, 285.12 $[M-H]^-$.

**Example 18-30 compound 30**

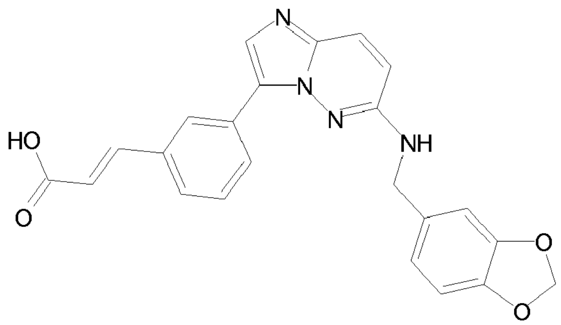

Mw.: 414.13, LCMS-ESI (*m/z*) - found 415.13 $[M+H]^+$, 413.13 $[M-H]^-$.

**Example 18-31 compound 31**

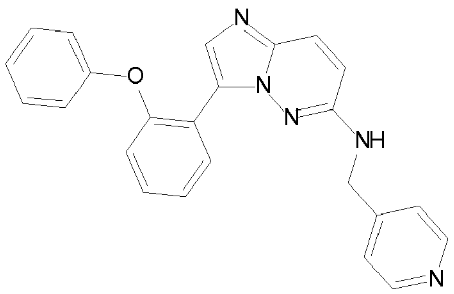

Mw.: 393.16, LCMS-ESI (*m/z*) - found 394.15 $[M+H]^+$, 392.15 $[M-H]^-$.

**Example 18-32 compound 32**

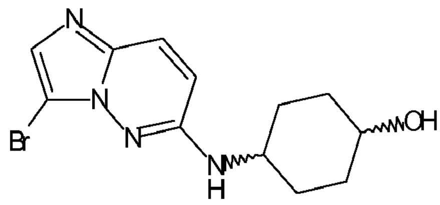

Mw.: 310.04, LCMS-ESI (*m/z*) - found 311.04 $[M+H]^+$, 309.04 $[M-H]^-$.

**Example 18-33 compound 33**

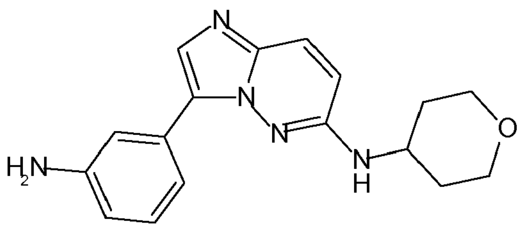

Mw.: 309.16, LCMS-ESI (*m/z*) - found 310.15 $[M+H]^+$, 308.15 $[M-H]^-$.

**Example 18-34 compound 34**

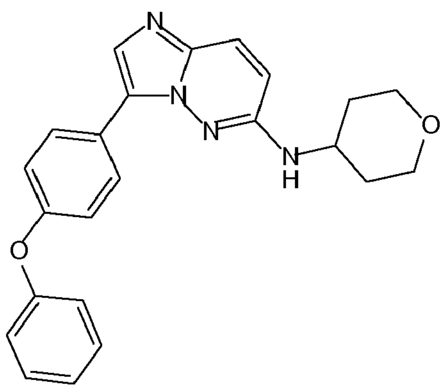

Mw.: 386.17, LCMS-ESI (*m/z*) - found 387.17 $[M+H]^+$, 385.17 $[M-H]^-$.

**Example 18-35 compound 35**

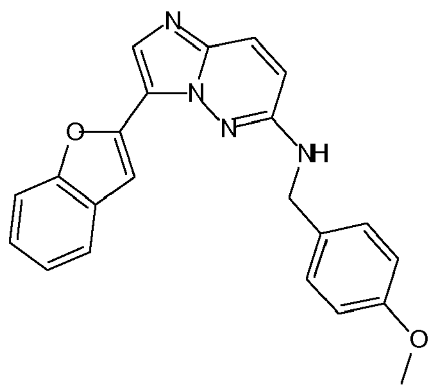

Mw.: 370.14, LCMS-ESI (*m*/*z*) - found 371.14 $[M+H]^+$, 369.14 $[M-H]^-$.

**Example 18-36 compound 36**

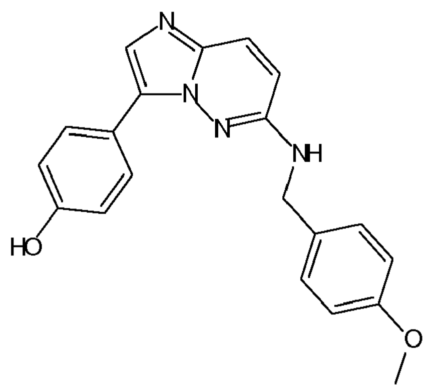

Mw.: 346.14, LCMS-ESI (*m*/*z*) - found 347.14 $[M+H]^+$, 345.14 $[M-H]^-$.

**Example 18-37 compound 37**

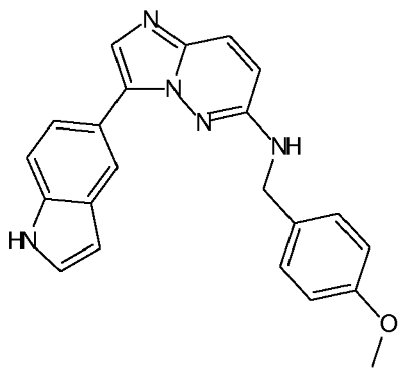

Mw.: 369.16, LCMS-ESI (*m*/*z*) - found 370.15 $[M+H]^+$, 368.15 $[M-H]^-$.

**Example 18-38 compound 38**

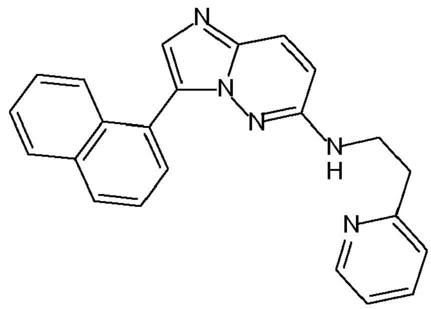

Mw.: 365.16, LCMS-ESI (*m/z*) - found 367.16 $[M+H]^+$, 364.16 $[M-H]^-$.

**Example 18-39 compound 39**

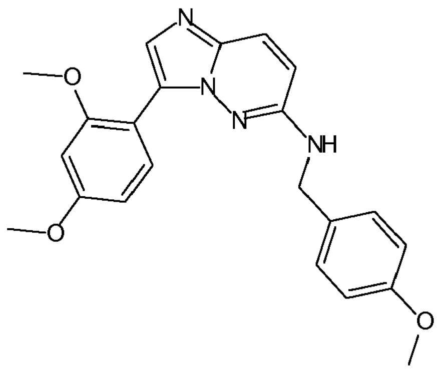

Mw.: 390.17, LCMS-ESI (*m/z*) - found 391.16 $[M+H]^+$, 389.16 $[M-H]^-$.

**Example 18-40 compound 40**

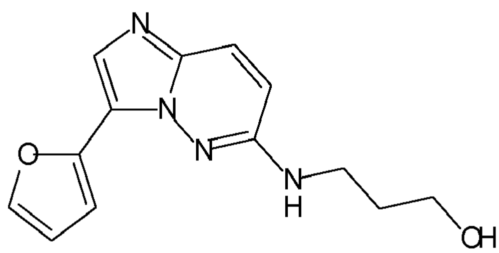

Mw.: 258.11, LCMS-ESI (*m/z*) - found 259.11 $[M+H]^+$, 257.11 $[M-H]^-$.

**Example 18-41 compound 41**

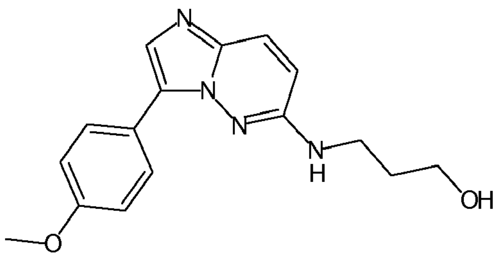

Mw.: 298.14, LCMS-ESI (*m/z*) - found 299.14 $[M+H]^+$, 297.14 $[M-H]^-$.

**Example 18-42 compound 42**

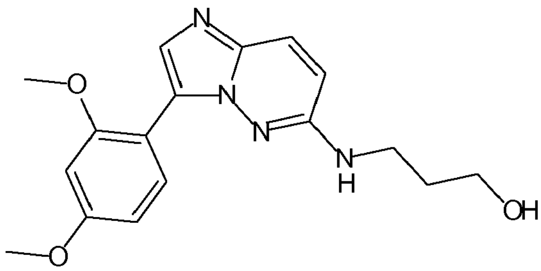

Mw.: 328.15, LCMS-ESI (*m/z*) - found 329.15 $[M+H]^+$, 327.15 $[M-H]^-$.

**Example 18-43 compound 43**

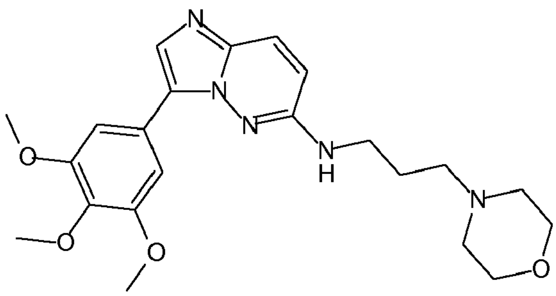

Mw.: 427.22, LCMS-ESI (*m/z*) - found 428.22 $[M+H]^+$, 426.22 $[M-H]^-$.

**Example 18-44 compound 44**

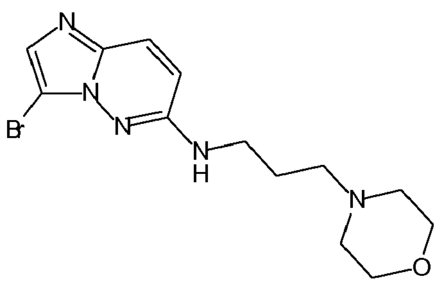

Mw.: 339.07, LCMS-ESI (*m*/*z*) - found 340.06 [M+H]+, 338.06 [M-H]-.

**Example 18-45 compound 45**

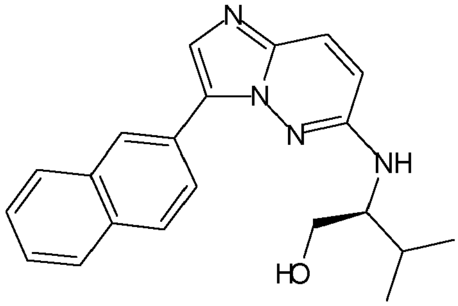

Mw.: 346.18, LCMS-ESI (*m*/*z*) - found 347.17 [M+H]+, 345.17 [M-H]-.

**Example 18-46 compound 46**

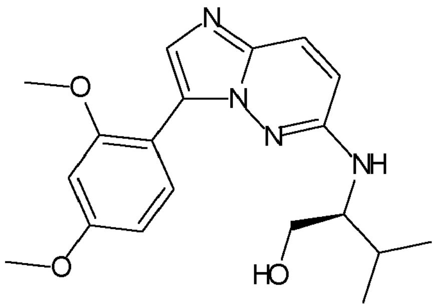

Mw.: 356.18, LCMS-ESI (*m*/*z*) - found 357.18 [M+H]+, 355.18 [M-H]-.

**Example 18-47 compound 47**

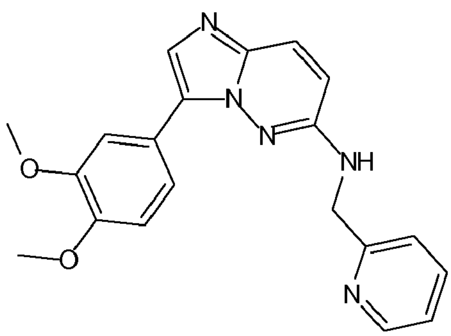

Mw.: 361.15, LCMS-ESI (*m*/*z*) - found 362.15 [M+H]+, 360.15 [M-H]-.

**Example 18-48 compound 48**

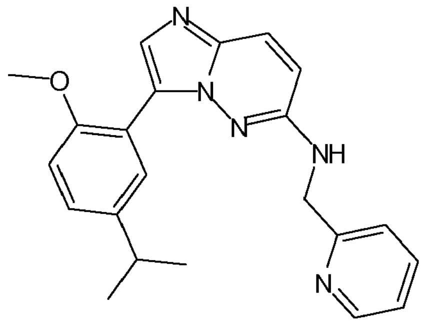

Mw.: 373.19, LCMS-ESI (*m/z*) - found 374.19 $[M+H]^+$, 372.19 $[M-H]^-$.

**Example 18-49 compound 49**

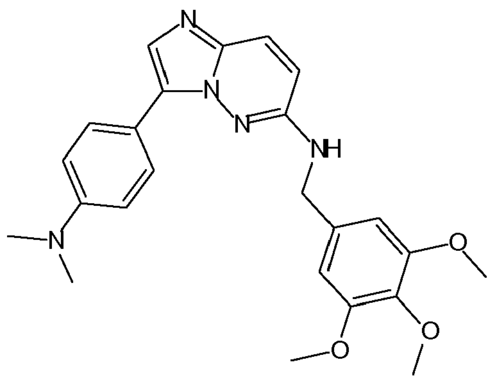

Mw.: 433.21, LCMS-ESI (*m/z*) - found 434.21 $[M+H]^+$, 432.21 $[M-H]^-$.

**Example 18-50 compound 50**

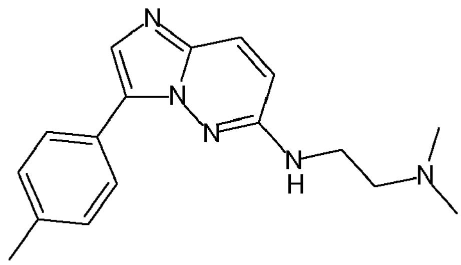

Mw.: 295.18, LCMS-ESI (*m/z*) - found 296.18 $[M+H]^+$, 294.18 $[M-H]^-$.

**Example 18-51 compound 51**

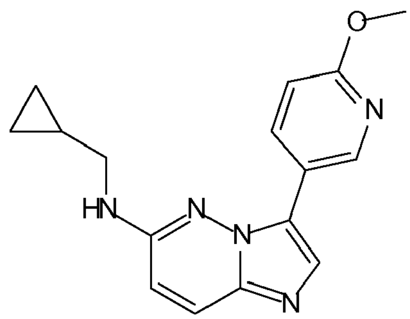

Mw.: 295.14, LCMS-ESI (*m/z*) - found 296.14 $[M+H]^+$, 294.14 $[M-H]^-$.

**Example 18-52 compound 52**

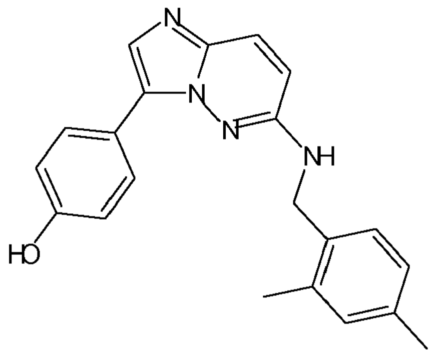

Mw.: 344.16, LCMS-ESI (*m/z*) - found 345.16 $[M+H]^+$, 343.16 $[M-H]^-$.

**Example 18-53 compound 53**

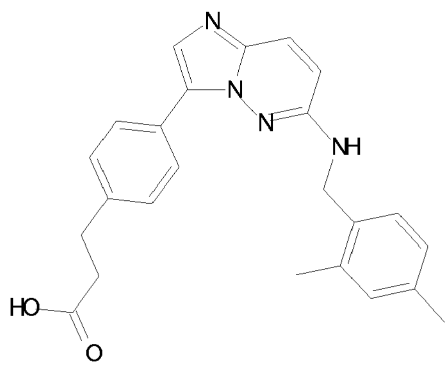

Mw.: 400.19, LCMS-ESI (*m/z*) - found 401.19 $[M+H]^+$, 39.19 $[M-H]^-$.

**Example 18-54 compound 54**

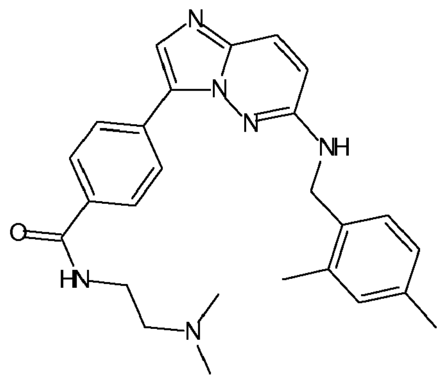

Mw.: 442.25, LCMS-ESI (*m*/*z*) - found 443.24 $[M+H]^+$, 441.24 $[M-H]^-$.

**Example 18-55 compound 55**

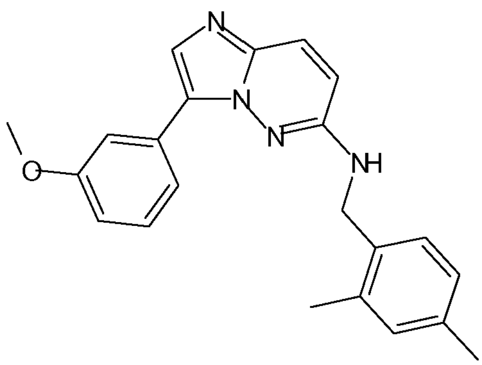

Mw.: 358.18, LCMS-ESI (*m*/*z*) - found 359.17 $[M+H]^+$, 357.17 $[M-H]^-$.

**Example 18-56 compound 56**

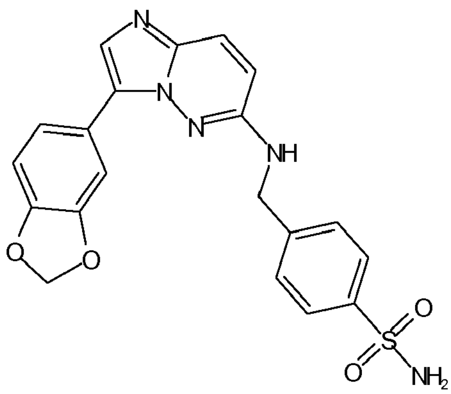

Mw.: 423.10, LCMS-ESI (*m*/*z*) - found 424.10 $[M+H]^+$, 422.10 $[M-H]^-$.

**Example 18-57 compound 57**

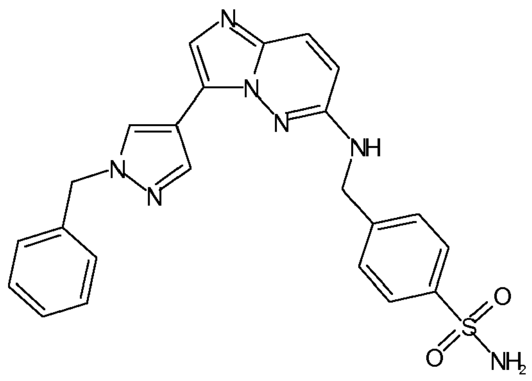

Mw.: 459.15, LCMS-ESI (*m/z*) - found 460.14 $[M+H]^+$, 458.14 $[M-H]^-$.

**Example 18-58 compound 58**

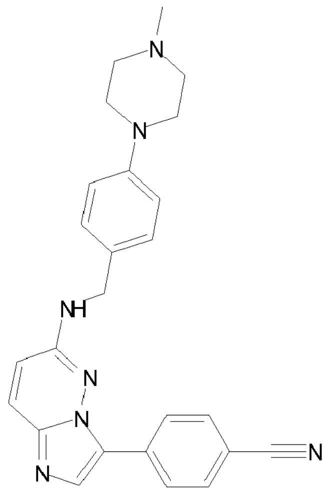

Mw.: 423.22, LCMS-ESI (*m/z*) - found 424.21 $[M+H]^+$, 422.21 $[M-H]^-$.

**Example 18-59 compound 59**

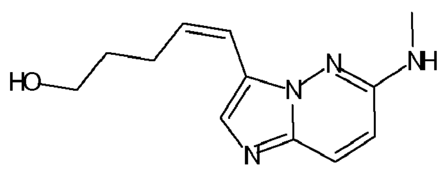

Mw.: 232.13, LCMS-ESI (*m/z*) - found 233.13 $[M+H]^+$, 231.13 $[M-H]^-$.

**Example 18-60 compound 60**

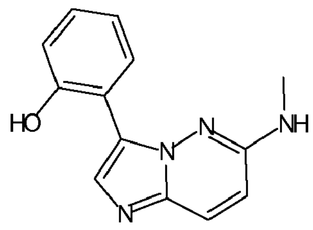

Mw.: 240.10, LCMS-ESI (*m*/*z*) - found 241.10 $[M+H]^+$, 239.10 $[M-H]^-$.

**Example 18-61 compound 61**

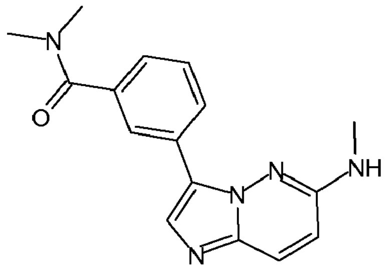

Mw.: 295.14, LCMS-ESI (*m*/*z*) - found 296.14 $[M+H]^+$, 294.14 $[M-H]^-$.

**Example 18-62 compound 62**

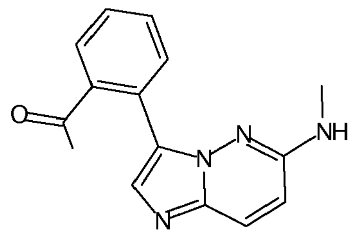

Mw.: 266.12, LCMS-ESI (*m*/*z*) - found 297.11 $[M+H]^+$, 265.11 $[M-H]^-$.

**Example 18-63 compound 63**

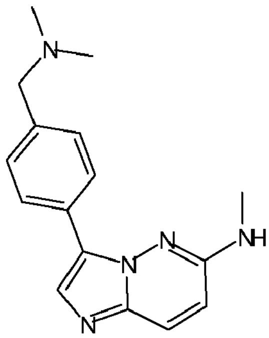

Mw.: 281.16, LCMS-ESI (*m*/*z*) - found 282.16 $[M+H]^+$, 280.16 $[M-H]^-$.

**Example 18-64 compound 64**

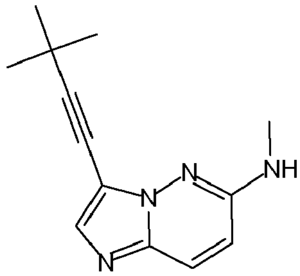

Mw.: 228.14, LCMS-ESI (*m*/*z*) - found 229.13 $[M+H]^+$, 227.13 $[M-H]^-$.

**Example 18-65 compound 65**

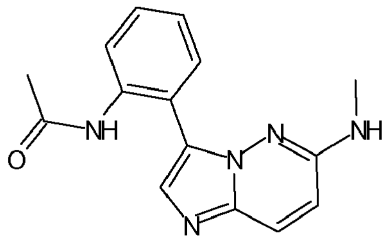

Mw.: 281.13, LCMS-ESI (*m*/*z*) - found 282.12 $[M+H]^+$, 280.12 $[M-H]^-$.

**Example 18-66 compound 66**

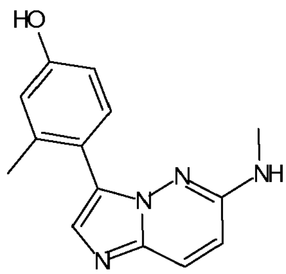

Mw.: 254.12, LCMS-ESI (*m*/*z*) - found 255.11 $[M+H]^+$, 253.11 $[M-H]^-$.

**Example 18-67 compound 67**

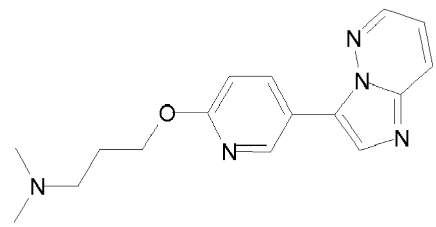

Mw.: 297.16, LCMS-ESI (*m*/*z*) - found 298.16 $[M+H]^+$.

**Example 18-68 compound 68**

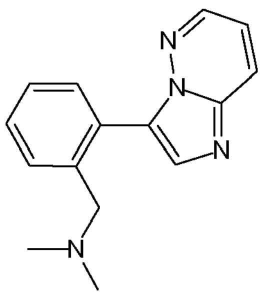

Mw.: 252.14, LCMS-ESI (*m*/*z*) - found 253.14 $[M+H]^+$.

**Example 18-69 compound 69**

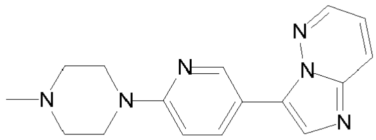

Mw.: 294.16, LCMS-ESI (*m*/*z*) - found 295.16 $[M+H]^+$.

**Example 18-70 compound 70**

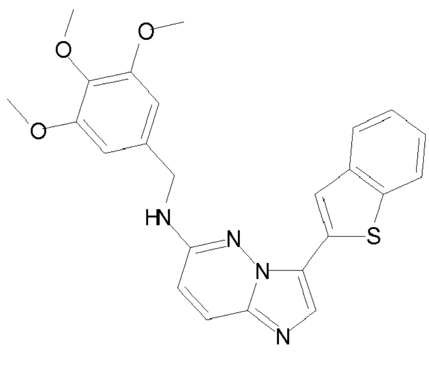

Mw.: 446.14, LCMS-ESI (*m*/*z*) - found 447.14 $[M+H]^+$, 445.14 $[M-H]^-$.

**Example 18-71 compound 71**

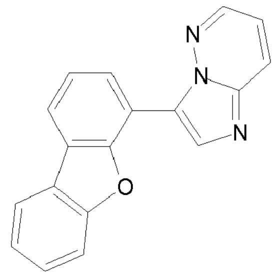

Mw.: 285.09, LCMS-ESI (*m*/*z*) - found 286.09 $[M+H]^+$.

**Example 18-72 compound 72**

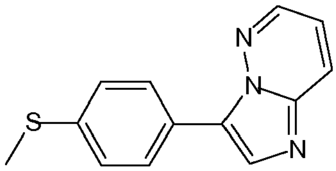

Mw.: 241.07, LCMS-ESI (*m*/*z*) - found 242.07 $[M+H]^+$.

**Example 18-73 compound 73**

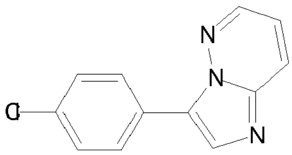

Mw.: 229.04, LCMS-ESI (*m*/*z*) - found 230.04 $[M+H]^+$.

**Example 18-74 compound 74**

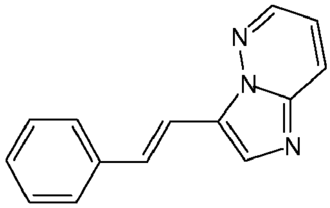

Mw.: 221.10, LCMS-ESI (*m*/*z*) - found 222.10 $[M+H]^+$.

**Example 18-75 compound 75**

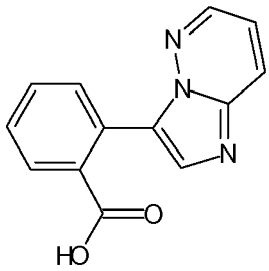

Mw.: 239.07, LCMS-ESI (*m/z*) - found 240.07 $[M+H]^+$, 238.07 $[M-H]^-$.

**Example 18-76 compound 76**

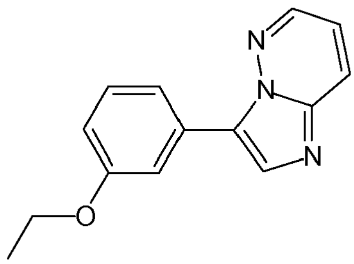

Mw.: 239.11, LCMS-ESI (*m/z*) - found 240.11 $[M+H]^+$.

**Example 18-77 compound 77**

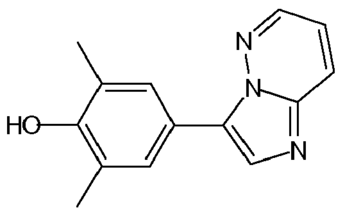

Mw.: 239.11, LCMS-ESI (*m/z*) - found 240.11 $[M+H]^+$, 238.11 $[M-H]^-$.

**Example 18-78 compound 78**

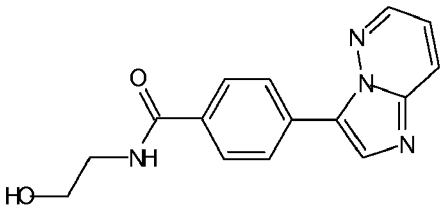

Mw.: 282.11, LCMS-ESI (*m/z*) - found 283.11 $[M+H]^+$, 281.11 $[M-H]^-$.

**Example 18-79 compound 79**

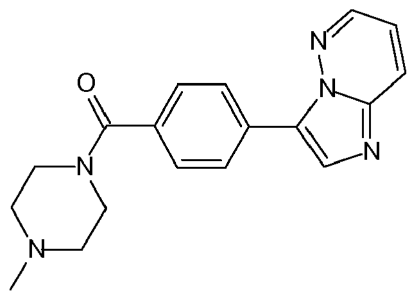

Mw.: 321.16, LCMS-ESI (*m*/*z*) - found 322.16 $[M+H]^+$.

**Example 18-80 compound 80**

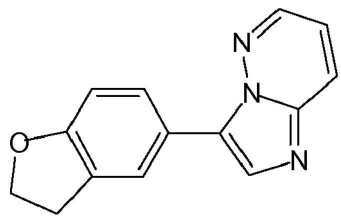

Mw.: 237.09, LCMS-ESI (*m*/*z*) - found 238.09 $[M+H]^+$.

**Example 18-81 compound 81**

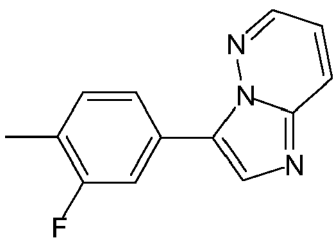

Mw.: 227.09, LCMS-ESI (*m*/*z*) - found 228.09 $[M+H]^+$.

**Example 18-82 compound 82**

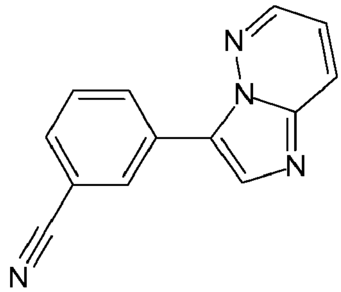

Mw.: 220.07, LCMS-ESI (*m*/*z*) - found 221.08 $[M+H]^+$.

**Example 18-83 compound 83**

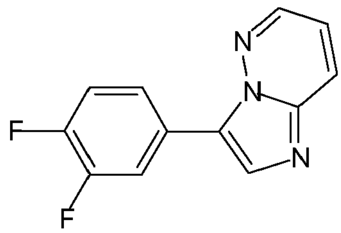

Mw.: 231.06, LCMS-ESI (*m*/*z*) - found 232.06 $[M+H]^+$.

**Example 18-84 compound 84**

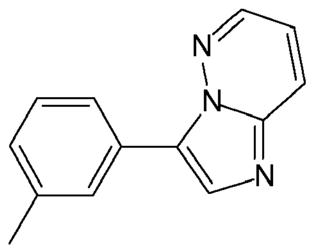

Mw.: 209.10, LCMS-ESI (*m*/*z*) - found 210.10 $[M+H]^+$.

**Example 18-85 compound 85**

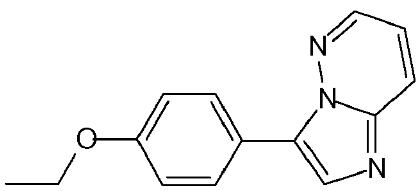

Mw.: 239.11, LCMS-ESI (*m*/*z*) - found 240.11 $[M+H]^+$.

**Example 18-86 compound 86**

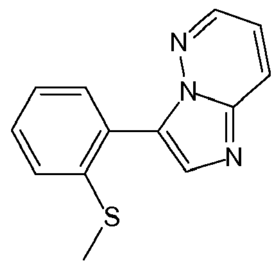

Mw.: 241.07, LCMS-ESI (*m*/*z*) - found 242.07 $[M+H]^+$.

**Example 18-87 compound 87**

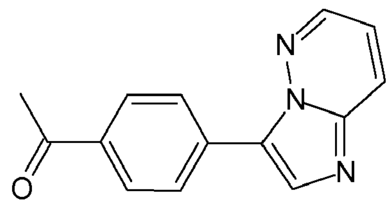

Mw.: 237.09, LCMS-ESI (*m*/*z*) - found 238.09 $[M+H]^+$.

**Example 18-88 compound 88**

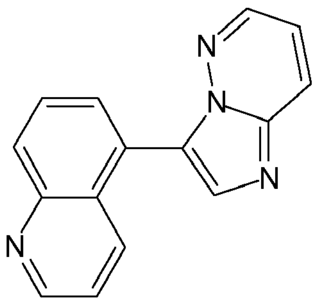

Mw.: 246.09, LCMS-ESI (*m*/*z*) - found 247.09 $[M+H]^+$.

**Example 18-89 compound 89**

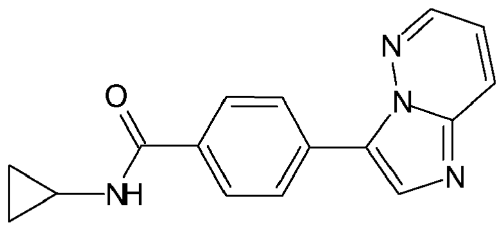

Mw.: 278.12, LCMS-ESI (*m/z*) - found 279.12 $[M+H]^+$, 277.12 $[M-H]^-$.

**Example 18-90 compound 90**

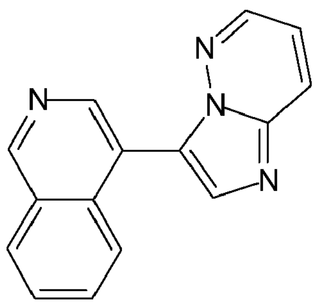

Mw.: 246.09, LCMS-ESI (*m/z*) - found 247.09 $[M+H]^+$.

**Example 18-91 compound 91**

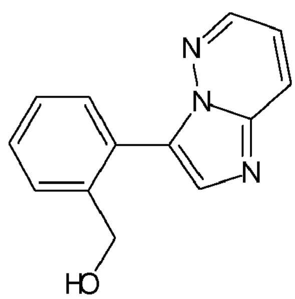

Mw.: 225.09, LCMS-ESI (*m/z*) - found 226.09 $[M+H]^+$.

**Example 18-92 compound 92**

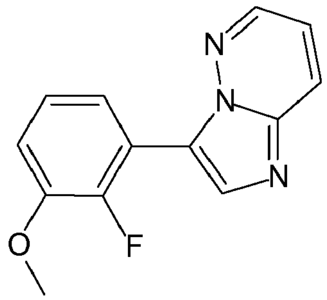

Mw.: 243.08, LCMS-ESI (*m/z*) - found 244.08 $[M+H]^+$.

**Example 18-93 compound 93**

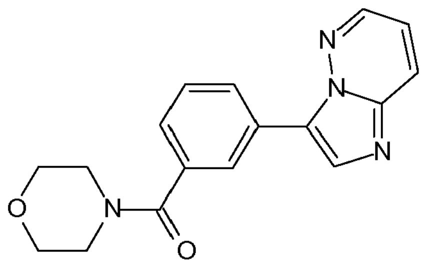

Mw.: 308.13, LCMS-ESI (*m/z*) - found 309.13 $[M+H]^+$.

**Example 18-94 compound 94**

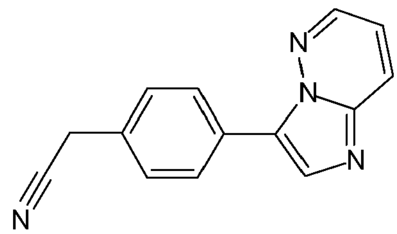

Mw.: 234.09, LCMS-ESI (*m/z*) - found 235.09 $[M+H]^+$.

**Example 18-95 compound 95**

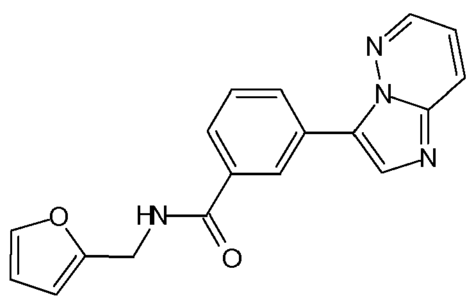

Mw.: 318.11, LCMS-ESI (m/z) - found 319.11 $[M+H]^+$, 317.11 $[M-H]^-$.

**Example 18-96 compound 96**

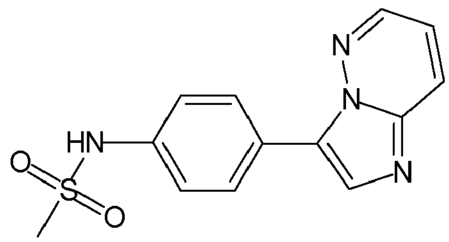

Mw.: 288.07, LCMS-ESI (*m/z*) - found 289.07 $[M+H]^+$, 287.07 $[M-H]^-$.

**Example 18-97 compound 97**

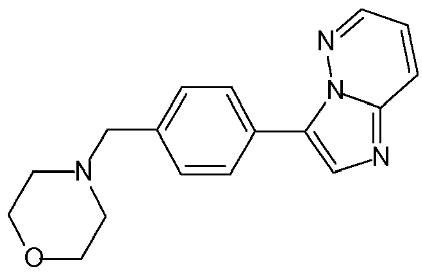

Mw.: 294.15, LCMS-ESI (*m/z*) - found 295.15 $[M+H]^+$.

**Example 18-98 compound 98**

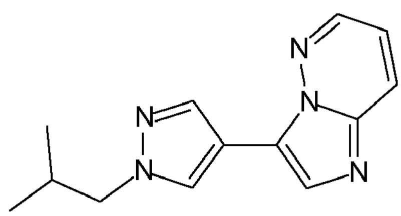

Mw.: 241.13, LCMS-ESI (*m/z*) - found 242.13 $[M+H]^+$.

**Example 18-99 compound 99**

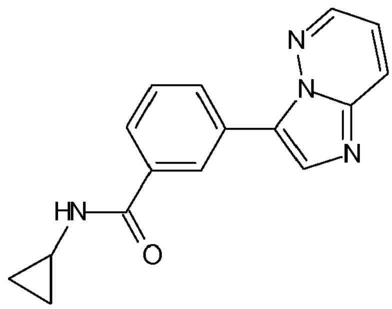

Mw.: 278.12, LCMS-ESI (*m/z*) - found 279.12 $[M+H]^+$, 277.12 $[M-H]^-$.

**Example 18-100 compound 100**

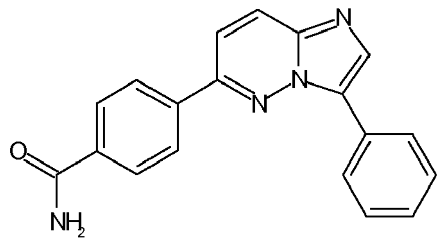

Mw.: 314.12, LCMS-ESI (*m/z*) - found 315.11 $[M+H]^+$, 313.11 $[M-H]^-$.

**Example 18-101 compound 101**

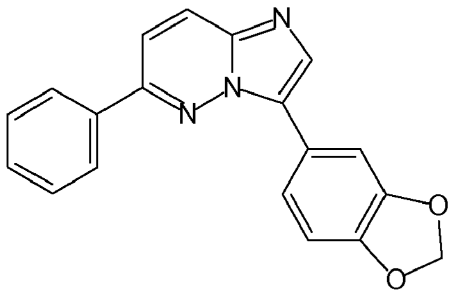

Mw.: 315.10, LCMS-ESI (*m/z*) - found 316.10 $[M+H]^+$, 314.10 $[M-H]^-$.

**Example 18-102 compound 102**

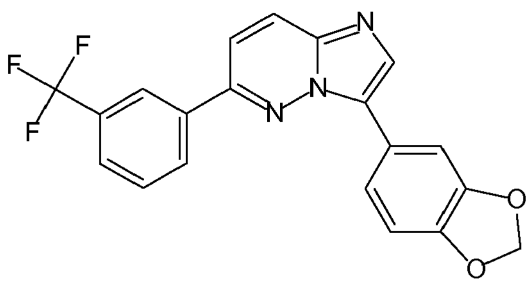

Mw.: 383.09, LCMS-ESI (*m*/*z*) - found 384.08 $[M+H]^+$, 382.08 $[M-H]^-$.

**Example 18-103 compound 103**

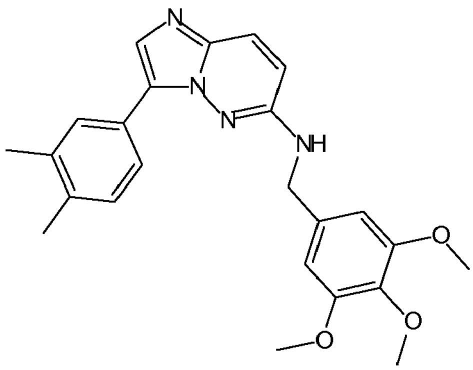

Mw.: 418.20, LCMS-ESI (*m*/*z*) - found 419.20 $[M+H]^+$, 417.20 $[M-H]^-$.

**Example 18-104 compound 104**

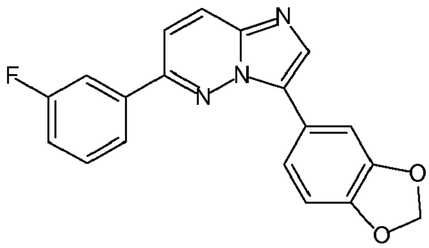

Mw.: 333.09, LCMS-ESI (*m*/*z*) - found 334.09 $[M+H]^+$, 332.09 $[M-H]^-$.

**Example 18-105 compound 105**

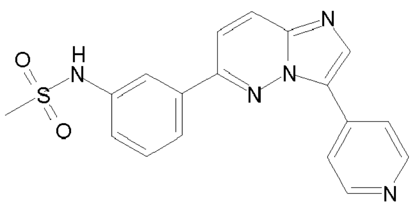

Mw.: 365.09, LCMS-ESI (*m*/*z*) - found 366.09 $[M+H]^+$, 364.09 $[M-H]^-$.

**Example 18-106 compound 106**

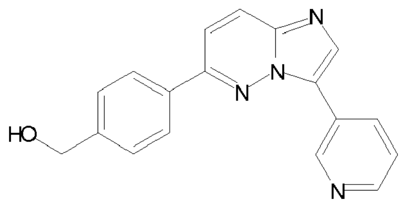

Mw.: 302.12, LCMS-ESI (*m/z*) - found 303.11 $[M+H]^+$, 301.11 $[M-H]^-$.

**Example 18-107 compound 107**

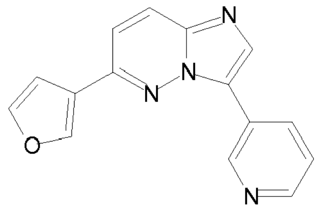

Mw.: 262.09, LCMS-ESI (*m/z*) - found 263.08 $[M+H]^+$, 261.08 $[M-H]^-$.

**Example 18-108 compound 108**

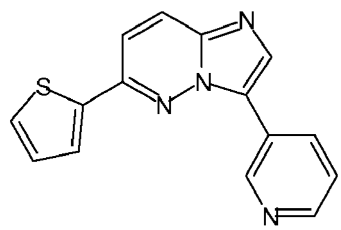

Mw.: 278.06, LCMS-ESI (*m/z*) - found 279.06 $[M+H]^+$, 277.06 $[M-H]^-$.

**Example 18-109 compound 109**

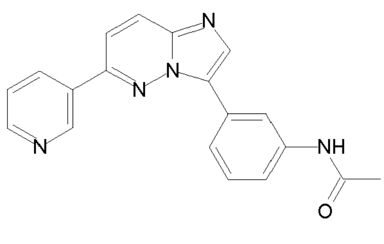

Mw.: 329.13, LCMS-ESI (*m/z*) - found 330.12 $[M+H]^+$, 328.12 $[M-H]^-$.

**Example 18-110 compound 110**

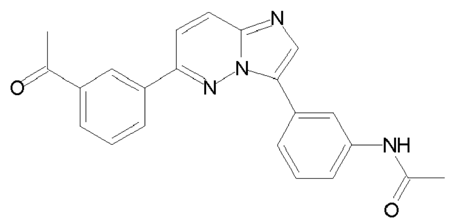

Mw.: 370.14, LCMS-ESI (*m/z*) - found 371.14 $[M+H]^+$, 369.14 $[M-H]^-$.

**Example 18-111 compound 111**

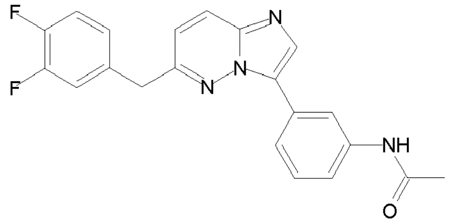

Mw.: 378.13, LCMS-ESI (*m/z*) - found 379.12 $[M+H]^+$, 377.12 $[M-H]^-$.

**Example 18-112 compound 112**

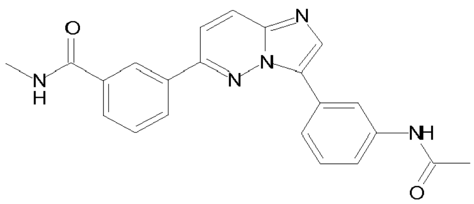

Mw.: 385.15, LCMS-ESI (*m/z*) - found 386.15 $[M+H]^+$, 384.15 $[M-H]^-$.

**Example 18-113 compound 113**

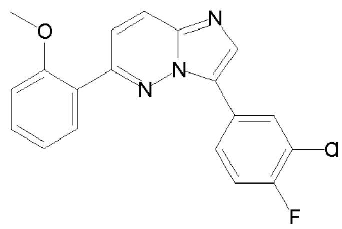

Mw.: 353.07, LCMS-ESI (*m/z*) - found 354.07 $[M+H]^+$, 352.07 $[M-H]^-$.

**Example 18-114 compound 114**

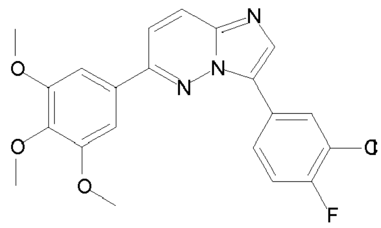

Mw.: 413.09, LCMS-ESI (*m*/*z*) - found 414.09 [M+H]$^+$.

**Example 18-115 compound 115**

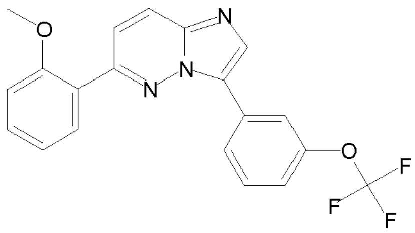

Mw.: 385.10, LCMS-ESI (*m*/*z*) - found 386.10 [M+H]$^+$, 384.10 [M-H]$^-$.

**Example 18-116 compound 116**

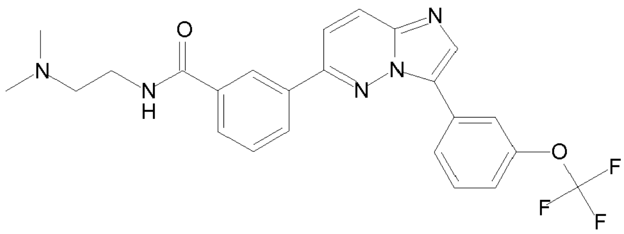

Mw.: 469.17, LCMS-ESI (*m*/*z*) - found 470.17 [M+H]$^+$, 468.17 [M-H]$^-$.

**Example 18-117 compound 117**

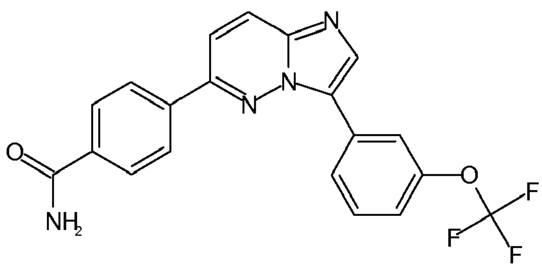

Mw.: 398.10, LCMS-ESI (*m*/*z*) - found 399.09 [M+H]$^+$, 397.09 [M-H]$^-$.

**Example 18-118 compound 118**

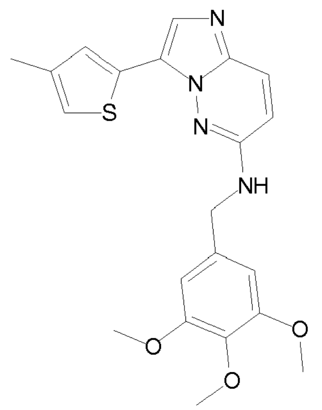

Mw.: 410.14, LCMS-ESI (*m/z*) - found 411.14 $[M+H]^+$, 409.14 $[M-H]^-$.

**Example 18-119 compound 119**

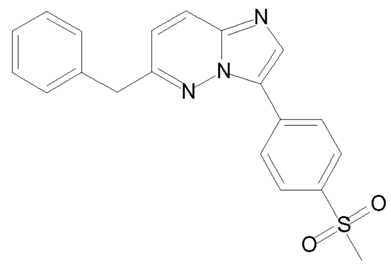

Mw.: 363.10, LCMS-ESI (*m/z*) - found 364.10 $[M+H]^+$, 362.10 $[M-H]^-$.

**Example 18-120 compound 120**

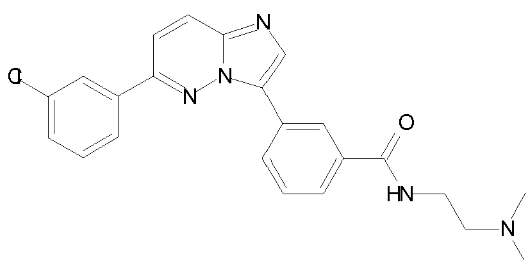

Mw.: 419.15, LCMS-ESI (*m/z*) - found 420.15 $[M+H]^+$, 418.15 $[M-H]^-$.

**Example 18-121 compound 121**

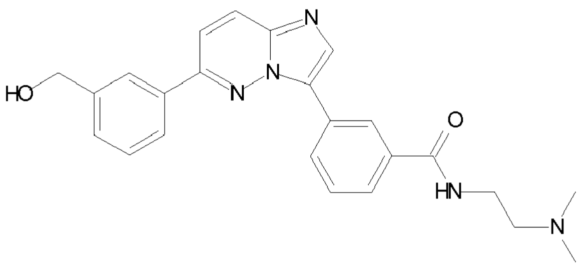

Mw.: 415.20, LCMS-ESI (*m/z*) - found 416.20 $[M+H]^+$, 414.20 $[M-H]^-$.

**Example 18-122 compound 122**

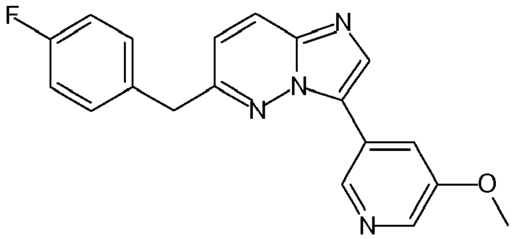

Mw.: 334.12, LCMS-ESI (*m/z*) - found 335.12 $[M+H]^+$, 333.12 $[M-H]^-$.

**Example 18-123 compound 123**

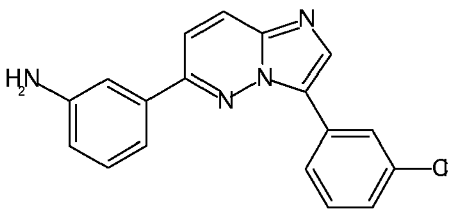

Mw.: 320.08, LCMS-ESI (*m/z*) - found 321.08 $[M+H]^+$, 319.08 $[M-H]^-$.

**Example 18-124 compound 124**

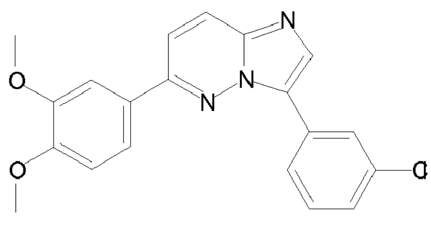

Mw.: 365.09, LCMS-ESI (*m/z*) - found 366.09 $[M+H]^+$, 364.09 $[M-H]^-$.

**Example 18-125 compound 125**

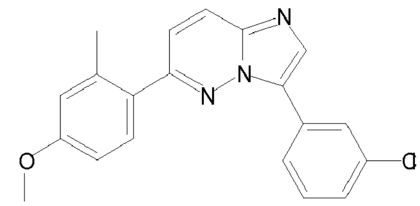

Mw.: 349.10, LCMS-ESI (*m*/*z*) - found 350.09 $[M+H]^+$, 348.09 $[M-H]^-$.

**Example 18-126 compound 126**

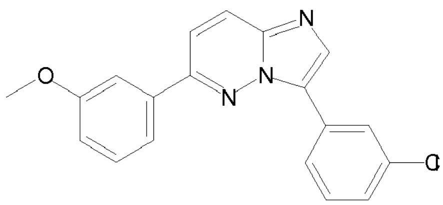

Mw.: 335.08, LCMS-ESI (*m*/*z*) - found 336.08 $[M+H]^+$, 334.08 $[M-H]^-$.

**Example 18-127 compound 127**

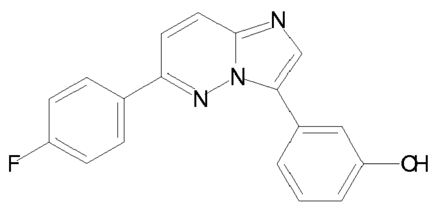

Mw.: 305.10, LCMS-ESI (*m*/*z*) - found 306.09 $[M+H]^+$, 304.09 $[M-H]^-$.

**Example 18-128 compound 128**

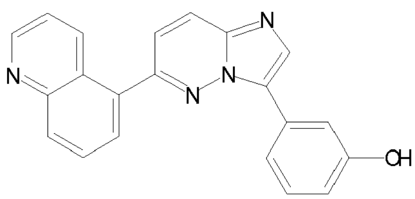

Mw.: 338.12, LCMS-ESI (*m*/*z*) - found 339.11 $[M+H]^+$, 337.11 $[M-H]^-$.

**Example 18-129 compound 129**

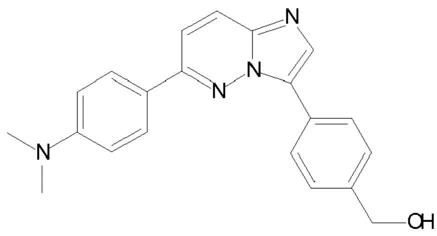

Mw.: 344.16, LCMS-ESI (*m*/*z*) - found 345.16 $[M+H]^+$, 346.16 $[M-H]^-$.

**Example 18-130 compound 130**

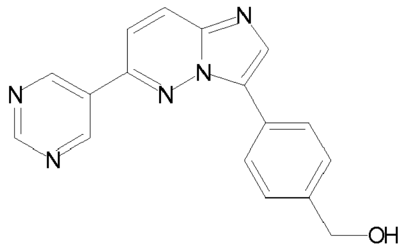

Mw.: 303.11, LCMS-ESI (*m*/*z*) - found 304.11 $[M+H]^+$, 302.11 $[M-H]^-$.

**Example 18-131 compound 131**

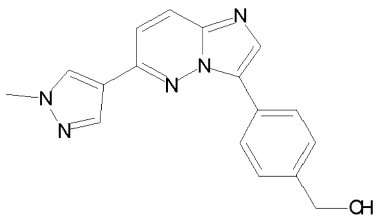

Mw.: 305.13, LCMS-ESI (*m*/*z*) - found 306.12 $[M+H]^+$, 304.12 $[M-H]^-$.

**Example 18-132 compound 132**

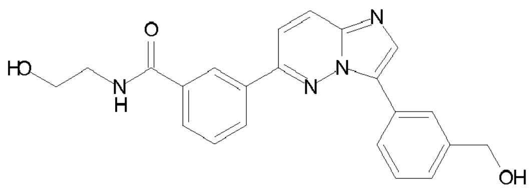

Mw.: 388.15, LCMS-ESI (*m*/*z*) - found 389.15 $[M+H]^+$, 387.15 $[M-H]^-$.

**Example 18-133 compound 133**

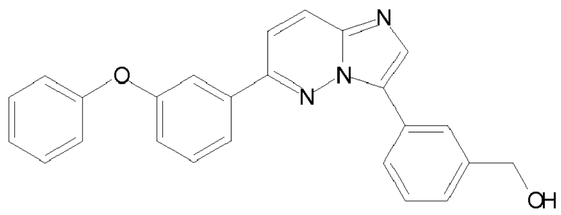

Mw.: 393.15, LCMS-ESI (*m/z*) - found 394.14 $[M+H]^+$, 392.14 $[M-H]^-$.

**Example 18-134 compound 134**

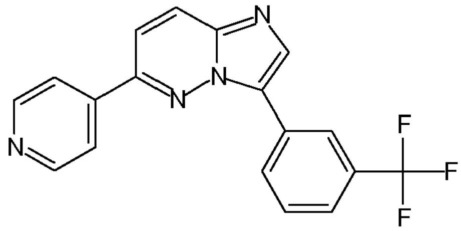

Mw.: 340.09, LCMS-ESI (*m/z*) - found 341.09 $[M+H]^+$, 339.09 $[M-H]^-$.

**Example 18-135 compound 135**

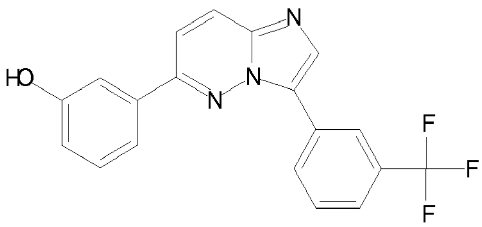

Mw.: 355.09, LCMS-ESI (*m/z*) - found 356.09 $[M+H]^+$, 354.09 $[M-H]^-$.

**Example 18-136 compound 136**

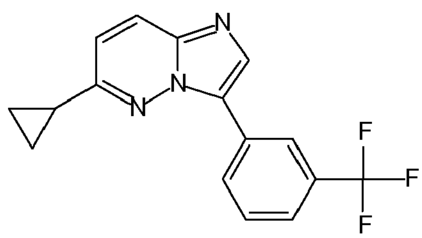

Mw.: 303.10, LCMS-ESI (*m/z*) - found 304.09 $[M+H]^+$, 302.09 $[M-H]^-$.

**Example 18-137 compound 137**

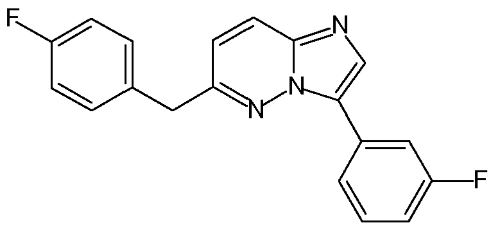

Mw.: 321.11, LCMS-ESI (*m*/*z*) - found 322.11 [M+H]$^+$.

**Example 18-138 compound 138**

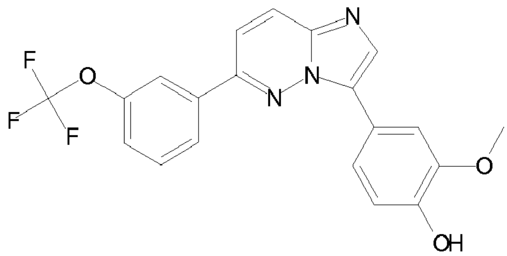

Mw.: 401.10, LCMS-ESI (*m*/*z*) - found 402.09 [M+H]$^+$, 400.09 [M-H]$^-$.

**Example 18-139 compound 139**

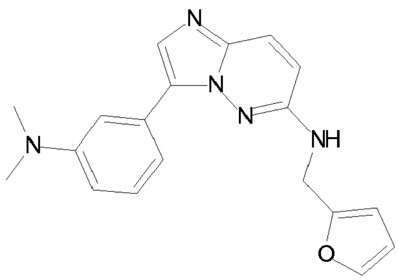

Mw.: 333.16, LCMS-ESI (*m*/*z*) - found 334.15 [M+H]$^+$, 332.15 [M-H]$^-$.

**Example 18-140 compound 140**

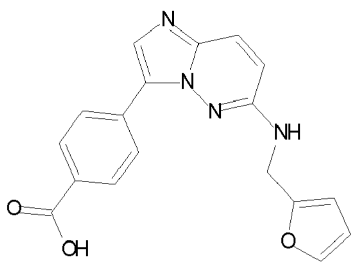

Mw.: 334.11, LCMS-ESI (*m*/*z*) - found 335.107 [M+H]$^+$, 333.107 [M-H]$^-$.

**Example 18-141 compound 141**

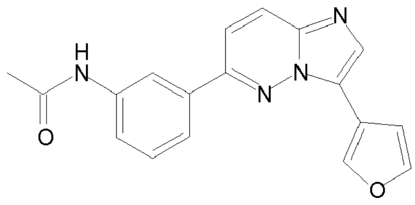

Mw.: 318.11, LCMS-ESI (*m*/*z*) - found 319.11 $[M+H]^+$, 317.11 $[M-H]^-$.

**Example 18-142 compound 142**

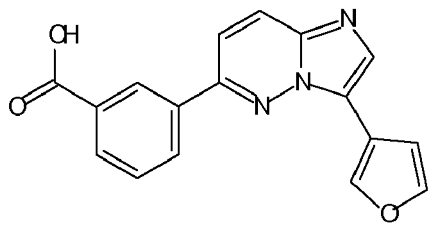

Mw.: 305.08, LCMS-ESI (*m*/*z*) - found 306.08 $[M+H]^+$, 304.08 $[M-H]^-$.

**Example 18-143 compound 143**

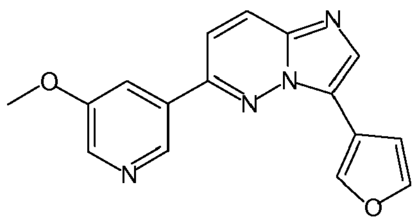

Mw.: 292.10, LCMS-ESI (*m*/*z*) - found 293.09 $[M+H]^+$, 291.09 $[M-H]^-$.

**Example 18-144 compound 144**

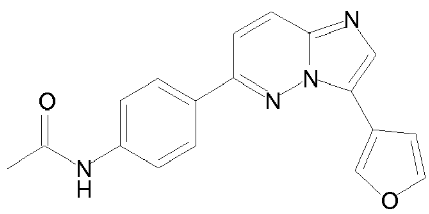

Mw.: 318.11, LCMS-ESI (*m*/*z*) - found 319.11 $[M+H]^+$, 317.11 $[M-H]^-$.

**Example 18-145 compound 145**

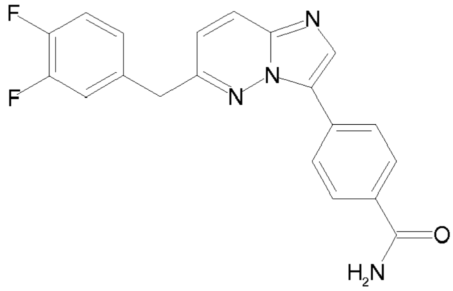

Mw.: 364.11, LCMS-ESI (*m/z*) - found 365.11 $[M+H]^+$, 363.11 $[M-H]^-$.

**Example 18-146 compound 146**

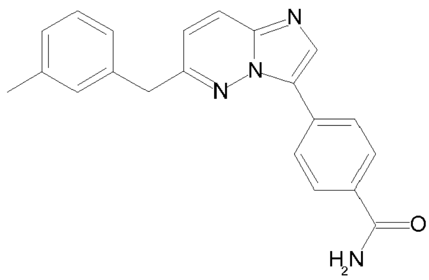

Mw.: 342.15, LCMS-ESI (*m/z*) - found 343.14 $[M+H]^+$, 341.14 $[M-H]^-$.

**Example 18-147 compound 147**

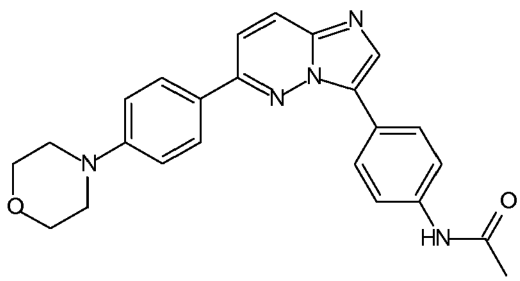

Mw.: 413.19, LCMS-ESI (*m/z*) - found 414.18 $[M+H]^+$, 412.18 $[M-H]^-$.

**Example 18-148 compound 148**

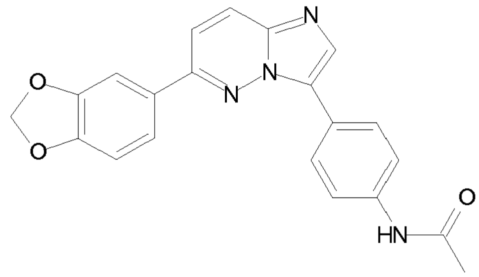

Mw.: 372.12, LCMS-ESI (*m/z*) - found 373.12 $[M+H]^+$, 371.12 $[M-H]^-$.

**Example 18-149 compound 149**

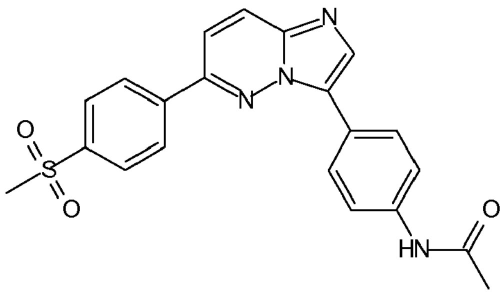

Mw.: 406.11, LCMS-ESI (*m/z*) - found 407.11 $[M+H]^+$, 405.11 $[M-H]^-$.

**Example 18-150 compound 150**

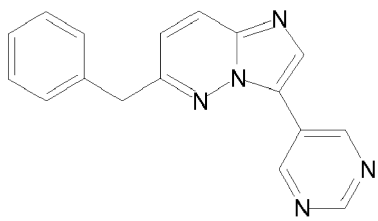

Mw.: 287.12, LCMS-ESI (*m/z*) - found 288.11 $[M+H]^+$, 286.11 $[M-H]^-$.

**Example 18-151 compound 151**

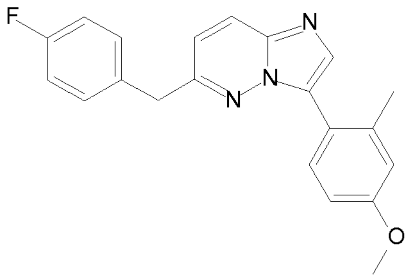

Mw.: 347.14, LCMS-ESI (*m/z*) - found 348.14 $[M+H]^+$, 346.14 $[M-H]^-$.

**Example 18-152 compound 152**

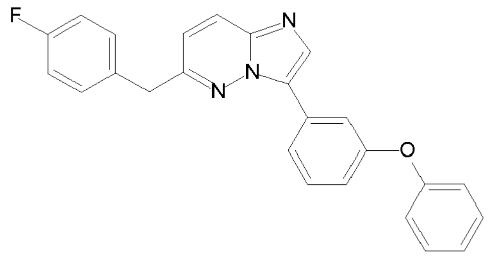

Mw.: 395.14, LCMS-ESI (*m*/*z*) - found 396.14 $[M+H]^+$, 394.14 $[M-H]^-$.

**Example 18-153 compound 153**

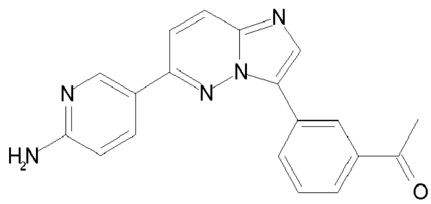

Mw.: 329.13, LCMS-ESI (*m*/*z*) - found 330.12 $[M+H]^+$, 328.12 $[M-H]^-$.

**Example 18-154 compound 154**

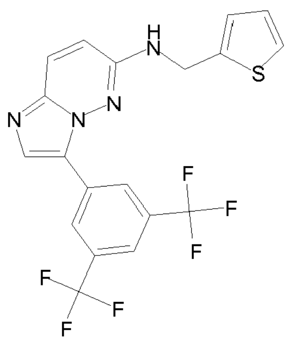

Mw.: 442.07, LCMS-ESI (*m*/*z*) - found 443.06 $[M+H]^+$, 441.06 $[M-H]^-$.

**Example 18-155 compound 155**

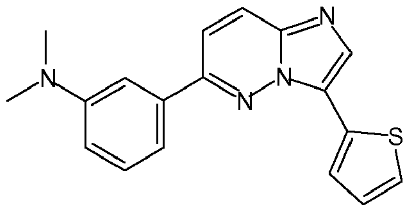

Mw.: 320.11, LCMS-ESI (*m/z*) - found 321.11 $[M+H]^+$, 319.11 $[M-H]^-$.

**Example 18-156 compound 156**

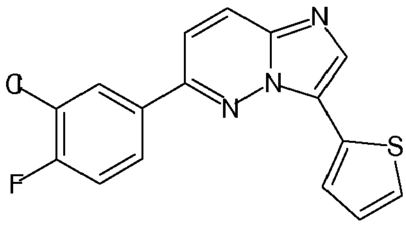

Mw.: 329.02, LCMS-ESI (*m/z*) - found 330.01 $[M+H]^+$, 328.01 $[M-H]^-$.

**Example 18-157 compound 157**

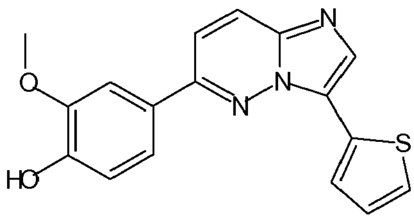

Mw.: 323.07, LCMS-ESI (*m/z*) - found 324.07 $[M+H]^+$, 322.07 $[M-H]^-$.

**Example 18-158 compound 158**

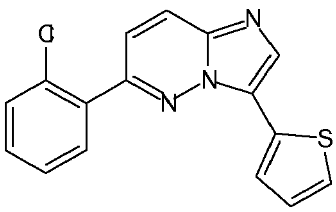

Mw.: 311.03, LCMS-ESI (*m/z*) - found 312.02 $[M+H]^+$, 310.02 $[M-H]^-$.

**Example 18-159 compound 159**

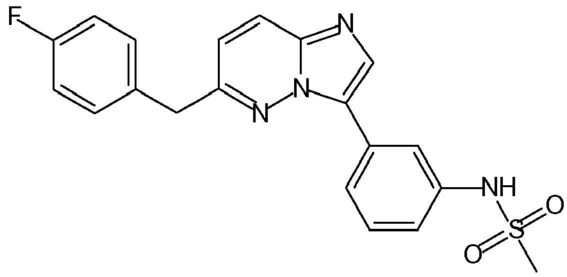

Mw.: 396.11, LCMS-ESI (*m/z*) - found 397.10 $[M+H]^+$, 395.10 $[M-H]^-$.

**Example 18-160 compound 160**

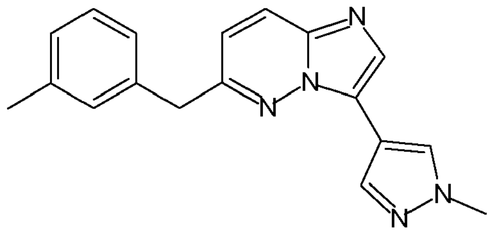

Mw.: 303.15, LCMS-ESI (*m/z*) - found 304.14 $[M+H]^+$, 302.14 $[M-H]^-$.

**Example 18-161 compound 161**

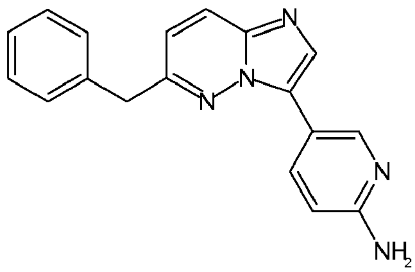

Mw.: 301.13, LCMS-ESI (*m/z*) - found 302.13 $[M+H]^+$, 300.13 $[M-H]^-$.

**Example 18-162 compound 162**

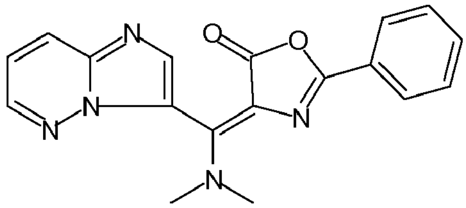

Mw.: 333.34.

**Example 18-163 compound 163**

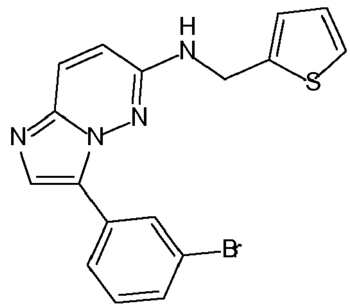

Mw.: 384.00, LCMS-ESI (*m/z*) - found 385.00 $[M+H]^+$, 383.00 $[M-H]^-$.

**Example 18-164 compound 164**

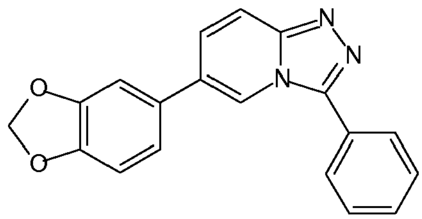

Mw.: 315.10, LCMS-ESI (*m/z*) - found 316.10 $[M+H]^+$, 314.10 $[M-H]^-$.

**Example 18-165 compound 165**

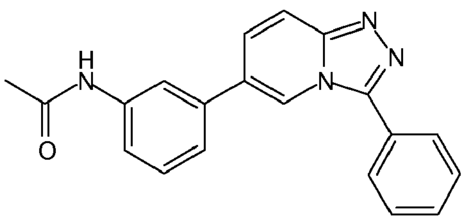

Mw.: 328.13, LCMS-ESI (*m/z*) - found 329.13 $[M+H]^+$, 327.13 $[M-H]^-$.

**Example 18-166 compound 166**

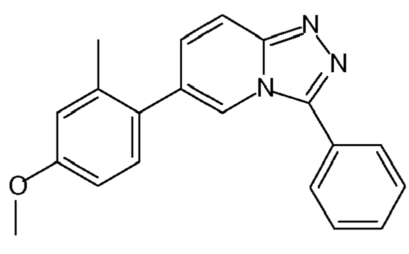

Mw.: 315.14, LCMS-ESI (*m*/*z*) - found 316.13 $[M+H]^+$, 314.13 $[M-H]^-$.

**Example 18-167 compound 167**

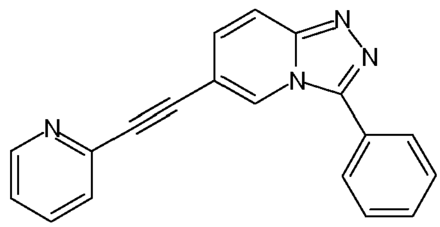

Mw.: 296.11, LCMS-ESI (*m*/*z*) - found 297.11 $[M+H]^+$.

**Example 18-168 compound 168**

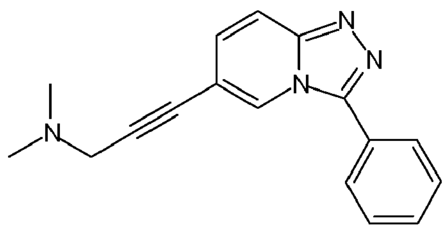

Mw.: 276.14, LCMS-ESI (*m*/*z*) - found 277.13 $[M+H]^+$, 275.13 $[M-H]^-$.

**Example 18-169 compound 169**

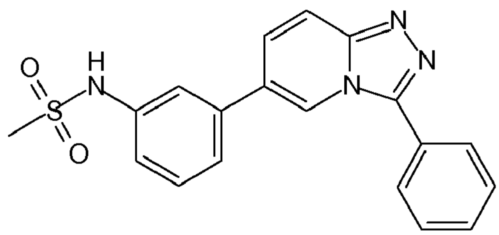

Mw.: 364.10, LCMS-ESI (*m*/*z*) - found 365.09 $[M+H]^+$, 363.09 $[M-H]^-$.

**Example 18-170 compound 170**

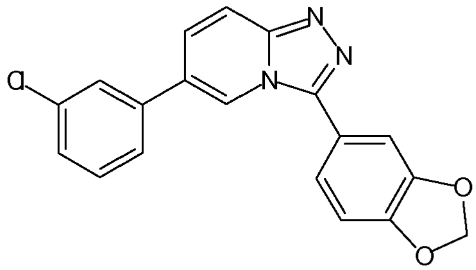

Mw.: 349.06, LCMS-ESI (*m/z*) - found 350.06 $[M+H]^+$, 348.06 $[M-H]^-$.

**Example 18-171 compound 171**

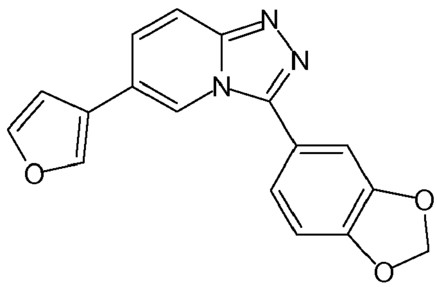

Mw.: 305.08, LCMS-ESI (*m/z*) - found 306.08 $[M+H]^+$, 304.08 $[M-H]^-$.

**Example 18-172 compound 172**

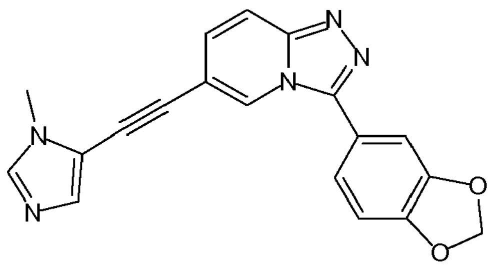

Mw.: 343.11, LCMS-ESI (*m/z*) - found 344.10 $[M+H]^+$, 342.10 $[M-H]^-$.

**Example 18-173 compound 173**

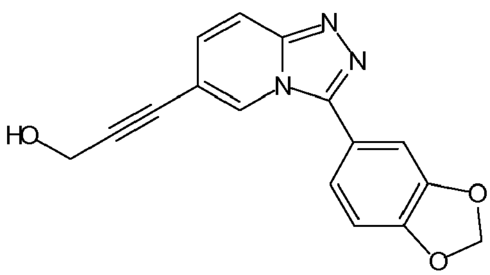

Mw.: 293.08, LCMS-ESI (*m/z*) - found 294.08 $[M+H]^+$, 292.08 $[M-H]^-$.

**Example 18-174 compound 174**

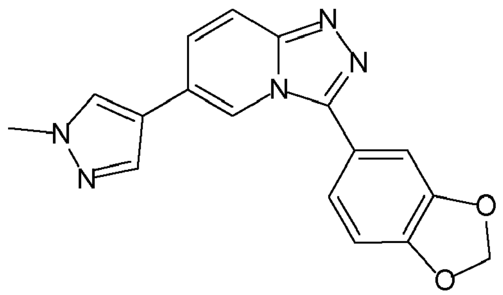

Mw.: 319.11, LCMS-ESI (*m/z*) - found 320.10 $[M+H]^+$, 318.10 $[M-H]^-$.

**Example 18-175 compound 175**

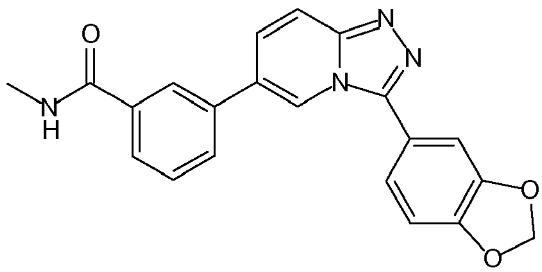

Mw.: 372.12, LCMS-ESI (*m/z*) - found 373.12 $[M+H]^+$, 371.12 $[M-H]^-$.

**Example 18-176 compound 176**

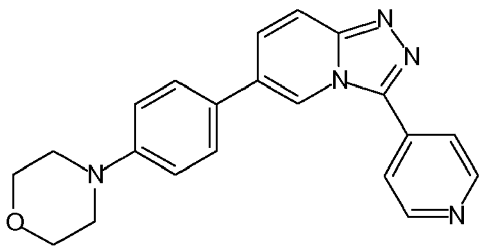

Mw.: 357.16, LCMS-ESI (*m/z*) - found 358.15 $[M+H]^+$, 356.15 $[M-H]^-$.

**Example 18-177 compound 177**

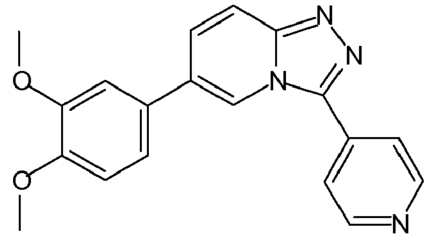

Mw.: 332.13, LCMS-ESI (*m*/*z*) - found 333.12 [M+H]$^+$, 331.12 [M-H]$^-$.

**Example 18-178 compound 178**

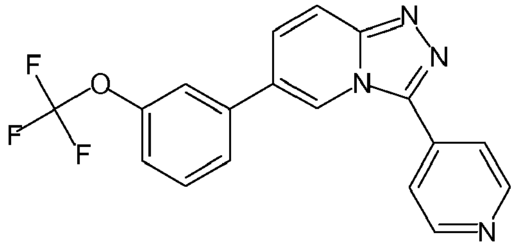

Mw.: 356.09, LCMS-ESI (*m*/*z*) - found 357.08 [M+H]$^+$, 355.08 [M-H]$^-$.

**Example 18-179 compound 179**

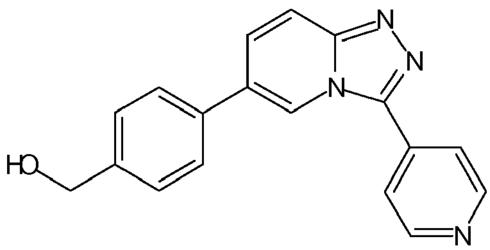

Mw.: 302.12, LCMS-ESI (*m*/*z*) - found 303.11 [M+H]$^+$, 301.11 [M-H]$^-$.

**Example 18-180 compound 180**

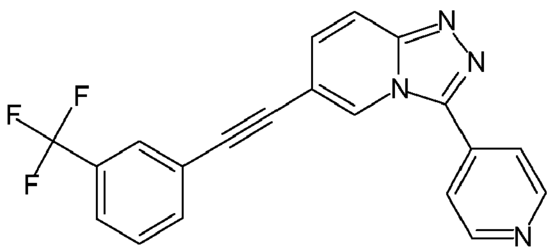

Mw.: 364.09, LCMS-ESI (*m*/*z*) - found 365.09 [M+H]$^+$, 362.09 [M-H]$^-$.

**Example 18-181 compound 181**

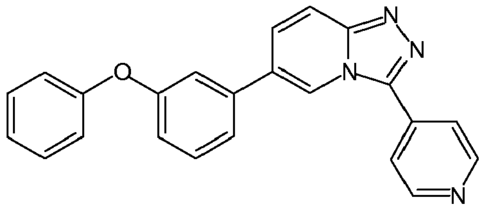

Mw.: 364.13, LCMS-ESI (*m/z*) - found 365.13 $[M+H]^+$, 363.13 $[M-H]^-$.

**Example 18-182 compound 182**

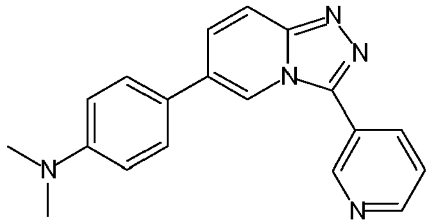

Mw.: 315.15, LCMS-ESI (*m/z*) - found 316.14 $[M+H]^+$, 314.14 $[M-H]^-$.

**Example 18-183 compound 183**

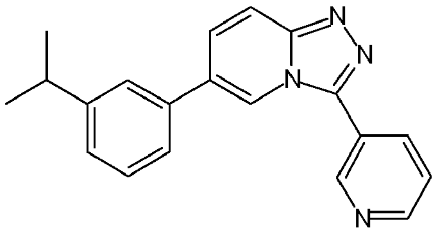

Mw.: 314.15, LCMS-ESI (*m/z*) - found 315.15 $[M+H]^+$, 313.15 $[M-H]^-$.

**Example 18-184 compound 184**

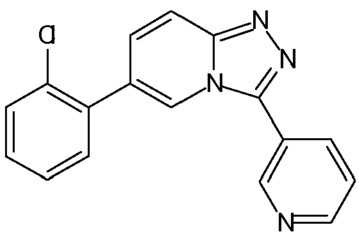

Mw.: 306.07, LCMS-ESI (*m/z*) - found 307.06 $[M+H]^+$, 305.06 $[M-H]^-$.

**Example 18-185 compound 185**

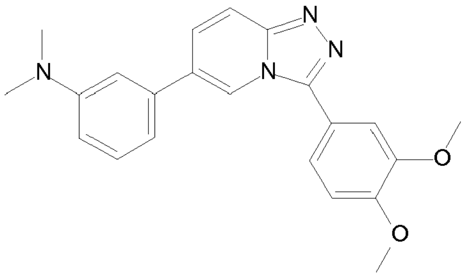

Mw.: 374.17, LCMS-ESI (*m/z*) - found 375.17 $[M+H]^+$, 373.17 $[M-H]^-$.

**Example 18-186 compound 186**

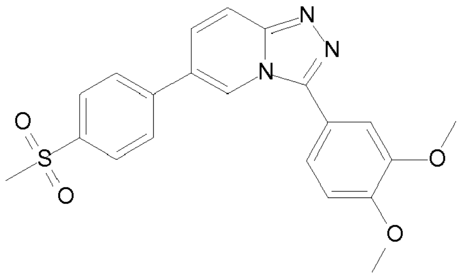

Mw.: 409.11, LCMS-ESI (*m/z*) - found 410.11 $[M+H]^+$, 408.11 $[M-H]^-$.

**Example 18-187 compound 187**

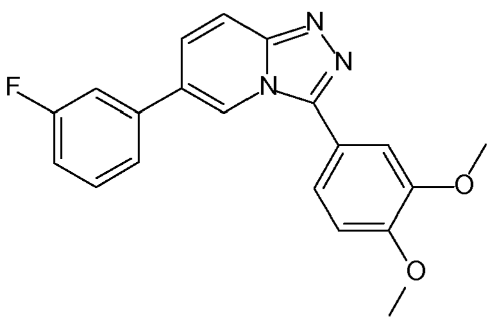

Mw.: 349.12, LCMS-ESI (*m/z*) - found 350.12 $[M+H]^+$, 348.12 $[M-H]^-$.

**Example 18-188 compound 188**

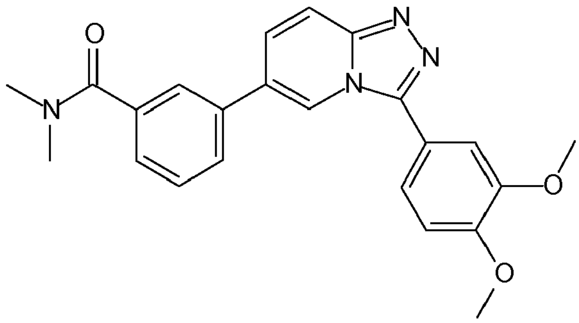

Mw.: 402.17, LCMS-ESI (*m*/*z*) - found 403.16 $[M+H]^+$, 401.16 $[M-H]^-$.

**Example 18-189 compound 189**

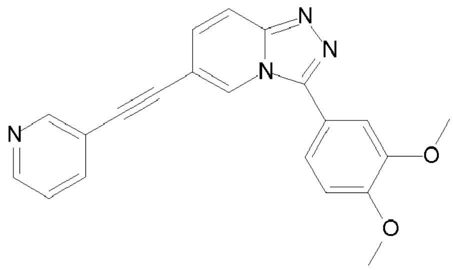

Mw.: 356.13, LCMS-ESI (*m*/*z*) - found 357.12 $[M+H]^+$, 355.12 $[M-H]^-$.

**Example 18-190 compound 190**

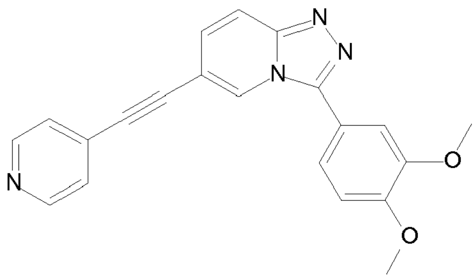

Mw.: 356.13, LCMS-ESI (*m*/*z*) - found 357.12 $[M+H]^+$, 355.12 $[M-H]^-$.

**Example 18-191 compound 191**

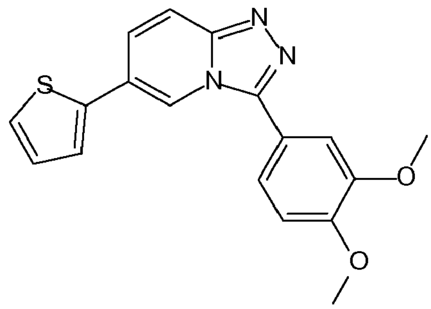

Mw.: 337.09, LCMS-ESI (*m/z*) - found 338.08 $[M+H]^+$, 336.08 $[M-H]^-$.

**Example 18-192 compound 192**

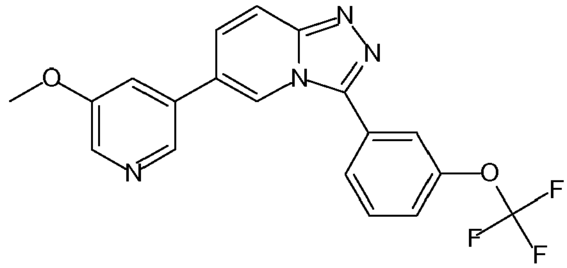

Mw.: 386.10, LCMS-ESI (*m/z*) - found 387.09 $[M+H]^+$, 385.09 $[M-H]^-$.

**Example 18-193 compound 193**

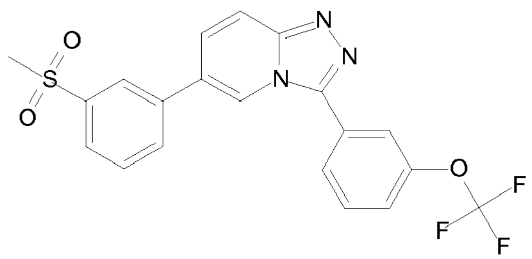

Mw.: 433.07, LCMS-ESI (*m/z*) - found 434.07 $[M+H]^+$, 432.07 $[M-H]^-$.

**Example 18-194 compound 194**

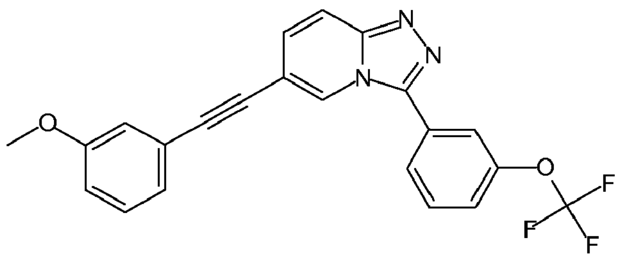

Mw.: 409.10, LCMS-ESI (*m*/*z*) - found 410.10 [M+H]$^+$.

**Example 18-195 compound 195**

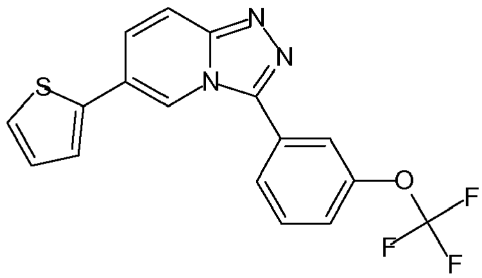

Mw.: 361.05, LCMS-ESI (*m*/*z*) - found 362.05 [M+H]$^+$, 360.05 [M-H]$^-$.

**Example 18-196 compound 196**

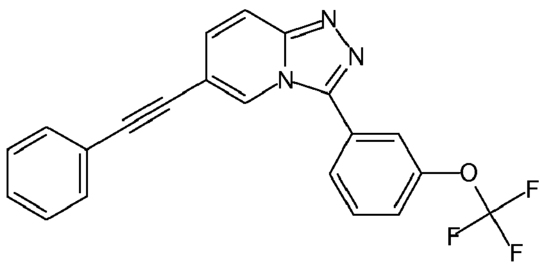

Mw.: 379.09, LCMS-ESI (*m*/*z*) - found 380.09 [M+H]$^+$, 378.09 [M-H]$^-$.

**Example 18-197 compound 197**

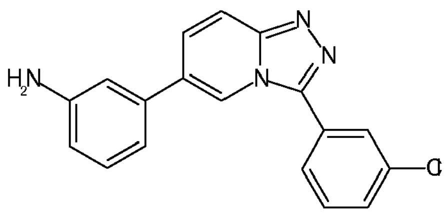

Mw.: 320.08, LCMS-ESI (*m*/*z*) - found 321.08 [M+H]$^+$, 319.08 [M-H]$^-$.

**Example 18-198 compound 198**

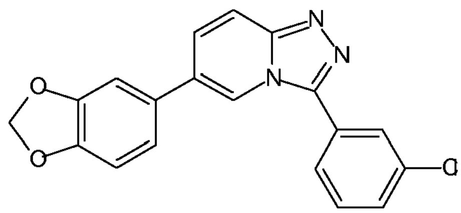

Mw.: 349.06, LCMS-ESI (*m/z*) - found 350.06 $[M+H]^+$, 348.06 $[M-H]^-$.

**Example 18-199 compound 199**

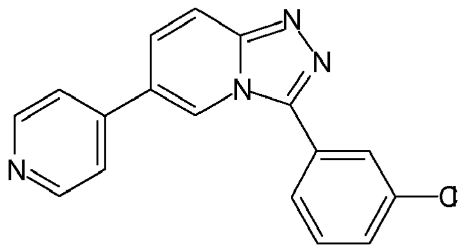

Mw.: 306.07, LCMS-ESI (*m/z*) - found 307.06 $[M+H]^+$, 305.06 $[M-H]^-$.

**Example 18-200 compound 200**

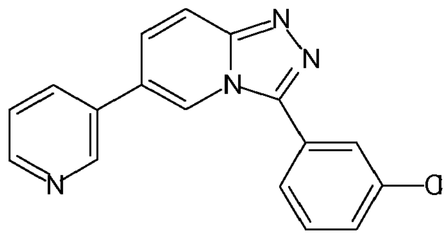

Mw.: 306.07, LCMS-ESI (*m/z*) - found 307.06 $[M+H]^+$, 305.06 $[M-H]^-$.

**Example 18-201 compound 201**

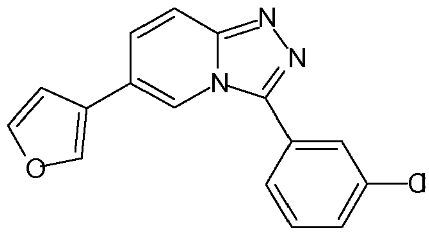

Mw.: 295.05, LCMS-ESI (*m/z*) - found 296.05 $[M+H]^+$, 294.05 $[M-H]^-$.

**Example 18-202 compound 202**

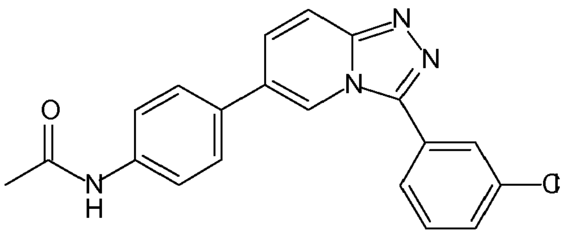

Mw.: 362.09, LCMS-ESI (*m*/*z*) - found 363.09 $[M+H]^+$, 361.09 $[M-H]^-$.

**Example 18-203 compound 203**

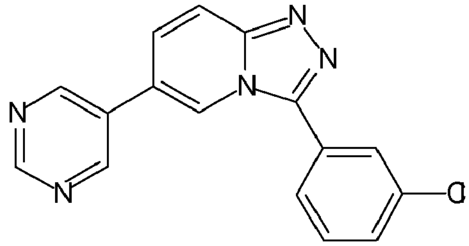

Mw.: 307.06, LCMS-ESI (*m*/*z*) - found 308.06 $[M+H]^+$, 306.06 $[M-H]^-$.

**Example 18-204 compound 204**

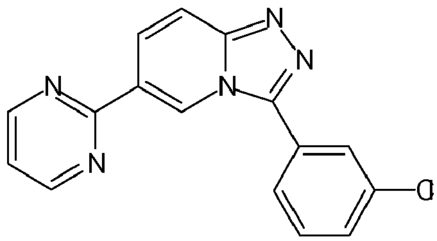

Mw.: 307.06, LCMS-ESI (*m*/*z*) - found 308.06 $[M+H]^+$, 306.06 $[M-H]^-$.

**Example 18-205 compound 205**

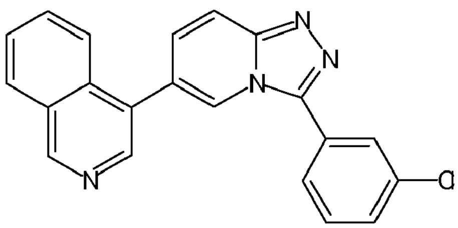

Mw.: 356.08, LCMS-ESI (*m*/*z*) - found 357.08 $[M+H]^+$, 355.08 $[M-H]^-$.

**Example 18-206 compound 206**

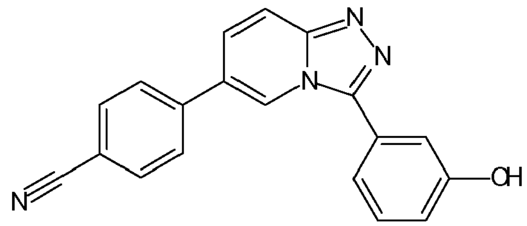

Mw.: 312.10, LCMS-ESI (*m*/*z*) - found 313.10 $[M+H]^+$, 311.10 $[M-H]^-$.

**Example 18-207 compound 207**

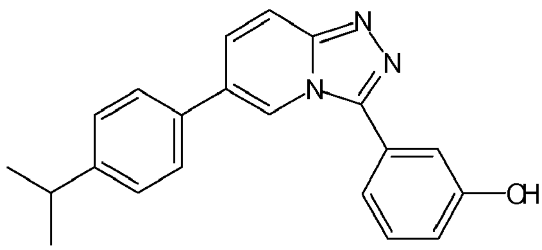

Mw.: 329.15, LCMS-ESI (*m*/*z*) - found 330.15 $[M+H]^+$, 328.15 $[M-H]^-$.

**Example 18-208 compound 208**

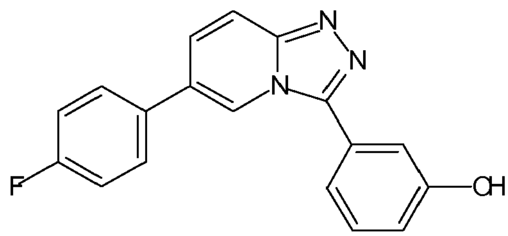

Mw.: 305.10, LCMS-ESI (*m*/*z*) - found 306.09 $[M+H]^+$, 304.09 $[M-H]^-$.

**Example 18-209 compound 209**

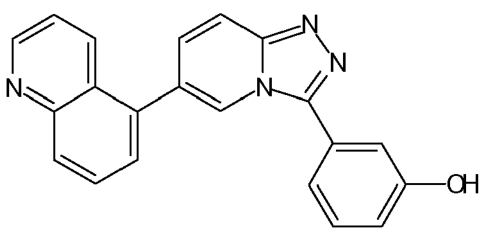

Mw.: 338.12, LCMS-ESI (*m/z*) - found 339.11 $[M+H]^+$, 337.11 $[M-H]^-$.

**Example 18-210 compound 210**

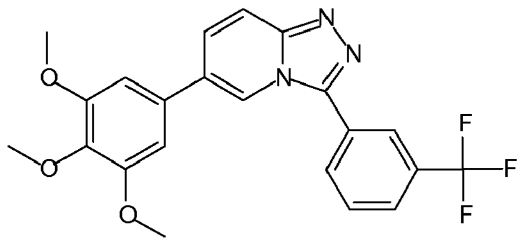

Mw.: 429.13, LCMS-ESI (*m/z*) - found 430.13 $[M+H]^+$, 428.13 $[M-H]^-$.

**Example 18-211 compound 211**

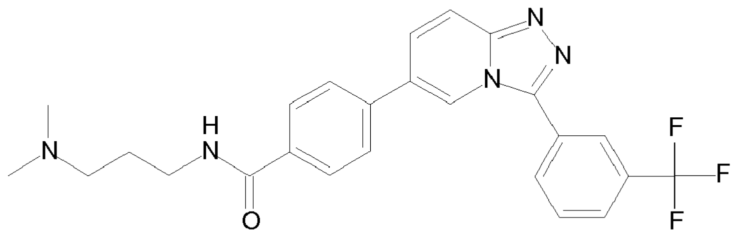

Mw.: 467.19, LCMS-ESI (*m/z*) - found 468.19 $[M+H]^+$, 466.19 $[M-H]^-$.

**Example 18-212 compound 212**

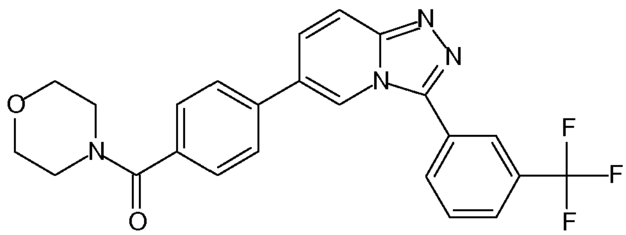

Mw.: 452.15, LCMS-ESI (*m/z*) - found 453.14 $[M+H]^+$, 451.14 $[M-H]^-$.

**Example 18-213 compound 213**

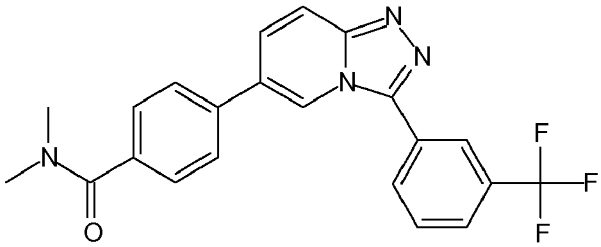

Mw.: 410.14, LCMS-ESI (*m*/*z*) - found 411.13 $[M+H]^+$, 409.13 $[M-H]^-$.

**Example 18-214 compound 214**

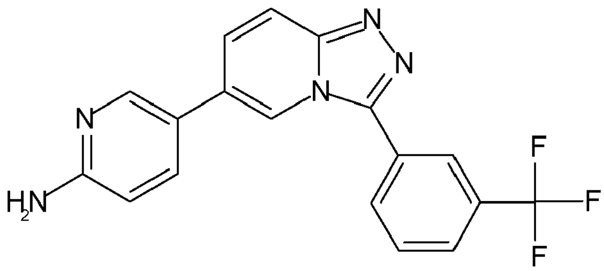

Mw.: 355.10, LCMS-ESI (*m*/*z*) - found 356.10 $[M+H]^+$, 354.10 $[M-H]^-$.

**Example 18-215 compound 215**

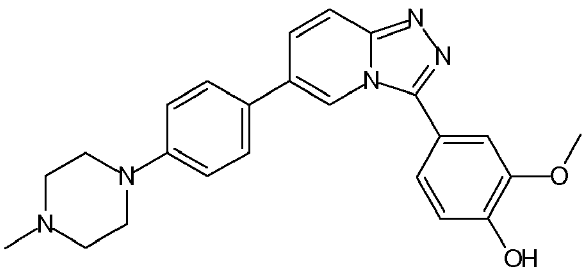

Mw.: 415.20, LCMS-ESI (*m*/*z*) - found 416.20 $[M+H]^+$, 414.20 $[M-H]^-$.

**Example 18-216 compound 216**

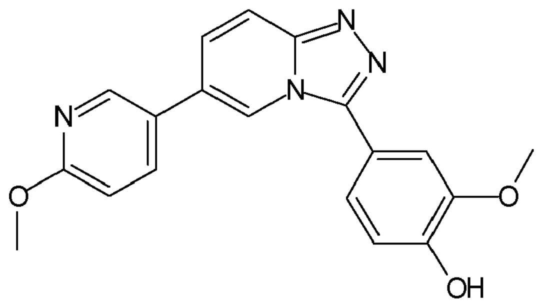

Mw.: 348.12, LCMS-ESI (*m/z*) - found 349.12 $[M+H]^+$, 347.12 $[M-H]^-$.

**Example 18-217 compound 217**

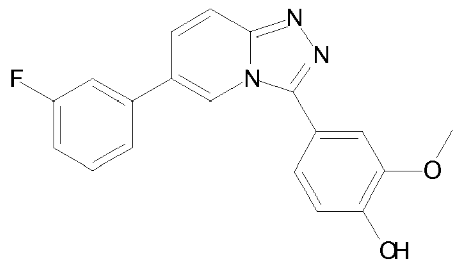

Mw.: 335.11, LCMS-ESI (*m/z*) - found 336.10 $[M+H]^+$, 334.10 $[M-H]^-$.

**Example 18-218 compound 218**

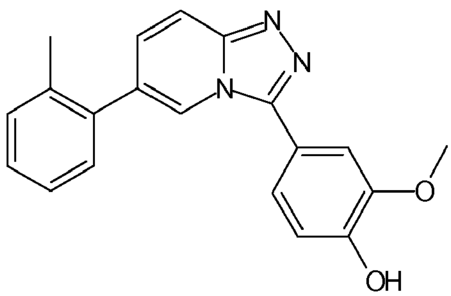

Mw.: 331.13, LCMS-ESI (*m/z*) - found 332.12 $[M+H]^+$, 330.13 $[M-H]^-$.

**Example 18-219 compound 219**

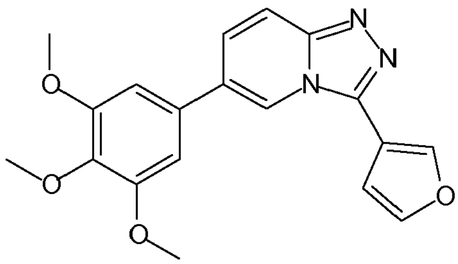

Mw.: 351.12, LCMS-ESI (*m/z*) - found 352.12 $[M+H]^+$, 350.12 $[M-H]^-$.

**Example 18-220 compound 220**

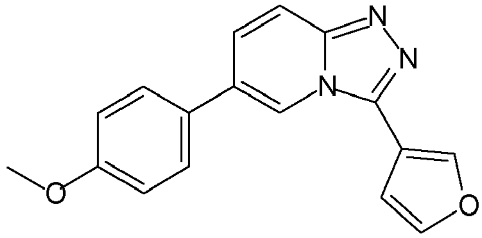

Mw.: 291.10, LCMS-ESI (*m/z*) - found 292.10 $[M+H]^+$, 290.10 $[M-H]^-$.

**Example 18-221 compound 221**

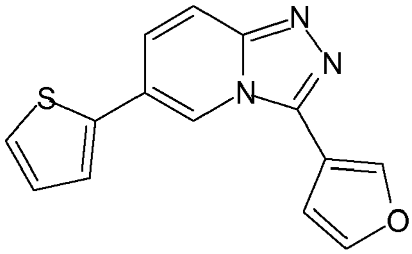

Mw.: 267.05, LCMS-ESI (*m/z*) - found 268.04 $[M+H]^+$, 266.04 $[M-H]^-$.

**Example 18-222 compound 222**

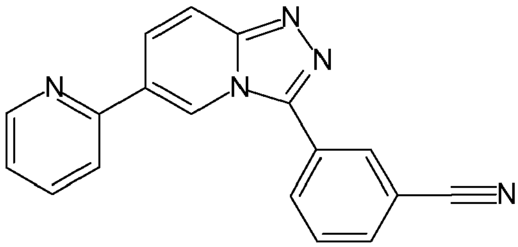

Mw.: 297.10, LCMS-ESI (*m/z*) - found 298.10 $[M+H]^+$, 296.10 $[M-H]^-$.

**Example 18-223 compound 223**

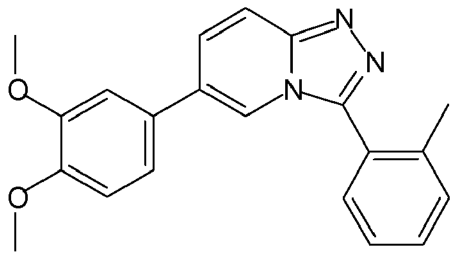

Mw.: 345.15, LCMS-ESI (*m/z*) - found 346.14 $[M+H]^+$, 344.14 $[M-H]^-$.

**Example 18-224 compound 224**

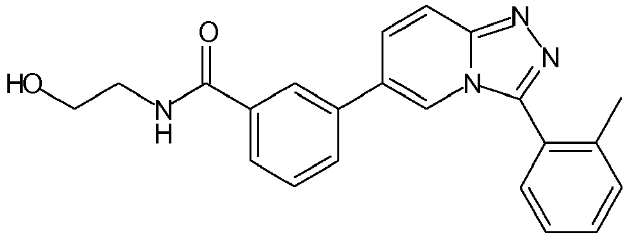

Mw.: 372.16, LCMS-ESI (*m*/*z*) - found 373.15 $[M+H]^+$, 371.15 $[M-H]^-$.

**Example 18-225 compound 225**

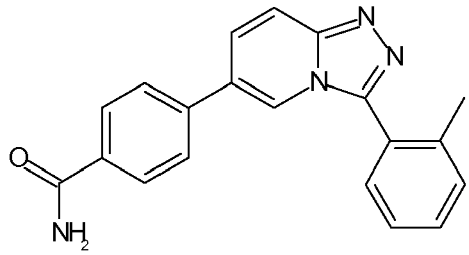

Mw.: 328.13, LCMS-ESI (*m*/*z*) - found 329.13 $[M+H]^+$, 327.13 $[M-H]^-$.

**Example 18-226 compound 226**

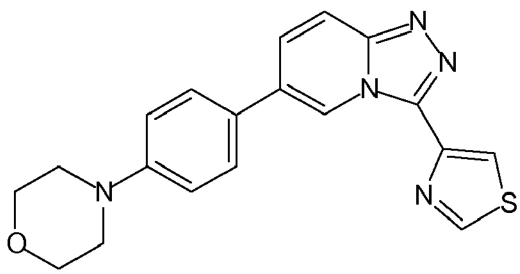

Mw.: 363.12, LCMS-ESI (*m*/*z*) - found 364.11 $[M+H]^+$, 362.11 $[M-H]^-$.

**Example 18-227 compound 227**

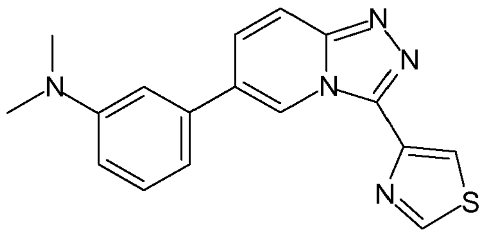

Mw.: 321.10, LCMS-ESI (*m*/*z*) - found 322.10 $[M+H]^+$, 320.10 $[M-H]^-$.

**Example 18-228 compound 228**

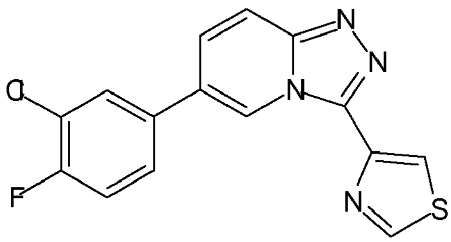

Mw.: 330.01, LCMS-ESI (*m*/*z*) - found 331.01 $[M+H]^+$, 329.01 $[M-H]^-$.

**Example 18-229 compound 229**

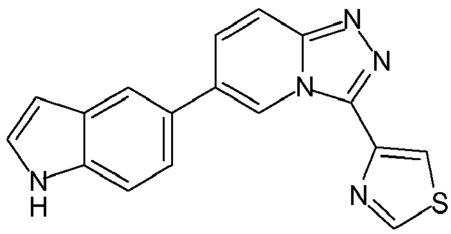

Mw.: 317.07, LCMS-ESI (*m*/*z*) - found 318.07 $[M+H]^+$, 316.07 $[M-H]^-$.

**Example 18-230 compound 230**

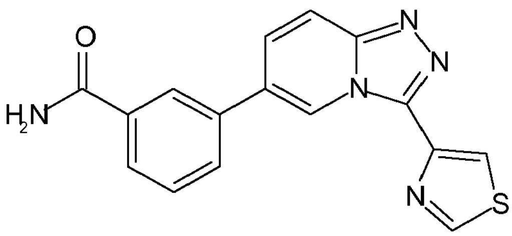

Mw.: 321.07, LCMS-ESI (*m*/*z*) - found 322.06 $[M+H]^+$, 320.06 $[M-H]^-$.

**Example 18-231 compound 231**

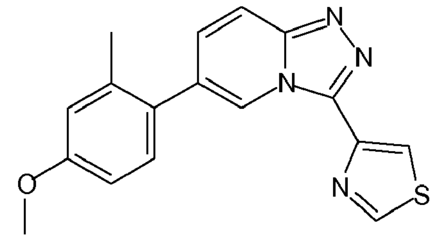

Mw.: 322.09, LCMS-ESI (*m/z*) - found 323.08 $[M+H]^+$, 321.08 $[M-H]^-$.

**Example 18-232 compound 232**

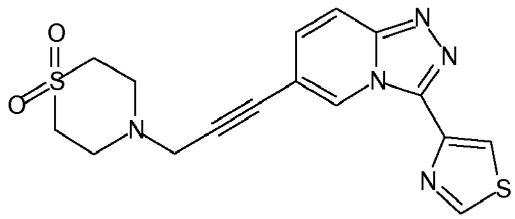

Mw.: 373.07, LCMS-ESI (*m/z*) - found 374.06 $[M+H]^+$, 372.06 $[M-H]^-$.

**Example 18-233 compound 233**

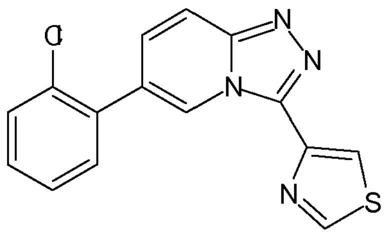

Mw.: 312.02, LCMS-ESI (*m/z*) - found 313.02 $[M+H]^+$, 311.02 $[M-H]^-$.

**Example 18-234 compound 234**

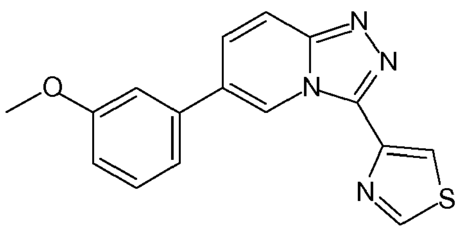

Mw.: 308.07, LCMS-ESI (*m/z*) - found 309.07 $[M+H]^+$, 307.07 $[M-H]^-$.

**Example 18-235 compound 235**

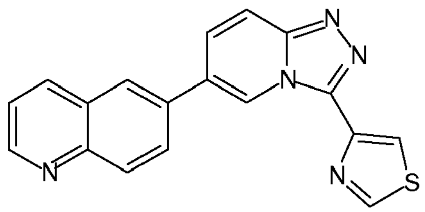

Mw.: 329.07, LCMS-ESI (*m*/*z*) - found 330.07 $[M+H]^+$, 328.07 $[M-H]^-$.

**Example 18-236 compound 236**

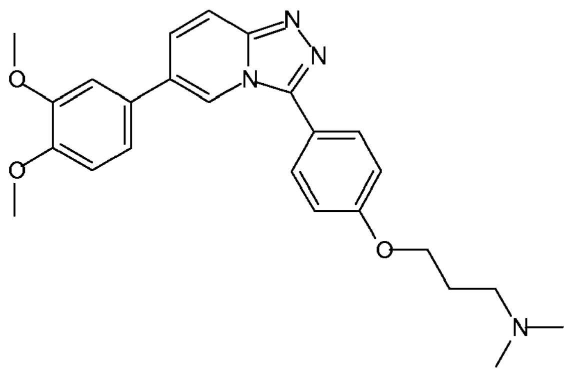

Mw.: 432.22, LCMS-ESI (*m*/*z*) - found 433.21 $[M+H]^+$, 431.21 $[M-H]^-$.

**Example 18-237 compound 237**

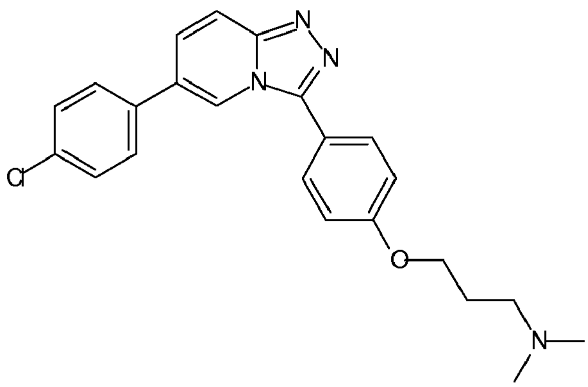

Mw.: 406.16, LCMS-ESI (*m*/*z*) - found 407.15 $[M+H]^+$, 405.15 $[M-H]^-$.

**Example 18-238 compound 238**

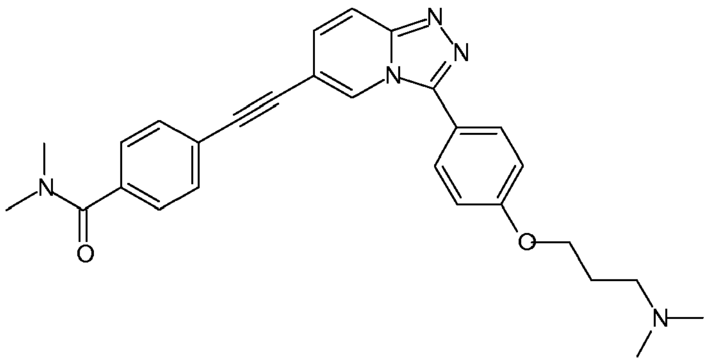

Mw.: 467.23, LCMS-ESI (*m*/*z*) - found 468.23 $[M+H]^+$, 466.23 $[M-H]^-$.

**Example 18-239 compound 239**

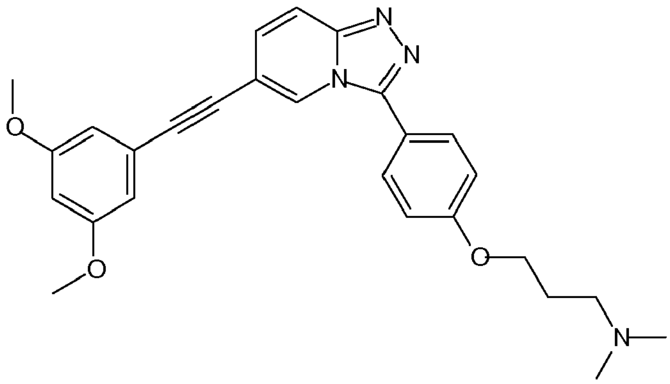

Mw.: 456.22, LCMS-ESI (*m*/*z*) - found 457.21 $[M+H]^+$, 455.21 $[M-H]^-$.

**Example 18-240 compound 240**

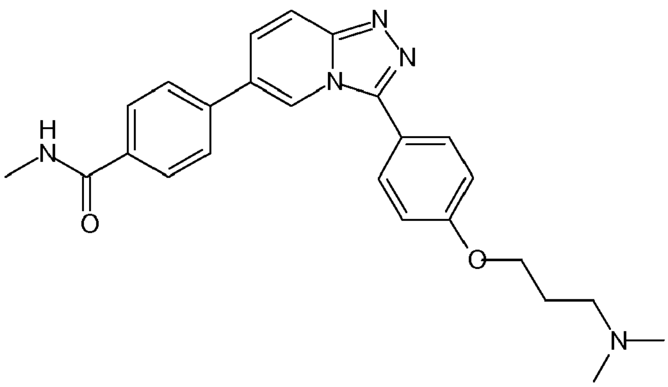

Mw.: 429.22, LCMS-ESI (*m*/*z*) - found 430.21 $[M+H]^+$, 428.21 $[M-H]^-$.

**Example 18-241 compound 241**

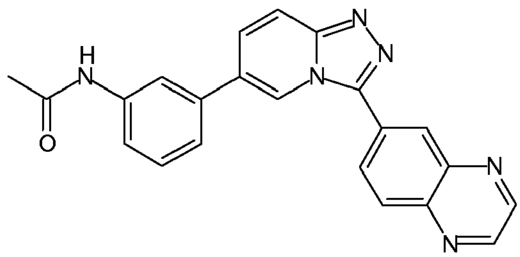

Mw.: 380.14, LCMS-ESI (*m*/*z*) - found 381.13 $[M+H]^+$, 379.13 $[M-H]^-$.

**Example 18-242 compound 242**

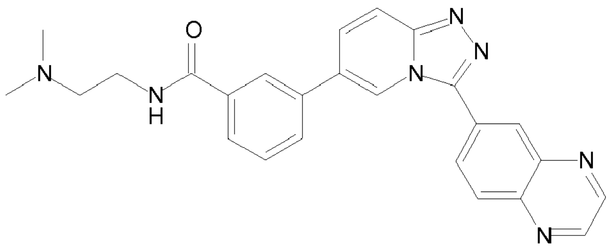

Mw.: 437.20, LCMS-ESI (*m*/*z*) - found 438.19 $[M+H]^+$, 436.19 $[M-H]^-$.

**Example 18-243 compound 243**

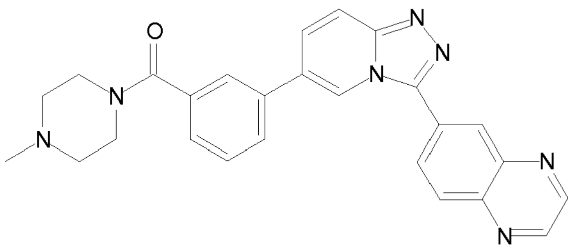

Mw.: 449.20, LCMS-ESI (*m*/*z*) - found 450.19 $[M+H]^+$, 448.19 $[M-H]^-$.

**Example 18-244 compound 244**

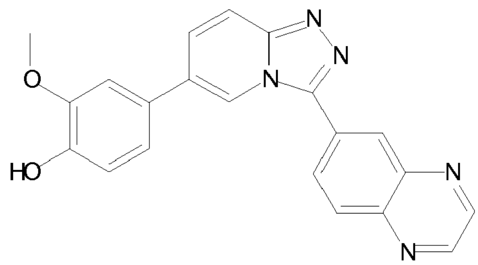

Mw.: 369.12, LCMS-ESI (*m*/*z*) - found 370.12 $[M+H]^+$, 368.12 $[M-H]^-$.

**Example 18-245 compound 245**

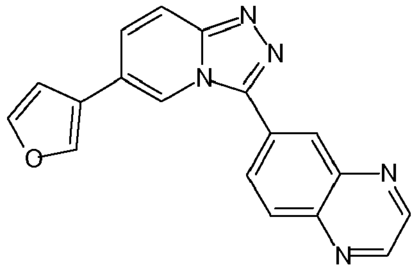

Mw.: 313.10, LCMS-ESI (*m/z*) - found 314.09 $[M+H]^+$, 312.09 $[M-H]^-$.

**Example 18-246 compound 246**

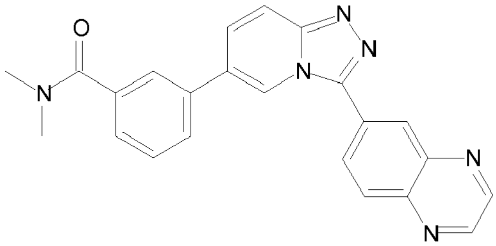

Mw.: 394.15, LCMS-ESI (*m/z*) - found 395.15 $[M+H]^+$, 393.15 $[M-H]^-$.

**Example 18-247 compound 247**

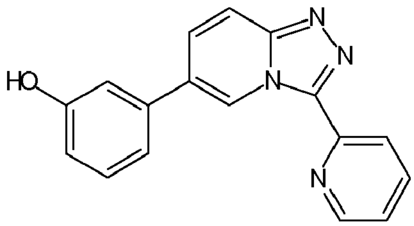

Mw.: 288.10, LCMS-ESI (*m/z*) - found 289.10 $[M+H]^+$, 287.10 $[M-H]^-$.

**Example 18-248 compound 248**

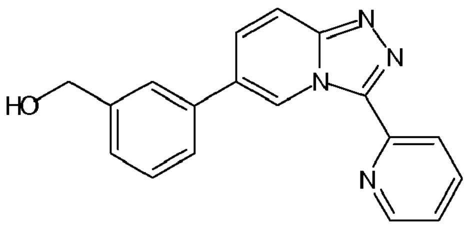

Mw.: 302.12, LCMS-ESI (*m*/*z*) - found 303.11 $[M+H]^+$, 301.11 $[M-H]^-$.

**Example 18-249 compound 249**

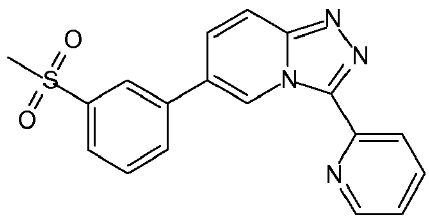

Mw.: 350.08, LCMS-ESI (*m*/*z*) - found 351.08 $[M+H]^+$, 349.08 $[M-H]^-$.

**Example 18-250 compound 250**

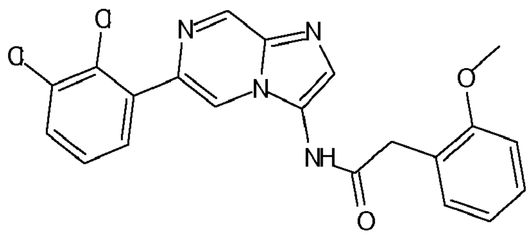

Mw.: 426.06, LCMS-ESI (*m*/*z*) - found 427.06 $[M+H]^+$, 425.06 $[M-H]^-$.

**Example 18-251 compound 251**

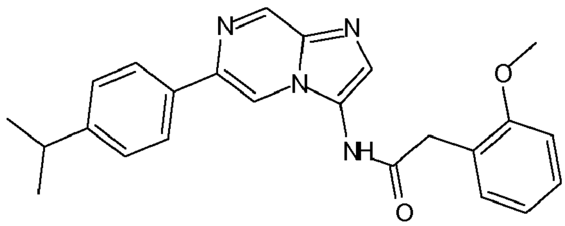

Mw.: 400.19, LCMS-ESI (*m*/*z*) - found 401.19 $[M+H]^+$, 399.19 $[M-H]^-$.

**Example 18-252 compound 252**

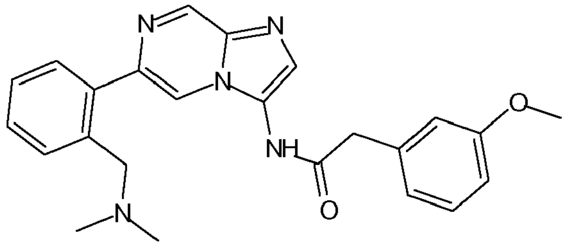

Mw.: 415.20, LCMS-ESI (*m*/*z*) - found 416.20 $[M+H]^+$, 414.20 $[M-H]^-$.

**Example 18-253 compound 253**

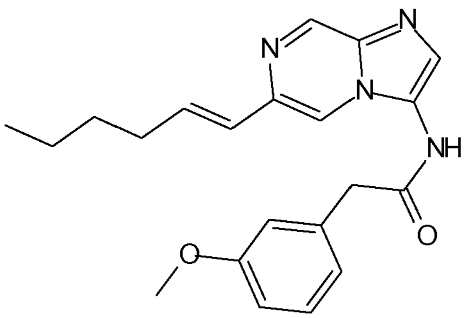

Mw.: 364.19, LCMS-ESI (*m*/*z*) - found 365.19 $[M+H]^+$, 363.19 $[M-H]^-$.

**Example 18-254 compound 254**

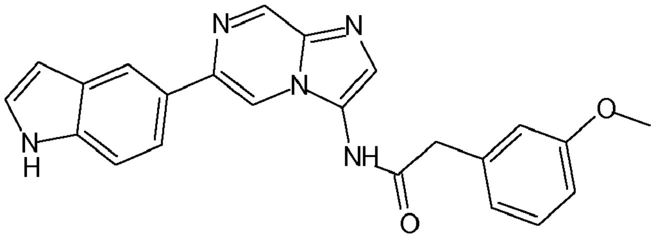

Mw.: 397.15, LCMS-ESI (*m*/*z*) - found 398.15 $[M+H]^+$, 396.15 $[M-H]^-$.

**Example 18-255 compound 255**

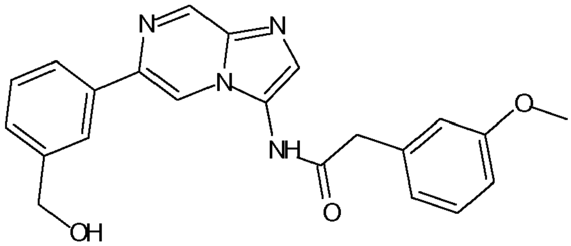

Mw.: 388.15, LCMS-ESI (*m*/*z*) - found 389.15 $[M+H]^+$, 387.15 $[M-H]^-$.

**Example 18-256 compound 256**

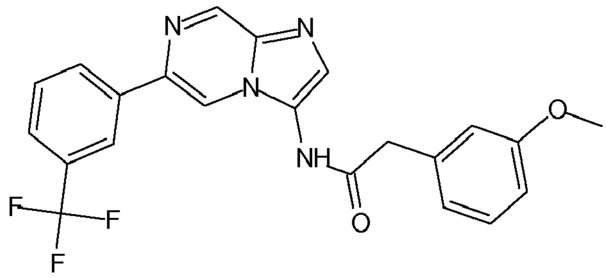

Mw.: 426.13, LCMS-ESI (*m/z*) - found 427.13 $[M+H]^+$, 425.13 $[M-H]^-$.

**Example 18-257 compound 257**

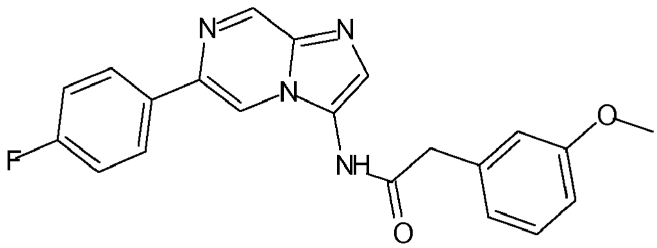

Mw.: 376.13, LCMS-ESI (*m/z*) - found 377.13 $[M+H]^+$, 375.13 $[M-H]^-$.

**Example 18-258 compound 258**

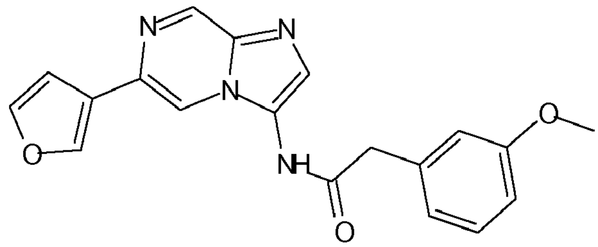

Mw.: 348.12, LCMS-ESI (*m/z*) - found 349.12 $[M+H]^+$, 347.12 $[M-H]^-$.

**Example 18-259 compound 259**

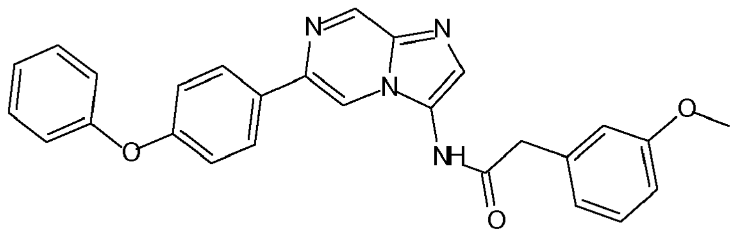

Mw.: 450.17, LCMS-ESI (*m/z*) - found 451.16 $[M+H]^+$, 449.16 $[M-H]^-$.

**Example 18-260 compound 260**

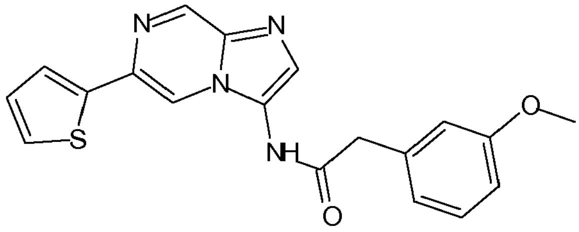

Mw.: 364.10, LCMS-ESI (*m*/*z*) - found 365.09 $[M+H]^+$, 363.09 $[M-H]^-$.

**Example 18-261 compound 261**

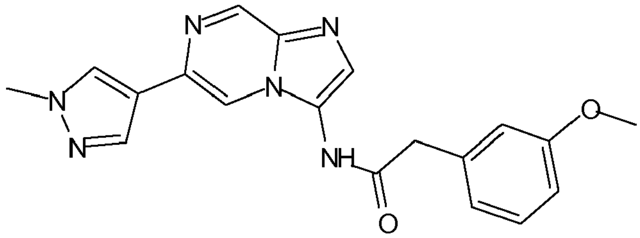

Mw.: 362.15, LCMS-ESI (*m*/*z*) - found 363.14 $[M+H]^+$, 361.14 $[M-H]^-$.

**Example 18-262 compound 262**

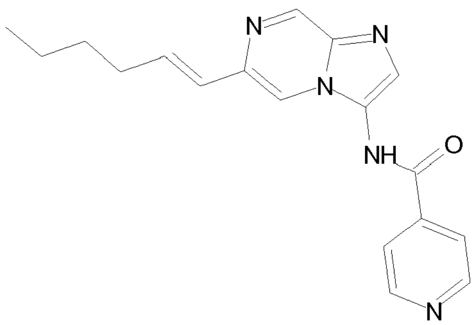

Mw.: 321.16, LCMS-ESI (*m*/*z*) - found 322.15 $[M+H]^+$, 320.15 $[M-H]^-$.

**Example 18-263 compound 263**

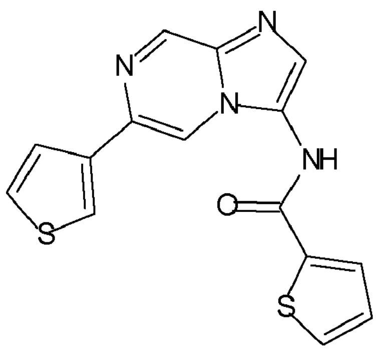

Mw.: 326.03, LCMS-ESI (*m/z*) - found 327.03 $[M+H]^+$, 325.03 $[M-H]^-$.

**Example 18-264 compound 264**

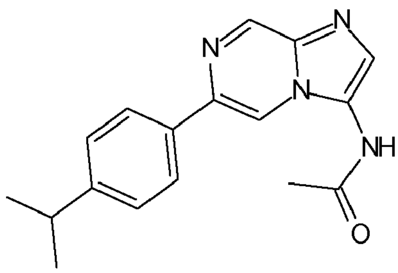

Mw.: 294.15, LCMS-ESI (*m/z*) - found 295.14 $[M+H]^+$, 293.14 $[M-H]^-$.

**Example 18-265 compound 265**

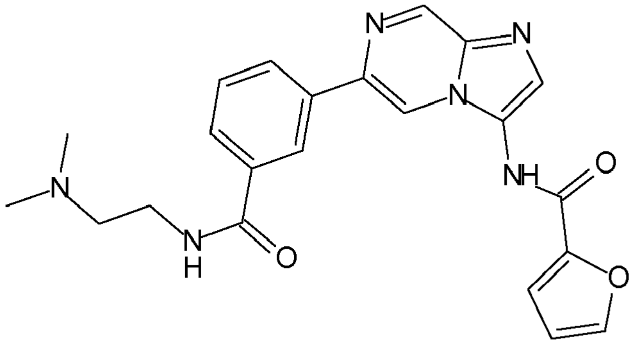

Mw.: 418.18, LCMS-ESI (*m/z*) - found 419.17 $[M+H]^+$, 417.17 $[M-H]^-$.

**Example 18-266 compound 266**

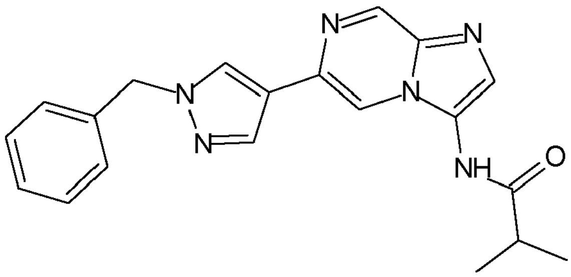

Mw.: 360.17, LCMS-ESI (*m/z*) - found 361.17 $[M+H]^+$, 359.17 $[M-H]^-$.

**Example 18-267 compound 267**

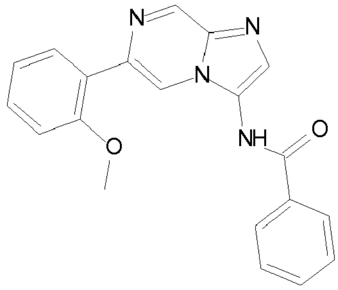

Mw.: 344.13, LCMS-ESI (*m/z*) - found 345.12 $[M+H]^+$, 343.12 $[M-H]^-$.

**Example 18-268 compound 268**

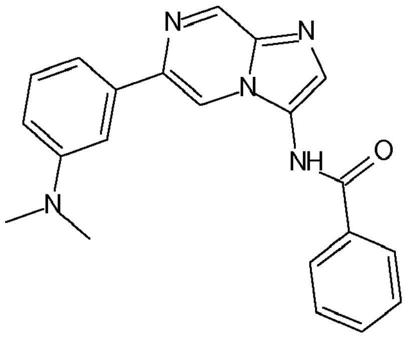

Mw.: 357.16, LCMS-ESI (*m/z*) - found 358.15 $[M+H]^+$, 356.15 $[M-H]^-$.

**Example 18-269 compound 269**

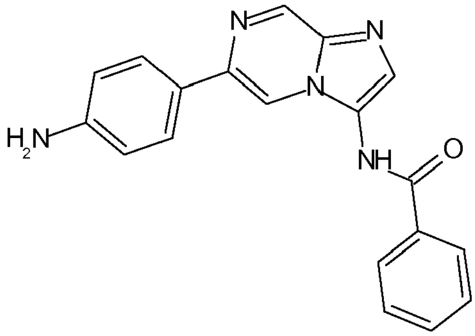

Mw.: 329.13, LCMS-ESI (*m/z*) - found 330.12 $[M+H]^+$, 328.12 $[M-H]^-$.

**Example 18-270 compound 270**

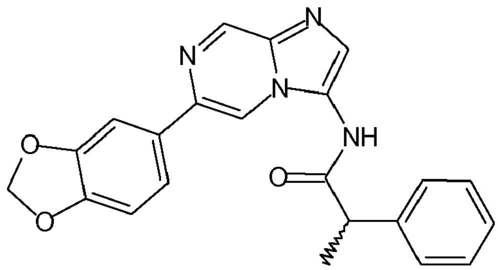

Mw.: 386.14, LCMS-ESI (*m/z*) - found 387.13 $[M+H]^+$, 385.13 $[M-H]^-$.

**Example 18-271 compound 271**

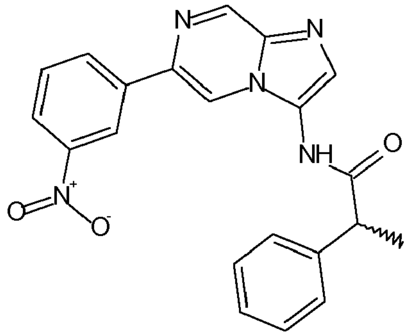

Mw.: 387.13, LCMS-ESI (*m/z*) - found 388.13 $[M+H]^+$, 386.13 $[M-H]^-$.

**Example 18-272 compound 272**

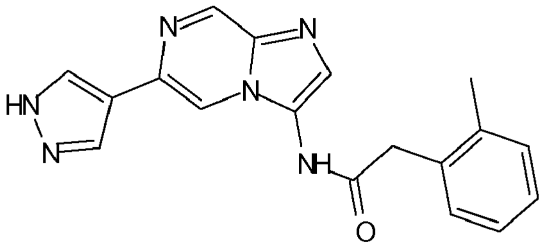

Mw.: 332.14, LCMS-ESI (*m/z*) - found 333.13 $[M+H]^+$, 331.13 $[M-H]^-$.

**Example 18-273 compound 273**

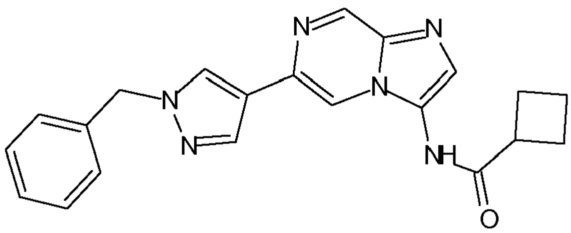

Mw.: 372.17, LCMS-ESI (*m/z*) - found 373.17 $[M+H]^+$, 371.17 $[M-H]^-$.

**Example 18-274 compound 274**

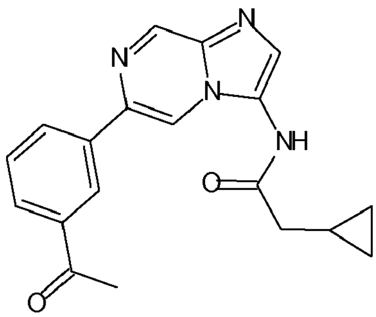

Mw.: 334.14, LCMS-ESI (*m/z*) - found 335.14 $[M+H]^+$, 333.14 $[M-H]^-$.

**Example 18-275 compound 275**

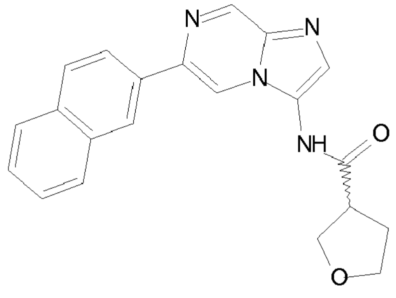

Mw.: 358.14, LCMS-ESI (*m*/*z*) - found 359.14 $[M+H]^+$, 357.14 $[M-H]^-$.

**Example 18-276 compound 276**

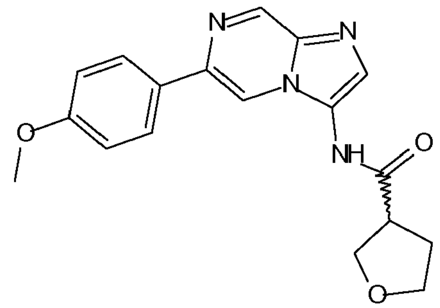

Mw.: 338.14, LCMS-ESI (*m*/*z*) - found 339.13 $[M+H]^+$, 337.13 $[M-H]^-$.

**Example 18-277 compound 277**

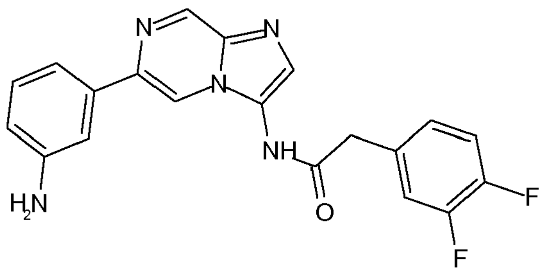

Mw.: 379.12, LCMS-ESI (*m*/*z*) - found 380.12 $[M+H]^+$, 378.12 $[M-H]^-$.

**Example 18-278 compound 278**

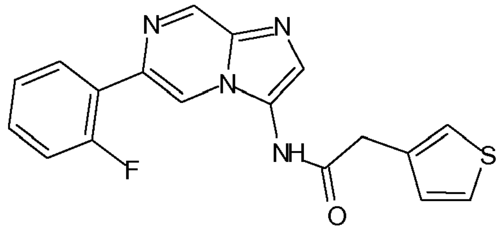

Mw.: 352.08, LCMS-ESI (*m*/*z*) - found 353.07 $[M+H]^+$, 351.07 $[M-H]^-$.

**Example 18-279 compound 279**

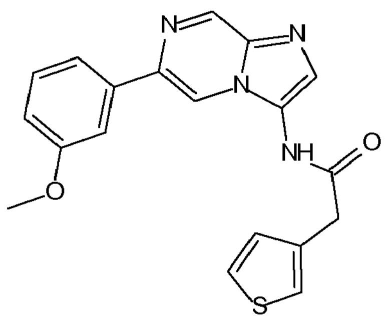

Mw.: 364.10, LCMS-ESI (*m/z*) - found 365.09 $[M+H]^+$, 363.09 $[M-H]^-$.

**Example 18-280 compound 280**

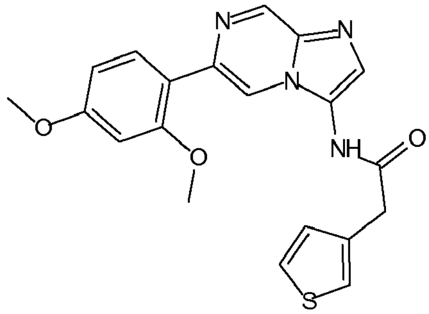

Mw.: 394.11, LCMS-ESI (*m/z*) - found 395.11 $[M+H]^+$, 393.11 $[M-H]^-$.

**Example 18-281 compound 281**

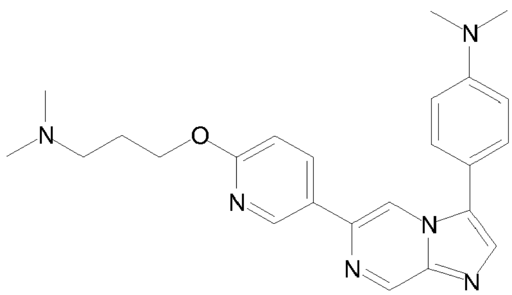

Mw.: 416.23, LCMS-ESI (*m/z*) - found 417.23 $[M+H]^+$, 415.23 $[M-H]^-$.

**Example 18-282 compound 282**

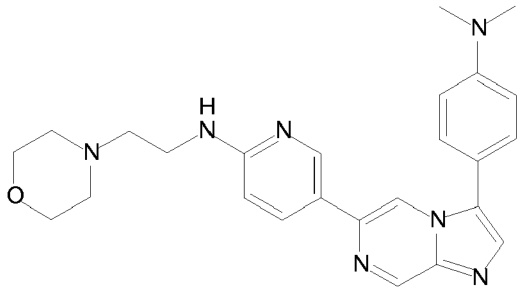

Mw.: 443.24, LCMS-ESI (*m/z*) - found 444.24 $[M+H]^+$, 442.24 $[M-H]^-$.

**Example 18-283 compound 283**

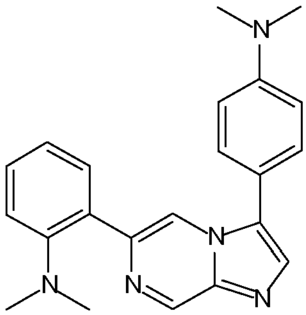

Mw.: 357.20, LCMS-ESI (*m/z*) - found 358.19 $[M+H]^+$, 356.19 $[M-H]^-$.

**Example 18-284 compound 284**

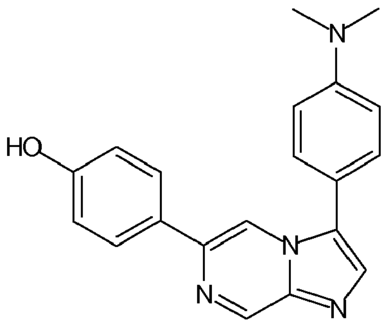

Mw.: 330.15, LCMS-ESI (*m/z*) - found 331.14 $[M+H]^+$, 329.14 $[M-H]^-$.

**Example 18-285 compound 285**

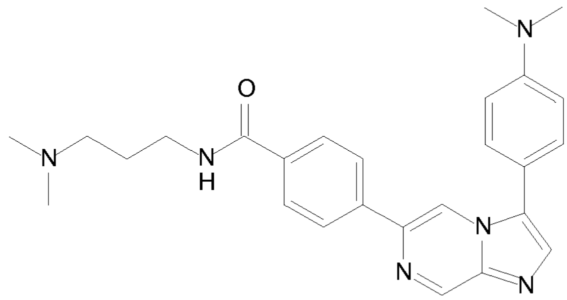

Mw.: 442.25, LCMS-ESI (*m*/*z*) - found 443.24 [M+H]$^+$, 441.24 [M-H]$^-$.

**Example 18-286 compound 286**

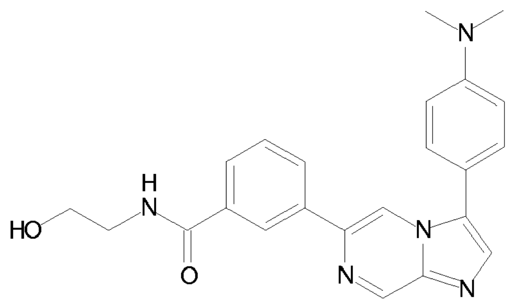

Mw.: 401.19, LCMS-ESI (*m*/*z*) - found 402.18 [M+H]$^+$, 400.18 [M-H]$^-$.

**Example 18-287 compound 287**

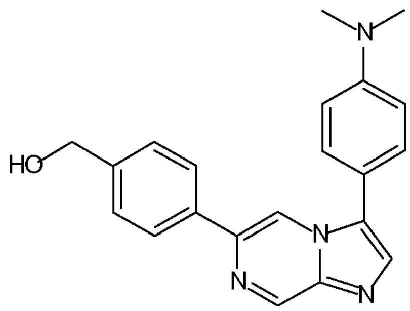

Mw.: 344.16, LCMS-ESI (*m*/*z*) - found 345.16 [M+H]$^+$, 343.16 [M-H]$^-$.

**Example 18-288 compound 288**

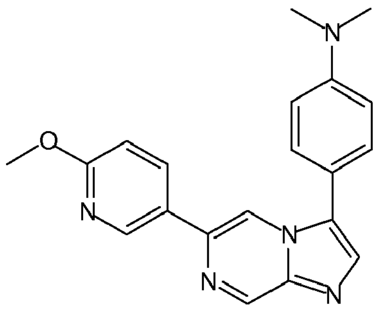

Mw.: 345.16, LCMS-ESI (*m*/*z*) - found 346.15 $[M+H]^+$, 344.15 $[M-H]^-$.

**Example 18-289 compound 289**

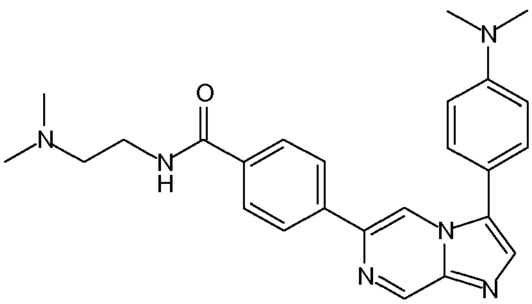

Mw.: 428.23, LCMS-ESI (*m*/*z*) - found 429.23 $[M+H]^+$, 427.23 $[M-H]^-$.

**Example 18-290 compound 290**

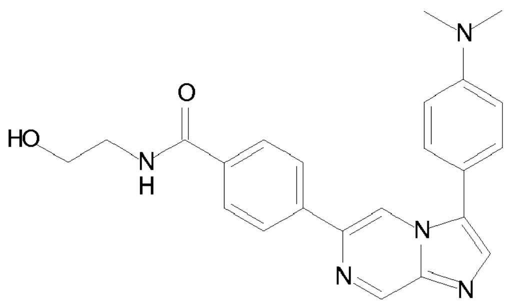

Mw.: 401.19, LCMS-ESI (*m*/*z*) - found 402.18 $[M+H]^+$, 400.18 $[M-H]^-$.

**Example 18-291 compound 291**

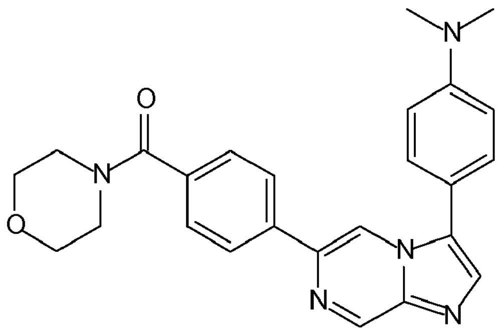

Mw.: 427.20, LCMS-ESI (*m*/*z*) - found 428.20 $[M+H]^+$, 426.20 $[M-H]^-$.

**Example 18-292 compound 292**

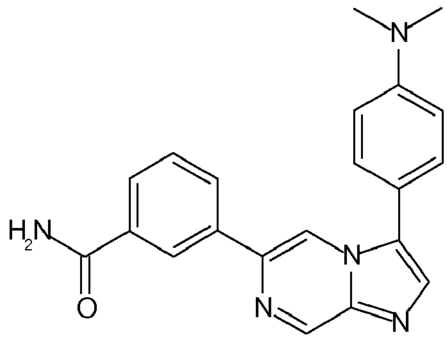

Mw.: 357.16, LCMS-ESI (*m*/*z*) - found 358.15 $[M+H]^+$, 356.15 $[M-H]^-$.

**Example 18-293 compound 293**

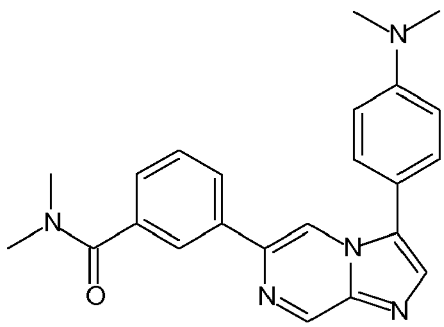

Mw.: 385.19, LCMS-ESI (*m*/*z*) - found 386.19 $[M+H]^+$, 384.19 $[M-H]^-$.

**Example 18-294 compound 294**

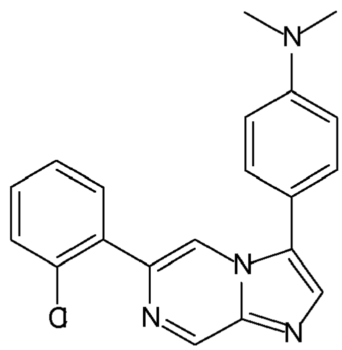

Mw.: 348.11, LCMS-ESI (*m/z*) - found 349.11 $[M+H]^+$, 347.11 $[M-H]^-$.

**Example 18-295 compound 295**

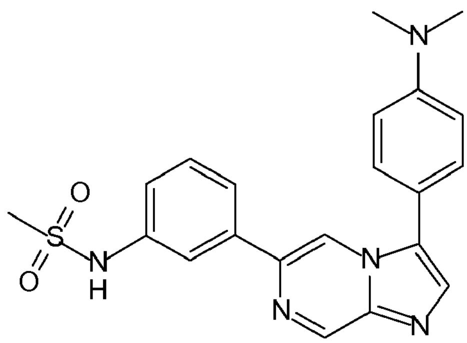

Mw.: 407.14, LCMS-ESI (*m/z*) - found 408.14 $[M+H]^+$, 406.14 $[M-H]^-$.

**Example 18-296 compound 296**

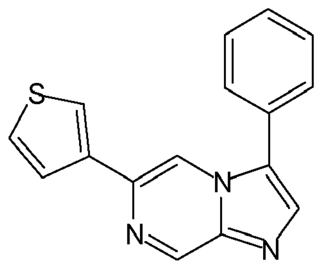

Mw.: 277.07, LCMS-ESI (*m/z*) - found 278.06 $[M+H]^+$, 276.06 $[M-H]^-$.

**Example 18-297 compound 297**

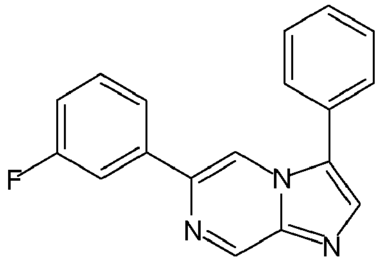

Mw.: 289.10, LCMS-ESI (*m/z*) - found 290.10 $[M+H]^+$, 288.10 $[M-H]^-$.

**Example 18-298 compound 298**

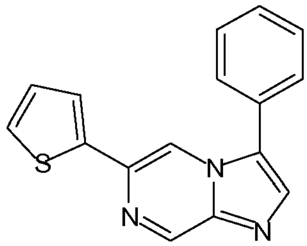

Mw.: 277.07, LCMS-ESI (*m/z*) - found 278.06 $[M+H]^+$, 276.06 $[M-H]^-$.

**Example 18-299 compound 299**

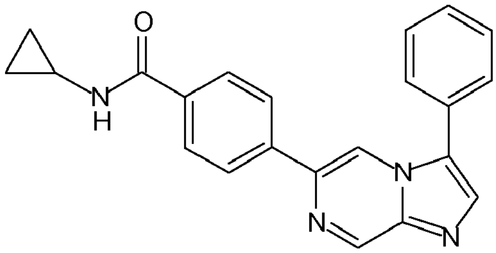

Mw.: 354.15, LCMS-ESI (*m/z*) - found 355.14 $[M+H]^+$, 353.14 $[M-H]^-$.

**Example 18-300 compound 300**

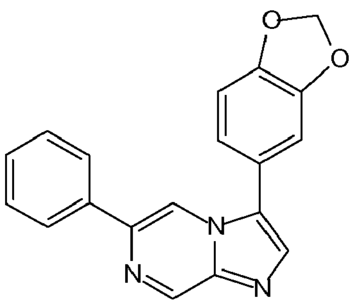

Mw.: 315.10, LCMS-ESI (*m/z*) - found 316.10 $[M+H]^+$, 314.10 $[M-H]^-$.

**Example 18-301 compound 301**

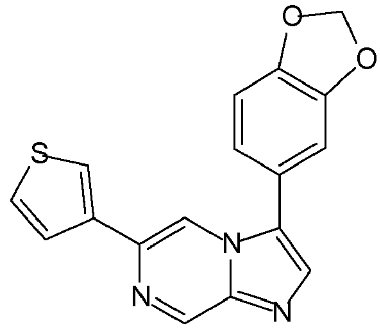

Mw.: 321.06, LCMS-ESI (*m/z*) - found 322.05 $[M+H]^+$, 320.05 $[M-H]^-$.

**Example 18-302 compound 302**

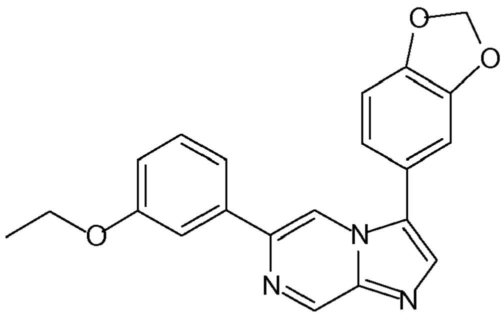

Mw.: 359.13, LCMS-ESI (*m/z*) - found 360.12 $[M+H]^+$, 358.12 $[M-H]^-$.

**Example 18-303 compound 303**

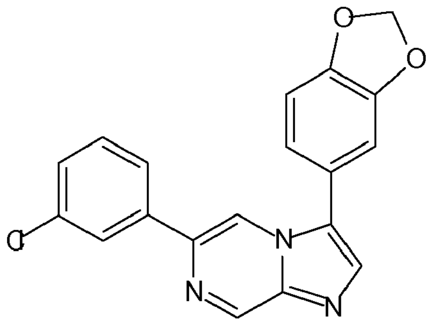

Mw.: 349.06, LCMS-ESI (*m/z*) - found 350.06 $[M+H]^+$, 348.06 $[M-H]^-$.

**Example 18-304 compound 304**

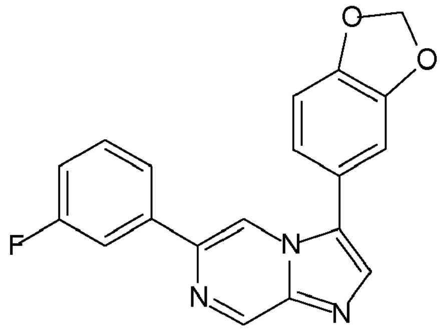

Mw.: 333.09, LCMS-ESI (*m/z*) - found 334.09 $[M+H]^+$, 332.09 $[M-H]^-$.

**Example 18-305 compound 305**

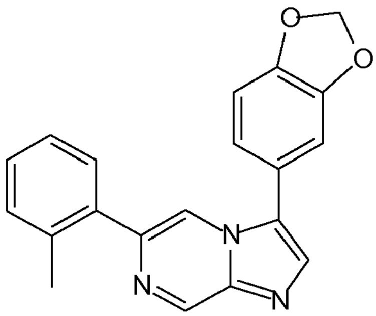

Mw.: 329.12, LCMS-ESI (*m/z*) - found 330.11 $[M+H]^+$, 328.11 $[M-H]^-$.

**Example 18-306 compound 306**

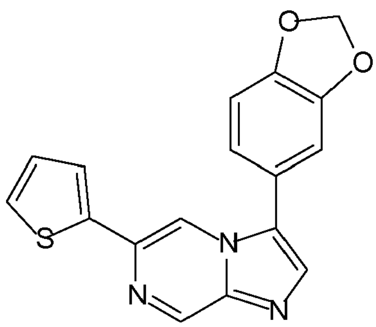

Mw.: 321.06, LCMS-ESI (*m/z*) - found 322.05 $[M+H]^+$, 320.05 $[M-H]^-$.

**Example 18-307 compound 307**

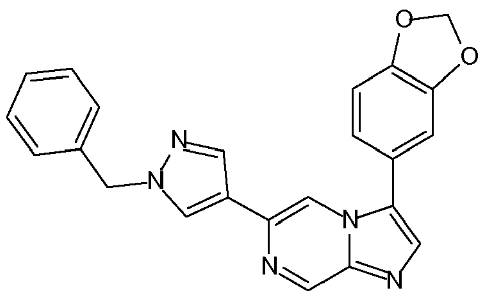

Mw.: 395.14, LCMS-ESI (*m/z*) - found 396.13 $[M+H]^+$, 394.13 $[M-H]^-$.

**Example 18-308 compound 308**

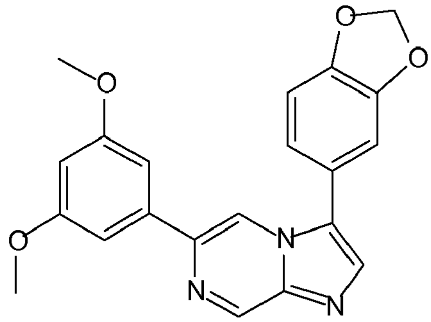

Mw.: 375.12, LCMS-ESI (*m/z*) - found 376.12 $[M+H]^+$, 374.12 $[M-H]^-$.

**Example 18-309 compound 309**

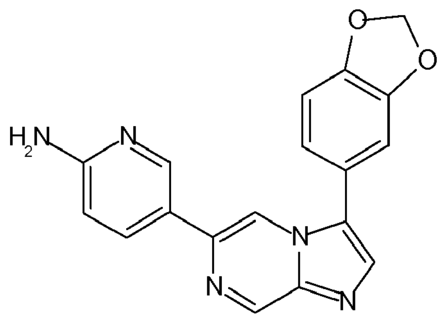

Mw.: 331.11, LCMS-ESI (*m/z*) - found 332.10 $[M+H]^+$, 330.10 $[M-H]^-$.

**Example 18-310 compound 310**

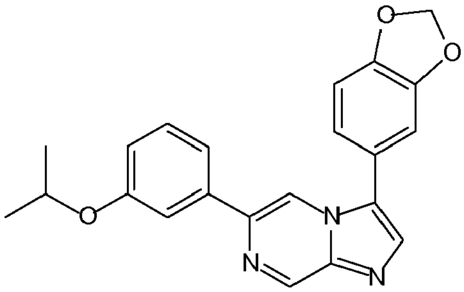

Mw.: 373.14, LCMS-ESI (*m/z*) - found 374.14 $[M+H]^+$, 372.14 $[M-H]^-$.

**Example 18-311 compound 311**

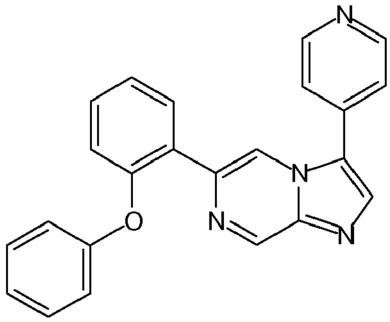

Mw.: 364.13, LCMS-ESI (*m/z*) - found 365.13 $[M+H]^+$, 365.13 $[M-H]^-$.

**Example 18-312 compound 312**

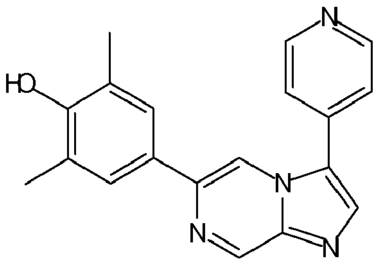

Mw.: 316.13, LCMS-ESI (*m/z*) - found 317.13 $[M+H]^+$, 315.13 $[M-H]^-$.

**Example 18-313 compound 313**

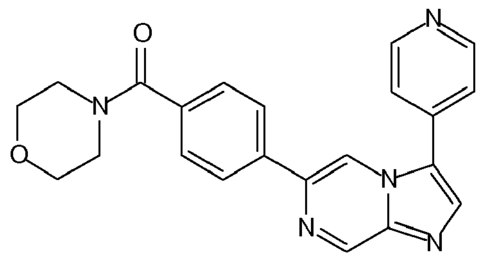

Mw.: 385.15, LCMS-ESI (*m/z*) - found 386.15 $[M+H]^+$, 384.15 $[M-H]^-$.

**Example 18-314 compound 314**

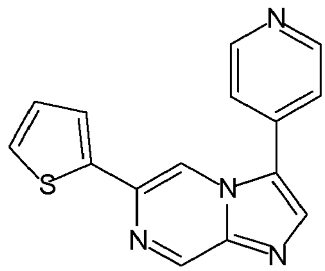

Mw.: 278.06, LCMS-ESI (*m/z*) - found 279.06 $[M+H]^+$, 277.06 $[M-H]^-$.

**Example 18-315 compound 315**

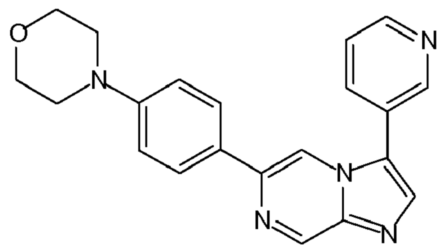

Mw.: 357.16, LCMS-ESI (*m/z*) - found 358.15 $[M+H]^+$, 356.15 $[M-H]^-$.

**Example 18-316 compound 316**

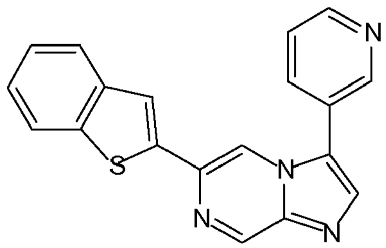

Mw.: 328.08, LCMS-ESI (*m/z*) - found 329.07 $[M+H]^+$, 327.07 $[M-H]^-$.

**Example 18-317 compound 317**

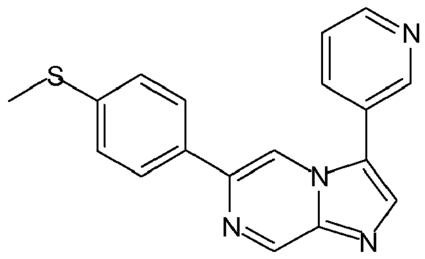

Mw.: 318.09, LCMS-ESI (*m/z*) - found 319.09 $[M+H]^+$, 317.09 $[M-H]^-$.

**Example 18-318 compound 318**

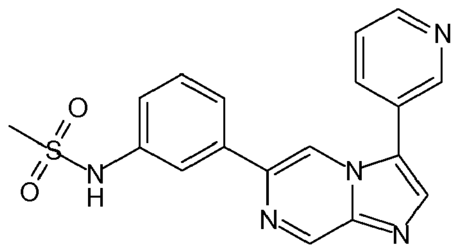

Mw.: 365.09, LCMS-ESI (*m/z*) - found 366.09 $[M+H]^+$, 364.09 $[M-H]^-$.

**Example 18-319 compound 319**

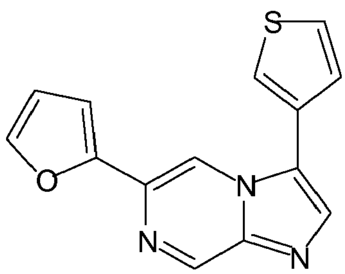

Mw.: 267.05, LCMS-ESI (*m/z*) - found 268.04 $[M+H]^+$, 266.04 $[M-H]^-$.

**Example 18-320 compound 320**

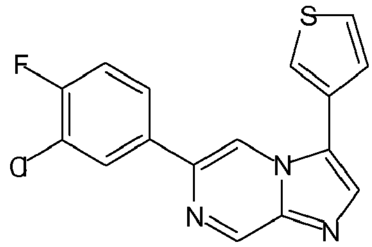

Mw.: 329.02, LCMS-ESI (*m*/*z*) - found 330.01 $[M+H]^+$, 328.01 $[M-H]^-$.

**Example 18-321 compound 321**

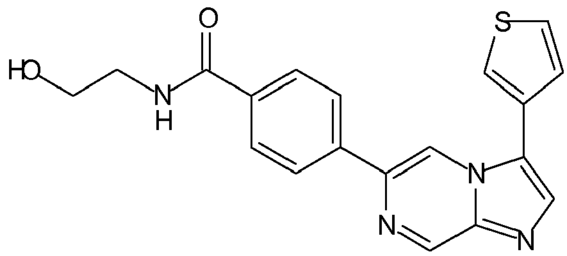

Mw.: 364.10, LCMS-ESI (*m*/*z*) - found 365.09 $[M+H]^+$, 363.09 $[M-H]^-$.

**Example 18-322 compound 322**

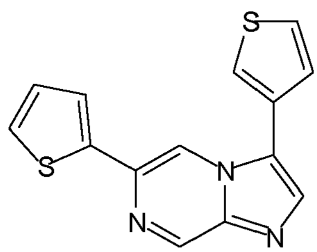

Mw.: 283.02, LCMS-ESI (*m*/*z*) - found 284.02 $[M+H]^+$, 282.02 $[M-H]^-$.

**Example 18-323 compound 323**

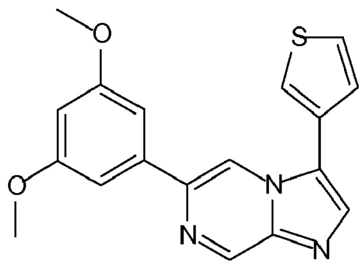

Mw.: 337.09, LCMS-ESI (*m*/*z*) - found 338.08 $[M+H]^+$, 336.08 $[M-H]^-$.

**Example 18-324 compound 324**

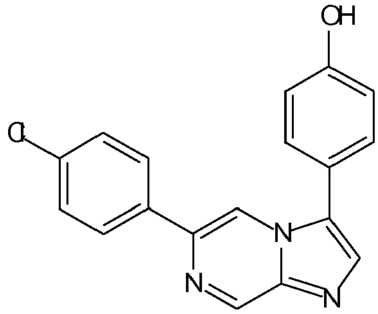

Mw.: 321.07, LCMS-ESI (*m/z*) - found 322.06 $[M+H]^+$, 320.06 $[M-H]^-$.

**Example 18-325 compound 325**

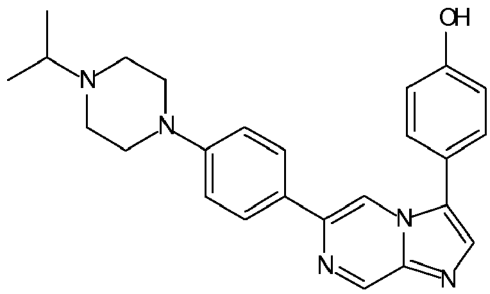

Mw.: 413.22, LCMS-ESI (*m/z*) - found 414.22 $[M+H]^+$, 412.22 $[M-H]^-$.

**Example 18-326 compound 326**

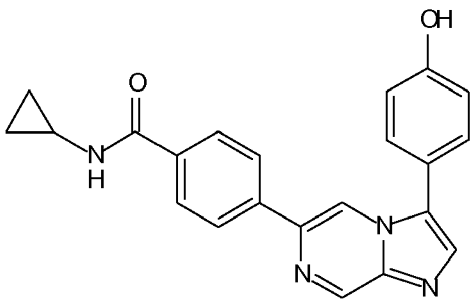

Mw.: 370.14, LCMS-ESI (*m/z*) - found 371.14 $[M+H]^+$, 369.14 $[M-H]^-$.

**Example 18-327 compound 327**

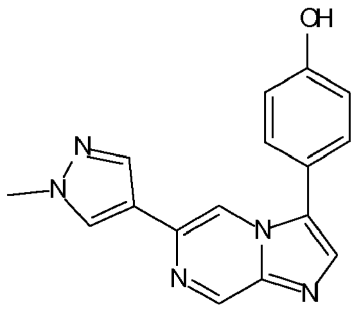

Mw.: 291.11, LCMS-ESI (*m/z*) - found 292.11 $[M+H]^+$, 290.11 $[M-H]^-$.

**Example 18-328 compound 328**

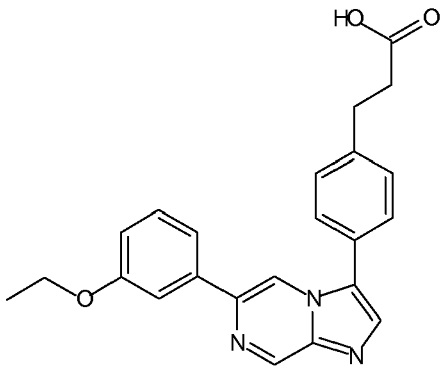

Mw.: 387.16, LCMS-ESI (*m/z*) - found 388.15 $[M+H]^+$, 386.15 $[M-H]^-$.

**Example 18-329 compound 329**

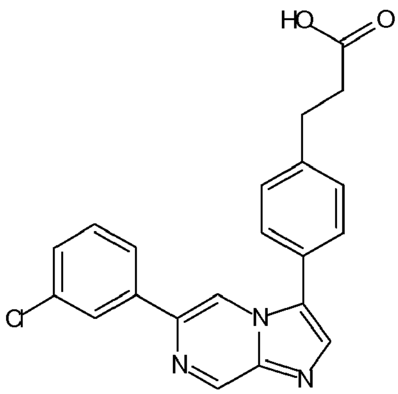

Mw.: 377.09, LCMS-ESI (*m/z*) - found 378.09 $[M+H]^+$, 376.09 $[M-H]^-$.

**Example 18-330 compound 330**

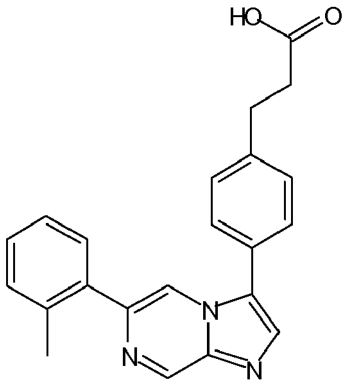

Mw.: 357.15, LCMS-ESI (*m*/*z*) - found 358.14 $[M+H]^+$, 356.14 $[M-H]^-$.

**Example 18-331 compound 331**

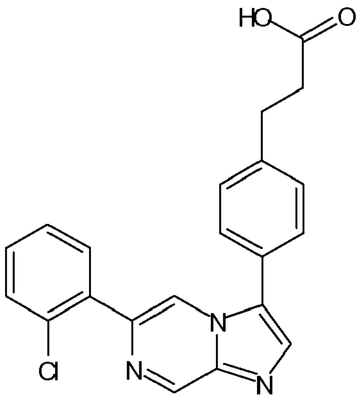

Mw.: 377.09, LCMS-ESI (*m*/*z*) - found 378.09 $[M+H]^+$, 376.09 $[M-H]^-$.

**Example 18-332 compound 332**

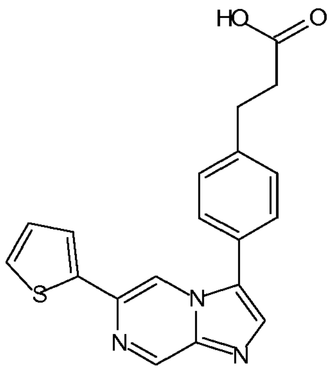

Mw.: 349.09, LCMS-ESI (*m*/*z*) - found 350.09 $[M+H]^+$, 348.08 $[M-H]^-$.

**Example 18-333 compound 333**

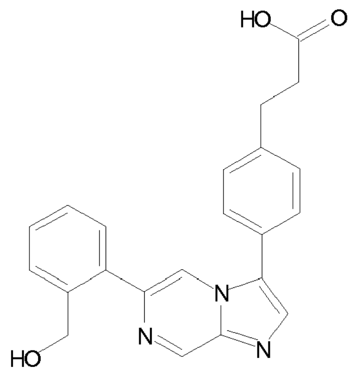

Mw.: 373.14, LCMS-ESI (*m/z*) - found 374.14 $[M+H]^+$, 372.14 $[M-H]^-$.

**Example 18-334 compound 334**

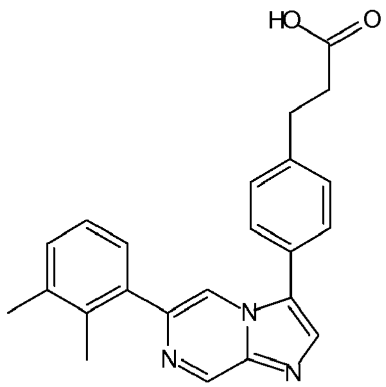

Mw.: 371.16, LCMS-ESI (*m/z*) - found 372.16 $[M+H]^+$, 370.16 $[M-H]^-$.

**Example 18-335 compound 335**

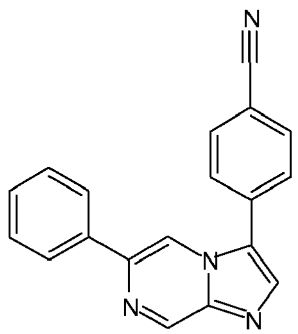

Mw.: 296.11, LCMS-ESI (*m/z*) - found 297.10 $[M+H]^+$, 295.10 $[M-H]^-$.

**Example 18-336 compound 336**

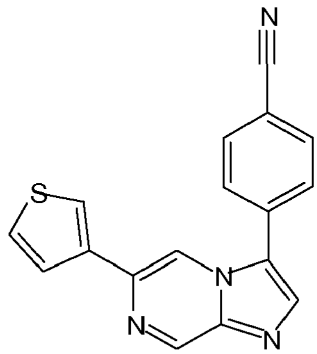

Mw.: 302.06, LCMS-ESI (*m/z*) - found 303.06 $[M+H]^+$, 301.06 $[M-H]^-$.

**Example 18-337 compound 337**

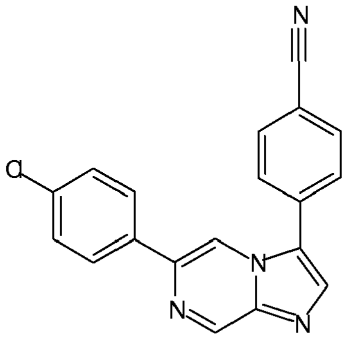

Mw.: 330.07, LCMS-ESI (*m/z*) - found 331.06 $[M+H]^+$, 329.06 $[M-H]^-$.

**Example 18-338 compound 338**

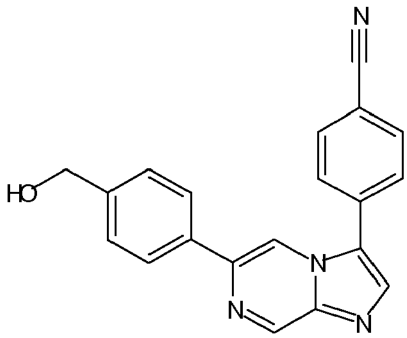

Mw.: 326.12, LCMS-ESI (*m/z*) - found 327.11 $[M+H]^+$, 325.11 $[M-H]^-$.

**Example 18-339 compound 339**

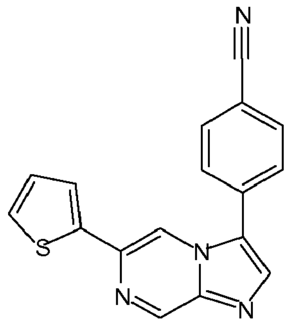

Mw.: 302.06, LCMS-ESI (*m*/*z*) - found 303.06 $[M+H]^+$, 301.06 $[M-H]^-$.

**Example 18-340 compound 340**

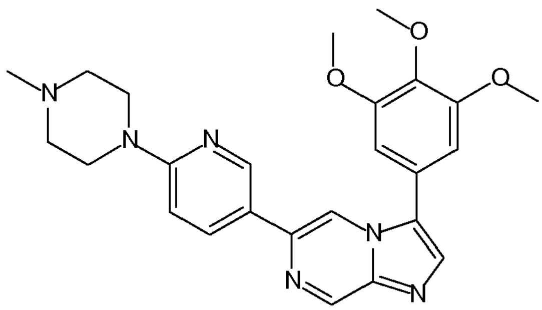

Mw.: 460.22, LCMS-ESI (*m*/*z*) - found 461.22 $[M+H]^+$, 459.22 $[M-H]^-$.

**Example 18-341 compound 341**

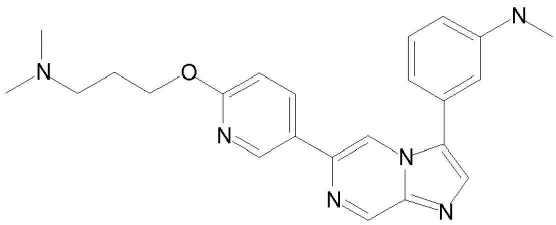

Mw.: 416.23, LCMS-ESI (*m*/*z*) - found 417.23 $[M+H]^+$, 415.23 $[M-H]^-$.

**Example 18-342 compound 342**

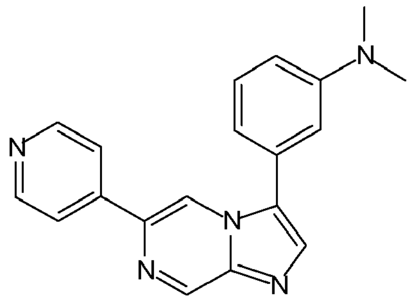

Mw.: 315.15, LCMS-ESI (*m*/*z*) - found 316.14 $[M+H]^+$, 314.14 $[M-H]^-$.

**Example 18-343 compound 343**

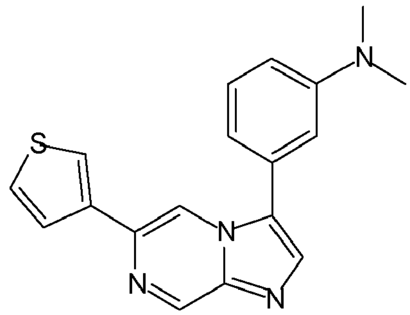

Mw.: 320.11, LCMS-ESI (*m*/*z*) - found 321.11 $[M+H]^+$, 319.11 $[M-H]^-$.

**Example 18-344 compound 344**

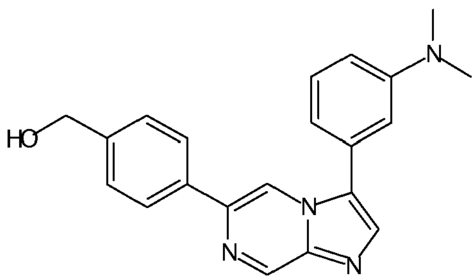

Mw.: 344.16, LCMS-ESI (*m*/*z*) - found 345.16 $[M+H]^+$, 343.16 $[M-H]^-$.

**Example 18-345 compound 345**

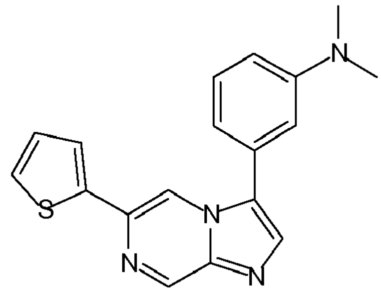

Mw.: 320.11, LCMS-ESI (*m*/*z*) - found 321.11 $[M+H]^+$, 319.11 $[M-H]^-$.

**Example 18-346 compound 346**

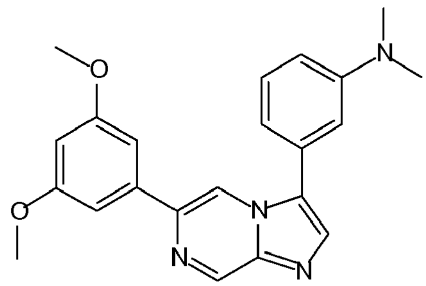

Mw.: 374.17, LCMS-ESI (*m*/*z*) - found 375.17 $[M+H]^+$, 373.17 $[M-H]^-$.

**Example 18-347 compound 347**

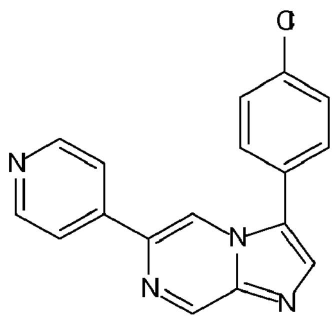

Mw.: 306.07, LCMS-ESI (*m*/*z*) - found 307.06 $[M+H]^+$, 305.06 $[M-H]^-$.

**Example 18-348 compound 348**

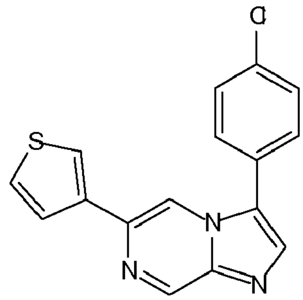

Mw.: 311.03, LCMS-ESI (*m*/*z*) - found 312.02 $[M+H]^+$, 310.02 $[M-H]^-$.

**Example 18-349 compound 349**

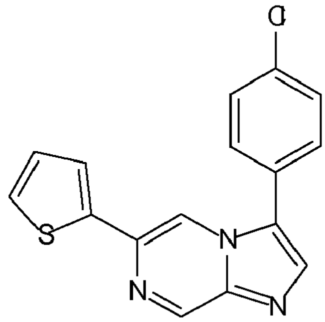

Mw.: 311.03, LCMS-ESI (*m*/*z*) - found 312.02 $[M+H]^+$, 310.02 $[M-H]^-$.

**Example 18-350 compound 350**

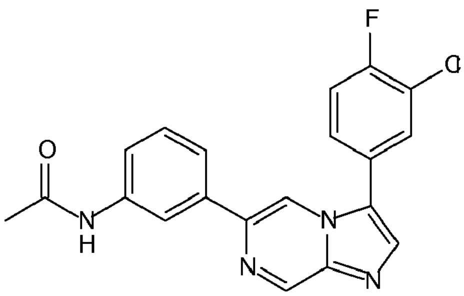

Mw.: 380.08, LCMS-ESI (*m*/*z*) - found 381.08 $[M+H]^+$, 379.08 $[M-H]^-$.

**Example 18-351 compound 351**

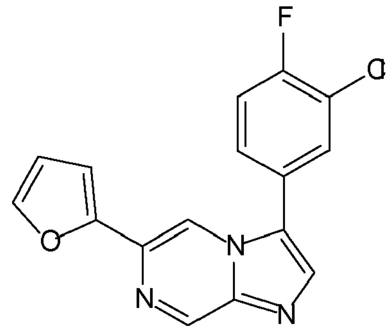

Mw.: 313.04, LCMS-ESI (*m/z*) - found 314.04 $[M+H]^+$, 312.04 $[M-H]^-$.

**Example 18-352 compound 352**

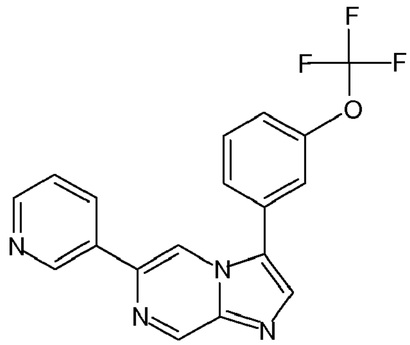

Mw.: 356.09, LCMS-ESI (*m/z*) - found 357.08 $[M+H]^+$, 355.08 $[M-H]^-$.

**Example 18-353 compound 353**

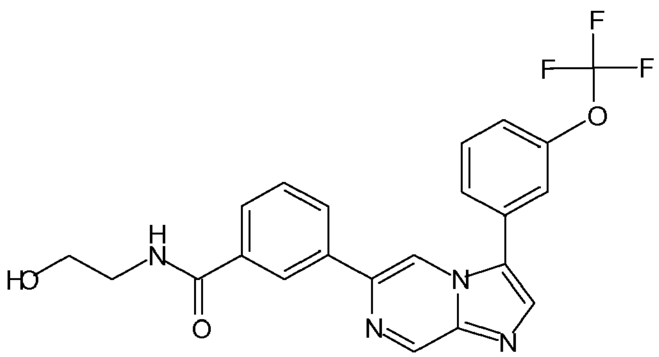

Mw.: 442.13, LCMS-ESI (*m/z*) - found 443.12 $[M+H]^+$, 441.12 $[M-H]^-$.

**Example 18-354 compound 354**

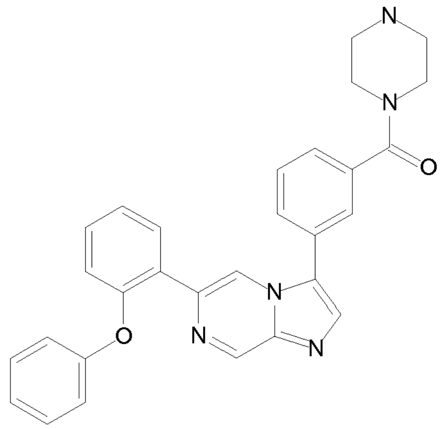

Mw.: 489.22, LCMS-ESI (*m*/*z*) - found 490.21 $[M+H]^+$, 488.21 $[M-H]^-$.

**Example 18-355 compound 355**

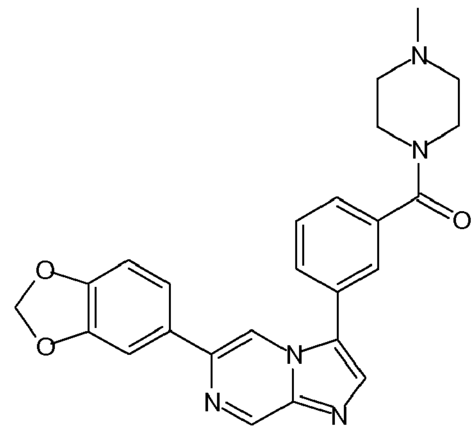

Mw.: 441.18, LCMS-ESI (*m*/*z*) - found 442.18 $[M+H]^+$, 440.18 $[M-H]^-$.

**Example 18-356 compound 356**

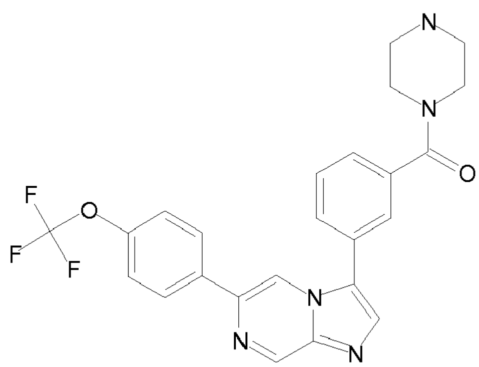

Mw.: 481.17, LCMS-ESI (*m*/*z*) - found 482.17 [M+H.

**Example 18-357 compound 357**

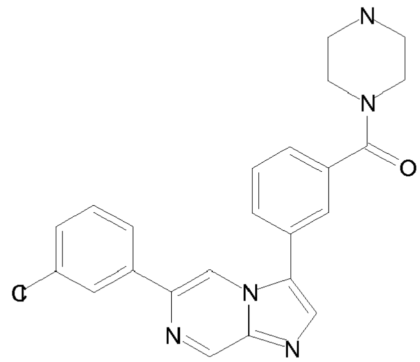

Mw.: 431.15, LCMS-ESI (*m/z*) - found 432.15 $[M+H]^+$, 430.15 $[M-H]^-$.

**Example 18-358 compound 358**

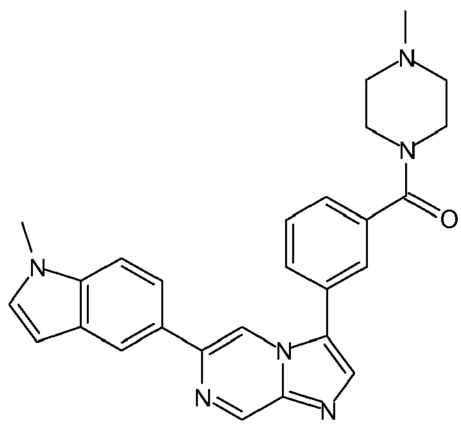

Mw.: 450.22, LCMS-ESI (*m/z*) - found 451.21 $[M+H]^+$, 449.21 $[M-H]^-$.

**Example 18-359 compound 359**

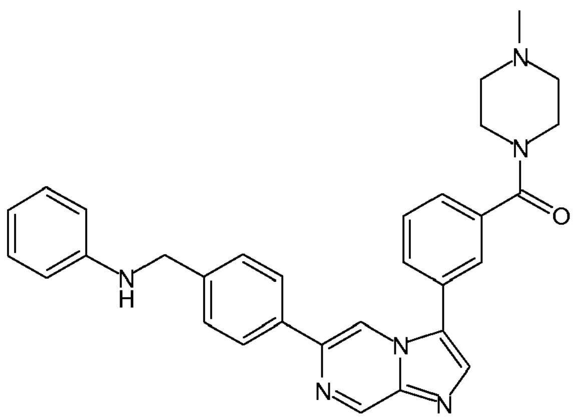

Mw.: 502.25, LCMS-ESI (*m/z*) - found 503.24 $[M+H]^+$, 501.24 $[M-H]^-$.

**Example 18-360 compound 360**

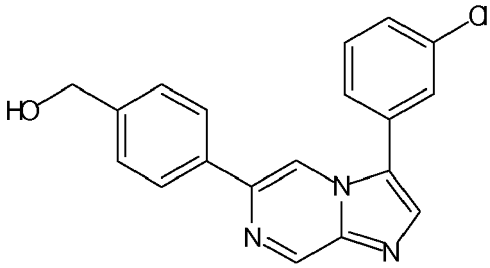

Mw.: 335.08, LCMS-ESI (*m/z*) - found 336.08 $[M+H]^+$, 334.08 $[M-H]^-$.

**Example 18-361 compound 361**

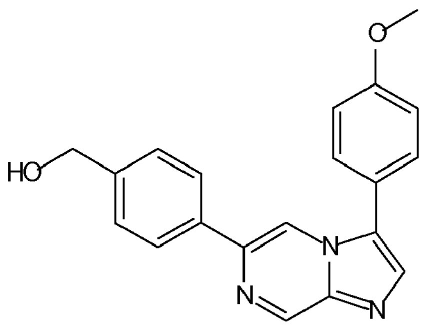

Mw.: 331.13, LCMS-ESI (*m/z*) - found 332.13 $[M+H]^+$, 330.13 $[M-H]^-$.

**Example 18-362 compound 362**

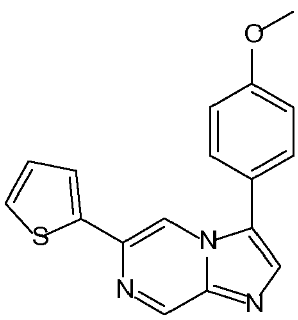

Mw.: 307.08, LCMS-ESI (*m/z*) - found 308.07 $[M+H]^+$, 306.07 $[M-H]^-$.

**Example 18-363 compound 363**

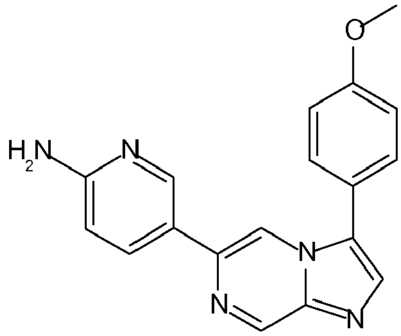

Mw.: 317.13, LCMS-ESI (*m/z*) - found 318.12 $[M+H]^+$, 316.12 $[M-H]^-$.

**Example 18-364 compound 364**

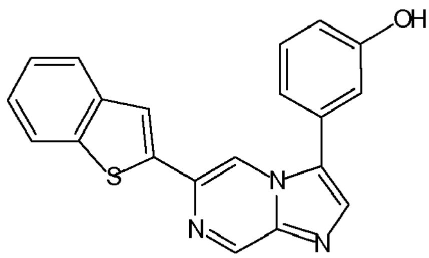

Mw.: 343.08, LCMS-ESI (*m/z*) - found 344.07 $[M+H]^+$, 342.07 $[M-H]^-$.

**Example 18-365 compound 365**

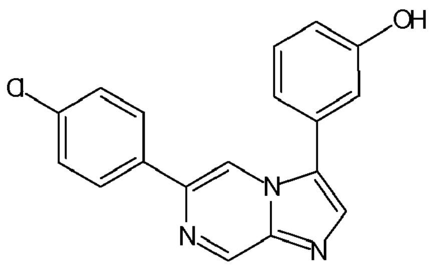

Mw.: 321.07, LCMS-ESI (*m/z*) - found 322.06 $[M+H]^+$, 4320.06 $[M-H]^-$.

**Example 18-366 compound 366**

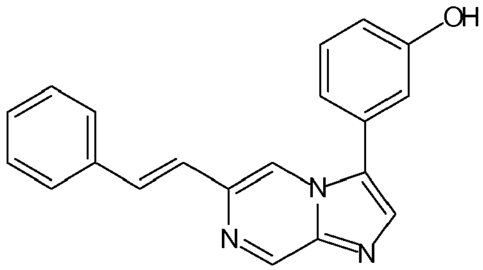

Mw.: 313.12, LCMS-ESI (*m/z*) - found 314.12 $[M+H]^+$, 312.12 $[M-H]^-$.

**Example 18-367 compound 367**

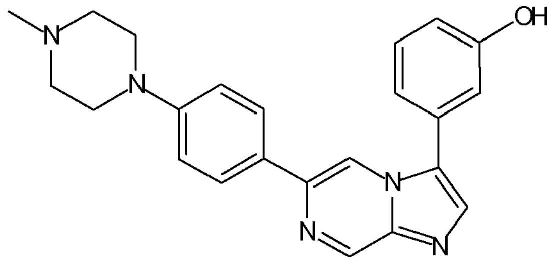

Mw.: 385.19, LCMS-ESI (*m/z*) - found 386.19 $[M+H]^+$, 384.19 $[M-H]^-$.

**Example 18-368 compound 368**

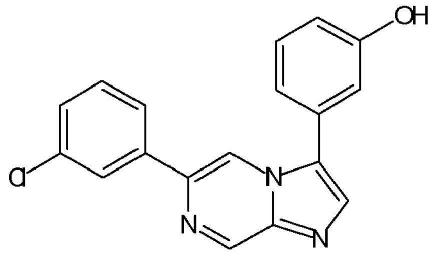

Mw.: 321.07, LCMS-ESI (*m/z*) - found 322.06$[M+H]^+$, 320.06 $[M-H]^-$.

**Example 18-369 compound 369**

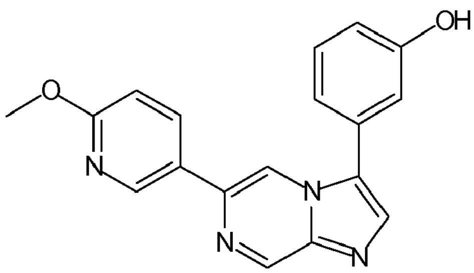

Mw.: 318.11, LCMS-ESI (*m/z*) - found 319.11 $[M+H]^+$, 317.11 $[M-H]^-$.

**Example 18-370 compound 370**

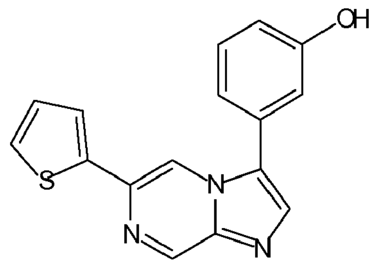

Mw.: 293.06, LCMS-ESI (*m/z*) - found 294.06 $[M+H]^+$, 292.06 $[M-H]^-$.

**Example 18-371 compound 371**

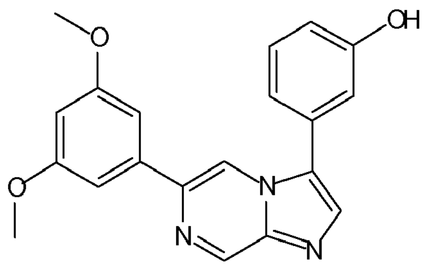

Mw.: 347.13, LCMS-ESI (*m/z*) - found 348.12 $[M+H]^+$, 346.12 $[M-H]^-$.

**Example 18-372 compound 372**

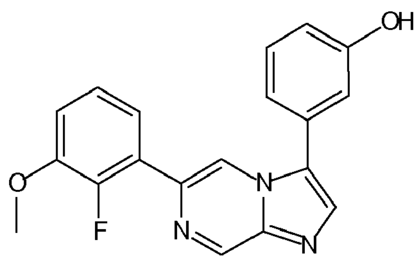

Mw.: 335.11, LCMS-ESI (*m/z*) - found 336.10 $[M+H]^+$, 334.10 $[M-H]^-$.

**Example 18-373 compound 373**

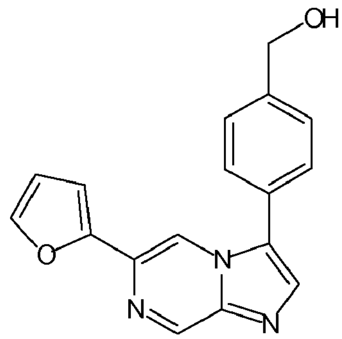

Mw.: 291.10, LCMS-ESI (*m*/*z*) - found 292.10 $[M+H]^+$, 290.10 $[M-H]^-$.

**Example 18-374 compound 374**

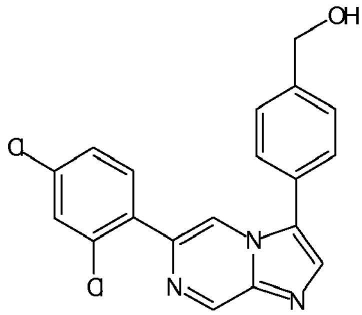

Mw.: 369.04, LCMS-ESI (*m*/*z*) - found 370.04 $[M+H]^+$, 368.04 $[M-H]^-$.

**Example 18-375 compound 375**

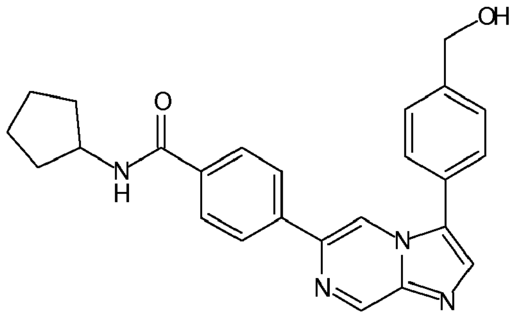

Mw.: 412.19, LCMS-ESI (*m*/*z*) - found 413.19 $[M+H]^+$, 411.19 $[M-H]^-$.

**Example 18-376 compound 376**

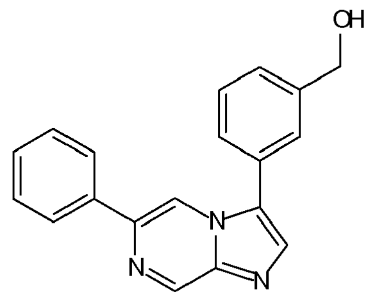

Mw.: 301.12, LCMS-ESI (*m/z*) - found 302.12 $[M+H]^+$, 300.12 $[M-H]^-$.

**Example 18-377 compound 377**

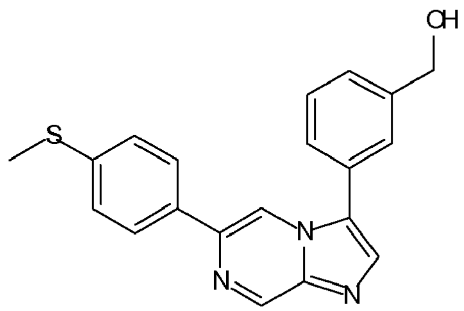

Mw.: 347.11, LCMS-ESI (*m/z*) - found 348.10 $[M+H]^+$, 346.10 $[M-H]^-$.

**Example 18-378 compound 378**

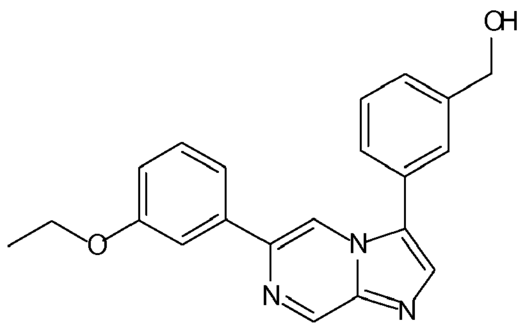

Mw.: 345.15, LCMS-ESI (*m/z*) - found 346.14 $[M+H]^+$, 344.14 $[M-H]^-$.

**Example 18-379 compound 379**

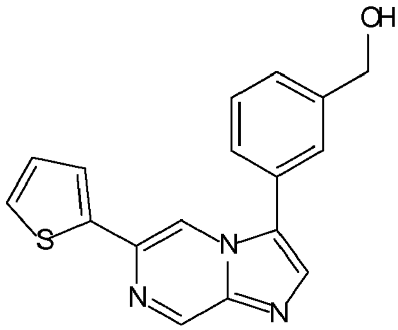

Mw.: 307.08, LCMS-ESI (*m/z*) - found 308.07 $[M+H]^+$, 306.07 $[M-H]^-$.

**Example 18-380 compound 380**

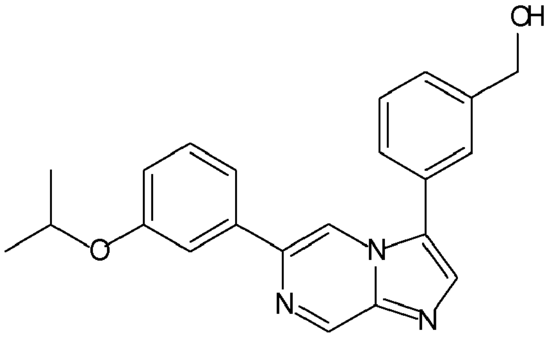

Mw.: 359.16, LCMS-ESI (*m/z*) - found 360.16 $[M+H]^+$, 358.16 $[M-H]^-$.

**Example 18-381 compound 381**

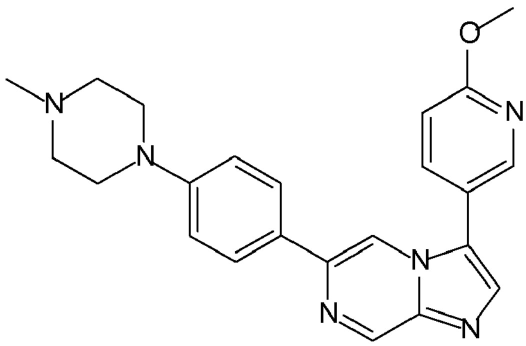

Mw.: 400.20, LCMS-ESI (*m/z*) - found 401.20 $[M+H]^+$, 399.20 $[M-H]^-$.

**Example 18-382 compound 382**

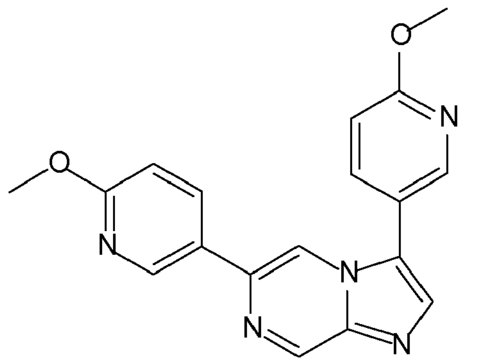

Mw.: 333.12, LCMS-ESI (*m/z*) - found 334.12 $[M+H]^+$, 332.12 $[M-H]^-$.

**Example 18-383 compound 383**

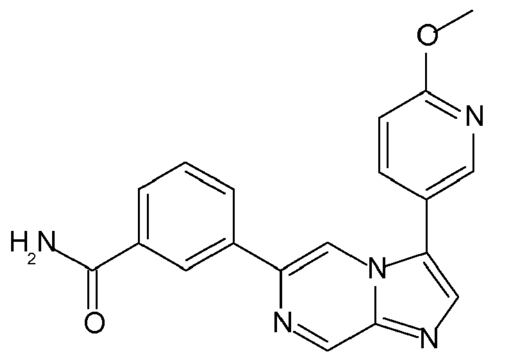

Mw.: 345.12, LCMS-ESI (*m/z*) - found 346.12 $[M+H]^+$, 344.12 $[M-H]^-$.

**Example 18-384 compound 384**

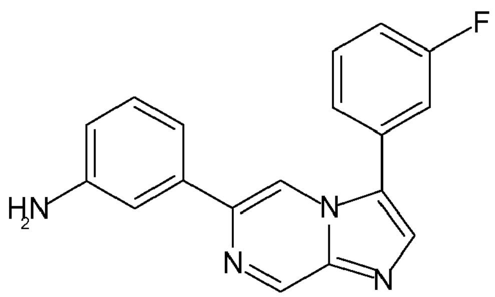

Mw.: 304.11, LCMS-ESI (*m/z*) - found 305.11 $[M+H]^+$, 303.11 $[M-H]^-$.

**Example 18-385 compound 385**

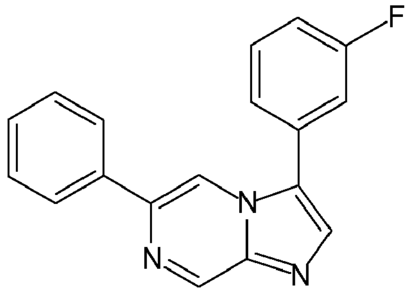

Mw.: 289.10, LCMS-ESI (*m*/*z*) - found 290.10 $[M+H]^+$, 288.10 $[M-H]^-$.

**Example 18-386 compound 386**

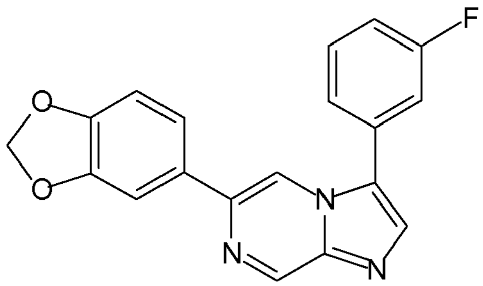

Mw.: 333.09, LCMS-ESI (*m*/*z*) - found 334.09 $[M+H]^+$, 332.09 $[M-H]^-$.

**Example 18-387 compound 387**

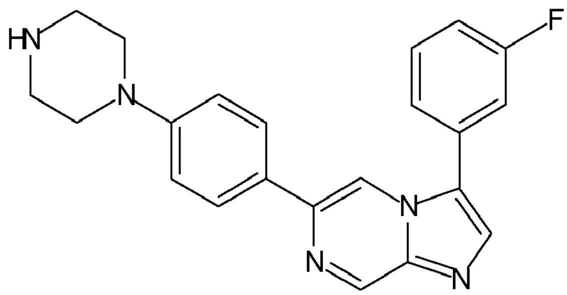

Mw.: 373.17, LCMS-ESI (*m*/*z*) - found 374.17 $[M+H]^+$, 372.17 $[M-H]^-$.

**Example 18-388 compound 388**

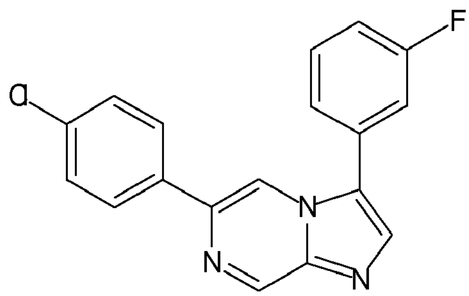

Mw.: 323.06, LCMS-ESI (*m/z*) - found 324.06 $[M+H]^+$, 322.06 $[M-H]^-$.

**Example 18-389 compound 389**

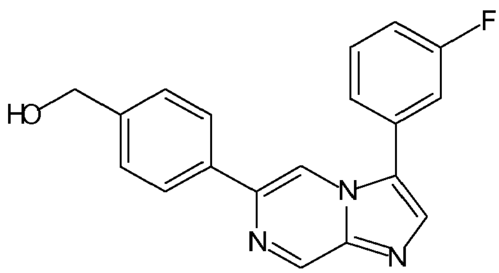

Mw.: 319.11, LCMS-ESI (*m/z*) - found 320.11 $[M+H]^+$, 318.11 $[M-H]^-$.

**Example 18-390 compound 390**

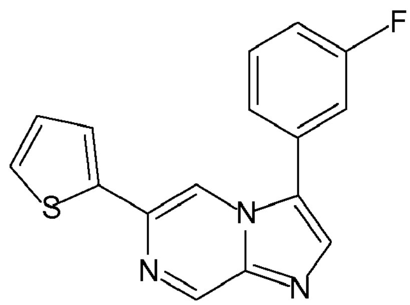

Mw.: 295.06, LCMS-ESI (*m/z*) - found 296.05 $[M+H]^+$, 294.05 $[M-H]^-$.

**Example 18-391 compound 391**

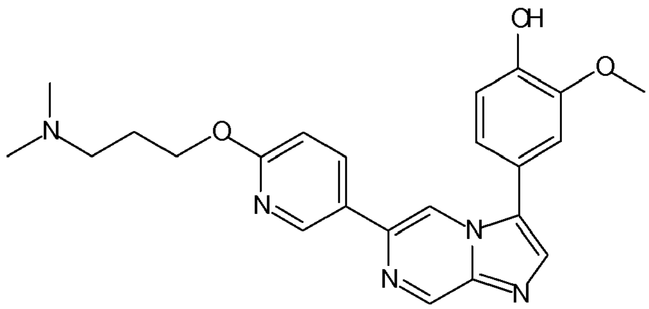

Mw.: 419.20, LCMS-ESI (*m/z*) - found 420.19 $[M+H]^+$, 418.19 $[M-H]^-$.

**Example 18-392 compound 392**

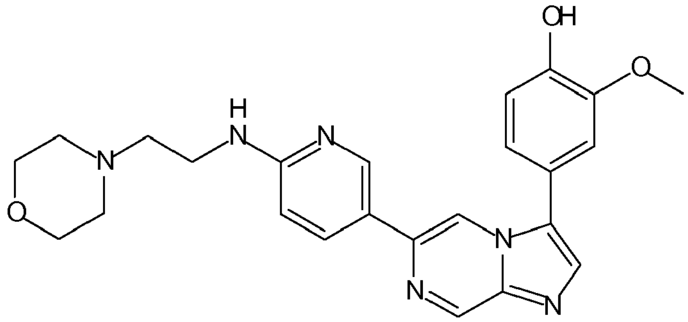

Mw.: 446.21, LCMS-ESI (*m/z*) - found 447.20 $[M+H]^+$, 445.20 $[M-H]^-$.

**Example 18-393 compound 393**

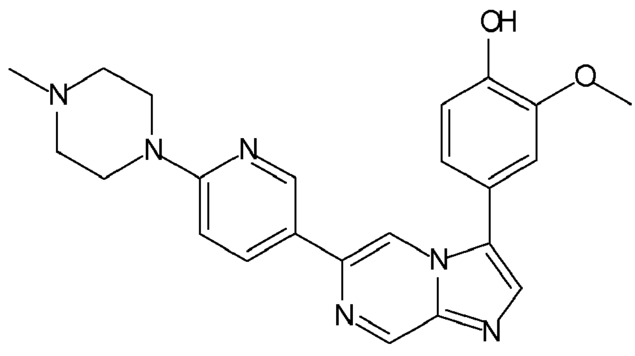

Mw.: 416.20, LCMS-ESI (*m/z*) - found 417.19 $[M+H]^+$, 415.19 $[M-H]^-$.

**Example 18-394 compound 394**

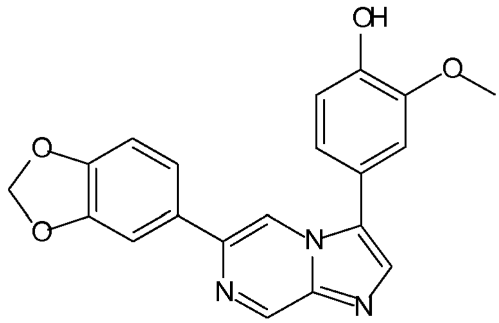

Mw.: 361.11, LCMS-ESI (*m*/*z*) - found 362.10 $[M+H]^+$, 360.10 $[M-H]^-$.

**Example 18-395 compound 395**

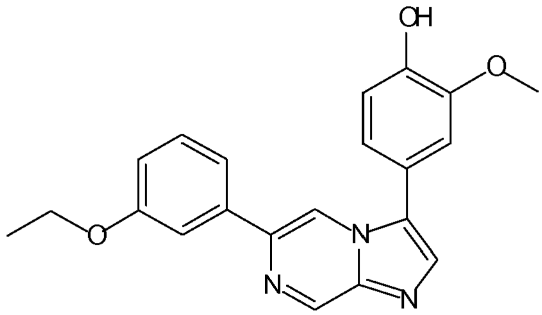

Mw.: 361.14, LCMS-ESI (*m*/*z*) - found 362.14 $[M+H]^+$, 360.14 $[M-H]^-$.

**Example 18-396 compound 396**

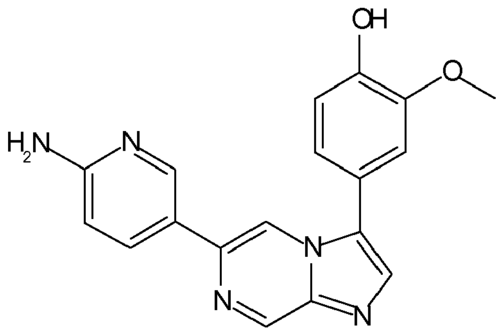

Mw.: 333.12, LCMS-ESI (*m*/*z*) - found 334.12 $[M+H]^+$, 332.12 $[M-H]^-$.

**Example 18-397 compound 397**

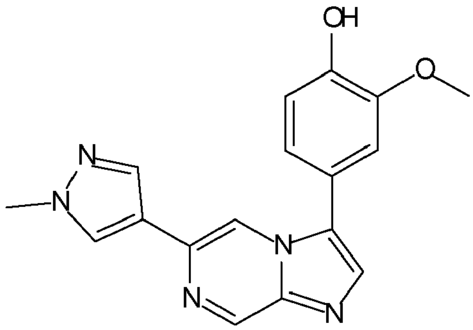

Mw.: 321.12, LCMS-ESI (*m*/*z*) - found 322.12 $[M+H]^+$, 320.12 $[M-H]^-$.

**Example 18-398 compound 398**

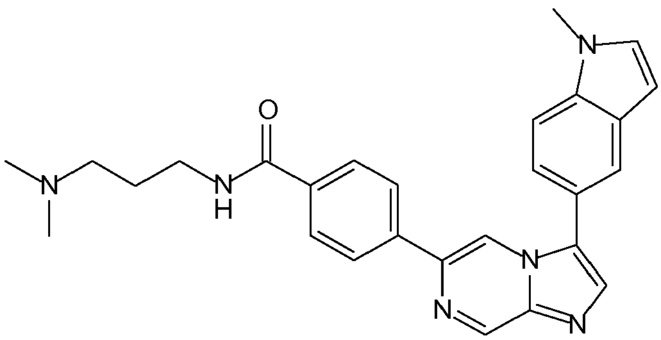

Mw.: 452.23, LCMS-ESI (*m*/*z*) - found 453.23 $[M+H]^+$, 451.23 $[M-H]^-$.

**Example 18-399 compound 399**

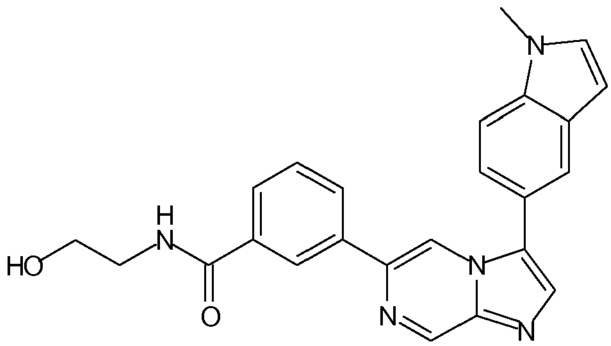

Mw.: 411.17, LCMS-ESI (*m*/*z*) - found 412.17 $[M+H]^+$, 410.17 $[M-H]^-$.

**Example 18-400 compound 400**

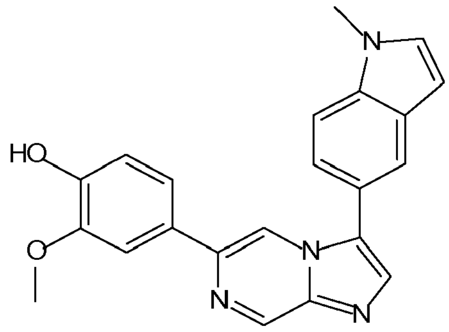

Mw.: 370.14, LCMS-ESI (*m*/*z*) - found 371.14 $[M+H]^+$, 369.14 $[M-H]^-$.

**Example 18-401 compound 401**

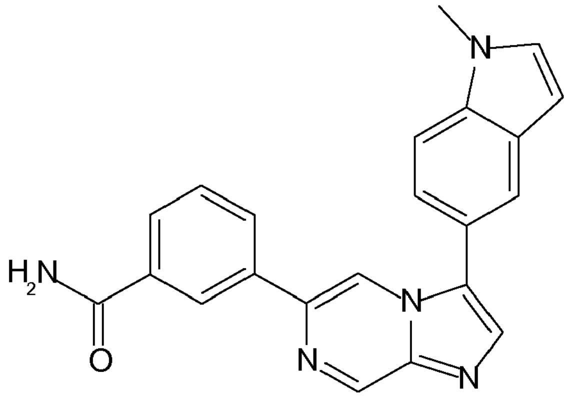

Mw.: 367.14, LCMS-ESI (*m*/*z*) - found 368.14 $[M+H]^+$, 366.14 $[M-H]^-$.

**Example 18-402 compound 402**

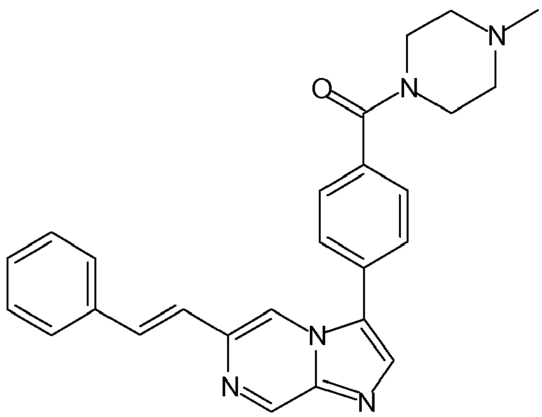

Mw.: 423.21, LCMS-ESI (*m*/*z*) - found 424.20 $[M+H]^+$, 422.20 $[M-H]^-$.

**Example 18-403 compound 403**

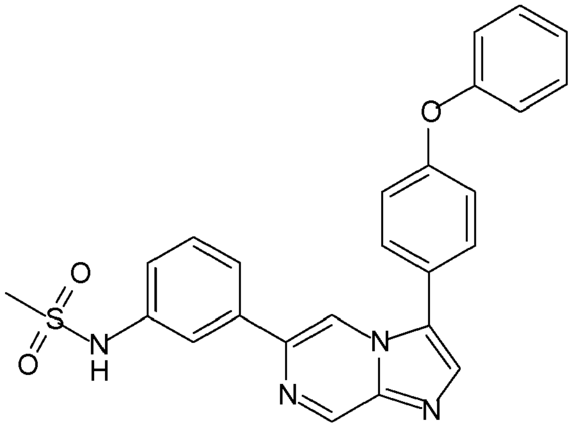

Mw.: 456.13, LCMS-ESI (*m/z*) - found 457.12 $[M+H]^+$, 455.12 $[M-H]^-$.

**Example 18-404 compound 404**

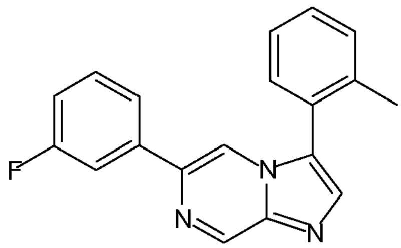

Mw.: 303.12, LCMS-ESI (*m/z*) - found 304.11 $[M+H]^+$, 302.11 $[M-H]^-$.

**Example 18-405 compound 405**

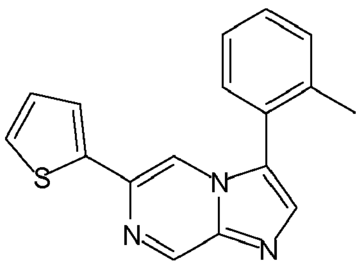

Mw.: 291.08, LCMS-ESI (*m/z*) - found 292.08 $[M+H]^+$, 290.08 $[M-H]^-$.

**Example 18-406 compound 406**

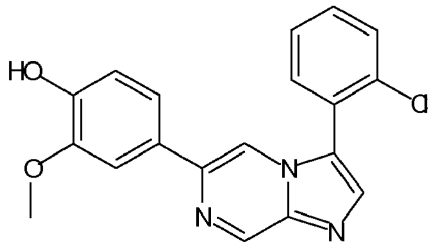

Mw.: 351.08, LCMS-ESI (*m*/*z*) - found 352.07 $[M+H]^+$, 350.07 $[M-H]^-$.

**Example 18-407 compound 407**

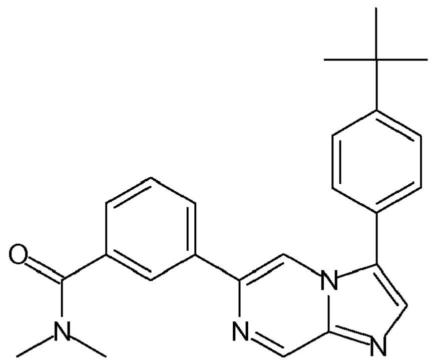

Mw.: 398.21, LCMS-ESI (*m*/*z*) - found 399.21 $[M+H]^+$, 397.21 $[M-H]^-$.

**Example 18-408 compound 408**

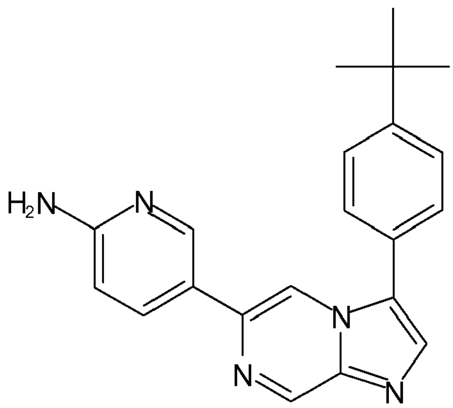

Mw.: 343.18, LCMS-ESI (*m*/*z*) - found 344.18 $[M+H]^+$, 342.18 $[M-H]^-$.

**Example 18-409 compound 409**

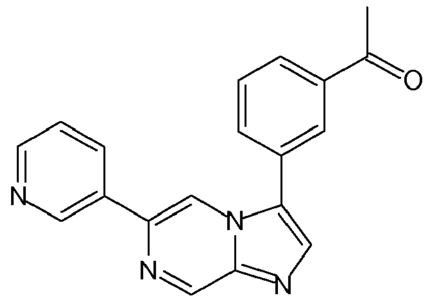

Mw.: 314.12, LCMS-ESI (*m/z*) - found 315.11 $[M+H]^+$, 313.11 $[M-H]^-$.

**Example 18-410 compound 410**

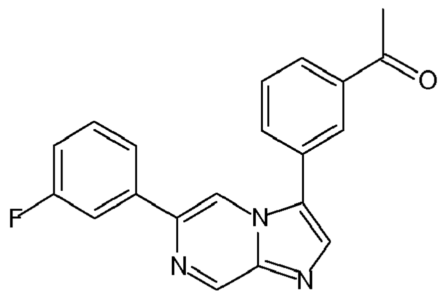

Mw.: 331.11, LCMS-ESI (*m/z*) - found 332.11 $[M+H]^+$, 330.11 $[M-H]^-$.

**Example 18-411 compound 411**

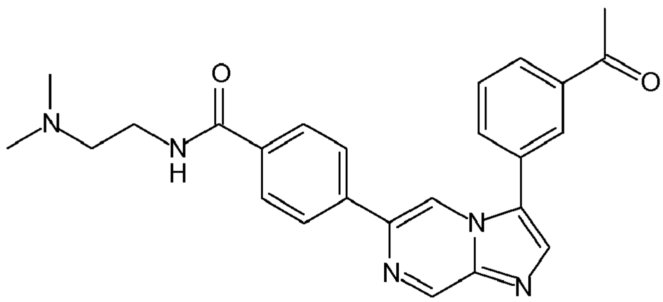

Mw.: 427.20, LCMS-ESI (*m/z*) - found 428.20 $[M+H]^+$, 426.20 $[M-H]^-$.

**Example 18-412 compound 412**

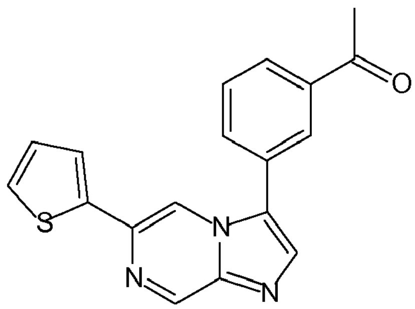

Mw.: 319.08, LCMS-ESI (*m*/*z*) - found 320.07 $[M+H]^+$, 318.07 $[M-H]^-$.

**Example 18-413 compound 413**

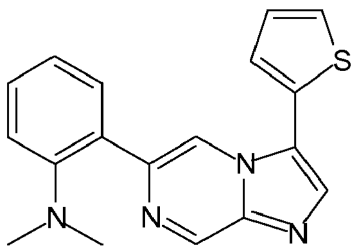

Mw.: 320.11, LCMS-ESI (*m*/*z*) - found 321.11 $[M+H]^+$, 319.11 $[M-H]^-$.

**Example 18-414 compound 414**

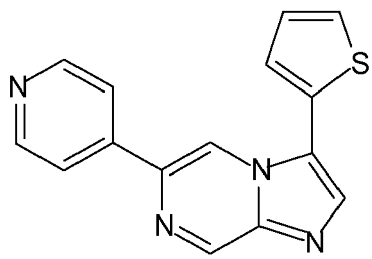

Mw.: 278.06, LCMS-ESI (*m*/*z*) - found 279.06 $[M+H]^+$, 277.06 $[M-H]^-$.

**Example 18-415 compound 415**

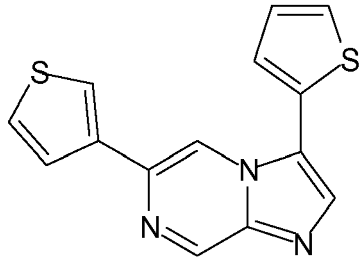

Mw.: 283.02, LCMS-ESI (*m*/*z*) - found 284.02 $[M+H]^+$, 282.02 $[M-H]^-$.

**Example 18-416 compound 416**

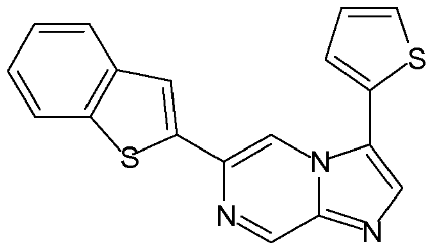

Mw.: 333.04, LCMS-ESI (*m*/*z*) - found 334.03 $[M+H]^+$, 332.03 $[M-H]^-$.

**Example 18-417 compound 417**

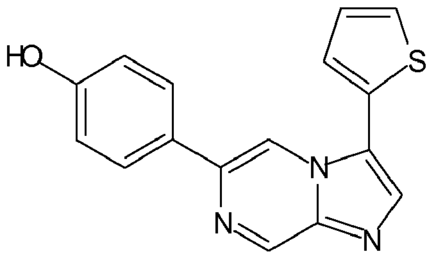

Mw.: 293.06, LCMS-ESI (*m*/*z*) - found 294.06 $[M+H]^+$, 292.06 $[M-H]^-$.

**Example 18-418 compound 418**

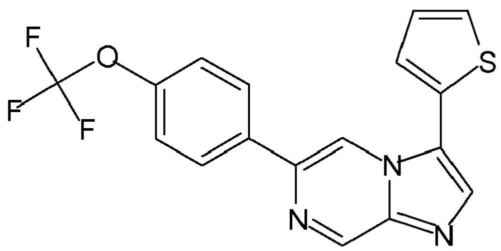

Mw.: 361.05, LCMS-ESI (*m*/*z*) - found 362.05 $[M+H]^+$, 360.05 $[M-H]^-$.

**Example 18-419 compound 419**

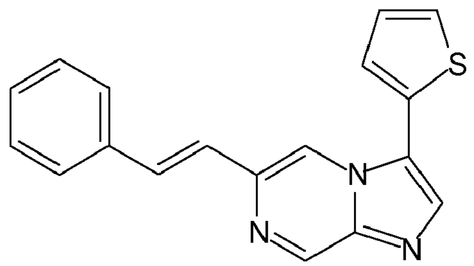

Mw.: 303.08, LCMS-ESI (*m/z*) - found 304.08 $[M+H]^+$, 302.08 $[M-H]^-$.

**Example 18-420 compound 420**

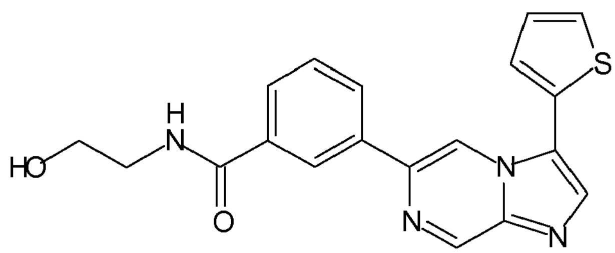

Mw.: 364.10, LCMS-ESI (*m/z*) - found 365.09 $[M+H]^+$, 363.09 $[M-H]^-$.

**Example 18-421 compound 421**

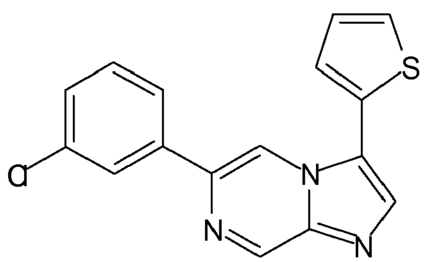

Mw.: 311.03, LCMS-ESI (*m/z*) - found 312.02 $[M+H]^+$, 310.02 $[M-H]^-$.

**Example 18-422 compound 422**

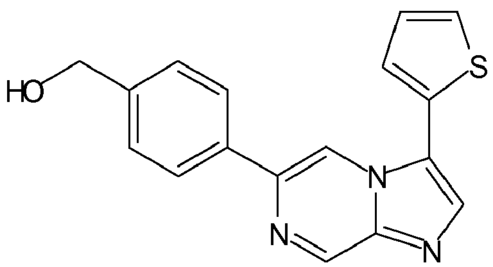

Mw.: 307.08, LCMS-ESI (*m/z*) - found 308.07 $[M+H]^+$, 306.07 $[M-H]^-$.

**Example 18-423 compound 423**

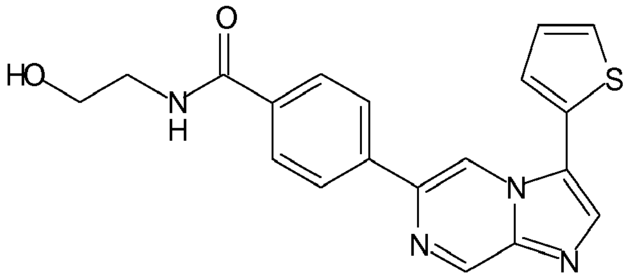

Mw.: 364.10, LCMS-ESI (*m*/*z*) - found 365.09 $[M+H]^+$, 363.09 $[M-H]^-$.

**Example 18-424 compound 424**

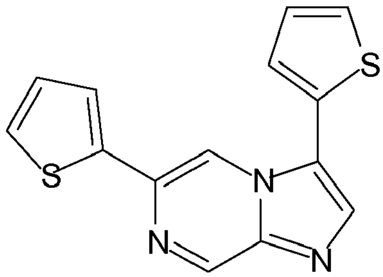

Mw.: 283.02, LCMS-ESI (*m*/*z*) - found 284.02 $[M+H]^+$, 282.02 $[M-H]^-$.

**Example 18-425 compound 425**

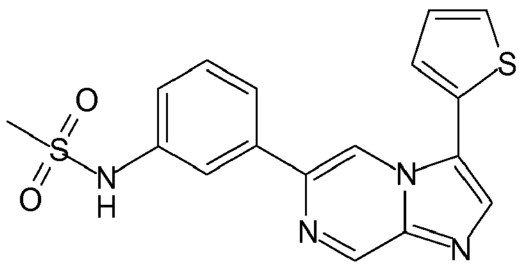

Mw.: 370.06, LCMS-ESI (*m*/*z*) - found 371.05 $[M+H]^+$, 369.05 $[M-H]^-$.

**Example 18-426 compound 426**

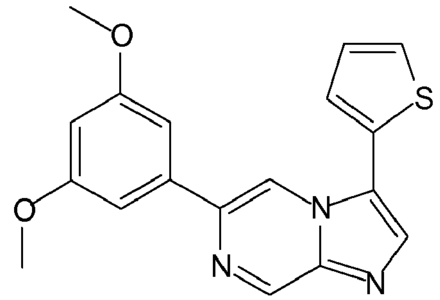

Mw.: 337.09, LCMS-ESI (*m*/*z*) - found 338.08 $[M+H]^+$, 336.08 $[M-H]^-$.

**Example 18-427 compound 427**

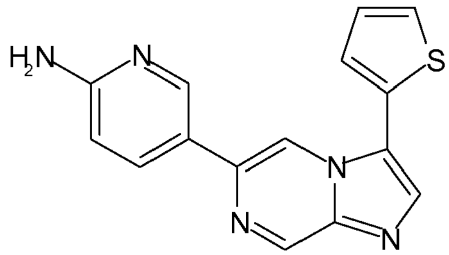

Mw.: 293.07, LCMS-ESI (*m*/*z*) - found 294.07 $[M+H]^+$, 292.07 $[M-H]^-$.

**Example 18-428 compound 428**

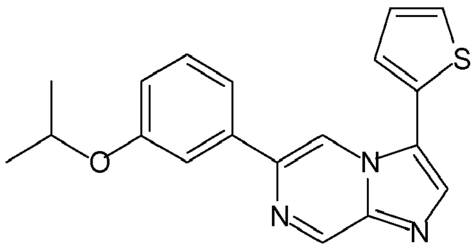

Mw.: 335.11, LCMS-ESI (*m*/*z*) - found 336.10 $[M+H]^+$, 334.10 $[M-H]^-$.

**Example 18-429 compound 429**

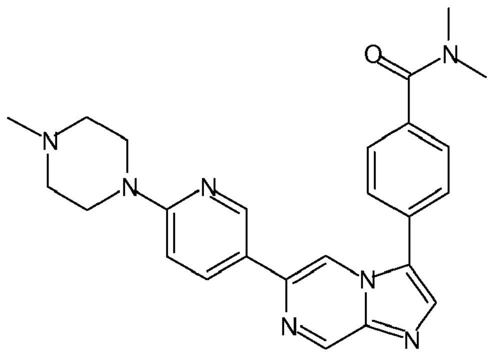

Mw.: 441.23, LCMS-ESI (*m/z*) - found 442.22 $[M+H]^+$, 440.22 $[M-H]^-$.

**Example 18-430 compound 430**

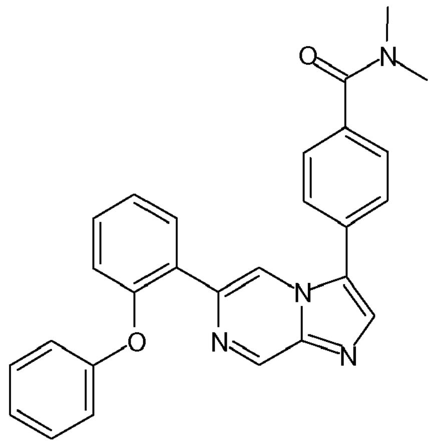

Mw.: 434.17, LCMS-ESI (*m/z*) - found 435.17 $[M+H]^+$, 433.17 $[M-H]^-$.

**Example 18-431 compound 431**

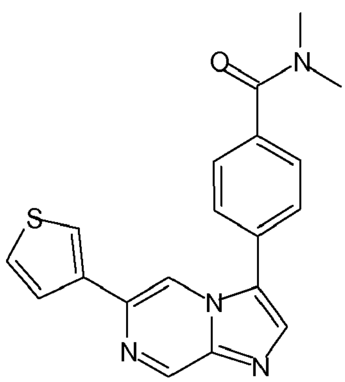

Mw.: 348.10, LCMS-ESI (*m/z*) - found 349.10 $[M+H]^+$, 347.10 $[M-H]^-$.

**Example 18-432 compound 432**

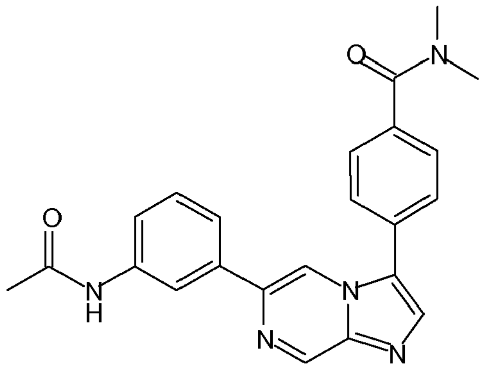

Mw.: 399.17, LCMS-ESI (*m/z*) - found 400.17 $[M+H]^+$, 398.17 $[M-H]^-$.

**Example 18-433 compound 433**

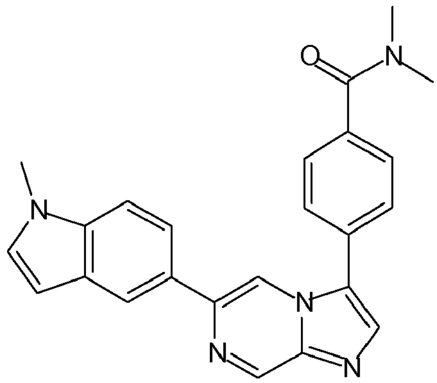

Mw.: 395.17, LCMS-ESI (*m/z*) - found 396.17 $[M+H]^+$, 394.17 $[M-H]^-$.

**Example 18-434 compound 434**

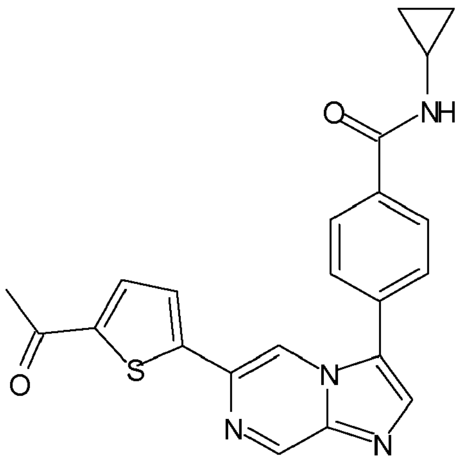

Mw.: 402.12, LCMS-ESI (*m*/*z*) - found 403.11 [M+H]$^+$, 401.11 [M-H]$^-$.

**Example 18-435 compound 435**

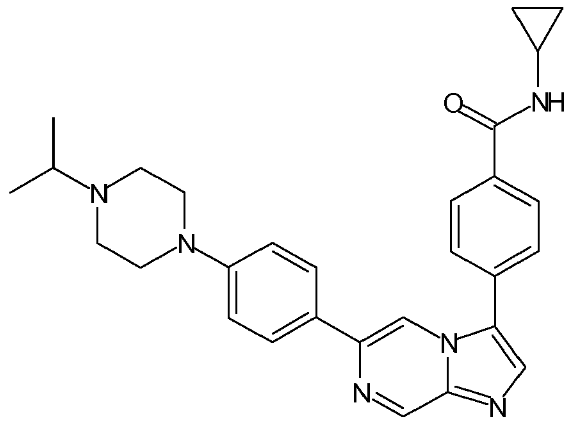

Mw.: 480.26, LCMS-ESI (*m*/*z*) - found 481.26 [M+H]$^+$, 479.26 [M-H]$^-$.

**Example 18-436 compound 436**

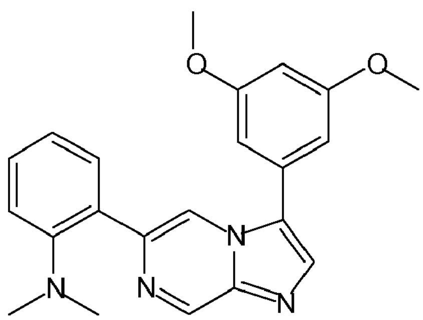

Mw.: 374.17, LCMS-ESI (*m*/*z*) - found 375.17 [M+H]$^+$, 373.17 [M-H]$^-$.

**Example 18-437 compound 437**

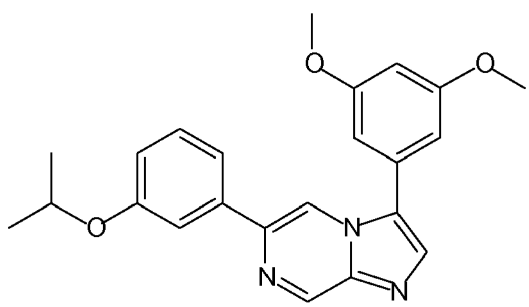

Mw.: 389.17, LCMS-ESI (*m*/*z*) - found 390.17 [M+H]$^+$, 388.17 [M-H]$^-$.

**Example 18-438 compound 438**

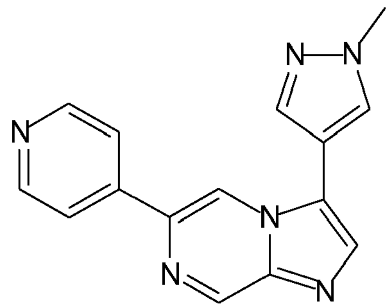

Mw.: 276.11, LCMS-ESI (*m/z*) - found 277.11 $[M+H]^+$, 275.11 $[M-H]^-$.

**Example 18-439 compound 439**

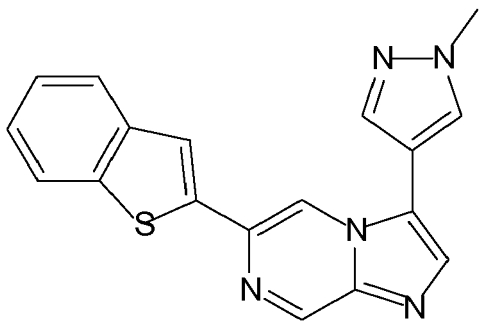

Mw.: 331.09, LCMS-ESI (*m/z*) - found 332.08 $[M+H]^+$, 330.08 $[M-H]^-$.

**Example 18-440 compound 440**

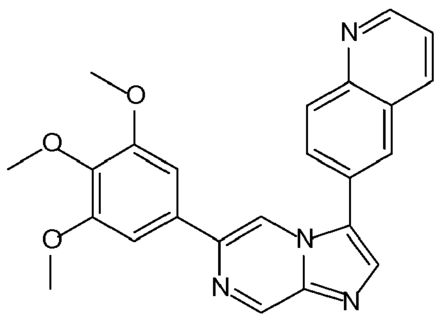

Mw.: 412.15, LCMS-ESI (*m/z*) - found 413.15 $[M+H]^+$, 411.15 $[M-H]^-$.

**Example 18-441 compound 441**

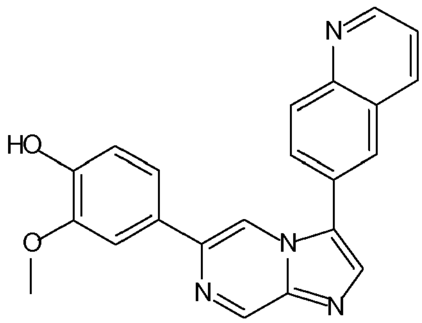

Mw.: 368.13, LCMS-ESI (*m/z*) - found 369.12 $[M+H]^+$, 367.12 $[M-H]^-$.

**Example 18-442 compound 442**

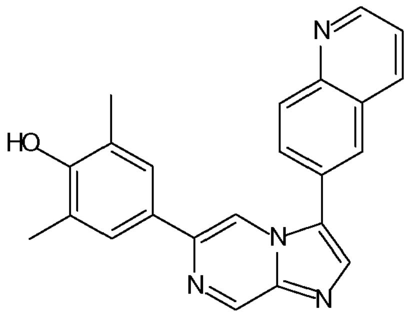

Mw.: 366.15, LCMS-ESI (*m/z*) - found 367.14 $[M+H]^+$, 365.14 $[M-H]^-$.

**Example 18-443 compound 443**

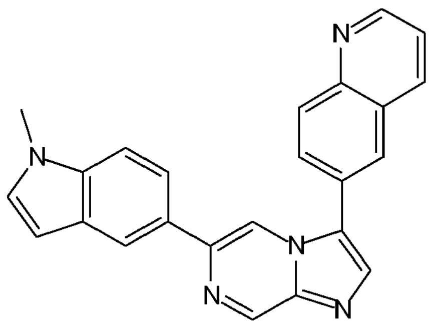

Mw.: 375.15, LCMS-ESI (*m/z*) - found 376.14 $[M+H]^+$, 374.14 $[M-H]^-$.

**Example 18-444 compound 444**

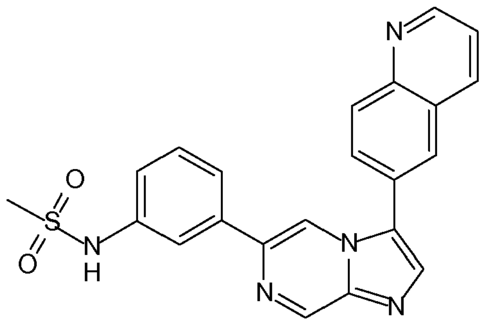

Mw.: 415.11, LCMS-ESI (*m*/*z*) - found 416.11 $[M+H]^+$, 414.11 $[M-H]^-$.

**Example 18-445 compound 445**

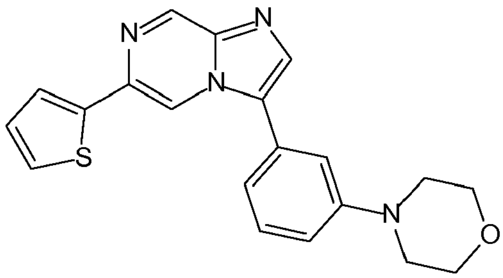

Mw.: 362.45, LCMS-ESI (*m*/*z*) - found 363 $[M+H]^+$.

**Example 18-446 compound 446**

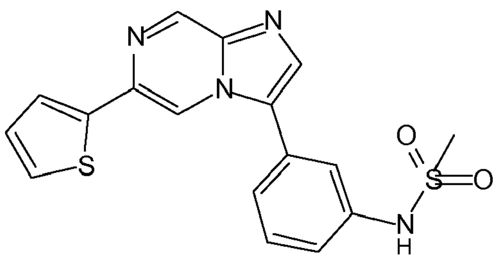

Mw.: 370.45, LCMS-ESI (*m*/*z*) - found 371 $[M+H]^+$.

**Example 18-447 compound 447**

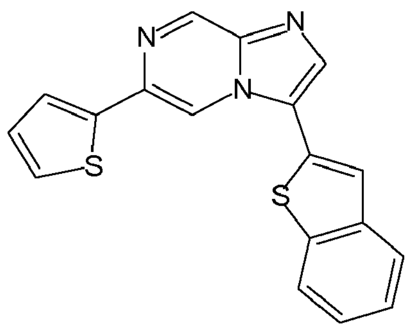

Mw.: 333.43, LCMS-ESI (*m*/*z*) - found 334 [M+H]+.

**Example 18-448 compound 448**

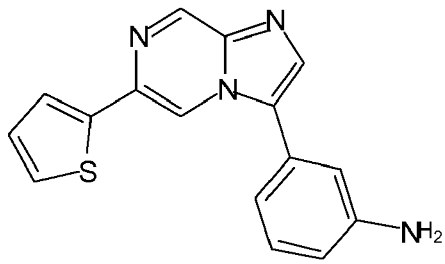

Mw.: 292.36, LCMS-ESI (*m*/*z*) - found 293 [M+H]+.

**Example 18-449 compound 449**

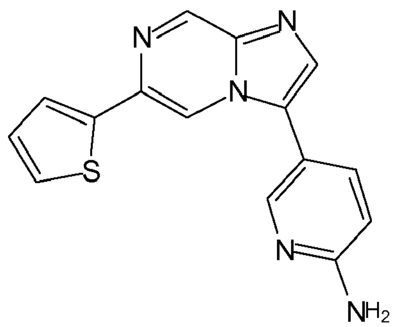

Mw.: 293.35, LCMS-ESI (*m*/*z*) - found 294 [M+H]+.

**Example 18-450 compound 450**

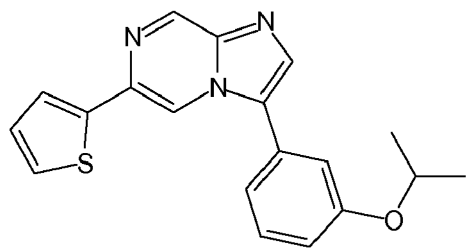

Mw.: 335.43, LCMS-ESI (*m*/*z*) - found 336 [M+H]+.
$^1$H-NMR (DMSO-d□): δ = 9.17 (1 H, ArH), 8.90 (1 H, d, ArH), 8.07 (1 H, s, ArH), 7.78 (1 H, dd, ArH), 7.63 (1 H, dd, ArH), 7.51 (1 H, m, ArH), 7.37-7.29 (2 H, m, ArH), 7.17 (1 H, dd, ArH), 7.07 (1 H, dd, ArH), 4.82-4.71 (1 H, m, CH), 1.33 (6 H, d, $2CH_3$).

**Example 18-451 compound 451**

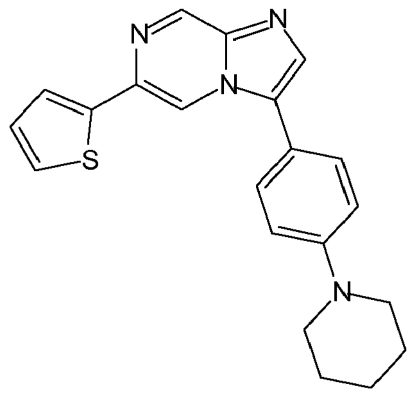

Mw.: 360.48, LCMS-ESI (*m*/*z*) - found 361 [M+H]+.

**Example 18-452 compound 452**

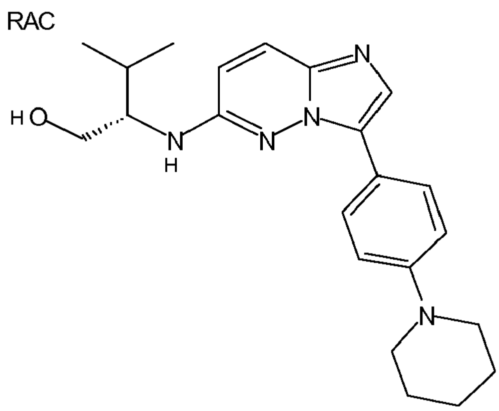

Mw.: 379.51, LCMS-ESI (*m*/*z*) - found 380 [M+H]+.

**Example 18-453 compound 453**

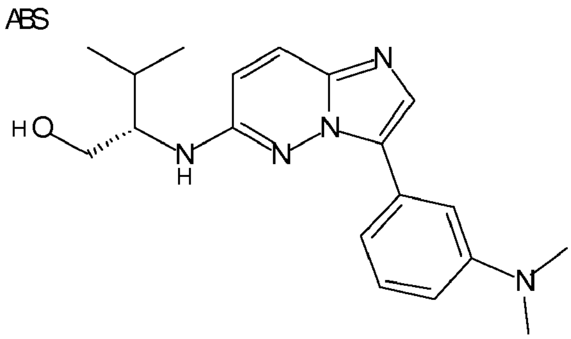

Mw.: 339.44, LCMS-ESI (*m*/*z*) - found 340 [M+H]+.

**Example 18-454 compound 454**

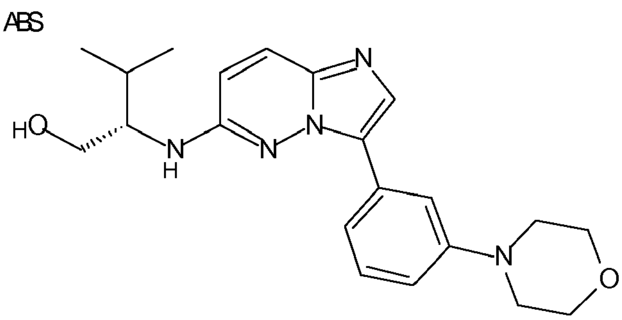

Mw.: 381.48, LCMS-ESI (*m*/*z*) - found 382 [M+H]+.

**Example 18-455 compound 455**

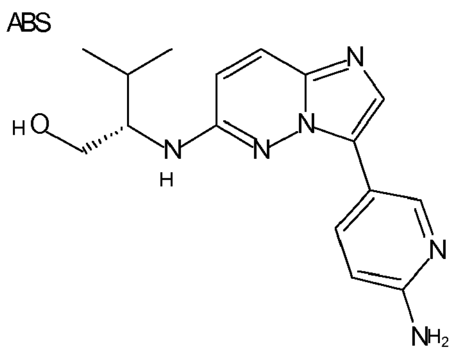

Mw.: 312.38, LCMS-ESI (*m*/*z*) - found 313 [M+H]+.

**Example 18-456 compound 456**

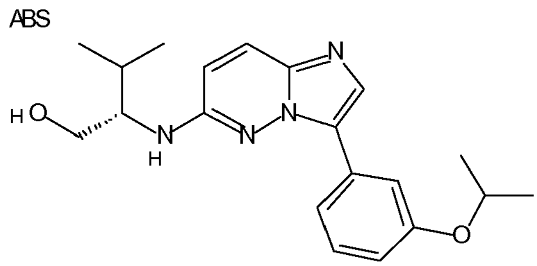

Mw.: 354.45, LCMS-ESI (*m*/*z*) - found 355 $[M+H]^+$.

**Example 18-457 compound 457**

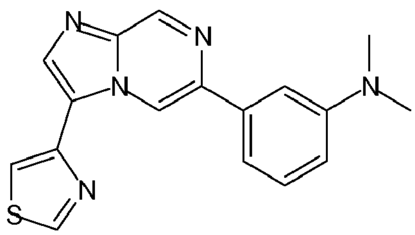

Mw.: 321.41, LCMS-ESI (*m*/*z*) - found 322 $[M+H]^+$.

**Example 18-458 compound 458**

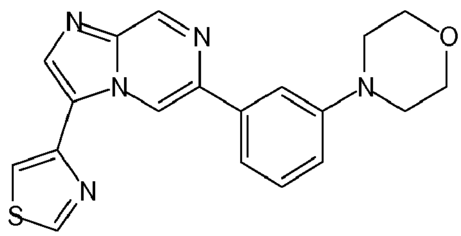

Mw.: 363.44, LCMS-ESI (*m*/*z*) - found 364 $[M+H]^+$.

**Example 18-459 compound 459**

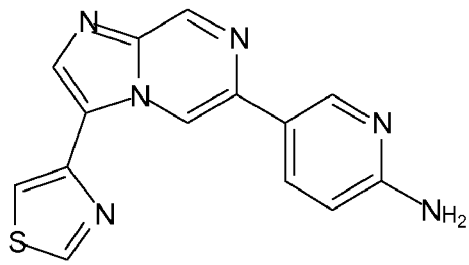

Mw.: 294.34, LCMS-ESI (*m*/*z*) - found 295 $[M+H]^+$.

**Example 18-460 compound 460**

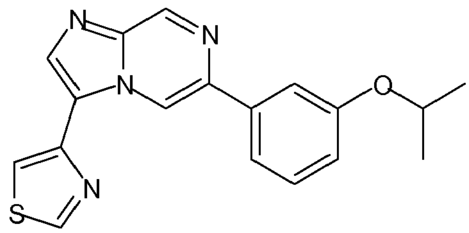

Mw.: 336.42, LCMS-ESI (*m*/*z*) - found 337 $[M+H]^+$.

**Example 18-461 compound 461**

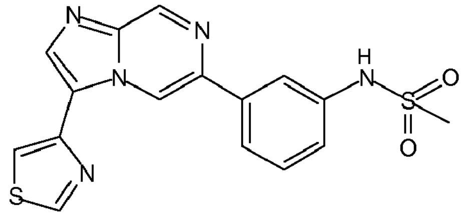

Mw.: 371.44, LCMS-ESI (*m*/*z*) - found 372 $[M+H]^+$.

**Example 18-462 compound 462**

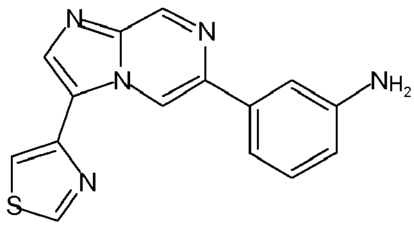

Mw.: 293.35, LCMS-ESI (*m*/*z*) - found 294 $[M+H]^+$.

## Claims

**1.** Compound of the general formula (I)

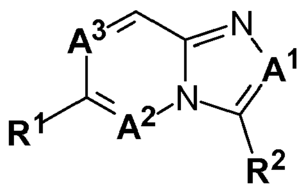

(I)

wherein
**$A^1$**, **$A^2$** and **$A^3$** represent independently of each other C-H or N, wherein one of **$A^1$**, **$A^2$** and **$A^3$** represents N;
**$R^1$** represents $-(CH_2)_n$-**$R^3$** or $-NH-(CH_2)_n$-**$R^3$**;
**$R^2$** represents $-(CH_2)_m$ **$R^4$** or $-NHCO-(CH_2)m$-**$R^4$**;
**$R^3$** and **$R^4$** are independently of each other
-H, -F, -Cl, -Br, -I, -CN, $-NO_2$, $-NHCH_3$, $-N(CH_3)_2$, $-CH=CH-C_4H_9$, $-CH=CH-C_5H_{11}$, -CH=CH-Ph, $-CH=CH-C_6H_{13}$, $-CH_2-OH$; $-C_2H_4-OH$; $-C_3H_6-OH$, $-C_4H_9-OH$, $-C_5H_{10}-OH$, $-C_6H_{12}-OH$, $-C_7H_{14}-OH$, $-C_8H_{16}-OH$, $-CH=CH-C_3H_6-OH$, $-CH=CH-C_4H_8-OH$, $-CH(CH_2OH)_2$, $-CH(C_2H_5)-CH_2-OH$, $-CH(CH_3)-C_2H_4-OH$, $-C(CH_3)_2OH$, $-C(CH_3)_2-CH_2-OH$, $-CH(CH_3)OH$, $-CH_2-CH(CH_3)OH$, $-C(OH)(CH_3)-C_2H_5$, $-C(OH)(CH_3)-C_3H_7$, $-CH_2-C(OH)(CH_3)-C_2H_5$, $-CH(CH_3)-CH(CH_3)OH$, $-C(CH_3)_2-C_2H_4OH$, $-CH_2-C(CH_3)_2OH$, $-C(OH)(C_2H_5)_2$, $-C_2H_4-C(OH)(CH_3)_2$, $-C(CH(CH_3)_2)CH_2OH$, $-C_3H_6-C(OH)(CH_3)_2$, $-CH(CH(CH_3)_2)CH_2-OH$, -OH, $-OCH_3$, $-OC_2H_5$, $-OC_3H_7$, -O-cyclo-$C_3H_5$, $-OCH(CH_3)_2$, $-OC(CH_3)_3$, $-OC_4H_9$, -OPh, $-OCH_2$-Ph, $-OCPh_3$, -SH, $-SCH_3$, $-SC_2H_5$, $-COCH_3$, $-COC_2H_5$, $-COC_3H_7$, -CO-cyclo-$C_3H_5$, $-COCH(CH_3)_2$, $-COC(CH_3)_3$, -COOH, $-OCF_3$, $-CH_2-OCF_3$, $-C_2H_4-OCF_3$, $-C_3H_6-OCF_3$, $-OC_2F_5$, $-COOCH_3$, $-COOC_2H_5$, $-COOC_3H_7$, -COO-cyclo-$C_3H_5$, $-COOCH(CH_3)_2$, $-COOC(CH_3)_3$, $-OOC-CH_3$, $-OOC-C_2H_5$, $-OOC-C_3H_7$, -OOC-cyclo-$C_3H_5$, $-OOC-CH(CH_3)_2$, $-OOC-C(CH_3)_3$, $-CONH_2$, $-CONHCH_3$, $-CONHC_2H_5$, $-CONHC_3H_7$, -CONH-cyclo-$C_3H_5$, $-CONH[CH(CH_3)_2]$, $-CONH[C(CH_3)_3]$, $-CON(CH_3)_2$, $-CON(C_2H_5)_2$, $-CON(C_3H_7)_2$, -CON(cyclo-$C_3H_5)_2$, $-CON[CH(CH_3)_2]_2$, $-CON[C(CH_3)_3]_2$, $-NHCOCH_3$,- $NHCOC_2H_5$, $-NHCOC_3H_7$, -NHCO-cyclo-$C_3H_5$, $-NHCO-CH(CH_3)_2$, $-NHCO-C(CH_3)_3$, $-NHCO-OCH_3$, $-NHCO-OC_2H_5$, $-NHCO-OC_3H_7$, -NHCO-O-cyclo-$C_3H_5$, $-NHCO-OCH(CH_3)_2$, $-NHCO-OC(CH_3)_3$, $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$, -N(cyclo-$C_3H_5)_2$, $-N[CH(CH_3)_2]_2$, $-N[C(CH_3)_3]_2$, -C≡C-**$R^5$**, **-$R^{11}$**, -**$R^{12}$**;

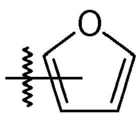 ,
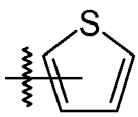 ,
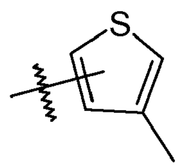 ,
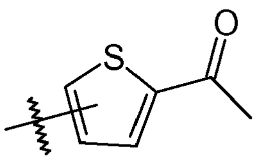 ,
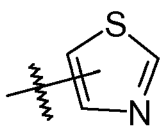 ,
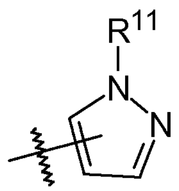 ,
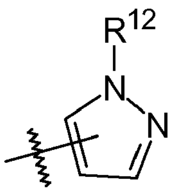 ,
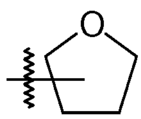 ,
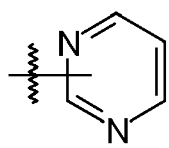 ,
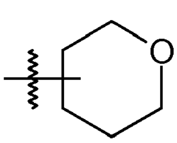 ,
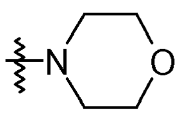 ,
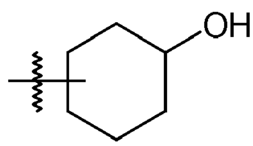 ,
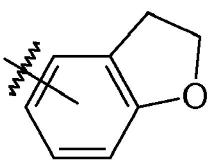 ,
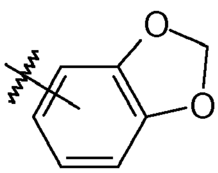 ,
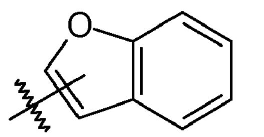 ,

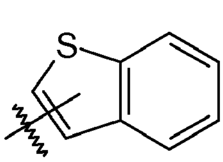, 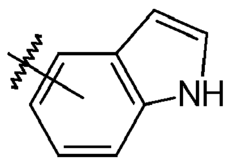, 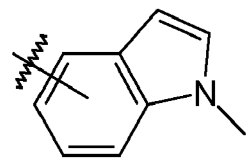,

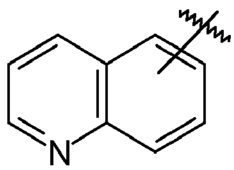, 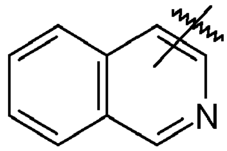, 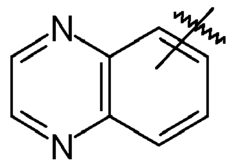,

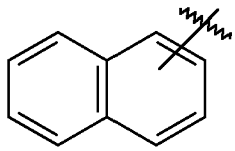, 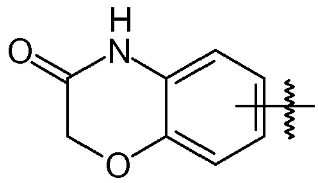, 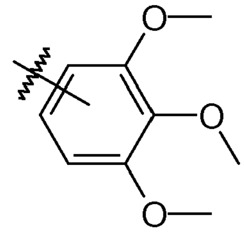,

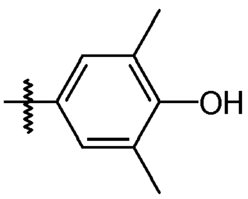, 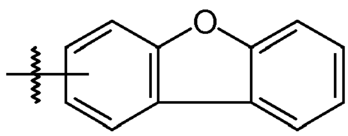, 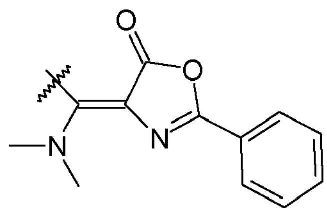,

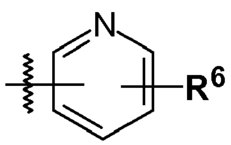, 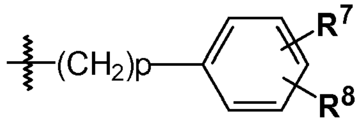;

**$R^5$** is -H, $-CH_2OH$, $-CH_2N(\mathbf{R^{13}})_2$, -**$R^{13}$**,

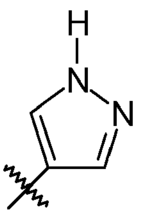, 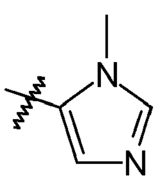, 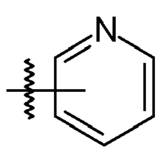, 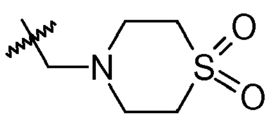,

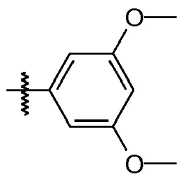, 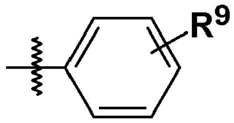;

$R^6$ is -H, $-NH_2$, -OMe, $-O-(CH_2)_3N(CH_3)_2$,

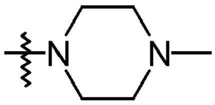, 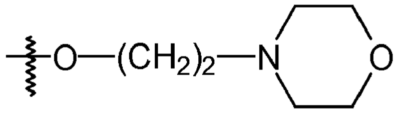;

$R^7$ and $R^8$ are independently of each other
-H, -F, -Br, -Cl, -OH, -CN, $-NO_2$, $-R^{14}$, $-R^{15}$, $-OR^{14}$, $-OR^{15}$, $-CH_2OH$, $-CH_2NH_2$, $-CH_2CN$, $-CH_2N(R^{14})_2$, $-CH_2N(R^{15})_2$, $-CH_2NH(R^{14})$, $-CH_2NH(R^{15})$, $-O(CH_2)_3N(CH_3)_2$, $-SCH_3$, $-NH_2$, $-NH(R^{14})$, $-NH(R^{15})$, $-NHCOCH_3$, $-NHSO_2CH_3$, $-N(R^{14})_2$, $-N(R^{15})_2$, $-SO_2CH_3$, $-SO_2NH_2$, $-CH_2CO_2H$, $-C_2H_4CO_2H$, $-CH=CH-CO_2H$, $-COR^{10}$,

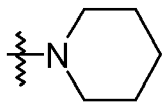, 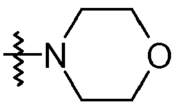, 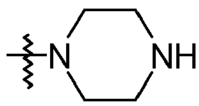, 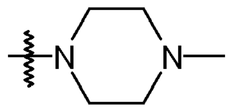,

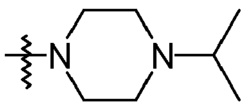, 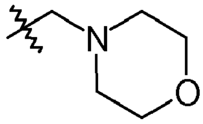;

$R^9$ is -H, -F, -Br, -Cl, -OH, -CN, $-R^{16}$, $-OR^{16}$, $-NHCOCH_3$, or $-CON(CH_3)_2$;
$R^{10}$ is -OH, $-R^{17}$, $-OR^{17}$, $-NH_2$, $-NHR^{17}$, $-N(R^{17})_2$, $-NHC_2H_4OH$, $-NH(CH_2)_qN(R^{17})_2$,

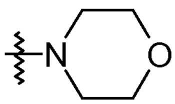, 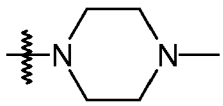, 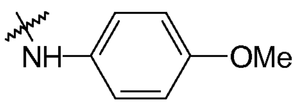, 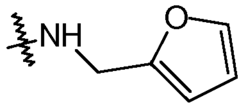;

$R^{11}$, $R^{12}$, $R^{13}$, $R^{14}$, $R^{15}$, $R^{16}$, and $R^{17}$ are independently of each other

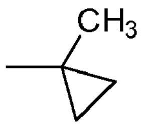,

cyclo-$C_3H_5$, cyclo-$C_4H_7$, cyclo-$C_5H_9$, cyclo-$C_6H_{11}$, cyclo-$C_7H_{13}$, -H, $-CH_2-OCH_3$, $-C_2H_4-OCH_3$, $-C_3H_6-OCH_3$, $-CH_2-OC_2H_5$, $-C_2H_4-OC_2H_5$, $-C_3H_6-OC_2H_5$, $-CH_2-OC_3H_7$, $-C_2H_4-OC_3H_7$, $-C_3H_6OC_3H_7$, $-CH_2$-O-cyclo-$C_3H_5$, $-C_2H_4$-O-cyclo-$C_3H_5$, $-C_3H_6$-O-cyclo-$C_3H_5$, $-CH_2-OCH(CH_3)_2$, $-C_2H_4-OCH(CH_3)_2$, $-C_3H_6OCH(CH_3)_2$, $-CH_2-OC(CH_3)_3$, $-C_2H_4-OC(CH_3)_3$, $-C_3H_6OC(CH_3)_3$, $-CH_2-OC_4H_9$, $-C_2H_4-OC_4H_9$, $-C_3H_6-OC_4H_9$, $-CH_2$-OPh, $-C_2H_4$-OPh, $-C_3H_6$-OPh, $-CH_2-OCH_2$-Ph, $-C_2H_4-OCH_2$-Ph, $-C_3H_6OCH_2$-Ph, $-CH_2F$, $-CHF_2$, $-CF_3$, $-CH_2Cl$, $-CH_2Br$, $-CH_2I$, $-CH_2-CH_2F$, $-CH_2-CHF_2$, $-CH_2-CF_3$, $-CH_2-CH_2Cl$, $-CH_2-CH_2Br$, $-CH_2-CH_2I$, cyclo-$C_8H_{15}$, -Ph, $-CH_2$-Ph, $-CH_2-CH_2$-Ph, -CH=CH-Ph, $-CPh_3$, $-CH_3$, $-C_2H_5$, $-C_3H_7$, $-CH(CH_3)_2$, $-C_4H_9$, $-CH_2-CH(CH_3)_2$, $-CH(CH_3)-C_2H_5$, $-C(CH_3)_3$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$,

$-CH(C_2H_5)_2$, $-C_2H_4-CH(CH_3)_2$, $-C_6H_{13}$, $-C_7H_{15}$, $-C_8H_{17}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH=CH_2$, $-CH_2-CH=CH_2$,- $C(CH_3)=CH_2$, $-CH=CH-CH_3$, $-C_2H_4-CH=CH_2$, $-CH_2-CH=CH-CH_3$, $-CH=CH-C_2H_5$, $-CH_2-C(CH_3)=CH_2$, $-CH(CH_3)-CH=CH$, $-CH=C(CH_3)_2$, $-C(CH_3)=CH-CH_3$, $-CH=CH-CH=CH_2$, $-C{\equiv}CH$, $-C{\equiv}C-CH_3$, $-CH_2-C{\equiv}CH$, $-C_2H_4-C{\equiv}CH$, $-CH_2-C{\equiv}C-CH_3$, $-C{\equiv}C-C_2H_5$, $-CH(CH_3)Ph$, or $-C(CH_3)_2Ph$;

m, n, p and q are independently of each other an integer from 0 to 3;

and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

**2.** Compound of according to claim 1 the general formula (II)

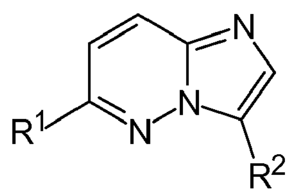

(II)

**$R^1$** represents $-(CH_2)_n-$**$R^3$** or $-NH-(CH_2)_n-$**$R^3$**;

**$R^2$** represents $-(CH_2)_m-$**$R^4$**; **$R^3$** and **$R^4$** are independently of each other

-H, -F, -Br, -Cl, -CN, -OH, $-OCH_3$, $-OC_2H_5$, $-NHCH_3$, $-N(CH_3)_2$, $-CH_3$, $-C_2H_5$, -cyclo-$C_3H_5$, $-C_3H_7$, $-CH(CH_3)_2$, -cyclo-$C_4H_7$, $-C_4H_9$, $-CH_2-CH(CH_3)_2$, $-CH(CH_3)-C_2H_5$, $-C(CH_3)_3$, -cyclo-$C_5H_9$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, -cyclo-$C_6H_{11}$, $-C_6H_{13}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH(CH_3)Ph$, $-CH=CH-C_4H_9$, $-CH=CH-C_5H_{11}$, $-CH=CH-Ph$, $-CH=CH-C_6H_{13}$, $-C{\equiv}C-C(CH_3)_3$, $-CH_2-OH$; $-C_2H_4-OH$; $-C_3H_6-OH$, $-C_4H_9-OH$, $-C_5H_{10}-OH$, $-C_6H_{12}-OH$, $-C_7H_{14}-OH$, $-C_8H_{16}-OH$, $-CH=CH-C_3H_6-OH$, $-CH=CH-C_4H_8-OH$, $-CH(CH_2OH)_2$,- $CH(C_2H_5)-CH_2-OH$, $-CH(CH_3)-C_2H_4-OH$, $-C(CH_3)_2-OH$, $-C(CH_3)_2-CH_2-OH$, $-CH(CH_3)OH$, $-CH_2-CH(CH_3)OH$, $-C(OH)(CH_3)-C_2H_5$, $-C(OH)(CH_3)-C_3H_7$, $-CH_2-C(OH)(CH_3)-C_2H_5$, $-CH(CH_3)-CH(CH_3)OH$, $-C(CH_3)_2-C_2H_4OH$, $-CH_2-C(CH_3)_2OH$, $-C(OH)(C_2H_5)_2$, $-C_2H_4-C(OH)(CH_3)_2$, $-C(CH(CH_3)_2)CH_2OH$, $-C_3H_6-C(OH)(CH_3)_2$, $-CH(CH(CH_3)_2)CH_2-OH$,

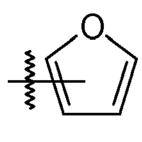 , 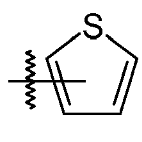 , 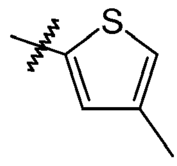 ,

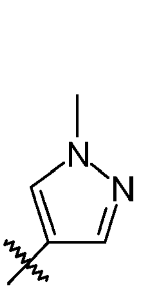 , 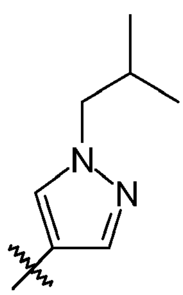 , 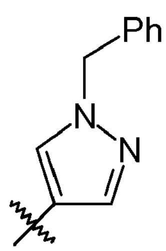 ,

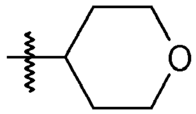 , 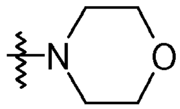 , 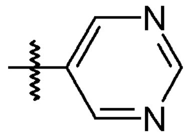 ,

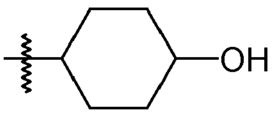 , 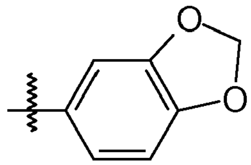 , 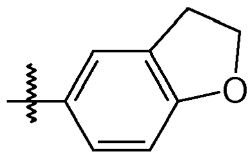 ,

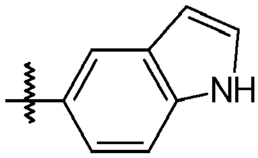 , 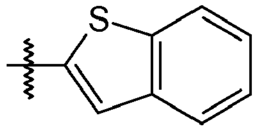 , 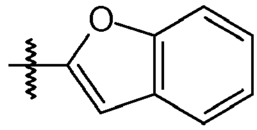 ,

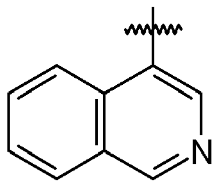 , 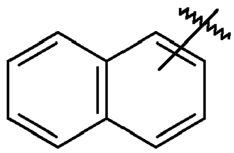 , 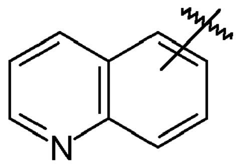 ,

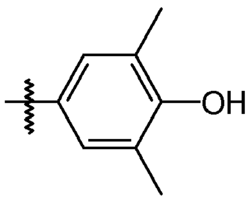 , 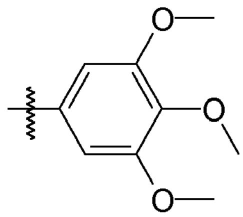 , 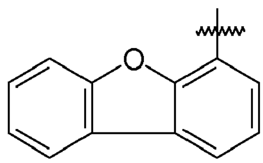 ,

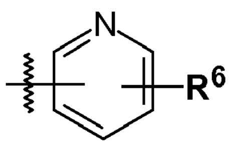 , 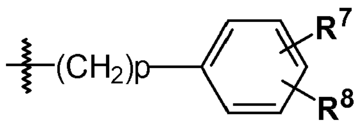 , 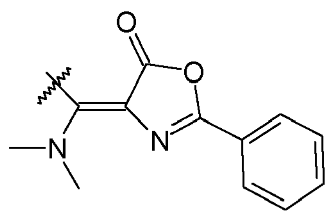 ;

**$R^6$ is** -H, $-NH_2$, -OMe, $-O-(CH_2)_3N(CH_3)_2$, or

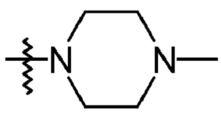

**$R^7$** and **$R^8$** are independently of each other
-H, -F, -Br, -Cl, -OH, -CN, $-NO_2$, $-CH_3$, $-CH(CH_3)_2$, $-OCH_3$, $-OC_2H_5$, $-CF_3$, $-OCF_3$, $-CH_2OH$, $-CH_2NH_2$, $-CH_2CN$,

$-CH_2N(CH_3)_2$, -OPh, $-SCH_3$, $-NH_2$, $-NHCH_3$, $-NHCOCH_3$, $-NHSO_2CH_3$, $-N(CH_3)_2$, $-SO_2CH_3$, $-SO_2NH_2$, $-C_2H_4CO_2H$, $-CH=CH-CO_2H$, -CO**R$^{10}$**,

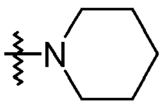 , 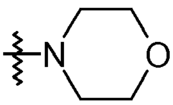 , 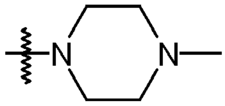 , 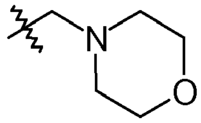 ;

**R$^{10}$** is -OH, $-CH_3$, $-OCH_3$, $-NH_2$, $-NHCH_3$, $-NHC_2H_4OH$, $-NHC_2H_4N(CH_3)_2$, -NH $(CH_2)_3N(CH_3)_2$,

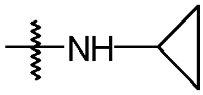 , 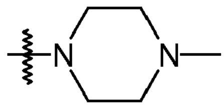 ,

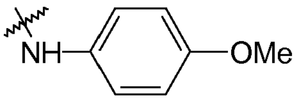 , 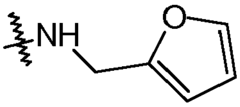 ;

m, n and p are independently of each other an integer from 0 to 3;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

**3.** Compound according to claim 1 of the general formula (III)

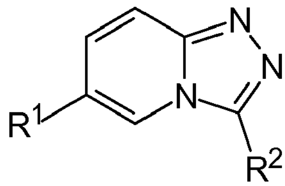

(III)

wherein
**R$^1$** represents $-(CH_2)_n$-**R$^3$**;
**R$^2$** represents $-(CH_2)_m$-**R$^4$**;
**R$^3$** and **R$^4$** are independently of each other -H, -F, -Br, -Cl, -CN, -OH, $-OCH_3$, $-OC_2H_5$, $-NHCH_3$, $-N(CH_3)_2$, $-CH_3$, $-C_2H_5$, -cyclo-$C_3H_5$, $-C_3H_7$, $-CH(CH_3)_2$, -cyclo-$C_4H_7$, $-C_4H_9$, $-CH_2-CH(CH_3)_2$, $-CH(CH_3)-C_2H_5$, $-C(CH_3)_3$, -cyclo-$C_5H_9$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, -cyclo-$C_6H_{11}$, $-C_6H_{13}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH(CH_3)Ph$, $-CH=CH-C_4H_9$, -CH=CH- $C_5H_{11}$, -CH=CH-Ph, $-CH=CH-C_6H_{13}$, $-CH_2-OH$; $-C_2H_4-OH$; $-C_3H_6-OH$, $-C_4H_9-OH$, $-C_5H_{10}-OH$, $-C_6H_{12}-OH$, $-C_7H_{14}-OH$, $-C_8H_{16}-OH$, $-CH=CH-C_3H_6-OH$, $-CH=CH-C_4H_8-OH$, $-CH(CH_2OH)_2$, $-CH(C_2H_5)-CH_2-OH$, $-CH(CH_3)-C_2H_4-OH$, $-C(CH_3)_2-OH$, $-C(CH_3)_2-CH_2-OH$, $-CH(CH_3)OH$, $-CH_2-CH(CH_3)OH$, $-C(OH)(CH_3)-C_2H_5$, $-C(OH)(CH_3)-C_3H_7$, $-CH_2-C(OH)(CH_3)-C_2H_5$, $-CH(CH_3)-CH(CH_3)OH$, $-C(CH_3)_2-C_2H_4OH$, $-CH_2-C(CH_3)_2OH$, $-C(OH)(C_2H_5)_2$, $-C_2H_4-C(OH)(CH_3)_2$, $-C(CH(CH_3)_2)CH_2OH$, $-C_3H_6-C(OH)(CH_3)_2$, $-CH(CH(CH_3)_2)CH_2-OH$, -C≡C-**R$^5$**,

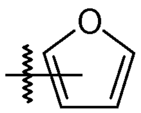 , 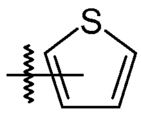 , 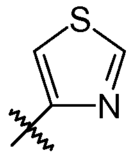 ,

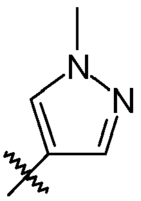 , 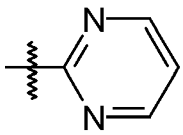 , 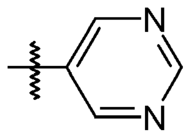 ,

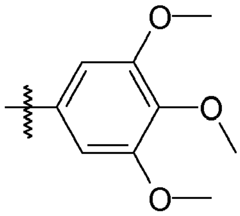 , 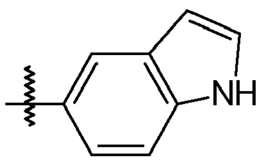 , 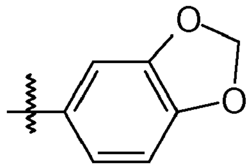 ,

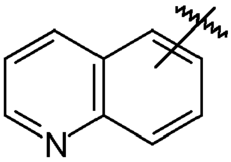 , 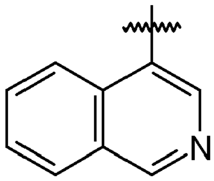 , 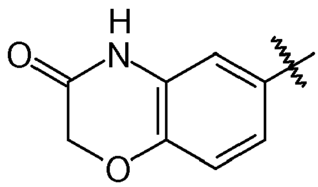 ,

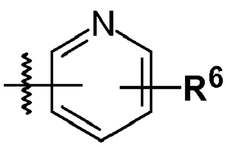 , 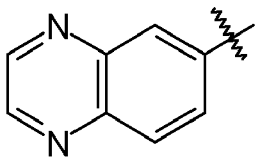 , 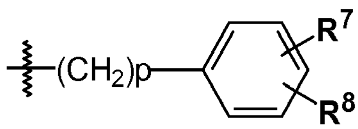 ;

**$R^5$** is selected from -H, $-CH_2OH$, $-CH_2N(CH_3)_2$,

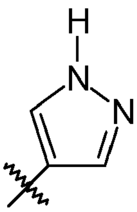 , 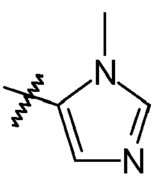 , 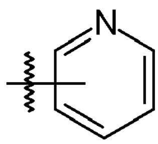 , 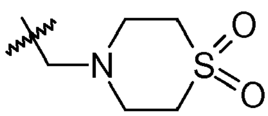 ,

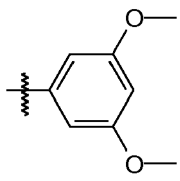
,
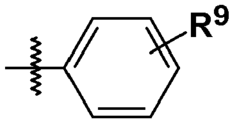
;

**$R^6$** is selected from -H, $-NH_2$, or -OMe;
**$R^7$** and **$R^8$** are independently of each other -H, -F, -Cl, -OH, -CN, $-NO_2$, $-CH_3$, $-CH(CH_3)_2$, $-OCH_3$, $-CF_3$, $-OCF_3$, $-CH_2OH$, $-CH_2N(CH_3)_2$, $-CH_2NHPh$, $-O(CH_2)_3N(CH_3)_2$, -OPh, $-SCH_3$, $-NH_2$, $-NHCOCH_3$, $-NHSO_2CH_3$, $-N(CH_3)_2$, $-SO_2CH_3$, -CO**$R^{10}$**,

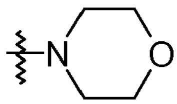
,
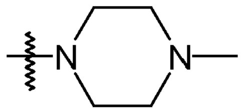
;

**$R^9$** is -H, -F, -Br, -Cl, -OH, -CN, $-CH_3$, $-CH(CH_3)_2$, $-OCH_3$, $-OC_2H_5$, $-CF_3$, $-OCF_3$, $-NHCOCH_3$, or $-CON(CH_3)_2$;
**$R^{10}$** is -OH, $-CH_3$, $-NH_2$, $-NHCH_3$, $-N(CH_3)_2$, $-NHC_2H_4OH$, $-NHC_2H_4N(CH_3)_2$, -NH $(CH_2)_3N(CH_3)_2$,

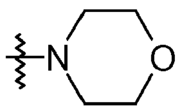
,
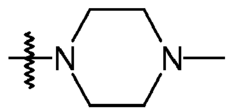
;

m, n and p are independently of each other 0 or 1;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

**4.** Compound according to claim 1 of the general formula (IV)

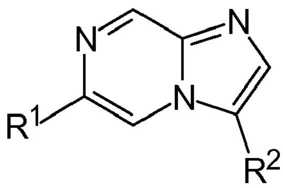

(IV)

wherein
**$R^1$** represents $-(CH_2)_n$-**$R^3$**;
**$R^2$** represents $-(CH_2)_m$-**$R^4$** or $-NHCO-(CH_2)_m$-**$R^4$**;
**$R^3$** and **$R^4$** are independently of each other -H, -F, -Br, -Cl, -CN, -OH, $-OCH_3$, $-OC_2H_5$, $-NHCH_3$, $-N(CH_3)_2$, $-CH_3$, $-C_2H_5$, -cyclo-$C_3H_5$, $-C_3H_7$, $-CH(CH_3)_2$, -cyclo-$C_4H_7$, $-C_4H_9$, $-CH_2-CH(CH_3)_2$, $-CH(CH_3)-C_2H_5$, $-C(CH_3)_3$, -cyclo-$C_5H_9$, $-C_5H_{11}$, $-CH(CH_3)-C_3H_7$, $-CH_2-CH(CH_3)-C_2H_5$, $-CH(CH_3)-CH(CH_3)_2$, $-C(CH_3)_2-C_2H_5$, $-CH_2-C(CH_3)_3$, $-CH(C_2H_5)_2$, $-C_2H_4-CH(CH_3)_2$, -cyclo-$C_6H_{11}$, $-C_6H_{13}$, $-C_3H_6-CH(CH_3)_2$, $-C_2H_4-CH(CH_3)-C_2H_5$, $-CH(CH_3)-C_4H_9$, $-CH_2-CH(CH_3)-C_3H_7$, $-CH(CH_3)-CH_2-CH(CH_3)_2$, $-CH(CH_3)-CH(CH_3)-C_2H_5$, $-CH_2-CH(CH_3)-CH(CH_3)_2$, $-CH_2-C(CH_3)_2-C_2H_5$, $-C(CH_3)_2-C_3H_7$, $-C(CH_3)_2-CH(CH_3)_2$, $-C_2H_4-C(CH_3)_3$, $-CH(CH_3)-C(CH_3)_3$, $-CH(CH_3)Ph$, $-CH=CH-C_4H_9$, $-CH=CH-C_5H_{11}$, -CH=CH-Ph, $-CH=CH-C_6H_{13}$, $-CH_2-OH$; $-C_2H_4-OH$; $-C_3H_6-OH$, $-C_4H_9-OH$, $-C_5H_{10}-OH$, $-C_6H_{12}-OH$, $-C_7H_{14}-OH$, $-C_8H_{16}-OH$, $-CH=CH-C_3H_6-OH$, $-CH=CH-C_4H_8-OH$, $-CH(CH_2OH)_2$,- $CH(C_2H_5)-CH_2-OH$, $-CH(CH_3)-C_2H_4-OH$, $-C(CH_3)_2-OH$, $-C(CH_3)_2-CH_2-OH$, $-CH(CH_3)OH$, $-CH_2-CH(CH_3)OH$, $-C(OH)(CH_3)-C_2H_5$,

$-C(OH)(CH_3)-C_3H_7$, $-CH_2-C(OH)(CH_3)-C_2H_5$, $-CH(CH_3)-CH(CH_3)OH$, $-C(CH_3)_2-C_2H_4OH$, $-CH_2-C(CH_3)_2OH$, $-C(OH)(C_2H_5)_2$, $-C_2H_4-C(OH)(CH_3)_2$, $-C(CH(CH_3)_2)CH_2OH$, $-C_3H_6-C(OH)(CH_3)_2$, $-CH(CH(CH_3)_2)CH_2-OH$,

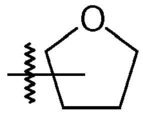 , 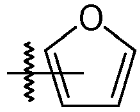 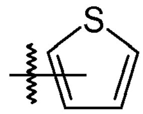 ,

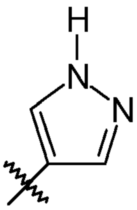 , 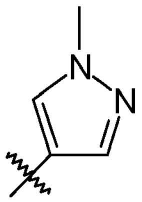 , 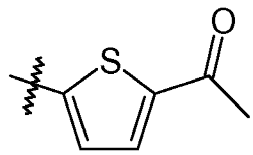 ,

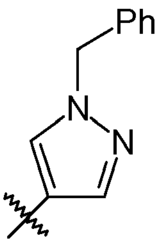 , 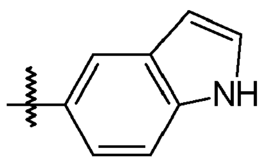 , 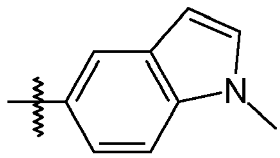 ,

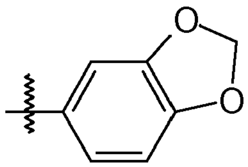 , 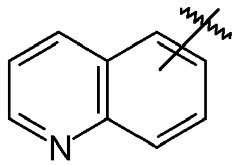 , 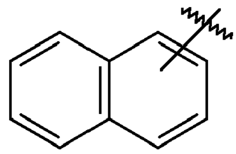 ,

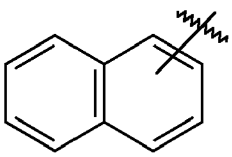 , 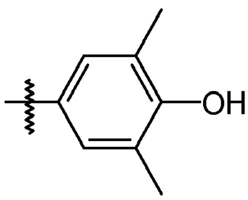 , 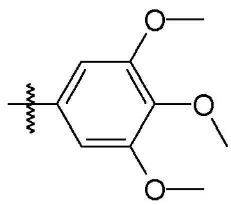 ,

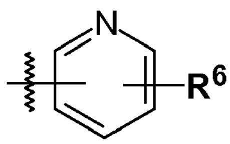 , 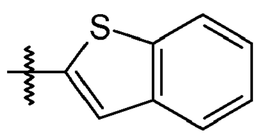 , 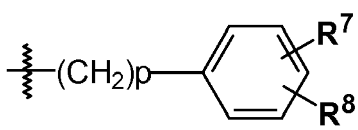 ;

**$R^6$** is selected from -H, $-NH_2$, -OMe, or $-O-(CH_2)_3-N(CH_3)_2$,

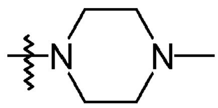 , 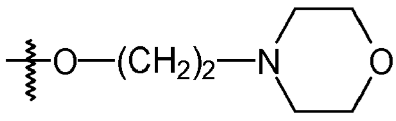 ;

$\mathbf{R^7}$ and $\mathbf{R^8}$ are independently of each other
-H, -F, -Br, -Cl, -OH, -CN, $-NO_2$, $-CH_3$, $-CH(CH_3)_2$, $-C(CH_3)_3$, $-OCH_3$, $-OC_2H_5$, $-OCH(CH_3)_2$, $-CF_3$, $-OCF_3$, $-CH_2OH$, $-CH_2N(CH_3)_2$, $-CH_2NHPh$, -OPh, $-O(CH_2)_3N(CH_3)_2$, $-SCH_3$, $-NH_2$, $-NHCH_3$, $-NHCOCH_3$, $-NHSO_2CH_3$, $-N(CH_3)_2$, $-C_2H_4CO_2H$, -CO$\mathbf{R^{10}}$,

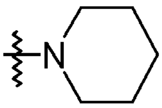 , 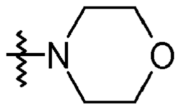 , 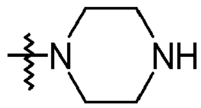 , 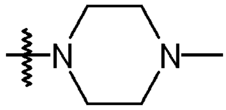 ,

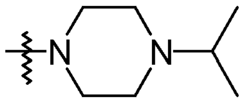 ;

$\mathbf{R^{10}}$ is selected from $-CH_3$, $-NH_2$, $-N(CH_3)_2$, $-NHC_2H_4N(CH_3)_2$, $-NHC_2H_4OH$, $-NH\ (CH_2)_3N(CH_3)_2$,

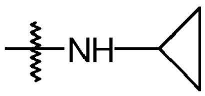 , 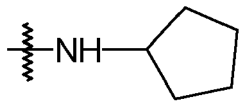 ,

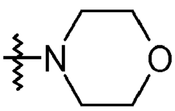 , 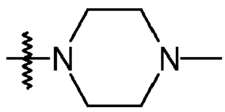 ;

m, n and p are independently of each other 0 or 1;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

**5.** Compound according to claim 1 selected from the group consisting of
4-[6-[(4-fluorophenyl)methyl]imidazo[1,2-b]pyridazin-3-yl]benzamide,
4-(6-benzylimidazo[1,2-b]pyridazin-3-yl)benzamide,
6-(1-methylpyrazol-4-yl)-3-(2-thienyl)imidazo[1,2-b]pyridazine,
N-(2-dimethylaminoethyl)-3-[6-(4-hydroxy-3-methoxy-phenyl)imidazo[1,2-b]pyridazin-3-yl]benzamide,
(2S)-2-[[3-(4-aminophenyl)imidazo[1,2-b]pyridazin-6-yl]amino]-3-methyl-butan-1-ol,
3-(2,4-dimethoxyphenyl)-N-(2-thienylmethyl)imidazo[1,2-b]pyridazin-6-amine,
4-[6-(2-methoxyethylamino)imidazo[1,2-b]pyridazin-3-yl]-N-(4-methoxyphenyl)benzamide,
2-[[3-[(E)-hex-1-enyl]imidazo[1,2-b]pyridazin-6-yl]amino]butan-1-ol,
2-[[3-(2-chlorophenyl)imidazo[1,2-b]pyridazin-6-yl]amino]butan-1-ol,
3-(3-pyridyl)-6-(3,4,5-trimethoxyphenyl)imidazo[1,2-b]pyridazine,
6-(3,4-dimethoxyphenyl)-3-(4-pyridyl)imidazo[1,2-b]pyridazine, N-[3-[3-(3-acetamidophenyl)imidazo[1,2-b]pyridazin-6-yl]phenyl]acetamide,
2-methoxy-4-[6-(3,4,5-trimethoxyphenyl)imidazo[1,2-b]pyridazin-3-yl]phenol,
N-(2-dimethylaminoethyl)-3-[6-[3-(methanesulfonamido)phenyl]imidazo[1,2-b]pyridazin-3-yl]benzamide,
N-[(3-chlorophenyl)methyl]-3-[4-(trifluoromethoxy)phenyl]imidazo[1,2-b]pyridazin-6-amine,

4-[6-[(3-chlorophenyl)methylamino]imidazo[1,2-b]pyridazin-3-yl]-2-methoxy-phenol,
methyl 4-[6-[(3-chlorophenyl)methylamino]imidazo[1,2-b]pyridazin-3-yl]benzoate,
N-[(4-fluorophenyl)methyl]-3-(3-thienyl)imidazo[1,2-b]pyridazin-6-amine,
N-[3-[6-[(4-fluorophenyl)methylamino]imidazo[1,2-b]pyridazin-3-yl]phenyl]acetamide,
4-[6-(propylamino)imidazo[1,2-b]pyridazin-3-yl]benzoic acid,
(E)-3-[3-[6-(propylamino)imidazo[1,2-b]pyridazin-3-yl]phenyl]prop-2-enoic acid,
3-(3-aminophenyl)-N-[(3,4-dichlorophenyl)methyl]imidazo[1,2-b]pyridazin-6-amine,
3-(4-fluorophenyl)-N-(2-methoxyethyl)imidazo[1,2-b]pyridazin-6-amine,
3-(4-morpholinophenyl)-N-[2-(3-pyridyl)ethyl]imidazo[1,2-b]pyridazin-6-amine,
3-(2-naphthyl)-N-[2-(3-pyridyl)ethyl]imidazo[1,2-b]pyridazin-6-amine,
N-(1,3-benzodioxol-5-ylmethyl)-3-(4-morpholinophenyl)imidazo[1,2-b]pyridazin-6-amine,
N-(1,3-benzodioxol-5-ylmethyl)-3-(8-quinolyl)imidazo[1,2-b]pyridazin-6-amine,
N-(1,3-benzodioxol-5-ylmethyl)-3-(4-chlorophenyl)imidazo[1,2-b]pyridazin-6-amine,
3-(2-fluorophenyl)-N-(2-methoxyethyl)imidazo[1,2-b]pyridazin-6-amine, (E)-3-[3-[6-(1,3-benzodioxol-5-ylmethylamino)imidazo[1,2-b]pyridazin-3-yl]phenyl]prop-2-enoic acid,
3-(2-phenoxyphenyl)-N-(4-pyridylmethyl)imidazo[1,2-b]pyridazin-6-amine,
4-[(3-bromoimidazo[1,2-b]pyridazin-6-yl)amino]cyclohexanol,
3-(3-aminophenyl)-N-tetrahydropyran-4-yl-imidazo[1,2-b]pyridazin-6-amine,
3-(4-phenoxyphenyl)-N-tetrahydropyran-4-yl-imidazo[1,2-b]pyridazin-6-amine,
3-(benzofuran-2-yl)-N-[(4-methoxyphenyl)methyl]imidazo[1,2-b]pyridazin-6-amine,
4-[6-[(4-methoxyphenyl)methylamino]imidazo[1,2-b]pyridazin-3-yl]phenol,
3-(1 H-indol-5-yl)-N-[(4-methoxyphenyl)methyl]imidazo[1,2-b]pyridazin-6-amine,
3-(1-naphthyl)-N-[2-(2-pyridyl)ethyl]imidazo[1,2-b]pyridazin-6-amine,
3-(2,4-dimethoxyphenyl)-N-[(4-methoxyphenyl)methyl]imidazo[1,2-b]pyridazin-6-amine,
3-[[3-(2-furyl)imidazo[1,2-b]pyridazin-6-yl]amino]propan-1-ol,
3-[[3-(4-methoxyphenyl)imidazo[1,2-b]pyridazin-6-yl]amino]propan-1-ol,
3-[[3-(2,4-dimethoxyphenyl)imidazo[1,2-b]pyridazin-6-yl]amino]propan-1-ol,
N-(3-morpholinopropyl)-3-(3,4,5-trimethoxyphenyl)imidazo[1,2-b]pyridazin-6-amine,
3-bromo-N-(3-morpholinopropyl)imidazo[1,2-b]pyridazin-6-amine
(2S)-3-methyl-2-[[3-(2-naphthyl)imidazo[1,2-b]pyridazin-6-yl]amino]butan-1-ol,
(2S)-2-[[3-(2,4-dimethoxyphenyl)imidazo[1,2-b]pyridazin-6-yl]amino]-3-methyl-butan-1-ol,
3-(3,4-dimethoxyphenyl)-N-(2-pyridylmethyl)imidazo[1,2-b]pyridazin-6-amine,
3-(5-isopropyl-2-methoxy-phenyl)-N-(2-pyridylmethyl)imidazo[1,2-b]pyridazin-6-amine,
3-(4-dimethylaminophenyl)-N-[(3,4,5-trimethoxyphenyl)methyl]imidazo[1,2-b]pyridazin-6-amine,
N',N'-dimethyl-N-[3-(p-tolyl)imidazo[1,2-b]pyridazin-6-yl]ethane-1,2-diamine,
N-(cyclopropylmethyl)-3-(6-methoxy-3-pyridyl)imidazo[1,2-b]pyridazin-6-amine,
4-[6-[(2,4-dimethylphenyl)methylamino]imidazo[1,2-b]pyridazin-3-yl]phenol,
3-[4-[6-[(2,4-dimethylphenyl)methylamino]imidazo[1,2-b]pyridazin-3-yl]phenyl]propanoic acid,
N-(2-dimethylaminoethyl)-4-[6-[(2,4-dimethylphenyl)methylamino] imidazo[1,2-b]pyridazin-3-yl]benzamide,
N-[(2,4-dimethylphenyl)methyl]-3-(3-methoxyphenyl)imidazo[1,2-b]pyridazin-6-amine,
4-[[[3-(1,3-benzodioxol-5-yl)imidazo[1,2-b]pyridazin-6-yl]amino]methyl]benzenesulfonamide,
4-[[[3-(1-benzylpyrazol-4-yl)imidazo[1,2-b]pyridazin-6-yl]amino]methyl]benzenesulfonamide,
4-[6-[[4-(4-methylpiperazin-1-yl)phenyl]methylamino]imidazo[1,2-b]pyridazin-3-yl]benzonitrile,
(Z)-5-[6-(methylamino)imidazo[1,2-b]pyridazin-3-yl]pent-4-en-1-ol,
2-[6-(methylamino)imidazo[1,2-b]pyridazin-3-yl]phenol,
N,N-dimethyl-3-[6-(methylamino)imidazo[1,2-b]pyridazin-3-yl]benzamide,
1-[2-[6-(methylamino)imidazo[1,2-b]pyridazin-3-yl]phenyl]ethanone,
3-[4-(dimethylaminomethyl)phenyl]-N-methyl-imidazo[1,2-b]pyridazin-6-amine,
3-(3,3-dimethylbut-1-ynyl)-N-methyl-imidazo[1,2-b]pyridazin-6-amine,
N-[2-[6-(methylamino)imidazo[1,2-b]pyridazin-3-yl]phenyl]acetamide,
3-methyl-4-[6-(methylamino)imidazo[1,2-b]pyridazin-3-yl]phenol,
3-[(5-imidazo[1,2-b]pyridazin-3-yl-2-pyridyl)oxy]-N,N-dimethyl-propan-1-amine,
1-(2-imidazo[1,2-b]pyridazin-3-ylphenyl)-N,N-dimethyl-methanamine,
3-[6-(4-methylpiperazin-1-yl)-3-pyridyl]imidazo[1,2-b]pyridazine,
3-(benzothiophen-2-yl)-N-[(3,4,5-trimethoxyphenyl)methyl]imidazo[1,2-b]pyridazin-6-amine,
3-dibenzofuran-4-ylimidazo[1,2-b]pyridazine,
3-(4-methylsulfanylphenyl)imidazo[1,2-b]pyridazine,
3-(4-chlorophenyl)imidazo[1,2-b]pyridazine,

3-[(E)-styryl]imidazo[1,2-b]pyridazine,
2-imidazo[1,2-b]pyridazin-3-ylbenzoic acid,
3-(3-ethoxyphenyl)imidazo[1,2-b]pyridazine,
4-imidazo[1,2-b]pyridazin-3-yl-2,6-dimethyl-phenol,
N-(2-hydroxyethyl)-4-imidazo[1,2-b]pyridazin-3-yl-benzamide,
(4-imidazo[1,2-b]pyridazin-3-ylphenyl)-(4-methylpiperazin-1-yl)methanone,
3-(2,3-dihydrobenzofuran-5-yl)imidazo[1,2-b]pyridazine,
3-(3-fluoro-4-methyl-phenyl)imidazo[1,2-b]pyridazine,
3-imidazo[1,2-b]pyridazin-3-ylbenzonitrile,
3-(3,4-difluorophenyl)imidazo[1,2-b]pyridazine,
3-(m-tolyl)imidazo[1,2-b]pyridazine,
3-(4-ethoxyphenyl)imidazo[1,2-b]pyridazine,
3-(2-methylsulfanylphenyl)imidazo[1,2-b]pyridazine,
1-(4-imidazo[1,2-b]pyridazin-3-ylphenyl)ethanone,
5-imidazo[1,2-b]pyridazin-3-ylquinoline,
N-cyclopropyl-4-imidazo[1,2-b]pyridazin-3-yl-benzamide,
4-imidazo[1,2-b]pyridazin-3-ylisoquinoline,
(2-imidazo[1,2-b]pyridazin-3-ylphenyl)methanol,
3-(2-fluoro-3-methoxy-phenyl)imidazo[1,2-b]pyridazine,
(3-imidazo[1,2-b]pyridazin-3-ylphenyl)-morpholino-methanone,
2-(4-imidazo[1,2-b]pyridazin-3-ylphenyl)acetonitrile,
N-(2-furylmethyl)-3-imidazo[1,2-b]pyridazin-3-yl-benzamide,
N-(4-imidazo[1,2-b]pyridazin-3-ylphenyl)methanesulfonamide,
4-[(4-imidazo[1,2-b]pyridazin-3-ylphenyl)methyl]morpholine,
3-(1-isobutylpyrazol-4-yl)imidazo[1,2-b]pyridazine,
N-cyclopropyl-3-imidazo[1,2-b]pyridazin-3-yl-benzamide,
4-(3-phenylimidazo[1,2-b]pyridazin-6-yl)benzamide,
3-(1,3-benzodioxol-5-yl)-6-phenyl-imidazo[1,2-b]pyridazine,
3-(1,3-benzodioxol-5-yl)-6-[3-(trifluoromethyl)phenyl]imidazo[1,2-b]pyridazine,
3-(3,4-dimethylphenyl)-N-[(3,4,5-trimethoxyphenyl)methyl]imidazo[1,2-b]pyridazin-6-amine,
3-(1,3-benzodioxol-5-yl)-6-(3-fluorophenyl)imidazo[1,2-b]pyridazine, N-[3-[3-(4-pyridyl)imidazo[1,2-b]pyridazin-6-yl]phenyl]methanesulfonamide,
[4-[3-(3-pyridyl)imidazo[1,2-b]pyridazin-6-yl]phenyl]methanol,
6-(3-furyl)-3-(3-pyridyl)imidazo[1,2-b]pyridazine,
3-(3-pyridyl)-6-(2-thienyl)imidazo[1,2-b]pyridazine,
N-[3-[6-(3-pyridyl)imidazo[1,2-b]pyridazin-3-yl]phenyl]acetamide,
N-[3-[6-(3-acetylphenyl)imidazo[1,2-b]pyridazin-3-yl]phenyl]acetamide,
N-[3-[6-[(3,4-difluorophenyl)methyl]imidazo[1,2-b]pyridazin-3-yl]phenyl]acetamide,
3-[3-(3-acetamidophenyl)imidazo[1,2-b]pyridazin-6-yl]-N-methyl-benzamide,
3-(3-chloro-4-fluoro-phenyl)-6-(2-methoxyphenyl)imidazo[1,2-b]pyridazine,
3-(3-chloro-4-fluoro-phenyl)-6-(3,4,5-trimethoxyphenyl)imidazo[1,2-b]pyridazine,
6-(2-methoxyphenyl)-3-[3-(trifluoromethoxy)phenyl]imidazo[1,2-b]pyridazine,
N-(2-dimethylaminoethyl)-3-[3-[3-(trifluoromethoxy)phenyl]imidazo[1,2-b]pyridazin-6-yl]benzamide,
4-[3-[3-(trifluoromethoxy)phenyl]imidazo[1,2-b]pyridazin-6-yl]benzamide,
3-(4-methyl-2-th ienyl)-N-[(3,4,5-trimethoxyphenyl)methyl]imidazo[1,2-b]pyridazin-6-amine,
6-benzyl-3-(4-methylsulfonylphenyl)imidazo[1,2-b]pyridazine,
3-[6-(3-chlorophenyl)imidazo[1,2-b]pyridazin-3-yl]-N-(2-dimethylaminoethyl)benzamide,
N-(2-dimethylaminoethyl)-3-[6-[3-(hydroxymethyl)phenyl]imidazo[1,2-b]pyridazin-3-yl]benzamide,
6-[(4-fluorophenyl)methyl]-3-(5-methoxy-3-pyridyl)imidazo[1,2-b]pyridazine,
3-[3-(3-chlorophenyl)imidazo[1,2-b]pyridazin-6-yl]aniline,
3-(3-chlorophenyl)-6-(3,4-dimethoxyphenyl)imidazo[1,2-b]pyridazine,
3-(3-chlorophenyl)-6-(4-methoxy-2-methyl-phenyl)imidazo[1,2-b]pyridazine,
3-(3-chlorophenyl)-6-(3-methoxyphenyl)imidazo[1,2-b]pyridazine,
3-[6-(4-fluorophenyl)imidazo[1,2-b]pyridazin-3-yl]phenol,
3-[6-(5-quinolyl)imidazo[1,2-b]pyridazin-3-yl]phenol,
[4-[6-(4-dimethylaminophenyl)imidazo[1,2-b]pyridazin-3-yl]phenyl]methanol,
[4-(6-pyrimidin-5-ylimidazo[1,2-b]pyridazin-3-yl)phenyl]methanol,
[4-[6-(1-methylpyrazol-4-yl)imidazo[1,2-b]pyridazin-3-yl]phenyl]methanol,

N-(2-hydroxyethyl)-3-[3-[3-(hydroxymethyl)phenyl]imidazo[1,2-b]pyridazin-6-yl]benzamide,
[3-[6-(3-phenoxyphenyl)imidazo[1,2-b]pyridazin-3-yl]phenyl]methanol,
6-(4-pyridyl)-3-[3-(trifluoromethyl)phenyl]imidazo[1,2-b]pyridazine,
3-[3-[3-(trifluoromethyl)phenyl]imidazo[1,2-b]pyridazin-6-yl]phenol,
6-cyclopropyl-3-[3-(trifluoromethyl)phenyl]imidazo[1,2-b]pyridazine,
3-(3-fluorophenyl)-6-[(4-fluorophenyl)methyl]imidazo[1,2-b]pyridazine,
2-methoxy-4-[6-[3-(trifluoromethoxy)phenyl]imidazo[1,2-b]pyridazin-3-yl]phenol,
3-[3-(dimethylamino)phenyl]-N-(2-furylmethyl)imidazo[1,2-b]pyridazin-6-amine,
4-[6-(2-furylmethylamino)imidazo[1,2-b]pyridazin-3-yl]benzoic acid,
N-[3-[3-(3-furyl)imidazo[1,2-b]pyridazin-6-yl]phenyl]acetamide,
3-[3-(3-furyl)imidazo[1,2-b]pyridazin-6-yl]benzoic acid,
3-(3-furyl)-6-(5-methoxy-3-pyridyl)imidazo[1,2-b]pyridazine,
N-[4-[3-(3-furyl)imidazo[1,2-b]pyridazin-6-yl]phenyl]acetamide,
4-[6-[(3,4-difluorophenyl)methyl]imidazo[1,2-b]pyridazin-3-yl]benzamide,
4-[6-(m-tolylmethyl)imidazo[1,2-b]pyridazin-3-yl]benzamide,
N-[4-[6-(4-morpholinophenyl)imidazo[1,2-b]pyridazin-3-yl]phenyl]acetamide,
N-[4-[6-(1,3-benzodioxol-5-yl)imidazo[1,2-b]pyridazin-3-yl]phenyl]acetamide,
N-[4-[6-(4-methylsulfonylphenyl)imidazo[1,2-b]pyridazin-3-yl]phenyl]acetamide,
6-benzyl-3-pyrimidin-5-yl-imidazo[1,2-b]pyridazine,
6-[(4-fluorophenyl)methyl]-3-(4-methoxy-2-methyl-phenyl)imidazo[1,2-b]pyridazine,
6-[(4-fluorophenyl)methyl]-3-(3-phenoxyphenyl)imidazo[1,2-b]pyridazine,
1-[3-[6-(6-amino-3-pyridyl)imidazo[1,2-b]pyridazin-3-yl]phenyl]ethanone,
3-[3,5-bis(trifluoromethyl)phenyl]-N-(2-thienylmethyl)imidazo[1,2-b]pyridazin-6-amine,
N,N-dimethyl-3-[3-(2-thienyl)imidazo[1,2-b]pyridazin-6-yl]aniline,
6-(3-chloro-4-fluoro-phenyl)-3-(2-thienyl)imidazo[1,2-b]pyridazine,
2-methoxy-4-[3-(2-thienyl)imidazo[1,2-b]pyridazin-6-yl]phenol,
6-(2-chlorophenyl)-3-(2-thienyl)imidazo[1,2-b]pyridazine,
N-[3-[6-[(4-fluorophenyl)methyl]imidazo[1,2-b]pyridazin-3-yl]phenyl]methanesulfonamide,
3-(1-methylpyrazol-4-yl)-6-(m-tolylmethyl)imidazo[1,2-b]pyridazine,
5-(6-benzylimidazo[1,2-b]pyridazin-3-yl)pyridin-2-amine,
(4Z)-4-[dimethylamino(imidazo[1,2-b]pyridazin-3-yl)methylene]-2-phenyl-oxazol-5-one,
3-(3-bromophenyl)-N-(2-thienylmethyl)imidazo[1,2-b]pyridazin-6-amine,
6-(1,3-benzodioxol-5-yl)-3-phenyl-[1,2,4]triazolo[4,3-a]pyridine,
N-[3-(3-phenyl-[1,2,4]triazolo[4,3-a]pyridin-6-yl)phenyl]acetamide,
6-(4-methoxy-2-methyl-phenyl)-3-phenyl-[1,2,4]triazolo[4,3-a]pyridine,
3-phenyl-6-[2-(2-pyridyl)ethynyl]-[1,2,4]triazolo[4,3-a]pyrid ine,
N,N-dimethyl-3-(3-phenyl-[1,2,4]triazolo[4,3-a]pyridin-6-yl)prop-2-yn-1-amine,
N-[3-(3-phenyl-[1,2,4]triazolo[4,3-a]pyridin-6-yl)phenyl]methanesulfonamide,
3-(1,3-benzodioxol-5-yl)-6-(3-chlorophenyl)-[1,2,4]triazolo[4,3-a]pyridine,
3-(1,3-benzodioxol-5-yl)-6-(3-furyl)-[1,2,4]triazolo[4,3-a]pyridine,
3-(1,3-benzodioxol-5-yl)-6-[2-(3-methylimidazol-4-yl)ethynyl]-[1,2,4]triazolo[4,3-a]pyridine,
3-[3-(1,3-benzodioxol-5-yl)-[1,2,4]thiazolo[4,3-a]pyridin-6-yl]prop-2-yn-1-ol,
3-(1,3-benzodioxol-5-yl)-6-(1-methylpyrazol-4-yl)-[1,2,4]triazolo[4,3-a]pyridine,
3-[3-(1,3-benzodioxol-5-yl)-[1,2,4]triazolo[4,3-a]pyridin-6-yl]-N-methyl-benzamide,
4-[4-[3-(4-pyridyl)-[1,2,4]triazolo[4,3-a]pyridin-6-yl]phenyl]morpholine,
6-(3,4-dimethoxyphenyl)-3-(4-pyridyl)-[1,2,4]triazolo[4,3-a]pyridine,
3-(4-pyridyl)-6-[3-(trifluoromethoxy)phenyl]-[1,2,4]triazolo[4,3-a]pyridine,
[4-[3-(4-pyridyl)-[1,2,4]triazolo[4,3-a]pyridin-6-yl]phenyl]methanol,
3-(4-pyridyl)-6-[2-[3-(trifluoromethyl)phenyl]ethynyl]-[1,2,4]triazolo[4,3-a]pyridine,
6-(3-phenoxyphenyl)-3-(4-pyridyl)-[1,2,4]triazolo[4,3-a]pyridine,
N,N-dimethyl-4-[3-(3-pyridyl)-[1,2,4]triazolo[4,3-a]pyridin-6-yl]aniline,
6-(3-isopropylphenyl)-3-(3-pyridyl)-[1,2,4]triazolo[4,3-a]pyridine,
6-(2-chlorophenyl)-3-(3-pyridyl)-[1,2,4]triazolo[4,3-a]pyridine,
3-[3-(3,4-dimethoxyphenyl)-[1,2,4]triazolo[4,3-a]pyridin-6-yl]-N,N-dimethyl-aniline,
3-(3,4-dimethoxyphenyl)-6-(4-methylsulfonylphenyl)-[1,2,4]triazolo[4,3-a]pyridine,
3-(3,4-dimethoxyphenyl)-6-(3-fluorophenyl)-[1,2,4]triazolo[4,3-a]pyridine,
3-[3-(3,4-d imethoxyphenyl)-[1,2,4]triazolo[4,3-a]pyrid in-6-yl]-N,N-dimethyl-benzamide,
3-(3,4-d imethoxyphenyl)-6-[2-(3-pyridyl)ethynyl]-[1,2,4]triazolo[4,3-a]pyridine,

3-(3,4-dimethoxyphenyl)-6-[2-(4-pyridyl)ethynyl]-[1,2,4]triazolo[4,3-a]pyridine,
3-(3,4-dimethoxyphenyl)-6-(2-thienyl)-[1,2,4]triazolo[4,3-a]pyridine,
6-(5-methoxy-3-pyridyl)-3-[3-(trifluoromethoxy)phenyl]-[1,2,4]triazolo[4,3-a]pyridine,
6-(3-methylsulfonylphenyl)-3-[3-(trifluoromethoxy)phenyl]-[1,2,4]triazolo[4,3-a]pyridine,
6-[2-(3-methoxyphenyl)ethynyl]-3-[3-(trifluoromethoxy)phenyl]-[1,2,4]triazolo[4,3-a]pyridine,
6-(2-thienyl)-3-[3-(trifluoromethoxy)phenyl]-[1,2,4]triazolo[4,3-a]pyridine,
6-(2-phenylethynyl)-3-[3-(trifluoromethoxy)phenyl]-[1,2,4]triazolo[4,3-a]pyridine,
3-[3-(3-chlorophenyl)-[1,2,4]triazolo[4,3-a]pyridin-6-yl]aniline,
6-(1,3-benzodioxol-5-yl)-3-(3-chlorophenyl)-[1,2,4]triazolo[4,3-a]pyridine,
3-(3-chlorophenyl)-6-(4-pyridyl)-[1,2,4]triazolo[4,3-a]pyridine,
3-(3-chlorophenyl)-6-(3-pyridyl)-[1,2,4]triazolo[4,3-a]pyridine,
3-(3-chlorophenyl)-6-(3-furyl)-[1,2,4]triazolo[4,3-a]pyridine,
N-[4-[3-(3-chlorophenyl)-[1,2,4]triazolo[4,3-a]pyridin-6-yl]phenyl]acetamide,
3-(3-chlorophenyl)-6-pyrimidin-5-yl-[1,2,4]triazolo[4,3-a]pyridine,
3-(3-chlorophenyl)-6-pyrimidin-2-yl-[1,2,4]triazolo[4,3-a]pyridine,
4-[3-(3-chlorophenyl)-[1,2,4]triazolo[4,3-a]pyridin-6-yl]isoquinoline,
4-[3-(3-hydroxyphenyl)-[1,2,4]triazolo[4,3-a]pyridin-6-yl]benzonitrile,
3-[6-(4-isopropylphenyl)-[1,2,4]triazolo[4,3-a]pyridin-3-yl]phenol,
3-[6-(4-fluorophenyl)-[1,2,4]triazolo[4,3-a]pyridin-3-yl]phenol,
3-[6-(5-quinolyl)-[1,2,4]triazolo[4,3-a]pyridin-3-yl]phenol,
3-[3-(trifluoromethyl)phenyl]-6-(3,4,5-trimethoxyphenyl)-[1,2,4]triazolo[4,3-a]pyridine,
N-[3-(dimethylamino)propyl]-4-[3-[3-(trifluoromethyl)phenyl]-[1,2,4]triazolo[4,3-a]pyridin-6-yl]benzamide,
morpholino-[4-[3-[3-(trifluoromethyl)phenyl]-[1,2,4]triazolo[4,3-a]pyridin-6-yl]phenyl]methanone,
N,N-dimethyl-4-[3-[3-(trifluoromethyl)phenyl]-[1,2,4]triazolo[4,3-a]pyridin-6-yl]benzamide,
5-[3-[3-(trifluoromethyl)phenyl]-[1,2,4]triazolo[4,3-a]pyridin-6-yl]pyridin-2-amine,
2-methoxy-4-[6-[4-(4-methylpiperazin-1-yl)phenyl]-[1,2,4]triazolo[4,3-a]pyridin-3-yl]phenol,
2-methoxy-4-[6-(6-methoxy-3-pyridyl)-[1,2,4]triazolo[4,3-a]pyridin-3-yl]phenol,
4-[6-(3-fluorophenyl)-[1,2,4]triazolo[4,3-a]pyridin-3-yl]-2-methoxy-phenol,
2-methoxy-4-[6-(o-tolyl)-[1,2,4]triazolo[4,3-a]pyridin-3-yl]phenol,
3-(3-furyl)-6-(3,4,5-trimethoxyphenyl)-[1,2,4]triazolo[4,3-a]pyridine,
3-(3-furyl)-6-(4-methoxyphenyl)-[1,2,4]triazolo[4,3-a]pyridine,
3-(3-furyl)-6-(2-thienyl)-[1,2,4]triazolo[4,3-a]pyridine,
3-[6-(2-pyridyl)-[1,2,4]triazolo[4,3-a]pyridin-3-yl]benzonitrile,
6-(3,4-dimethoxyphenyl)-3-(o-tolyl)-[1,2,4]triazolo[4,3-a]pyridine,
N-(2-hydroxyethyl)-3-[3-(o-tolyl)-[1,2,4]triazolo[4,3-a]pyridin-6-yl]benzamide,
4-[3-(o-tolyl)-[1,2,4]triazolo[4,3-a]pyridin-6-yl]benzamide,
4-[4-(3-thiazol-4-yl-[1,2,4]triazolo[4,3-a]pyridin-6-yl)phenyl]morpholine,
N,N-dimethyl-3-(3-thiazol-4-yl-[1,2,4]triazolo[4,3-a]pyridin-6-yl)aniline,
4-[6-(3-chloro-4-fluoro-phenyl)-[1,2,4]triazolo[4,3-a]pyridin-3-yl]thiazole,
4-[6-(1 H-indol-5-yl)-[1,2,4]triazolo[4,3-a]pyridin-3-yl]thiazole,
3-(3-thiazol-4-yl-[1,2,4]triazolo[4,3-a]pyridin-6-yl)benzamide,
4-[6-(4-methoxy-2-methyl-phenyl)-[1,2,4]triazolo[4,3-a]pyridin-3-yl]thiazole,
4-[3-(3-thiazol-4-yl-[1,2,4]triazolo[4,3-a]pyridin-6-yl)prop-2-ynyl]-1,4-thiazinane 1,1-dioxide,
4-[6-(2-chlorophenyl)-[1,2,4]triazolo[4,3-a]pyridin-3-yl]thiazole,
4-[6-(3-methoxyphenyl)-[1,2,4]triazolo[4,3-a]pyridin-3-yl]thiazole,
4-[6-(6-quinolyl)-[1,2,4]triazolo[4,3-a]pyridin-3-yl]thiazole,
3-[4-[6-(3,4-dimethoxyphenyl)-[1,2,4]triazolo[4,3-a]pyridin-3-yl]phenoxy]-N,N-dimethyl-propan-1-amine,
3-[4-[6-(4-chlorophenyl)-[1,2,4]triazolo[4,3-a]pyridin-3-yl]phenoxy]-N,N-dimethyl-propan-1-amine,
4-[2-[3-[4-[3-(dimethylamino)propoxy]phenyl]-[1,2,4]triazolo[4,3-a]pyridin-6-yl]ethynyl]-N, N-d imethyl-benzam ide,
3-[4-[6-[2-(3,5-dimethoxyphenyl)ethynyl]-[1,2,4]triazolo[4,3-a]pyridin-3-yl]phenoxy]-N,N-dimethyl-propan-1-amine,
4-[3-[4-[3-(dimethylamino)propoxy]phenyl]-[1,2,4]triazolo[4,3-a]pyridin-6-yl]-N-methyl-benzamide,
N-[3-(3-quinoxalin-6-yl-[1,2,4]triazolo[4,3-a]pyridin-6-yl)phenyl]acetamide,
N-(2-dimethylaminoethyl)-3-(3-quinoxalin-6-yl-[1,2,4]triazolo[4,3-a]pyridin-6-yl)benzamide,
(4-methylpiperazin-1-yl)-[3-(3-quinoxalin-6-yl-[1,2,4]triazolo[4,3-a]pyridin-6-yl)phenyl]methanone,
2-methoxy-4-(3-quinoxalin-6-yl-[1,2,4]triazolo[4,3-a]pyridin-6-yl)phenol,
6-[6-(3-furyl)-[1,2,4]triazolo[4,3-a]pyridin-3-yl]quinoxaline,
N,N-dimethyl-3-(3-quinoxalin-6-yl-[1,2,4]triazolo[4,3-a]pyridin-6-yl)benzamide,
3-[3-(2-pyridyl)-[1,2,4]triazolo[4,3-a]pyridin-6-yl]phenol,

[3-[3-(2-pyridyl)-[1,2,4]triazolo[4,3-a]pyridin-6-yl]phenyl]methanol,
6-(3-methylsulfonylphenyl)-3-(2-pyridyl)-[1,2,4]triazolo[4,3-a]pyridine,
N-[6-(2,3-dichlorophenyl)imidazo[1,2-a]pyrazin-3-yl]-2-(2-methoxyphenyl)acetamide,
N-[6-(4-isopropylphenyl)imidazo[1,2-a]pyrazin-3-yl]-2-(2-methoxyphenyl)acetamide,
N-[6-[2-(dimethylaminomethyl)phenyl]imidazo[1,2-a]pyrazin-3-yl]-2-(3-methoxyphenyl)acetamide,
N-[6-[(E)-hex-1-enyl]imidazo[1,2-a]pyrazin-3-yl]-2-(3-methoxyphenyl)acetamide,
N-[6-(1 H-indol-5-yl)imidazo[1,2-a]pyrazin-3-yl]-2-(3-methoxyphenyl)acetamide,
N-[6-[3-(hydroxymethyl)phenyl]imidazo[1,2-a]pyrazin-3-yl]-2-(3-methoxyphenyl)acetamide,
2-(3-methoxyphenyl)-N-[6-[3-(trifluoromethyl)phenyl]imidazo[1,2-a]pyrazin-3-yl]acetamide,
N-[6-(4-fluorophenyl)imidazo[1,2-a]pyrazin-3-yl]-2-(3-methoxyphenyl)acetamide,
N-[6-(3-furyl)imidazo[1,2-a]pyrazin-3-yl]-2-(3-methoxyphenyl)acetamide,
2-(3-methoxyphenyl)-N-[6-(4-phenoxyphenyl)imidazo[1,2-a]pyrazin-3-yl]acetamide,
2-(3-methoxyphenyl)-N-[6-(2-thienyl)imidazo[1,2-a]pyrazin-3-yl]acetamide,
2-(3-methoxyphenyl)-N-[6-(1-methylpyrazol-4-yl)imidazo[1,2-a]pyrazin-3-yl]acetamide,
N-[6-[(E)-hex-1-enyl]imidazo[1,2-a]pyrazin-3-yl]pyridine-4-carboxamide,
N-[6-(3-thienyl)imidazo[1,2-a]pyrazin-3-yl]thiophene-2-carboxamide,
N-[6-(4-isopropylphenyl)imidazo[1,2-a]pyrazin-3-yl]acetamide,
N-[6-[3-(2-dimethylaminoethylcarbamoyl)phenyl]imidazo[1,2-a]pyrazin-3-yl]furan-2-carboxamide,
N-[6-(1-benzylpyrazol-4-yl)imidazo[1,2-a]pyrazin-3-yl]-2-methyl-propanamide,
N-[6-(2-methoxyphenyl)imidazo[1,2-a]pyrazin-3-yl]benzamide,
N-[6-[3-(dimethylamino)phenyl]imidazo[1,2-a]pyrazin-3-yl]benzamide,
N-[6-(4-aminophenyl)imidazo[1,2-a]pyrazin-3-yl]benzamide,
N-[6-(1,3-benzodioxol-5-yl)imidazo[1,2-a]pyrazin-3-yl]-2-phenyl-propanamide,
N-[6-(3-nitrophenyl)imidazo[1,2-a]pyrazin-3-yl]-2-phenyl-propanamide,
2-(o-tolyl)-N-[6-(1 H-pyrazol-4-yl)imidazo[1,2-a]pyrazin-3-yl]acetamide,
N-[6-(1-benzylpyrazol-4-yl)imidazo[1,2-a]pyrazin-3-yl]cyclobutanecarboxam ide,
N-[6-(3-acetylphenyl)imidazo[1,2-a]pyrazin-3-yl]-2-cyclopropyl-acetamide,
N-[6-(2-naphthyl)imidazo[1,2-a]pyrazin-3-yl]tetrahydrofuran-3-carboxamide,
N-[6-(4-methoxyphenyl)imidazo[1,2-a]pyrazin-3-yl]tetrahydrofuran-3-carboxamide,
N-[6-(3-aminophenyl)imidazo[1,2-a]pyrazin-3-yl]-2-(3,4-difluorophenyl)acetamide,
N-[6-(2-fluorophenyl)imidazo[1,2-a]pyrazin-3-yl]-2-(3-thienyl)acetamide,
N-[6-(3-methoxyphenyl)imidazo[1,2-a]pyrazin-3-yl]-2-(3-thienyl)acetamide,
N-[6-(2,4-dimethoxyphenyl)imidazo[1,2-a]pyrazin-3-yl]-2-(3-thienyl)acetamide,
4-[6-[6-[3-(dimethylamino)propoxy]-3-pyridyl]imidazo[1,2-a]pyrazin-3-yl]-N,N-dimethyl-aniline,
5-[3-(4-dimethylaminophenyl)imidazo[1,2-a]pyrazin-6-yl]-N-(2-morpholinoethyl)pyridin-2-amine,
2-[3-(4-dimethylaminophenyl)imidazo[1,2-a]pyrazin-6-yl]-N,N-dimethylaniline,
4-[3-(4-dimethylaminophenyl)imidazo[1,2-a]pyrazin-6-yl]phenol,
4-[3-(4-dimethylaminophenyl)imidazo[1,2-a]pyrazin-6-yl]-N-[3-(dimethylamino)propyl]benzamide,
3-[3-(4-dimethylaminophenyl)imidazo[1,2-a]pyrazin-6-yl]-N-(2-hydroxyethyl)benzamide,
[4-[3-(4-dimethylaminophenyl)imidazo[1,2-a]pyrazin-6-yl]phenyl]methanol,
4-[6-(6-methoxy-3-pyridyl)imidazo[1,2-a]pyrazin-3-yl]-N,N-dimethyl-aniline,
N-(2-dimethylaminoethyl)-4-[3-(4-dimethylaminophenyl)imidazo[1,2-a]pyrazin-6-yl]benzamide,
4-[3-(4-dimethylaminophenyl)imidazo[1,2-a]pyrazin-6-yl]-N-(2-hydroxyethyl)benzamide,
[4-[3-(4-dimethylaminophenyl)imidazo[1,2-a]pyrazin-6-yl]phenyl]-morpholino-methanone,
3-[3-(4-dimethylaminophenyl)imidazo[1,2-a]pyrazin-6-yl]benzamide,
3-[3-(4-dimethylaminophenyl)imidazo[1,2-a]pyrazin-6-yl]-N,N-dimethyl-benzamide,
4-[6-(2-chlorophenyl)imidazo[1,2-a]pyrazin-3-yl]-N,N-dimethyl-aniline,
N-[3-[3-(4-dimethylaminophenyl)imidazo[1,2-a]pyrazin-6-yl]phenyl]methanesulfonamide,
3-phenyl-6-(3-thienyl)imidazo[1,2-a]pyrazine,
6-(3-fluorophenyl)-3-phenyl-imidazo[1,2-a]pyrazine,
3-phenyl-6-(2-thienyl)imidazo[1,2-a]pyrazine,
N-cyclopropyl-4-(3-phenylimidazo[1,2-a]pyrazin-6-yl)benzamide,
3-(1,3-benzodioxol-5-yl)-6-phenyl-imidazo[1,2-a]pyrazine,
3-(1,3-benzodioxol-5-yl)-6-(3-thienyl)imidazo[1,2-a]pyrazine,
3-(1,3-benzodioxol-5-yl)-6-(3-ethoxyphenyl)imidazo[1,2-a]pyrazine,
3-(1,3-benzodioxol-5-yl)-6-(3-chlorophenyl)imidazo[1,2-a]pyrazine,
3-(1,3-benzodioxol-5-yl)-6-(3-fluorophenyl)imidazo[1,2-a]pyrazine,
3-(1,3-benzodioxol-5-yl)-6-(o-tolyl)imidazo[1,2-a]pyrazine,

3-(1,3-benzodioxol-5-yl)-6-(2-thienyl)imidazo[1,2-a]pyrazine,
3-(1,3-benzodioxol-5-yl)-6-(1-benzylpyrazol-4-yl)imidazo[1,2-a]pyrazine,
3-(1,3-benzodioxol-5-yl)-6-(3,5-dimethoxyphenyl)imidazo[1,2-a]pyrazine,
5-[3-(1,3-benzodioxol-5-yl)imidazo[1,2-a]pyrazin-6-yl]pyridin-2-amine,
3-(1,3-benzodioxol-5-yl)-6-(3-isopropoxyphenyl)imidazo[1,2-a]pyrazine,
6-(2-phenoxyphenyl)-3-(4-pyridyl)imidazo[1,2-a]pyrazine,
2,6-dimethyl-4-[3-(4-pyridyl)imidazo[1,2-a]pyrazin-6-yl]phenol, morpholino-[4-[3-(4-pyridyl)imidazo[1,2-a]pyrazin-6-yl]phenyl]methanone,
3-(4-pyridyl)-6-(2-thienyl)imidazo[1,2-a]pyrazine,
4-[4-[3-(3-pyridyl)imidazo[1,2-a]pyrazin-6-yl]phenyl]morpholine,
6-(benzothiophen-2-yl)-3-(3-pyridyl)imidazo[1,2-a]pyrazine,
6-(4-methylsulfanylphenyl)-3-(3-pyridyl)imidazo[1,2-a]pyrazine,
N-[3-[3-(3-pyridyl)imidazo[1,2-a]pyrazin-6-yl]phenyl]methanesulfonamide,
6-(2-furyl)-3-(3-thienyl)imidazo[1,2-a]pyrazine,
6-(3-chloro-4-fluoro-phenyl)-3-(3-thienyl)imidazo[1,2-a]pyrazine,
N-(2-hydroxyethyl)-4-[3-(3-thienyl)imidazo[1,2-a]pyrazin-6-yl]benzamide,
6-(2-thienyl)-3-(3-thienyl)imidazo[1,2-a]pyrazine,
6-(3,5-dimethoxyphenyl)-3-(3-thienyl)imidazo[1,2-a]pyrazine,
4-[6-(4-chlorophenyl)imidazo[1,2-a]pyrazin-3-yl]phenol,
4-[6-[4-(4-isopropylpiperazin-1-yl)phenyl]imidazo[1,2-a]pyrazin-3-yl]phenol,
N-cyclopropyl-4-[3-(4-hydroxyphenyl)imidazo[1,2-a]pyrazin-6-yl]benzamide,
4-[6-(1-methylpyrazol-4-yl)imidazo[1,2-a]pyrazin-3-yl]phenol,
3-[4-[6-(3-ethoxyphenyl)imidazo[1,2-a]pyrazin-3-yl]phenyl]propanoic acid,
3-[4-[6-(3-chlorophenyl)imidazo[1,2-a]pyrazin-3-yl]phenyl]propanoic acid,
3-[4-[6-(o-tolyl)imidazo[1,2-a]pyrazin-3-yl]phenyl]propanoic acid,
3-[4-[6-(2-chlorophenyl)imidazo[1,2-a]pyrazin-3-yl]phenyl]propanoic acid,
3-[4-[6-(2-thienyl)imidazo[1,2-a]pyrazin-3-yl]phenyl]propanoic acid,
3-[4-[6-[2-(hydroxymethyl)phenyl]imidazo[1,2-a]pyrazin-3-yl]phenyl]propanoic acid,
3-[4-[6-(2,3-dimethylphenyl)imidazo[1,2-a]pyrazin-3-yl]phenyl]propanoic acid,
4-(6-phenylimidazo[1,2-a]pyrazin-3-yl)benzonitrile,
4-[6-(3-thienyl)imidazo[1,2-a]pyrazin-3-yl]benzonitrile,
4-[6-(4-chlorophenyl)imidazo[1,2-a]pyrazin-3-yl]benzonitrile,
4-[6-[4-(hydroxymethyl)phenyl]imidazo[1,2-a]pyrazin-3-yl]benzonitrile,
4-[6-(2-thienyl)imidazo[1,2-a]pyrazin-3-yl]benzonitrile,
6-[6-(4-methylpiperazin-1-yl)-3-pyridyl]-3-(3,4,5-trimethoxyphenyl)imidazo[1,2-a]pyrazine,
3-[6-[6-[3-(dimethylamino)propoxy]-3-pyridyl]imidazo[1,2-a]pyrazin-3-yl]-N,N-dimethyl-aniline,
N,N-dimethyl-3-[6-(4-pyridyl)imidazo[1,2-a]pyrazin-3-yl]aniline,
N,N-dimethyl-3-[6-(3-thienyl)imidazo[1,2-a]pyrazin-3-yl]aniline,
[4-[3-[3-(dimethylamino)phenyl]imidazo[1,2-a]pyrazin-6-yl]phenyl]methanol,
N,N-dimethyl-3-[6-(2-thienyl)imidazo[1,2-a]pyrazin-3-yl]aniline,
3-[6-(3,5-dimethoxyphenyl)imidazo[1,2-a]pyrazin-3-yl]-N,N-dimethylaniline,
3-(4-chlorophenyl)-6-(4-pyridyl)imidazo[1,2-a]pyrazine,
3-(4-chlorophenyl)-6-(3-thienyl)imidazo[1,2-a]pyrazine,
3-(4-chlorophenyl)-6-(2-thienyl)imidazo[1,2-a]pyrazine,
N-[3-[3-(3-chloro-4-fluoro-phenyl)imidazo[1,2-a]pyrazin-6-yl]phenyl]acetamide,
3-(3-chloro-4-fluoro-phenyl)-6-(2-furyl)imidazo[1,2-a]pyrazine,
6-(3-pyridyl)-3-[3-(trifluoromethoxy)phenyl]imidazo[1,2-a]pyrazine,
N-(2-hydroxyethyl)-3-[3-[3-(trifluoromethoxy)phenyl]imidazo[1,2-a]pyrazin-6-yl]benzamide,
(4-methylpiperazin-1-yl)-[3-[6-(2-phenoxyphenyl)imidazo[1,2-a]pyrazin-3-yl]phenyl]methanone,
[3-[6-(1,3-benzodioxol-5-yl)imidazo[1,2-a]pyrazin-3-yl]phenyl]-(4-methylpiperazin-1-yl)methanone,
(4-methylpiperazin-1-yl)-[3-[6-[4-(trifluoromethoxy)phenyl]imidazo[1,2-a]pyrazin-3-yl]phenyl]methanone,
[3-[6-(3-chlorophenyl)imidazo[1,2-a]pyrazin-3-yl]phenyl]-(4-methylpiperazin-1-yl)methanone,
[3-[6-(1-methylindol-5-yl)imidazo[1,2-a]pyrazin-3-yl]phenyl]-(4-methylpiperazin-1-yl)methanone,
[3-[6-[4-(anilinomethyl)phenyl]imidazo[1,2-a]pyrazin-3-yl]phenyl]-(4-methylpiperazin-1-yl)methanone,
[4-[3-(3-chlorophenyl)imidazo[1,2-a]pyrazin-6-yl]phenyl]methanol,
[4-[3-(4-methoxyphenyl)imidazo[1,2-a]pyrazin-6-yl]phenyl]methanol,
3-(4-methoxyphenyl)-6-(2-thienyl)imidazo[1,2-a]pyrazine,
5-[3-(4-methoxyphenyl)imidazo[1,2-a]pyrazin-6-yl]pyridin-2-amine,

3-[6-(benzothiophen-2-yl)imidazo[1,2-a]pyrazin-3-yl]phenol,
3-[6-(4-chlorophenyl)imidazo[1,2-a]pyrazin-3-yl]phenol,
3-[6-[(E)-styryl]imidazo[1,2-a]pyrazin-3-yl]phenol,
3-[6-[4-(4-methylpiperazin-1-yl)phenyl]imidazo[1,2-a]pyrazin-3-yl]phenol,
3-[6-(3-chlorophenyl)imidazo[1,2-a]pyrazin-3-yl]phenol,
3-[6-(6-methoxy-3-pyridyl)imidazo[1,2-a]pyrazin-3-yl]phenol,
3-[6-(2-thienyl)imidazo[1,2-a]pyrazin-3-yl]phenol,
3-[6-(3,5-dimethoxyphenyl)imidazo[1,2-a]pyrazin-3-yl]phenol,
3-[6-(2-fluoro-3-methoxy-phenyl)imidazo[1,2-a]pyrazin-3-yl]phenol,
[4-[6-(2-furyl)imidazo[1,2-a]pyrazin-3-yl]phenyl]methanol,
[4-[6-(2,4-dichlorophenyl)imidazo[1,2-a]pyrazin-3-yl]phenyl]methanol,
N-cyclopentyl-4-[3-[4-(hydroxymethyl)phenyl]imidazo[1,2-a]pyrazin-6-yl]benzamide,
[3-(6-phenylimidazo[1,2-a]pyrazin-3-yl)phenyl]methanol,
[3-[6-(4-methylsulfanylphenyl)imidazo[1,2-a]pyrazin-3-yl]phenyl]methanol,
[3-[6-(3-ethoxyphenyl)imidazo[1,2-a]pyrazin-3-yl]phenyl]methanol,
[3-[6-(2-thienyl)imidazo[1,2-a]pyrazin-3-yl]phenyl]methanol,
[3-[6-(3-isopropoxyphenyl)imidazo[1,2-a]pyrazin-3-yl]phenyl]methanol,
3-(6-methoxy-3-pyridyl)-6-[4-(4-methylpiperazin-1-yl)phenyl]imidazo[1,2-a]pyrazine,
3,6-bis(6-methoxy-3-pyridyl)imidazo[1,2-a]pyrazine,
3-[3-(6-methoxy-3-pyridyl)imidazo[1,2-a]pyrazin-6-yl]benzamide,
3-[3-(3-fluorophenyl)imidazo[1,2-a]pyrazin-6-yl]aniline,
3-(3-fluorophenyl)-6-phenyl-imidazo[1,2-a]pyrazine,
6-(1,3-benzodioxol-5-yl)-3-(3-fluorophenyl)imidazo[1,2-a]pyrazine,
3-(3-fluorophenyl)-6-(4-piperazin-1-ylphenyl)imidazo[1,2-a]pyrazine,
6-(4-chlorophenyl)-3-(3-fluorophenyl)imidazo[1,2-a]pyrazine,
[4-[3-(3-fluorophenyl)imidazo[1,2-a]pyrazin-6-yl]phenyl]methanol,
3-(3-fluorophenyl)-6-(2-thienyl)imidazo[1,2-a]pyrazine,
4-[6-[6-[3-(dimethylamino)propoxy]-3-pyridyl]imidazo[1,2-a]pyrazin-3-yl]-2-methoxy-phenol,
2-methoxy-4-[6-[6-(2-morpholinoethylamino)-3-pyridyl]imidazo[1,2-a]pyrazin-3-yl]phenol,
2-methoxy-4-[6-[6-(4-methylpiperazin-1-yl)-3-pyridyl]imidazo[1,2-a]pyrazin-3-yl]phenol,
4-[6-(1,3-benzodioxol-5-yl)imidazo[1,2-a]pyrazin-3-yl]-2-methoxy-phenol,
4-[6-(3-ethoxyphenyl)imidazo[1,2-a]pyrazin-3-yl]-2-methoxy-phenol,
4-[6-(6-amino-3-pyridyl)imidazo[1,2-a]pyrazin-3-yl]-2-methoxy-phenol,
2-methoxy-4-[6-(1-methylpyrazol-4-yl)imidazo[1,2-a]pyrazin-3-yl]phenol,
N-[3-(dimethylamino)propyl]-4-[3-(1-methylindol-5-yl)imidazo[1,2-a]pyrazin-6-yl]benzamide,
N-(2-hydroxyethyl)-3-[3-(1-methylindol-5-yl)imidazo[1,2-a]pyrazin-6-yl]benzamide,
2-methoxy-4-[3-(1-methylindol-5-yl)imidazo[1,2-a]pyrazin-6-yl]phenol,
3-[3-(1-methylindol-5-yl)imidazo[1,2-a]pyrazin-6-yl]benzamide,
(4-methylpiperazin-1-yl)-[4-[6-[(E)-styryl]imidazo[1,2-a]pyrazin-3-yl]phenyl]methanone,
N-[3-[3-(4-phenoxyphenyl)imidazo[1,2-a]pyrazin-6-yl]phenyl]methanesulfonamide,
6-(3-fluorophenyl)-3-(o-tolyl)imidazo[1,2-a]pyrazine,
3-(o-tolyl)-6-(2-thienyl)imidazo[1,2-a]pyrazine,
4-[3-(2-chlorophenyl)imidazo[1,2-a]pyrazin-6-yl]-2-methoxy-phenol,
3-[3-(4-tert-butylphenyl)imidazo[1,2-a]pyrazin-6-yl]-N,N-dimethyl-benzamide,
5-[3-(4-tert-butylphenyl)imidazo[1,2-a]pyrazin-6-yl]pyridin-2-amine,
1-[3-[6-(3-pyridyl)imidazo[1,2-a]pyrazin-3-yl]phenyl]ethanone,
1-[3-[6-(3-fluorophenyl)imidazo[1,2-a]pyrazin-3-yl]phenyl]ethanone,
4-[3-(3-acetylphenyl)imidazo[1,2-a]pyrazin-6-yl]-N-(2-dimethylaminoethyl)benzam ide,
1-[3-[6-(2-thienyl)imidazo[1,2-a]pyrazin-3-yl]phenyl]ethanone,
N,N-dimethyl-2-[3-(2-thienyl)imidazo[1,2-a]pyrazin-6-yl]aniline,
6-(4-pyridyl)-3-(2-thienyl)imidazo[1,2-a]pyrazine,
3-(2-thienyl)-6-(3-thienyl)imidazo[1,2-a]pyrazine,
6-(benzothiophen-2-yl)-3-(2-thienyl)imidazo[1,2-a]pyrazine,
4-[3-(2-thienyl)imidazo[1,2-a]pyrazin-6-yl]phenol,
3-(2-thienyl)-6-[4-(trifluoromethoxy)phenyl]imidazo[1,2-a]pyrazine,
6-[(E)-styryl]-3-(2-thienyl)imidazo[1,2-a]pyrazine,
N-(2-hydroxyethyl)-3-[3-(2-thienyl)imidazo[1,2-a]pyrazin-6-yl]benzamide,
6-(3-chlorophenyl)-3-(2-thienyl)imidazo[1,2-a]pyrazine,

[4-[3-(2-thienyl)imidazo[1,2-a]pyrazin-6-yl]phenyl]methanol,
N-(2-hydroxyethyl)-4-[3-(2-thienyl)imidazo[1,2-a]pyrazin-6-yl]benzamide,
3,6-bis(2-thienyl)imidazo[1,2-a]pyrazine,
N-[3-[3-(2-thienyl)imidazo[1,2-a]pyrazin-6-yl]phenyl] methanesulfonamide,
6-(3,5-dimethoxyphenyl)-3-(2-thienyl)imidazo[1,2-a]pyrazine,

5-[3-(2-thienyl)imidazo[1,2-a]pyrazin-6-yl]pyridin-2-amine,
6-(3-isopropoxyphenyl)-3-(2-thienyl)imidazo[1,2-a]pyrazine,
N,N-dimethyl-4-[6-[6-(4-methylpiperazin-1-yl)-3-pyridyl]imidazo[1,2-a]pyrazin-3-yl]benzamide,
N,N-dimethyl-4-[6-(2-phenoxyphenyl)imidazo[1,2-a]pyrazin-3-yl] benzamide,
N,N-dimethyl-4-[6-(3-thienyl)imidazo[1,2-a]pyrazin-3-yl]benzamide,
4-[6-(3-acetamidophenyl)imidazo[1,2-a]pyrazin-3-yl]-N,N-dimethyl-benzamide,
N,N-dimethyl-4-[6-(1-methylindol-5-yl)imidazo[1,2-a]pyrazin-3-yl] benzamide,
4-[6-(5-acetyl-2-thienyl)imidazo[1,2-a]pyrazin-3-yl]-N-cyclopropyl-benzamide,
N-cyclopropyl-4-[6-[4-(4-isopropylpiperazin-1-yl)phenyl]imidazo[1,2-a]pyrazin-3-yl]benzamide,
2-[3-(3,5-dimethoxyphenyl)imidazo[1,2-a]pyrazin-6-yl]-N,N-dimethylaniline,
3-(3,5-dimethoxyphenyl)-6-(3-isopropoxyphenyl)imidazo[1,2-a] pyrazine,
3-(1-methylpyrazol-4-yl)-6-(4-pyridyl)imidazo[1,2-a]pyrazine,
6-(benzothiophen-2-yl)-3-(1-methylpyrazol-4-yl)imidazo[1,2-a]pyrazine,
6-[6-(3,4,5-trimethoxyphenyl)imidazo[1,2-a]pyrazin-3-yl]quinoline,
2-methoxy-4-[3-(6-quinolyl)imidazo[1,2-a]pyrazin-6-yl]phenol,
2,6-dimethyl-4-[3-(6-quinolyl)imidazo[1,2-a]pyrazin-6-yl]phenol,
6-[6-(1-methylindol-5-yl)imidazo[1,2-a]pyrazin-3-yl]quinoline, and
N-[3-[3-(6-quinolyl)imidazo[1,2-a]pyrazin-6-yl]phenyl] methanesulfonamide,
4-[3-[6-(2-thienyl)imidazo[1,2-a]pyrazin-3-yl]phenyl]morpholine,
N-[3-[6-(2-thienyl)imidazo[1,2-a]pyrazin-3-yl]phenyl]methanesulfonamide,
3-(benzothiophen-2-yl)-6-(2-thienyl)imidazo[1,2-a]pyrazine,
3-[6-(2-thienyl)imidazo[1,2-a]pyrazin-3-yl]aniline,
5-[6-(2-thienyl)imidazo[1,2-a]pyrazin-3-yl]pyridin-2-amine,
3-(3-isopropoxyphenyl)-6-(2-thienyl)imidazo[1,2-a]pyrazine,
3-[4-(1-piperidyl)phenyl]-6-(2-thienyl)imidazo[1,2-a]pyrazine,
(2S)-3-methyl-2-[[3-[4-(1-piperidyl)phenyl]imidazo[1,2-b]pyridazin-6-yl]amino]butan-1-ol,
(2S)-2-[[3-[3-(dimethylamino)phenyl]imidazo[1,2-b]pyridazin-6-yl]amino]-3-methyl-butan-1-ol,
(2S)-3-methyl-2-[[3-(3-morpholinophenyl)imidazo[1,2-b]pyridazin-6-yl]amino]butan-1-ol,
(2S)-2-[[3-(6-amino-3-pyridyl)imidazo[1,2-b]pyridazin-6-yl]amino]-3-methyl-butan-1-ol,
(2S)-2-[[3-(3-isopropoxyphenyl)imidazo[1,2-b]pyridazin-6-yl]amino]-3-methyl-butan-1-ol,
N,N-dimethyl-3-(3-thiazol-4-ylimidazo[1,2-a]pyrazin-6-yl)aniline,
4-[3-(3-thiazol-4-ylimidazo[1,2-a]pyrazin-6-yl)phenyl]morpholine,
5-(3-thiazol-4-ylimidazo[1,2-a]pyrazin-6-yl)pyridin-2-amine,
4-[6-(3-isopropoxyphenyl)imidazo[1,2-a]pyrazin-3-yl]thiazole, N-[3-(3-thiazol-4-ylimidazo[1,2-a]pyrazin-6-yl)phenyl]methanesulfonamide,
3-(3-thiazol-4-ylimidazo[1,2-a]pyrazin-6-yl)aniline.

6. Compound according to any one of claims 1 - 5 for use as pharmaceutically active agent in medicine.

7. Compound according to any one of claims 1 - 5 for the inhibition of GRK5 and/or Scyl1.

8. Compound according to any one of claims 1 - 5 for use in treatment or prophylaxis of a metabolic disease.

9. Compound according to claim 8, wherein the metabolic disease is selected from diabetes, obesity and impaired adipogenesis.

10. The compound according to claim 8 or 9, wherein the disease is diabetes mellitus type 2.

11. Compound according to any one of claims 1 - 5 for use in glucose dependent regulation of insulin production and/or release of insulin.

12. Compound according to any one of claims 1 - 5 for increasing glucose uptake.

**13.** Pharmaceutical composition comprising at least one compound according to any one of claims 1 - 5 together with at least one pharmaceutically acceptable carrier, solvent or excipient.

**14.** Pharmaceutical composition according to claim 13 further comprising at least one active agent selected from the group consisting of an anti-diabetic drug.

**15.** Pharmaceutical composition according to claim 14 or 15, wherein the anti-diabetic drug is selected from thiazolidinediones, metformin and sulfonylurea.

**Figure 1A**

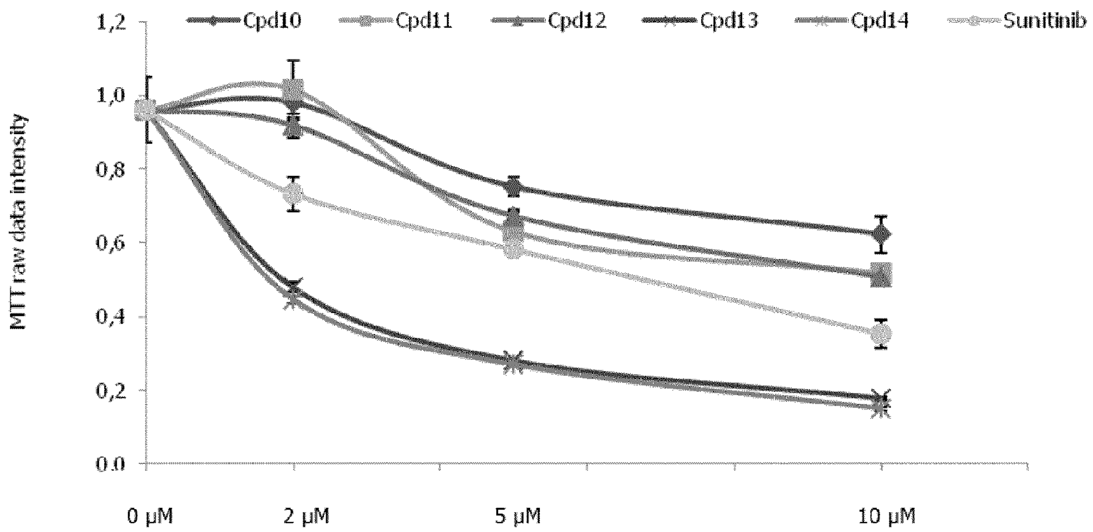


**Figure 1 B**

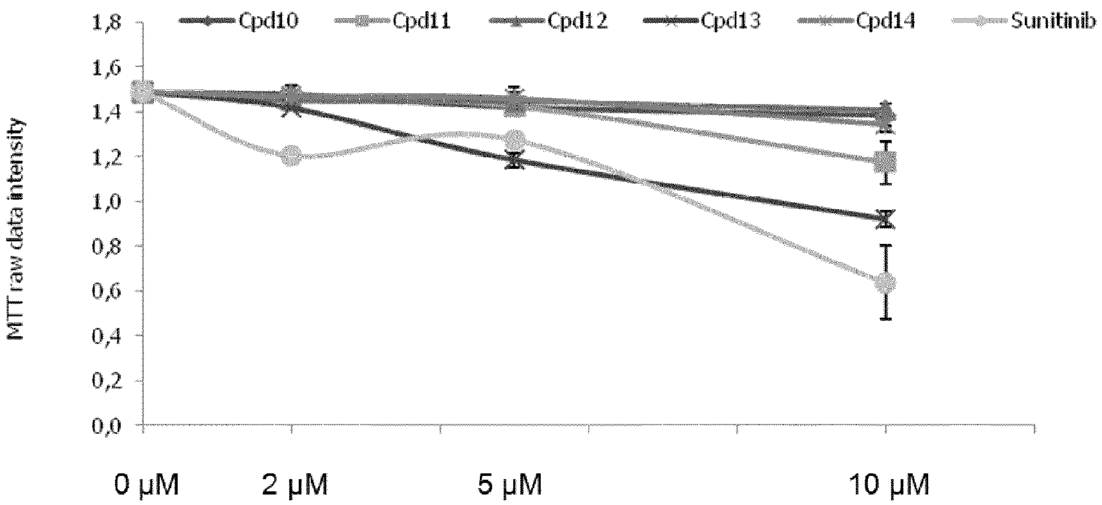

Figure 1 C

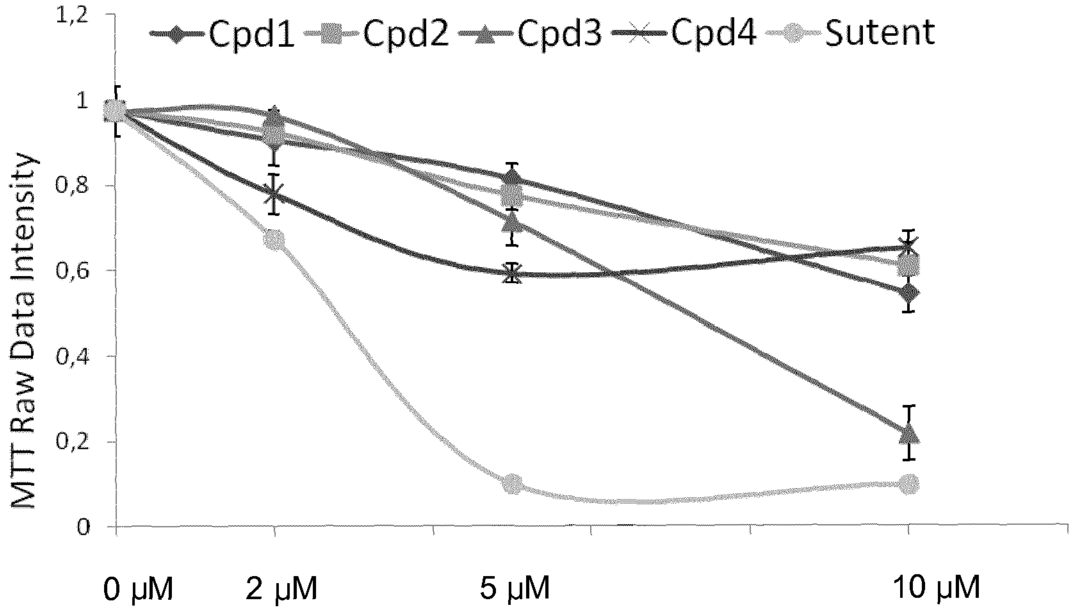


Figure 1 D

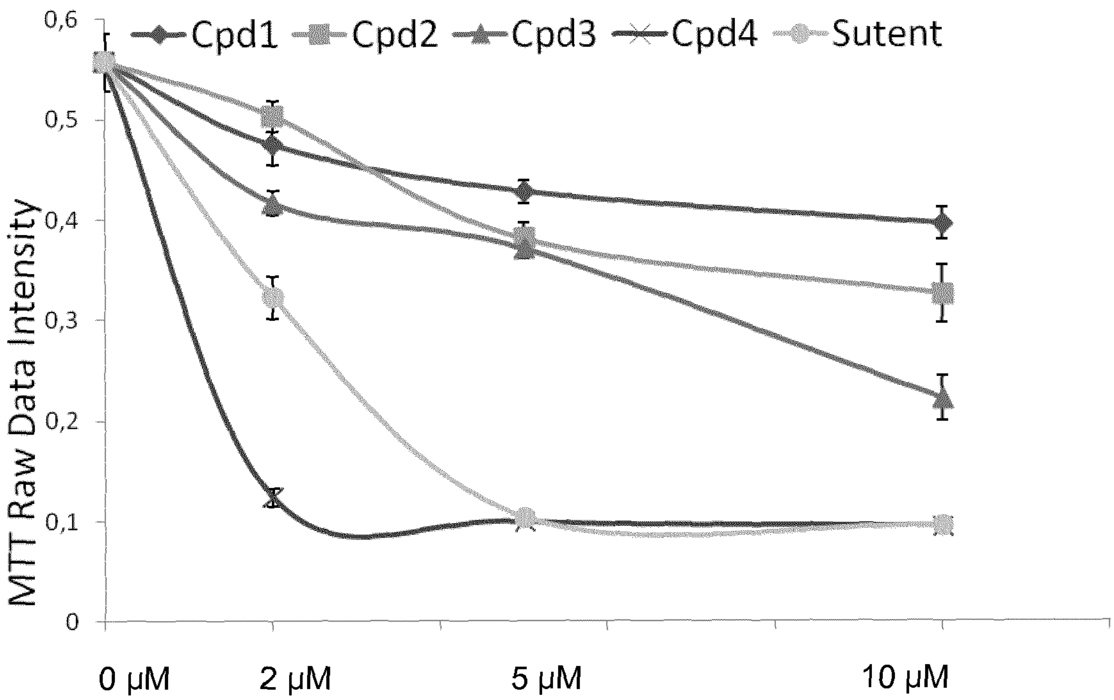

## Figure 2

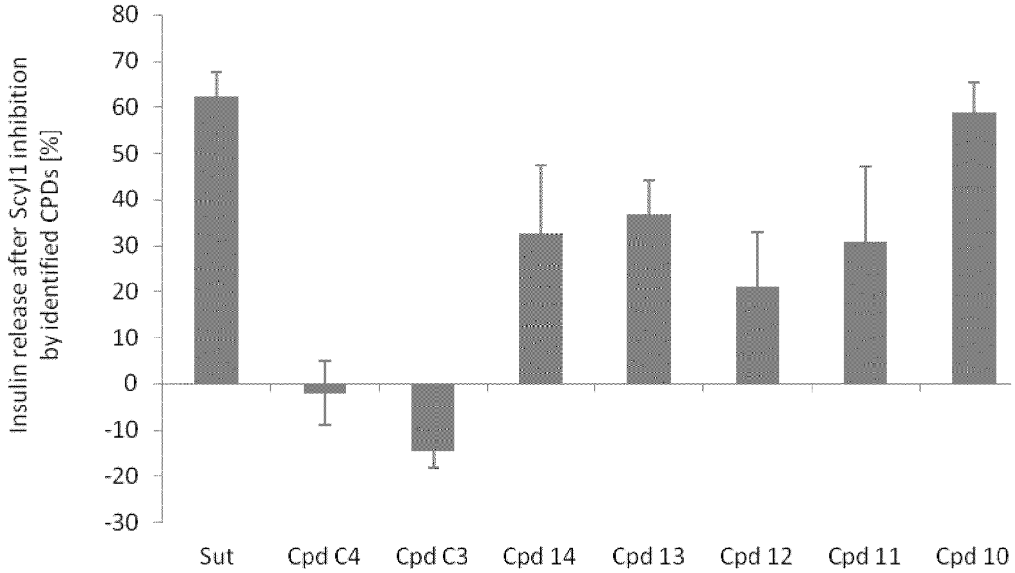


## Figure 3

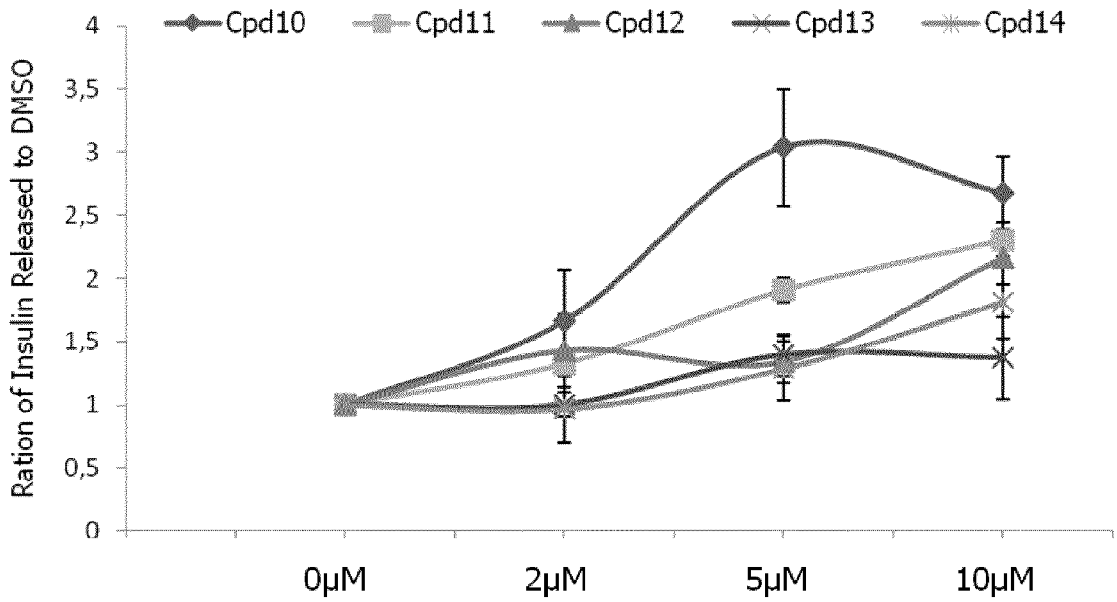

Figure 4 A

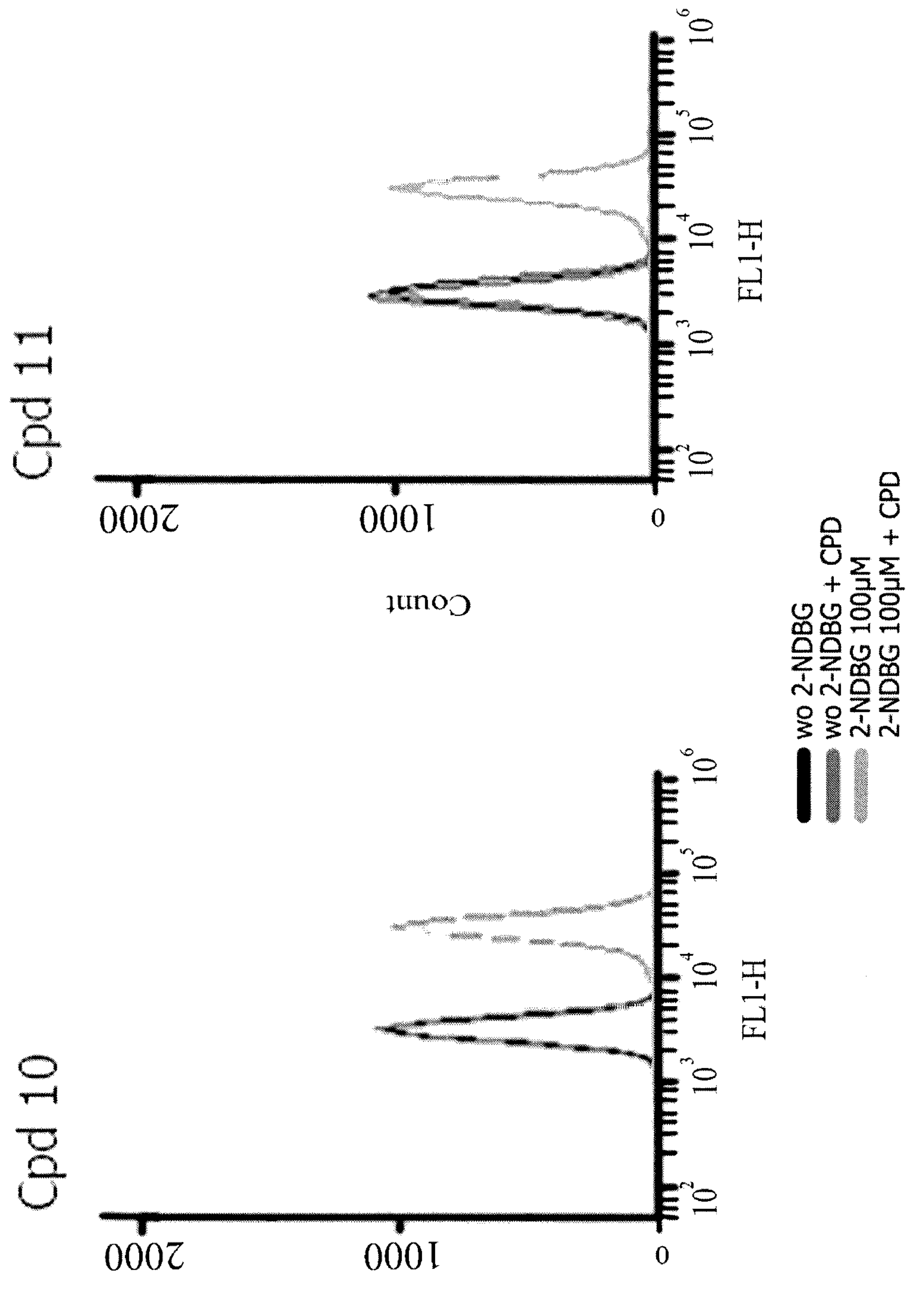

Cpd 10
Cpd 11
Count
2000
1000
0
$10^2$
$10^3$
$10^4$
$10^5$
$10^6$
FL1-H
wo 2-NDBG
wo 2-NDBG + CPD
2-NDBG 100µM
2-NDBG 100µM + CPD

Figure 4 A Continued

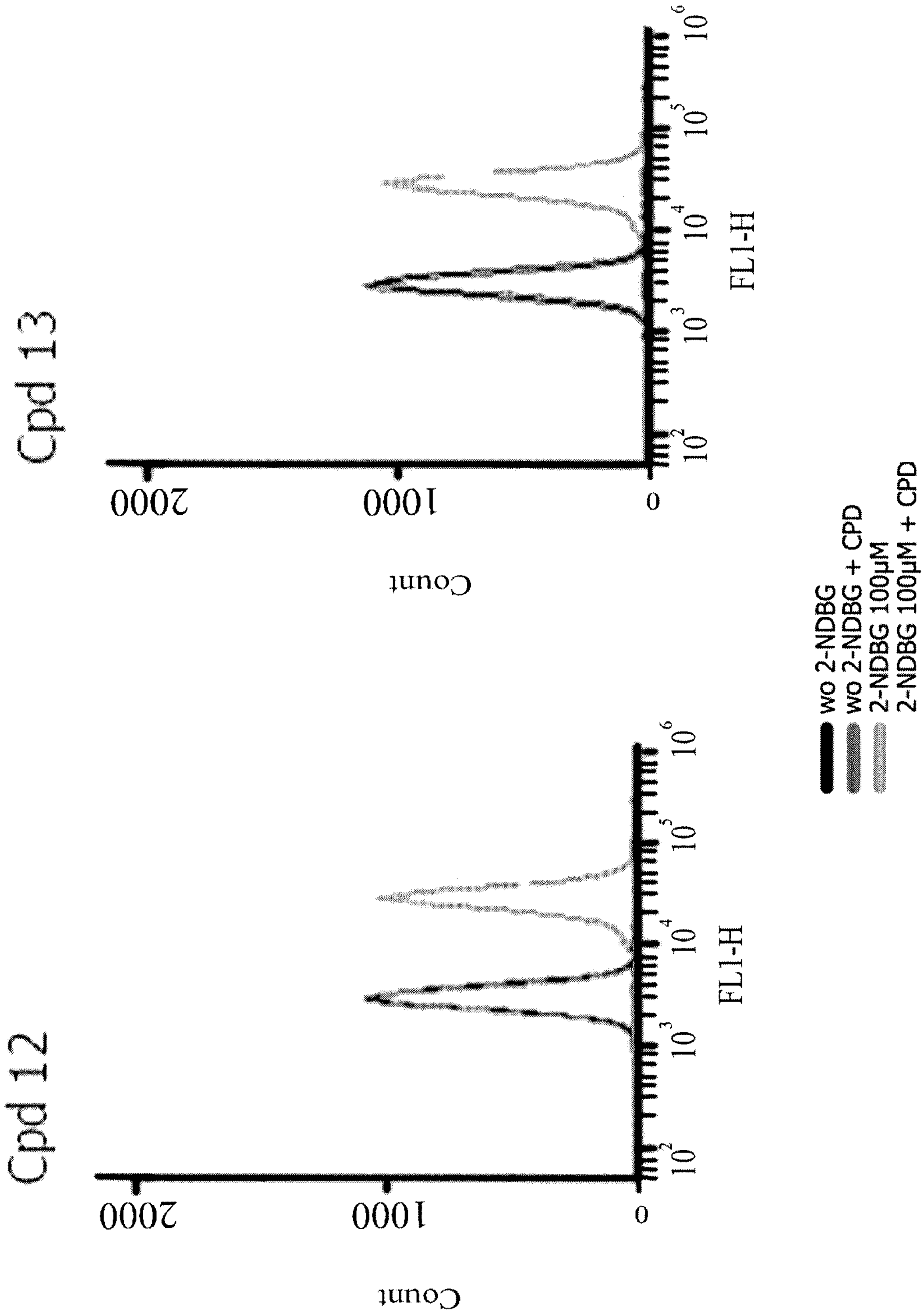

Figure 4 A Continued

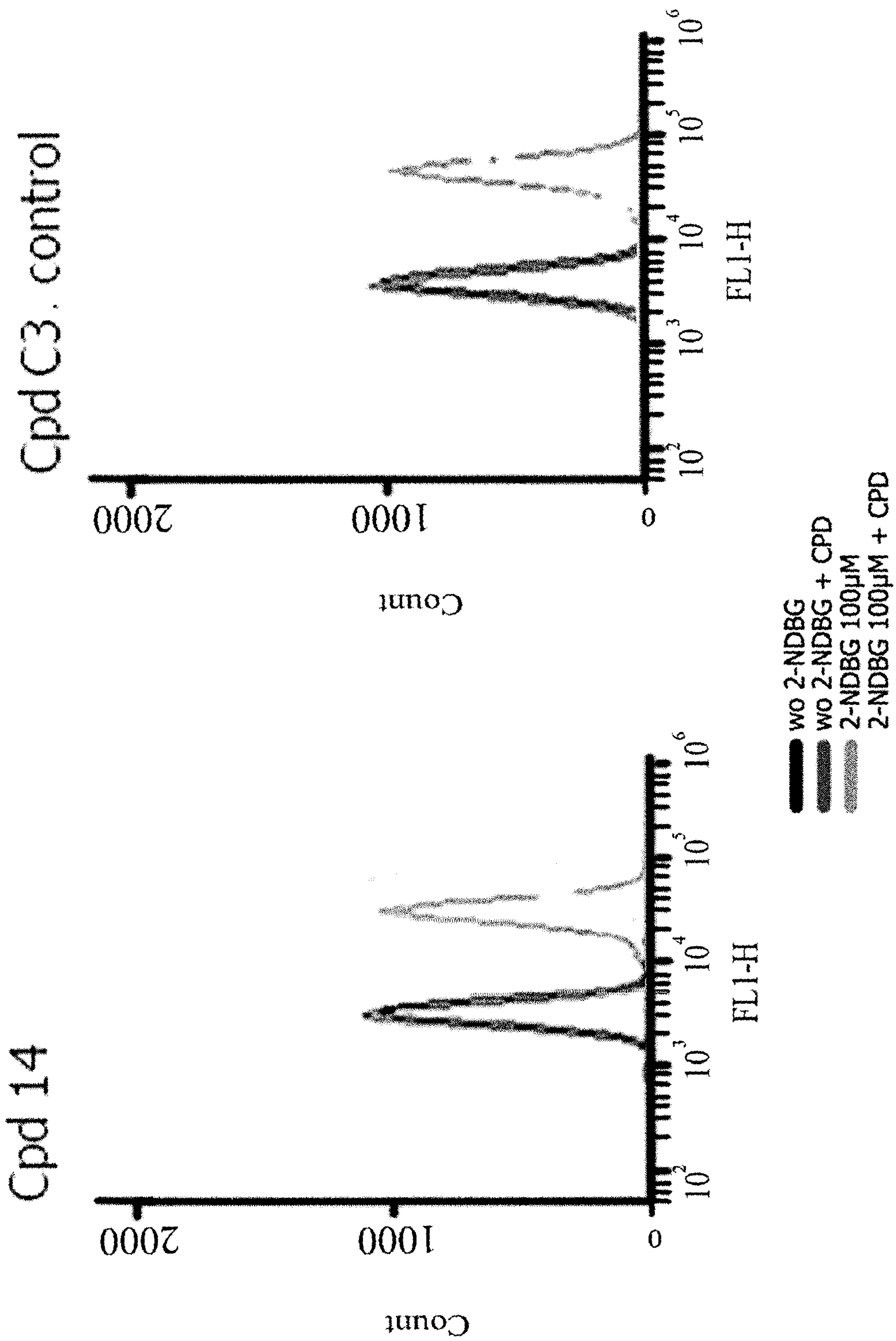

Figure 4 A Continued

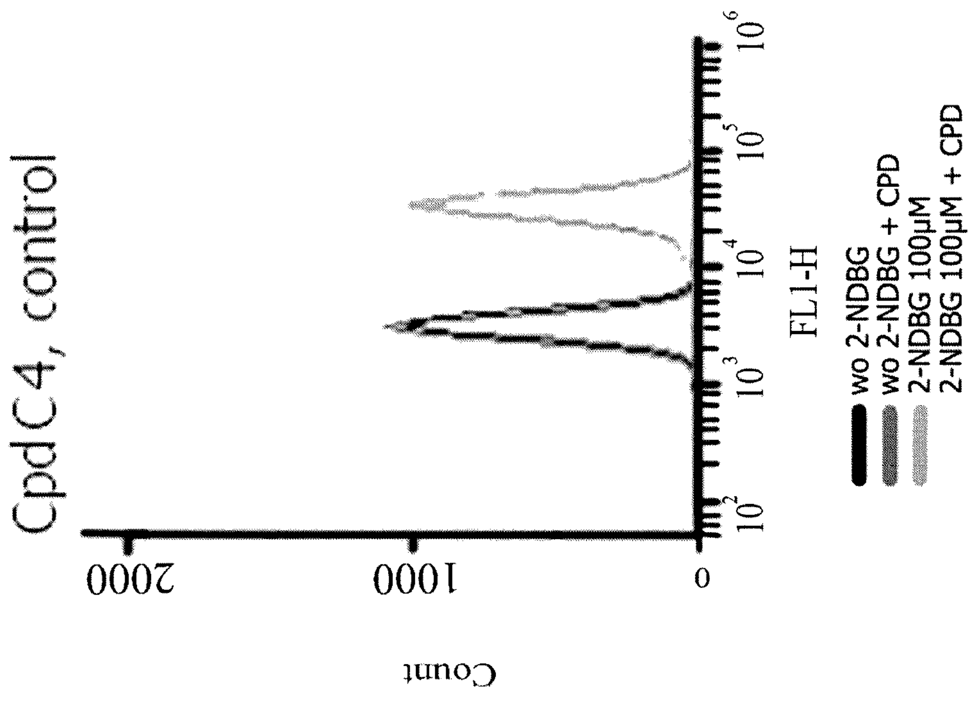

Figure 4 B

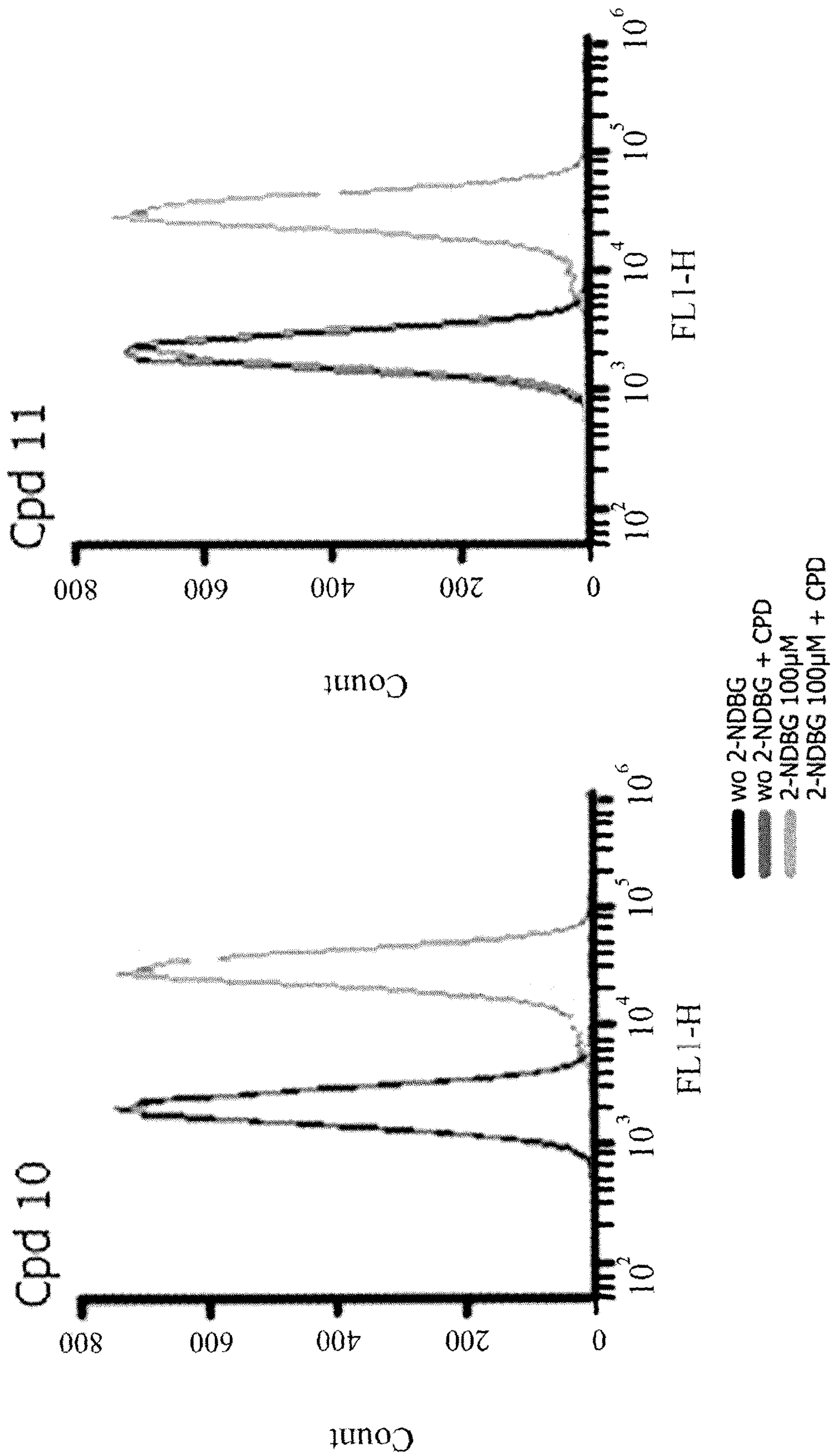
Cpd 10
Cpd 11
Count
0
200
400
600
800
FL1-H
$10^2$
$10^3$
$10^4$
$10^5$
$10^6$
wo 2-NDBG
wo 2-NDBG + CPD
2-NDBG 100µM
2-NDBG 100µM + CPD

Figure 4 B Continued

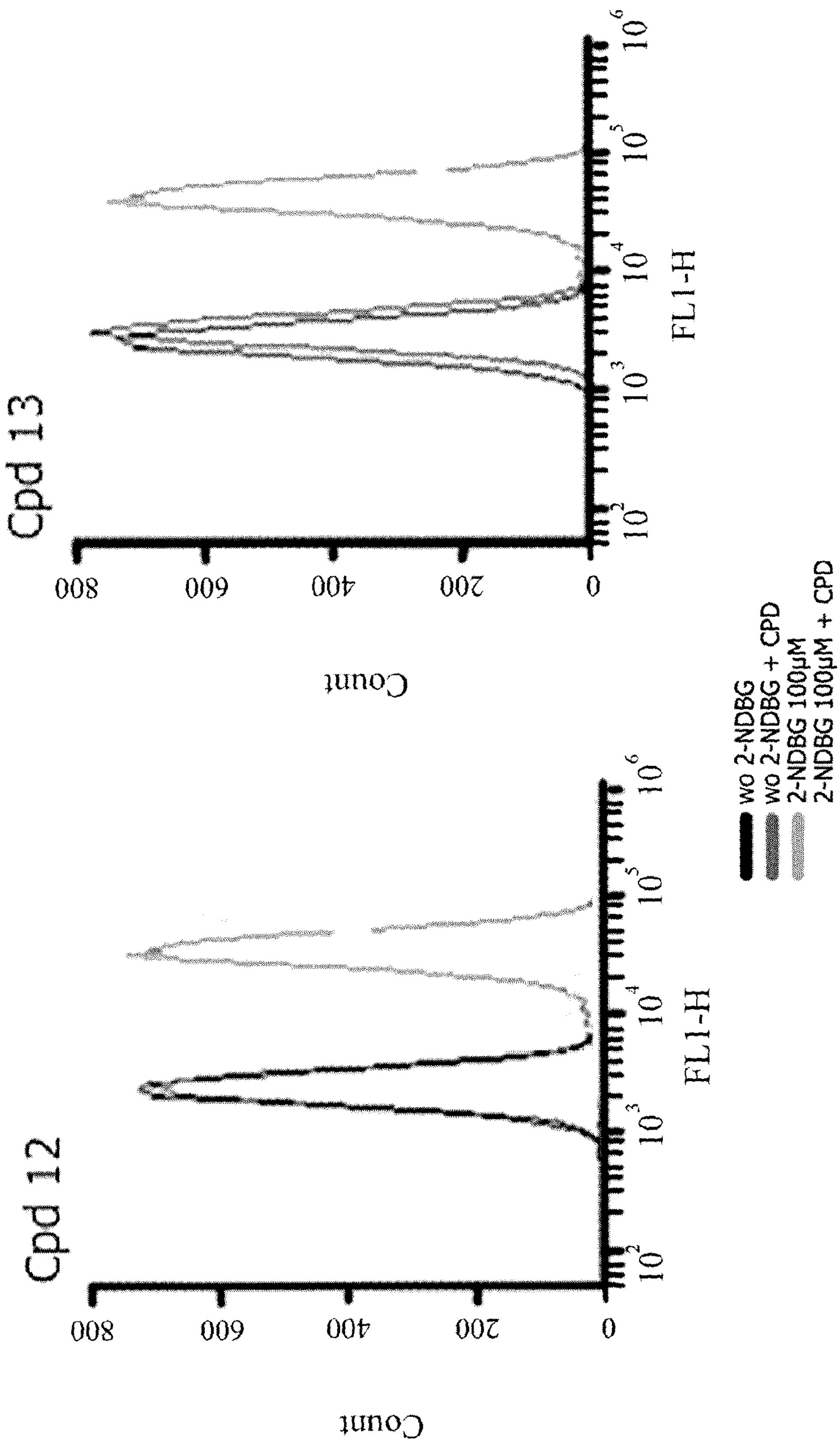

Figure 4 B Continued

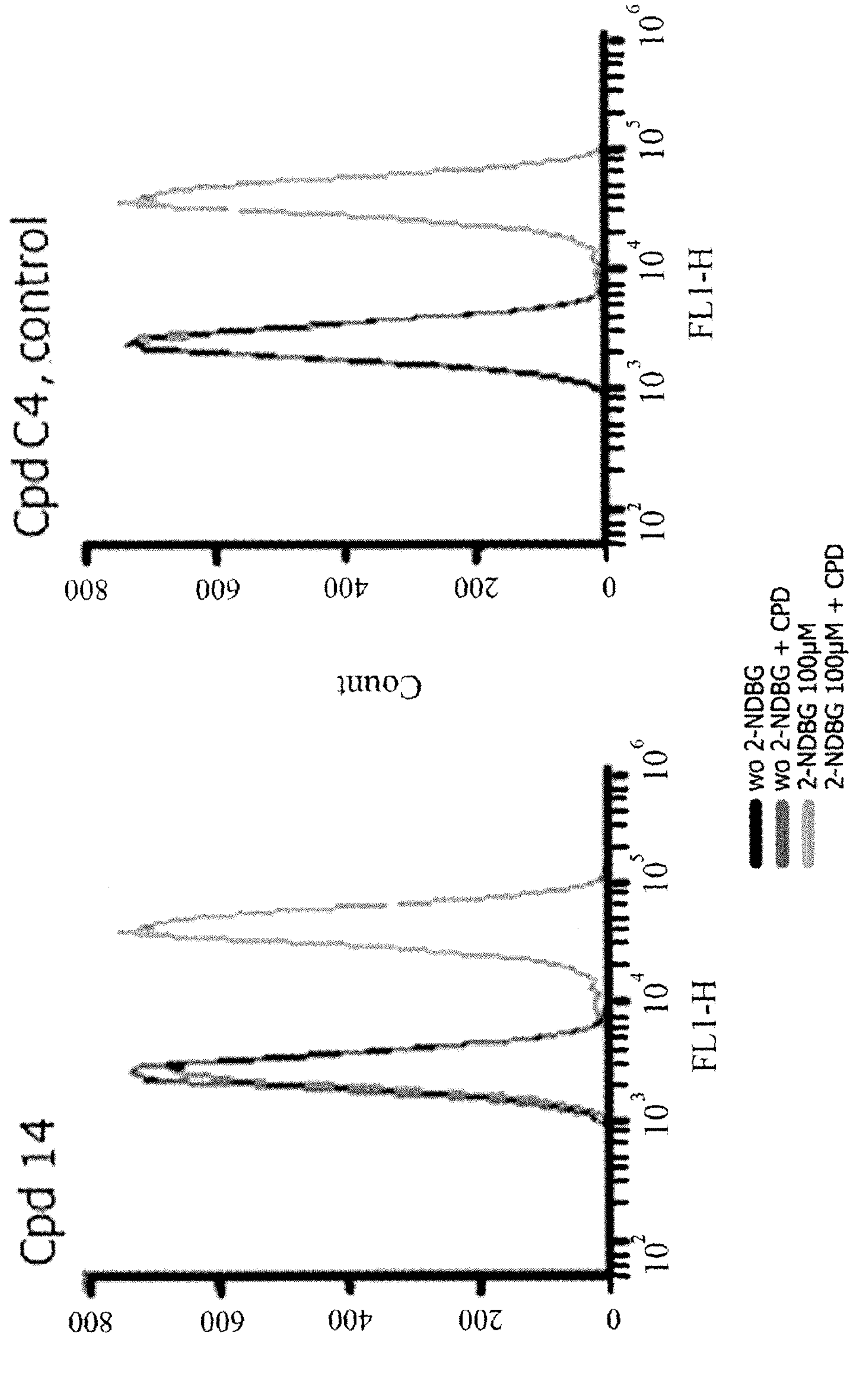

Figure 4 B Continued

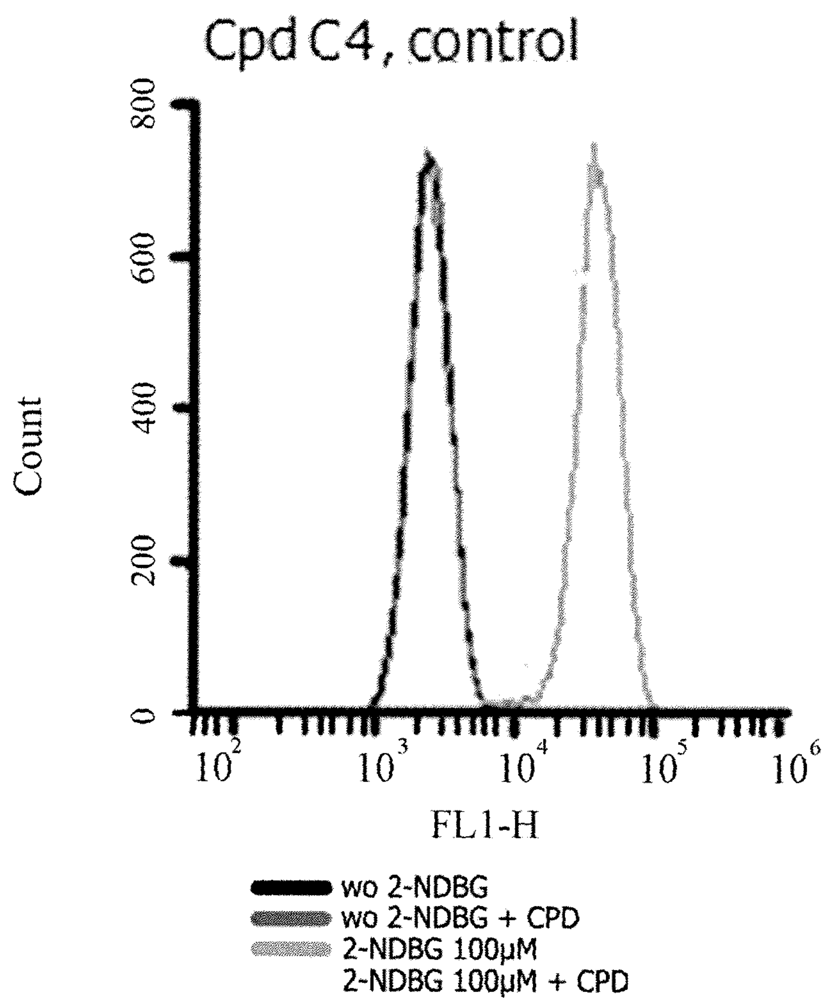

**Figure 5**

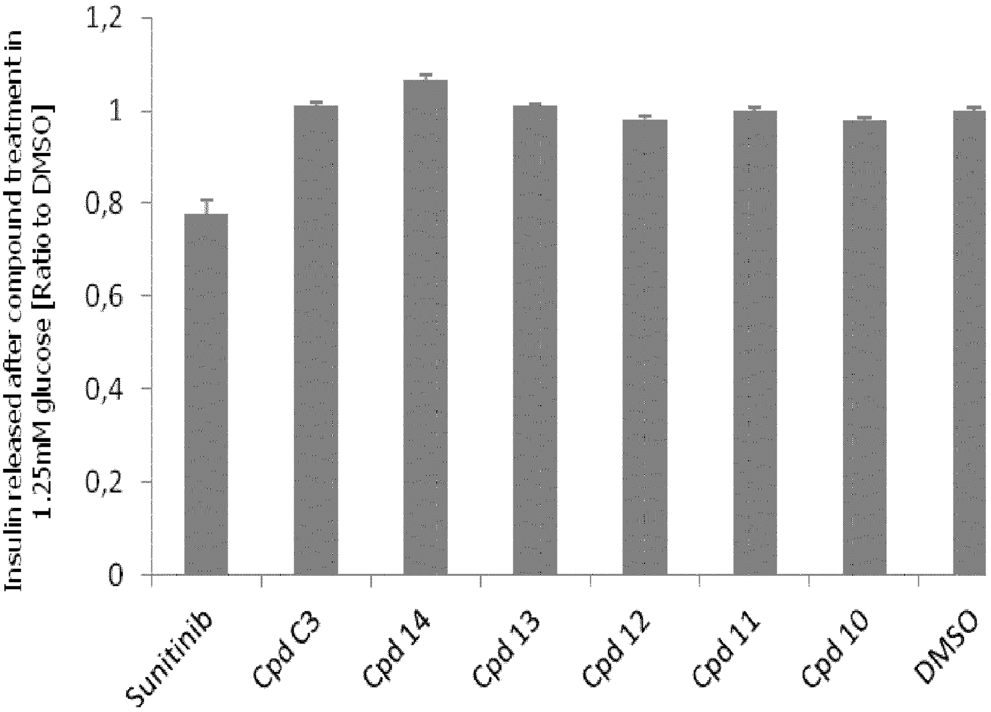


**Figure 6**

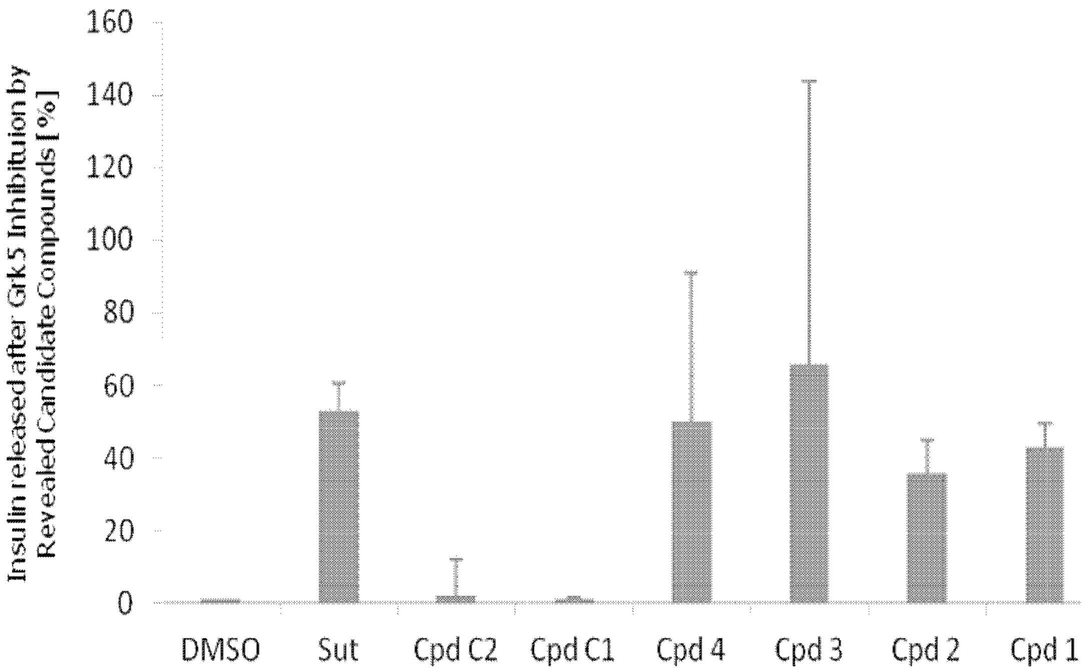

**Figure 7**

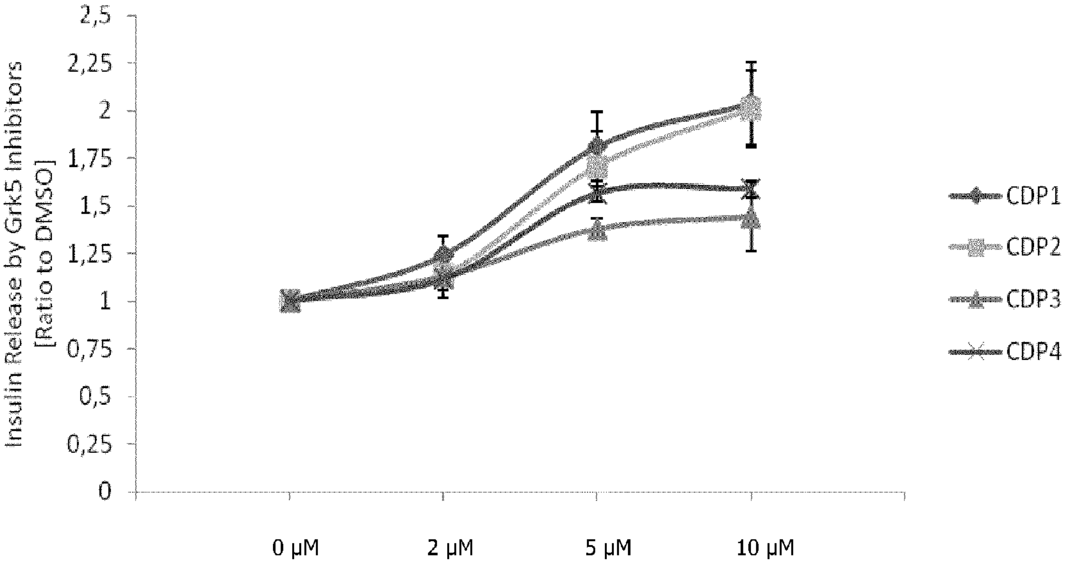
Insulin Release by Grk5 Inhibitors [Ratio to DMSO]
2,5
2,25
2
1,75
1,5
1,25
1
0,75
0,5
0,25
0
0 µM
2 µM
5 µM
10 µM
CDP1
CDP2
CDP3
CDP4

Figure 8

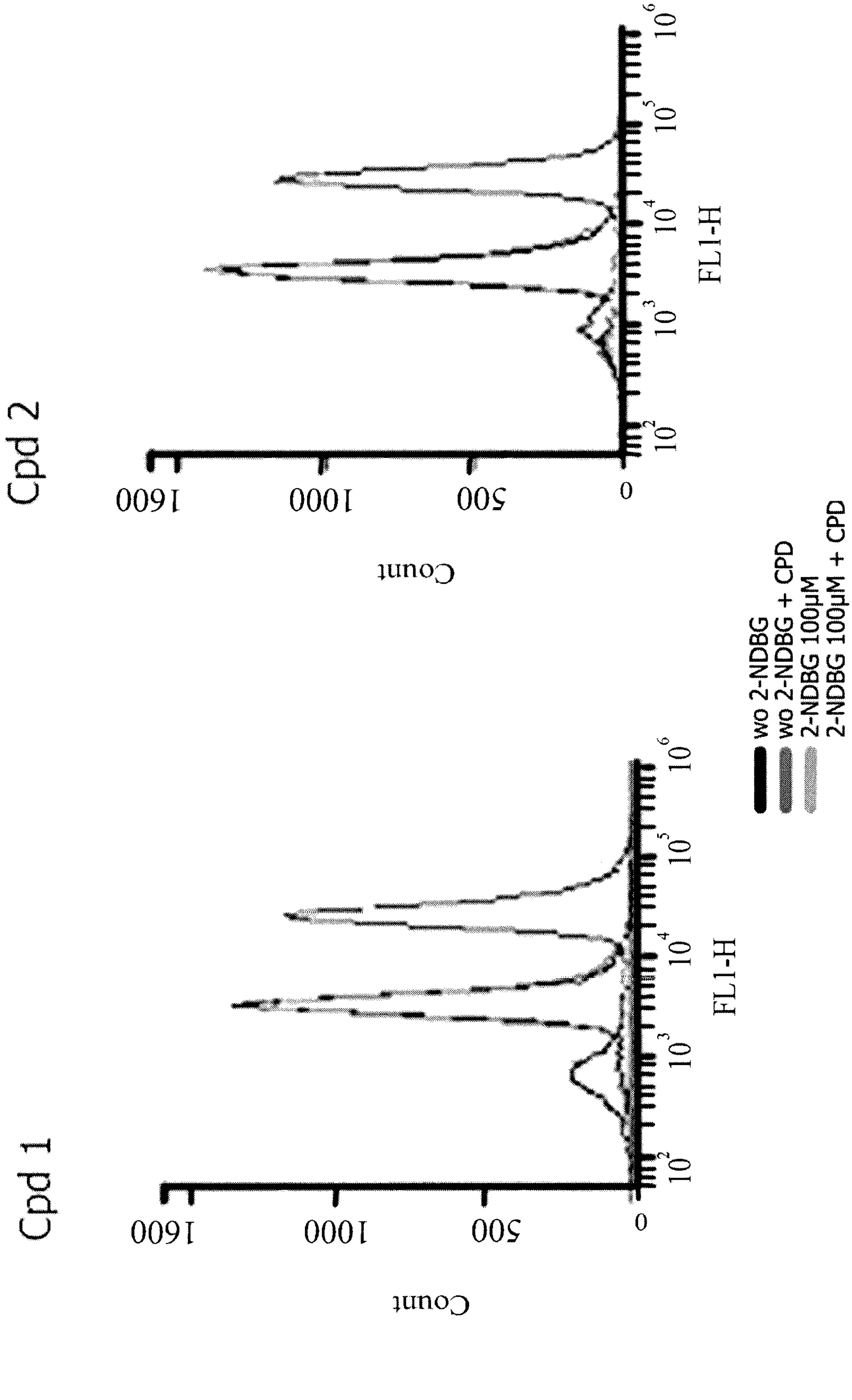
Cpd 1
Cpd 2
Count
FL1-H
1600
1000
500
0
$10^2$
$10^3$
$10^4$
$10^5$
$10^6$
wo 2-NDBG
wo 2-NDBG + CPD
2-NDBG 100µM
2-NDBG 100µM + CPD

Figure 8 Continued

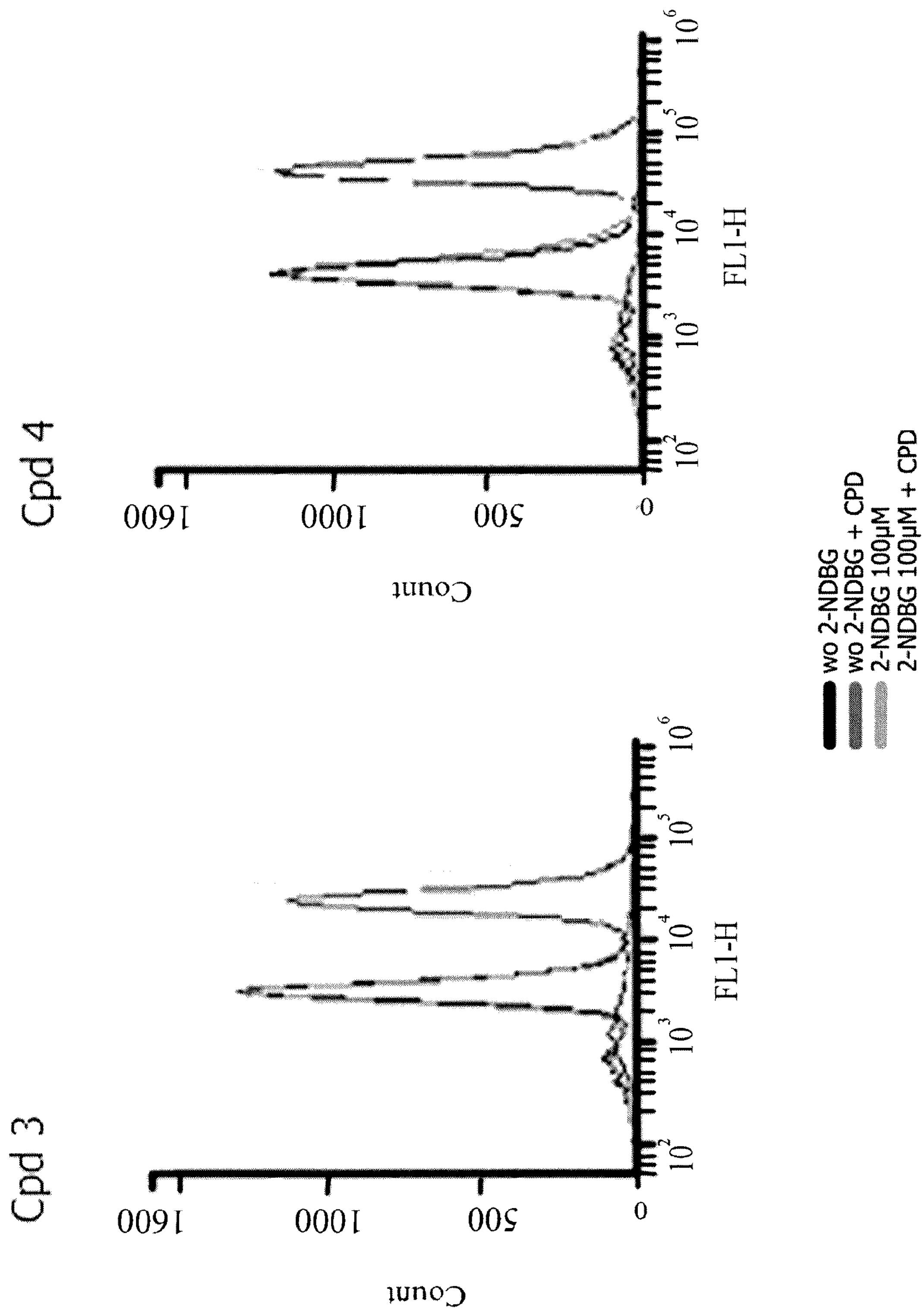

**Figure 8 Continued**

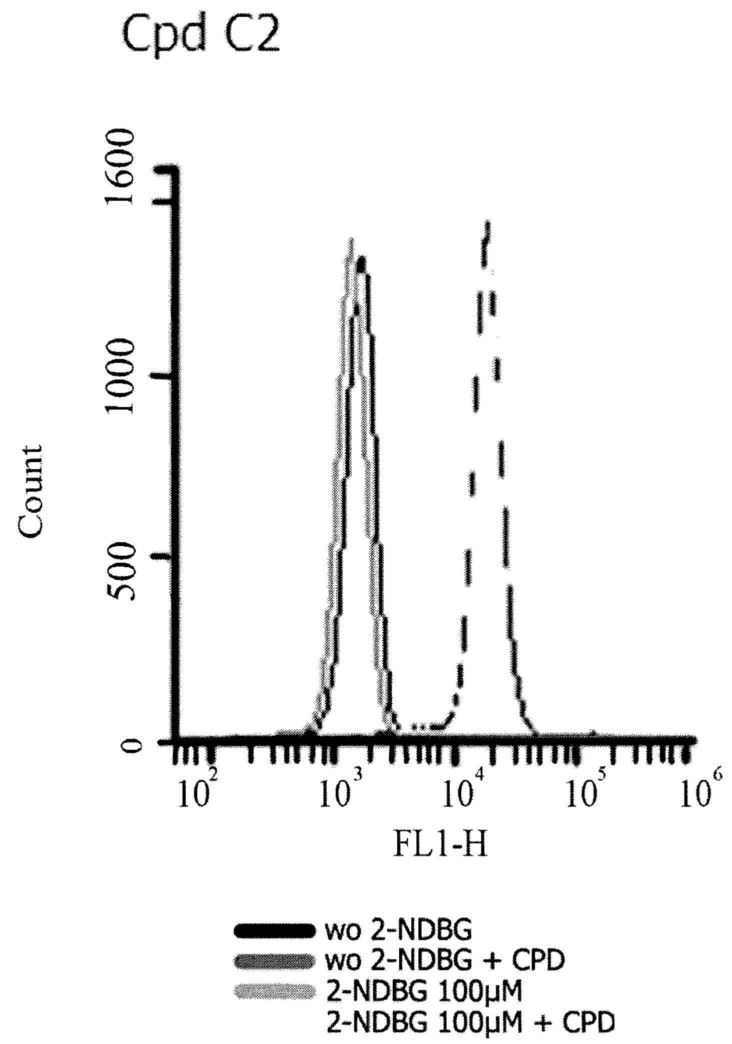

Figure 9

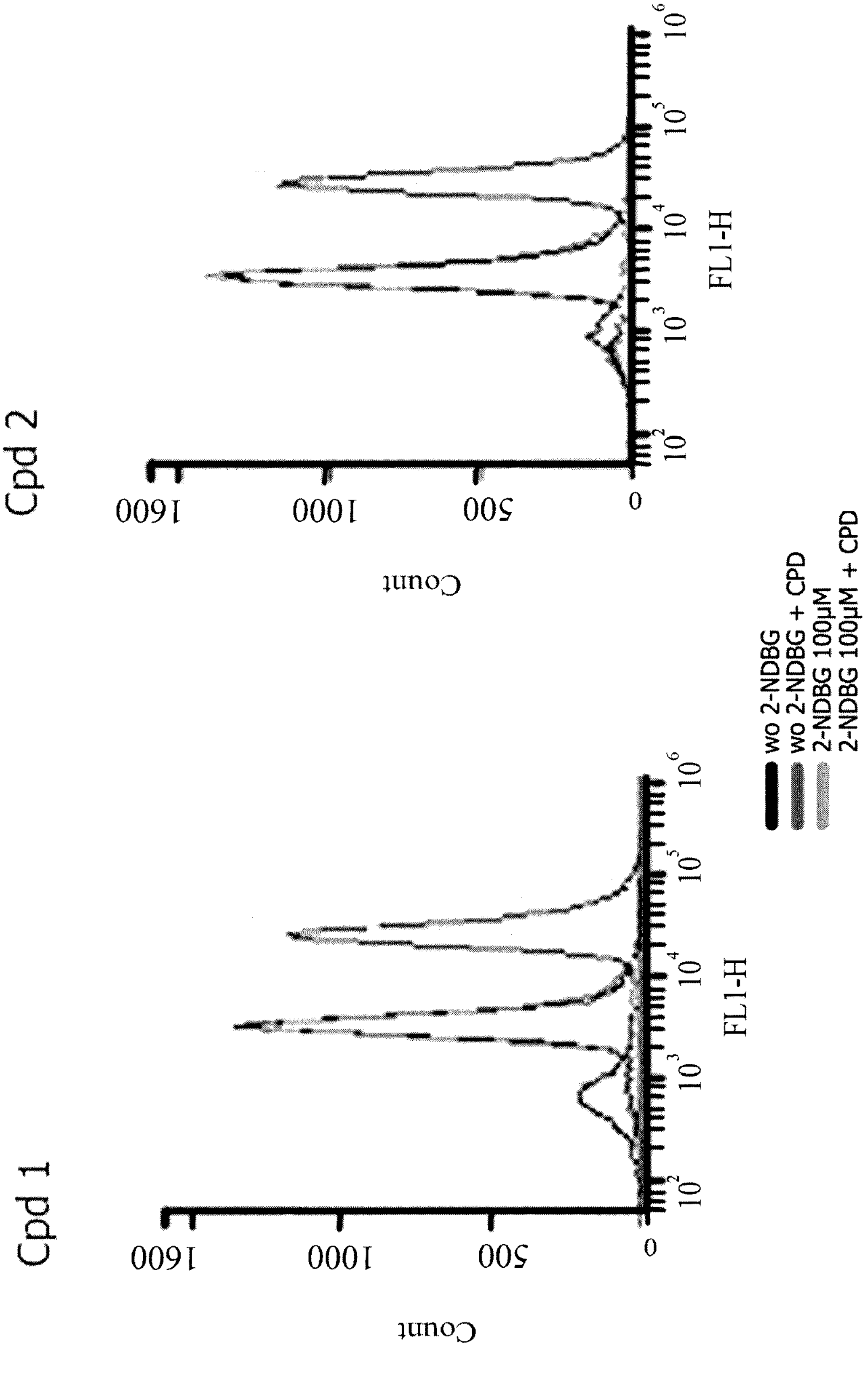
Cpd 1
Cpd 2
Count
0
500
1000
1600
FL1-H
wo 2-NDBG
wo 2-NDBG + CPD
2-NDBG 100µM
2-NDBG 100µM + CPD

**Figure 9 Continued**

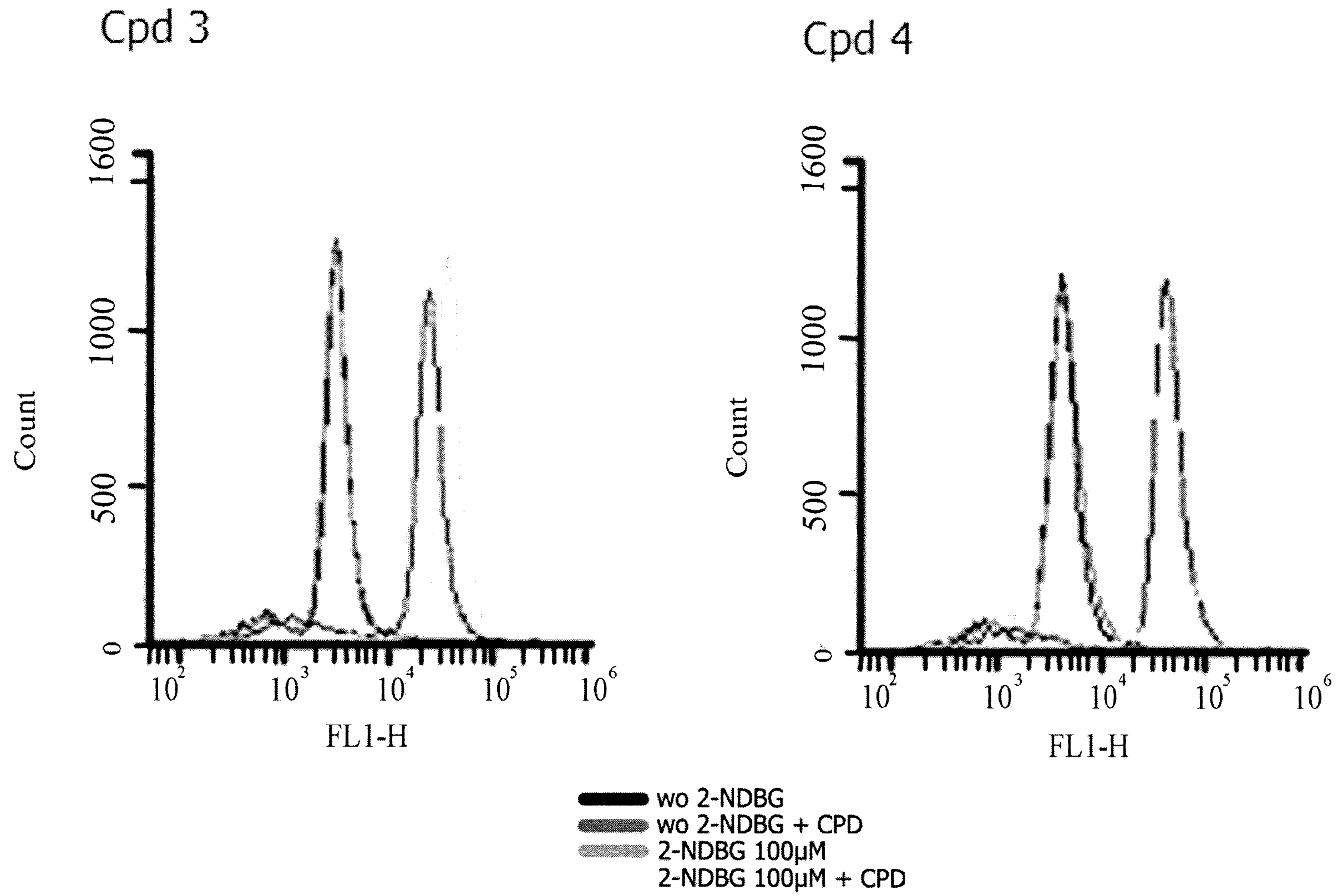

**Figure 9 Continued**

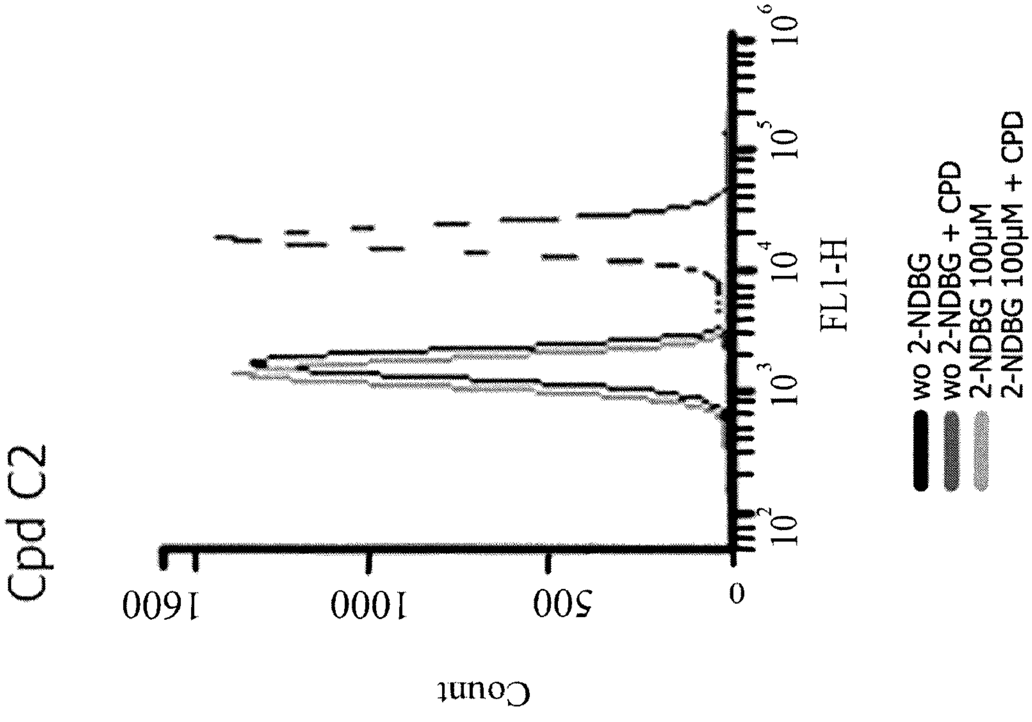

Figure 10

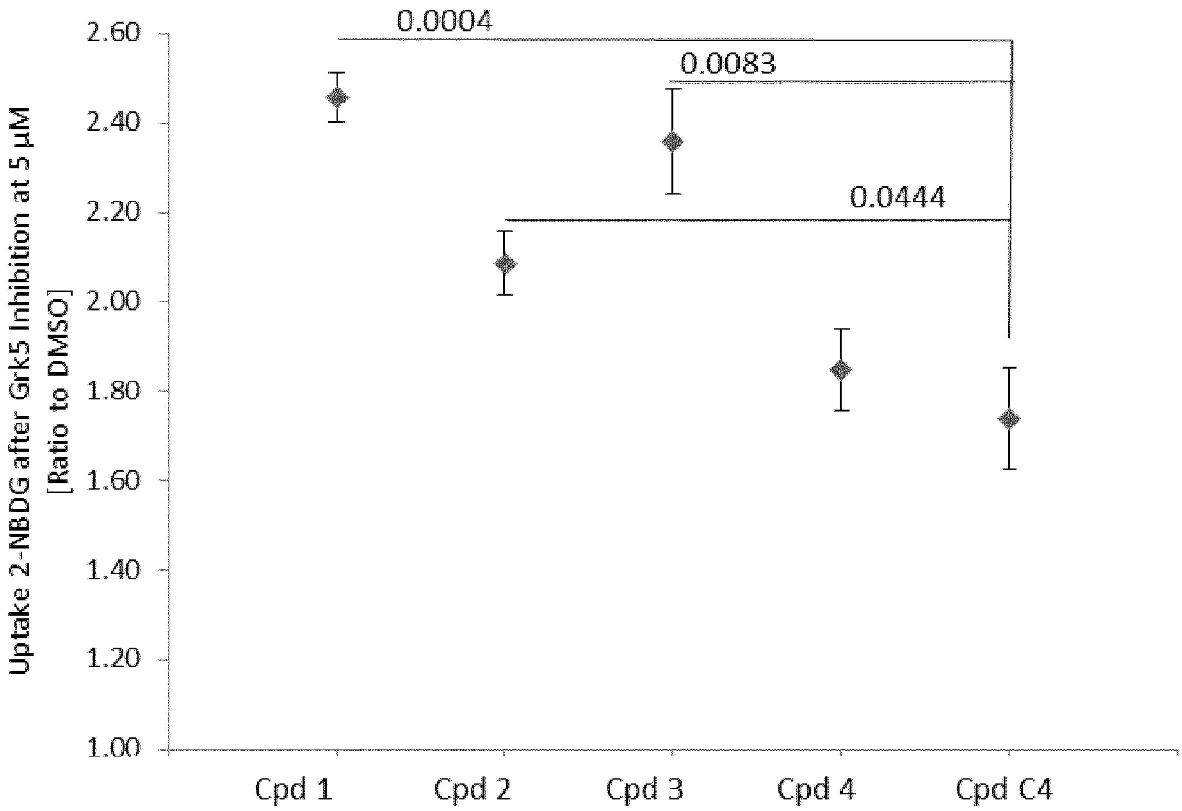
0.0004
0.0083
0.0444
2.60
2.40
2.20
2.00
1.80
1.60
1.40
1.20
1.00
Uptake 2-NBDG after Grk5 Inhibition at 5 µM [Ratio to DMSO]
Cpd 1
Cpd 2
Cpd 3
Cpd 4
Cpd C4

**Figure 11**

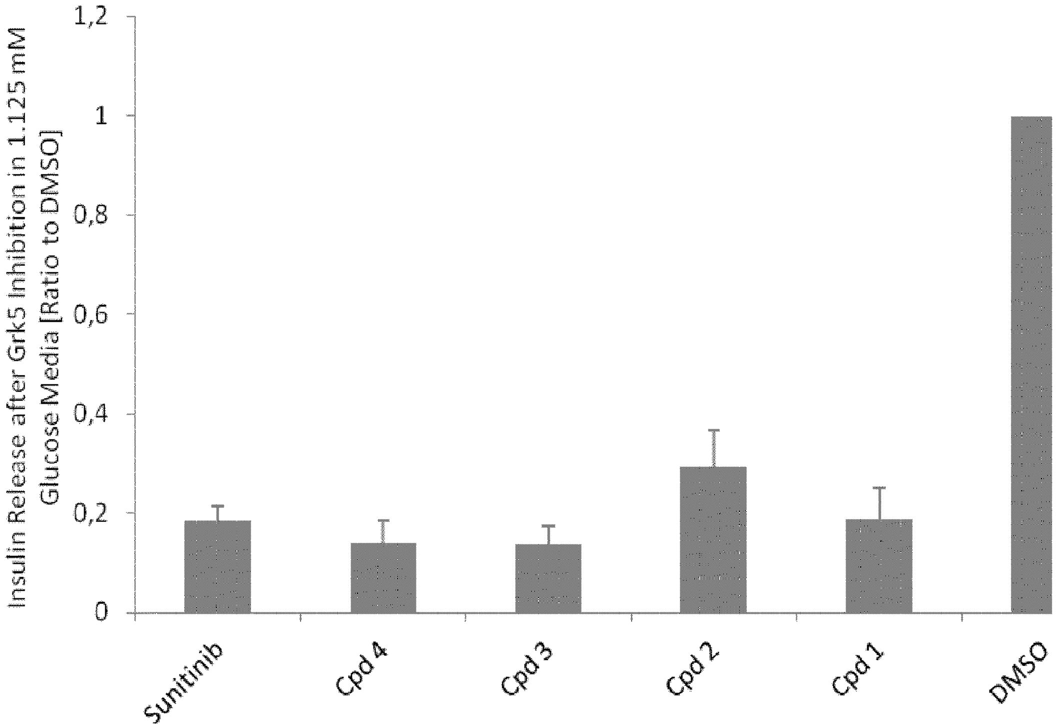


**Figure 12**

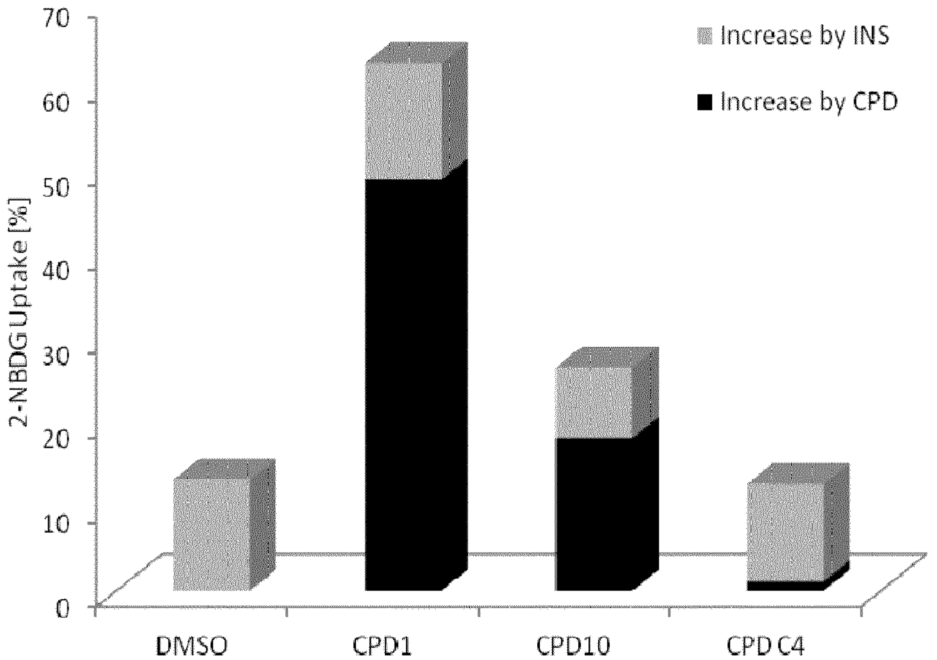

Figure 13

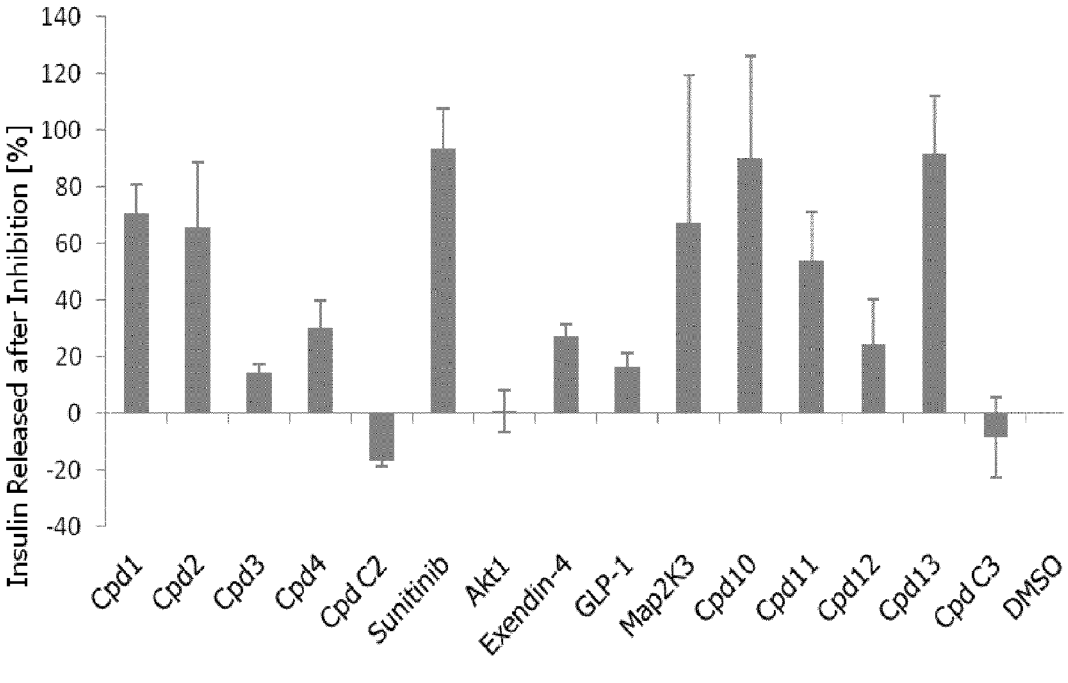

140
120
100
80
60
40
20
0
-20
-40
Insulin Released after Inhibition [%]
Cpd1
Cpd2
Cpd3
Cpd4
Cpd C2
Sunitinib
Akt1
Exendin-4
GLP-1
Map2K3
Cpd10
Cpd11
Cpd12
Cpd13
Cpd C3
DMSO

Europäisches Patentamt
European Patent Office
Office européen des brevets

# EUROPEAN SEARCH REPORT

Application Number
EP 13 17 3962

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2013/059589 A1 (SIRTRIS PHARMACEUTICALS INC [US]; BLUM CHARLES A [US]; SPRINGER STEPHA) 25 April 2013 (2013-04-25)<br>* page 3, line 1 *<br>* page 88 - page 95; table 1; compounds 6-41, 43-48, 50 *<br>* page 97; claim 1 *<br>----- | 1,4-15 | INV.<br>C07D471/04<br>C07D487/04<br>A61K31/437<br>A61K31/4985<br>A61K31/5025<br>A61P3/10 |
| A | WO 2008/069500 A1 (AMOREPACIFIC CORP [KR]; IND ACADEMIC COOP [KR]; CRYSTALGENOMICS INC [K) 12 June 2008 (2008-06-12)<br>* page 36; claim 1 *<br>----- | 1,4-15 | |
| X<br><br>A | US 2011/021521 A1 (CORKEY BRITTON [US] ET AL) 27 January 2011 (2011-01-27)<br>* page 1, paragraph 2 *<br>* page 29, paragraph 531 *<br>* page 32, paragraphs 556, 560, 569, 573, 576 - page 34; example 1 *<br>* page 53, paragraph 1002; example 13 *<br>* page 92; claim 1 *<br>----- | 1,3,5-15<br><br>4 | |
| | -/-- | | TECHNICAL FIELDS SEARCHED (IPC)<br>C07D<br>A61K<br>A61P |

~~The present search report has been drawn up for all claims~~

13

| Place of search | Date of completion of the search | Examiner |
|---|---|---|
| Munich | 27 March 2014 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 3962

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br><br>A | MATTHEWS T P ET AL: "Design and evaluation of 3,6-di(hetero)aryl imidazo[1,2-a]pyrazines as inhibitors of checkpoint and other kinases", BIOORGANIC & MEDICINAL CHEMISTRY LETTERS, PERGAMON, GB, vol. 20, no. 14, 15 July 2010 (2010-07-15), pages 4045-4049, XP027103981, ISSN: 0960-894X [retrieved on 2010-05-27]<br>* page 4046; compounds 5, 8, 9 *<br>* page 4046; table 1; compounds 12-14, 17-20 *<br>* page 4047; table 2; compounds 21-28 *<br>* page 4047; table 3; compounds 31a-31e, 31g, 32a-32i *<br>* page 4045 *<br>----- | 1,4,6,7,13<br><br><br><br><br><br><br><br><br><br>5,8-12,14,15 | |
| X<br><br><br>A | WO 2010/125101 A1 (GLAXO GROUP LTD [GB]; DEAN DAVID KENNETH [GB]; MUNOZ-MURIEDAS JORGE [G) 4 November 2010 (2010-11-04)<br>* page 50; compounds (I1), (I2) *<br>* page 54; compounds 3-(2-pyridinyl)imidazo[1,2-a]pyrazine *<br>----- | 1,4<br><br><br>5-15 | TECHNICAL FIELDS SEARCHED (IPC) |
| X | WO 2004/085409 A2 (BIOFOCUS DISCOVERY LTD [GB]; HARRIS JOHN [GB]; CHURCH NICOLA [GB]; PRO) 7 October 2004 (2004-10-07)<br>* page 39 - page 40; compounds (D), (I) *<br>* page 42; compounds (C), (1) *<br>* page 43; compound (2) *<br>* page 130; claim 5 *<br>* page 157 - page 158; claim 30 *<br>-----<br>-/-- | 1,4-15 | |

~~The present search report has been drawn up for all claims~~

13

| Place of search | Date of completion of the search | Examiner |
|---|---|---|
| Munich | 27 March 2014 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 3962

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | WO 2011/056652 A1 (NEWLINK GENETICS [US]; MAUTINO MARIO R [US]; KUMAR SANJEEV [US]; JAIPU) 12 May 2011 (2011-05-12)<br>* page 81, paragraph 312 *<br>* page 120; compounds 3-(5-chloro-2-methoxyphenyl)imidazo[1,2-a] pyrazine *<br>* page 150; compound 1038 *<br>----- | 1,4,6-14<br>5,15 | |
| X<br>A | ANTHONY R HARRIS ET AL: "Synthesis of 5-bromo-6-methyl imidazopyrazine, 5-bromo and 5-chloro-6-methyl imidazopyridine using electron density surface maps to guide synthetic strategy", TETRAHEDRON, ELSEVIER SCIENCE PUBLISHERS, AMSTERDAM, NL, vol. 67, no. 47, 29 August 2011 (2011-08-29), pages 9063-9066, XP028321817, ISSN: 0040-4020, DOI: 10.1016/J.TET.2011.08.090 [retrieved on 2011-09-03]<br>* page 9063; compound 3 *<br>* page 9064; compounds 7, 9-10 *<br>----- | 1,4<br>5-15 | TECHNICAL FIELDS SEARCHED (IPC) |
| X<br>A | YANYANG LI ET AL: "Design, synthesis and bioevalution of novel benzamides derivatives as HDAC inhibitors", BIOORGANIC & MEDICINAL CHEMISTRY LETTERS, vol. 23, no. 1, 1 January 2013 (2013-01-01), pages 179-182, XP055087099, ISSN: 0960-894X, DOI: 10.1016/j.bmcl.2012.10.114<br>* page 180; compound 1 *<br>-----<br>-/-- | 1<br>4-15 | |

~~The present search report has been drawn up for all claims~~

13

| Place of search | Date of completion of the search | Examiner |
|---|---|---|
| Munich | 27 March 2014 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 3962

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ASHISH T. BAVISKAR ET AL: "N-Fused Imidazoles As Novel Anticancer Agents That Inhibit Catalytic Activity of Topoisomerase II[alpha] and Induce Apoptosis in G1/S Phase", JOURNAL OF MEDICINAL CHEMISTRY, vol. 54, no. 14, 28 July 2011 (2011-07-28), pages 5013-5030, XP055087101, ISSN: 0022-2623, DOI: 10.1021/jm200235u | 1 | |
| A | * page 5015; figure 2; compound (22) * | 4-15 | |
| X | US 2012/245178 A1 (SU WEI-GUO [CN] ET AL) 27 September 2012 (2012-09-27) | 1,4 | |
| A | * page 30, paragraph 332; compounds 5, 5-2 * | 5-15 | |
| X | MINGJUAN SU ET AL: "A Bulky Biaryl Phosphine Ligand Allows for Palladium-Catalyzed Amidation of Five-Membered Heterocycles as Electrophiles", ANGEWANDTE CHEMIE INTERNATIONAL EDITION, vol. 51, no. 19, 7 May 2012 (2012-05-07), pages 4710-4713, XP055087017, ISSN: 1433-7851, DOI: 10.1002/anie.201201244 | 1,4 | TECHNICAL FIELDS SEARCHED (IPC) |
| A | * page 4712; table 2; compound 7 * | 5-15 | |
| X | WO 03/070732 A1 (UPJOHN CO [US]; ROGERS BRUCE N [US]; PIOTROWSKI DAVID W [US]; WALKER D) 28 August 2003 (2003-08-28) | 1 | |
| A | * ethyl imidazo[1,2-a]pyrazine-6-carboxylate; page 115, line 10 - line 11; compounds imidazo[1,2-a]pyrazine-6-carboxylic acid * | 4-15 | |

-/--

~~The present search report has been drawn up for all claims~~

13

| Place of search | Date of completion of the search | Examiner |
|---|---|---|
| Munich | 27 March 2014 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 3962

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X<br><br><br>A | WO 2013/034048 A1 (MERCK SHARP & DOHME [US]; MCCOMAS CASEY CAMERON [US]; LIVERTON NIGEL J) 14 March 2013 (2013-03-14)<br>* page 75; compounds 6-bromo-3-iodoimidazo[1,2-a]pyrazine *<br>* page 75; compounds 6-bromoimidazo[1,2-a]pyrazine *<br>* page 75; compounds 6-bromo-3-(4-fluorophenyl)imidazo[1,2-a]pyrazine *<br>----- | 1,4<br><br><br>5-15 | |
| X<br><br><br>A | WO 2013/033116 A1 (IRM LLC [US]; LIU XIAODONG [US]; LI XIAOLIN [US]; LOREN JON [US]; MOLT) 7 March 2013 (2013-03-07)<br>* page 95; compound 24m *<br>----- | 1<br><br><br>4-15 | |
| X<br><br><br>A | WO 2012/098416 A1 (SUN PHARMA ADVANCED RES CO LTD [IN]; SENGUPTA PRABAL [IN]; CHOKSHI HEM) 26 July 2012 (2012-07-26)<br>* page 48; example I.15; compounds 3-ethynylimidazo[1,2-a]pyrazine *<br>----- | 1<br><br><br>4-15 | TECHNICAL FIELDS SEARCHED (IPC) |
| X<br><br><br><br><br><br><br><br><br><br>A | LUMMA W C ET AL: "PIPERAZINYLIMIDAZOLÚ1,2-A 3/4 PYRAZINES WITH SELECTIVE AFFINITY FOR IN VITRO ALPHA-ADRENERGIC RECEPTOR SUBTYPES", JOURNAL OF MEDICINAL CHEMISTRY, AMERICAN CHEMICAL SOCIETY, US, vol. 26, no. 3, 1 January 1983 (1983-01-01), pages 357-363, XP002049103, ISSN: 0022-2623, DOI: 10.1021/JM00357A009<br>* page 358; compound 3g *<br>-----<br>-/-- | 1,4<br><br><br><br><br><br><br><br><br><br>5-15 | |

13

~~The present search report has been drawn up for all claims~~

| Place of search | Date of completion of the search | Examiner |
|---|---|---|
| Munich | 27 March 2014 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 3962

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br>A | WO 03/076442 A1 (LILLY CO ELI [US]; CLAYTON JOSHUA RYAN [US]; DIEFENBACHER CLIVE GIDEON) 18 September 2003 (2003-09-18)<br>* page 78; compounds 2-(imidazo[1,2-a]pyrazin-3-yl)acetamide * | 1<br><br><br><br>4-15 | |
| X<br><br><br><br><br><br><br><br><br>A | ISAO HYODO ET AL: "Catalytic Arylation of a CH Bond in Pyridine and Related Six-Membered N-Heteroarenes Using Organozinc Reagents", CHEMISTRY - AN ASIAN JOURNAL, vol. 7, no. 6, 22 June 2012 (2012-06-22), pages 1357-1365, XP055087027, ISSN: 1861-4728, DOI: 10.1002/asia.201100971<br>* page 1360; compound 11 * | 1,4<br><br><br><br><br><br><br><br><br>5-15 | |
| A | WO 2008/051405 A1 (MERCK & CO INC [US]; DOBBELAAR PETER H [US]; FRANKLIN CHRISTOPHER L [U) 2 May 2008 (2008-05-02)<br>* page 104; compound 160 * | 1,4-15 | TECHNICAL FIELDS SEARCHED (IPC) |
| X<br><br>A | US 2 917 511 A (BICKING JOHN B) 15 December 1959 (1959-12-15)<br>* column 2 - column 4; examples 1, 3-17 * | 1,3<br><br>5-15 | |
| X<br><br>A | US 3 050 525 A (BICKING JOHN B) 21 August 1962 (1962-08-21)<br>* column 2; example 5 * | 1<br><br>3,5-15 | |

-/--

~~The present search report has been drawn up for all claims~~

13

| Place of search | Date of completion of the search | Examiner |
|---|---|---|
| Munich | 27 March 2014 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 3962

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | WO 2012/065297 A1 (IMPACT THERAPEUTICS INC [CN]; DONG HAIJUN [CN]; CAI SUIXIONG [CN]; TIA) 24 May 2012 (2012-05-24)<br>* page 19 - page 21; examples 1-5, 8, 10 *<br>* page 22; examples 11-13, 15 *<br>* page 23 - page 27; examples 17-28, 31-33, 35-36 *<br>* page 28 - page 33; examples 39-53 *<br>* page 34 - page 35; examples 56-58 *<br>* page 36 - page 43; examples 60-68, 70-71, 73, 76-78, 81-84, 86-87, 89-91 *<br>* page 51; claim 1 *<br>* page 55; claim 7 *<br>----- | 1,3,6,13<br>5,7-12, 14,15 | |
| X<br>A | US 2013/150343 A1 (VAN NIEL MONIQUE BODIL [GB] ET AL) 13 June 2013 (2013-06-13)<br>* page 61, paragraphs 693, 695; compounds 24a, 24b *<br>----- | 1,3<br>5-15 | |
| X<br><br>A | POTTS K T ET AL: "1,2,4-Triazoles.XII. Derivatives of the sigma-Triazolo[4,3-alpha]pyridine Ring System",<br>THE JOURNAL OF ORGANIC CHEMISTRY, AMERICAN CHEMICAL SOCIETY, US,<br>vol. 31, no. 1,<br>1 January 1966 (1966-01-01), pages 251-260, XP002369654,<br>ISSN: 0022-3263, DOI: 10.1021/JO01339A057<br>* page 253; tables II-III *<br>* page 254; table V *<br>* page 256; table VII *<br>----- | 1,3<br><br>5-15 | TECHNICAL FIELDS SEARCHED (IPC) |
| X<br>A | US 4 244 953 A (TRUST RONALD I ET AL)<br>13 January 1981 (1981-01-13)<br>* column 7 - column 8; compounds XXVIII, XXIX *<br>* column 10; compound XXX *<br>-----<br>-/-- | 1,3<br>5-15 | |

13

~~The present search report has been drawn up for all claims~~

| Place of search | Date of completion of the search | Examiner |
|---|---|---|
| Munich | 27 March 2014 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 3962

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2011/098471 A1 (KATOH ISSEI [JP] ET AL) 28 April 2011 (2011-04-28)<br>* page 62, paragraph 515 - paragraph 518; table 1; compound 10 * | 1,3<br>5-15 | |
| X<br>A | WO 2010/019899 A1 (TAKEDA PHARMACEUTICAL [JP]; BRESSI JEROME C [US]; CHU SHAOSONG [US]; E) 18 February 2010 (2010-02-18)<br>* page 129, paragraph 413 - paragraph 414 * | 1<br>3,5-15 | |
| A | WO 2011/060235 A1 (SCHERING CORP [US]; RAO ASHWIN U [US]; PALANI ANANDAN [US]; ASLANIAN R) 19 May 2011 (2011-05-19)<br>* page 42; claim 1 * | 1,3,5-15 | |
| A | WO 2008/057855 A2 (SQUIBB BRISTOL MYERS CO [US]; DHAR T G MURALI [US]; XIAO HAI-YUN [US];) 15 May 2008 (2008-05-15)<br>* page 88; claim 11 * | 1,3,5-15 | TECHNICAL FIELDS SEARCHED (IPC) |
| X<br>A | WO 2004/043940 A1 (MERCK & CO INC [US]; COLANDREA VINCENT J [US]; EDMONDSON SCOTT D [US];) 27 May 2004 (2004-05-27)<br>* page 80; compound 12 * | 1<br>3,5-15 | |

~~The present search report has been drawn up for all claims~~

13

| Place of search | Date of completion of the search | Examiner |
|---|---|---|
| Munich | 27 March 2014 | Jeanjean, Fabien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 13 17 3962

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

3, 4(completely); 1, 5-15(partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 13 17 3962

The Search Division considers that the present European patent application does not comply with the requirements of unity of invention and relates to several inventions or groups of inventions, namely:

1. claims: 4(completely); 1, 5-15(partially)

   Compound of formula (I) wherein A1= CH, A2= CH and A3= N and its related subject-matter

   ---

2. claims: 3(completely); 1, 5-15(partially)

   Compound of formula (I) wherein A1= N, A2= CH and A3= CH and its related subject-matter

   ---

3. claims: 2(completely); 1, 5-15(partially)

   Compound of formula (I) wherein A1= CH, A2= N and A3= CH and its related subject-matter

   ---

## ANNEX TO THE EUROPEAN SEARCH REPORT ON EUROPEAN PATENT APPLICATION NO. EP 13 17 3962

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2014

| Patent document cited in search report | Publication date | Patent family member(s) | Publication date |
|---|---|---|---|
| WO 2013059589 A1 | 25-04-2013 | NONE | |
| WO 2008069500 A1 | 12-06-2008 | KR 20080052024 A | 11-06-2008 |
| | | WO 2008069500 A1 | 12-06-2008 |
| US 2011021521 A1 | 27-01-2011 | AR 077598 A1 | 07-09-2011 |
| | | AU 2010276537 A1 | 24-05-2012 |
| | | CA 2774715 A1 | 03-02-2011 |
| | | CN 102725290 A | 10-10-2012 |
| | | EA 201290121 A1 | 30-10-2012 |
| | | EP 2464645 A1 | 20-06-2012 |
| | | TW 201116528 A | 16-05-2011 |
| | | US 2011021521 A1 | 27-01-2011 |
| | | UY 32805 A | 28-02-2011 |
| | | WO 2011014462 A1 | 03-02-2011 |
| WO 2010125101 A1 | 04-11-2010 | EP 2424867 A1 | 07-03-2012 |
| | | ES 2439042 T3 | 21-01-2014 |
| | | JP 2012525351 A | 22-10-2012 |
| | | US 2012172366 A1 | 05-07-2012 |
| | | WO 2010125101 A1 | 04-11-2010 |
| WO 2004085409 A2 | 07-10-2004 | GB 2400101 A | 06-10-2004 |
| | | WO 2004085409 A2 | 07-10-2004 |
| WO 2011056652 A1 | 12-05-2011 | CA 2778115 A1 | 12-05-2011 |
| | | EP 2493862 A1 | 05-09-2012 |
| | | US 2012277217 A1 | 01-11-2012 |
| | | WO 2011056652 A1 | 12-05-2011 |
| US 2012245178 A1 | 27-09-2012 | NONE | |
| WO 03070732 A1 | 28-08-2003 | AU 2003219690 A1 | 09-09-2003 |
| | | BR 0307735 A | 25-01-2005 |
| | | CA 2476681 A1 | 28-08-2003 |
| | | EP 1476449 A1 | 17-11-2004 |
| | | JP 2005523288 A | 04-08-2005 |
| | | MX PA04008038 A | 26-11-2004 |
| | | US 2003236264 A1 | 25-12-2003 |
| | | US 2005215584 A1 | 29-09-2005 |
| | | WO 03070732 A1 | 28-08-2003 |
| WO 2013034048 A1 | 14-03-2013 | TW 201317230 A | 01-05-2013 |
| | | WO 2013033899 A1 | 14-03-2013 |
| | | WO 2013034048 A1 | 14-03-2013 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 13 17 3962

This annex lists the patent family members relating to the patent documents cited in the above-mentioned European search report. The members are as contained in the European Patent Office EDP file on
The European Patent Office is in no way liable for these particulars which are merely given for the purpose of information.

27-03-2014

| Patent document cited in search report | Publication date | Patent family member(s) | Publication date |
|---|---|---|---|
| WO 2013033116 A1 | 07-03-2013 | NONE | |
| WO 2012098416 A1 | 26-07-2012 | AU 2012208388 A1 | 22-08-2013 |
| | | CA 2825367 A1 | 26-07-2012 |
| | | CN 103502217 A | 08-01-2014 |
| | | EP 2665709 A1 | 27-11-2013 |
| | | KR 20140016889 A | 10-02-2014 |
| | | US 2013296557 A1 | 07-11-2013 |
| | | WO 2012098416 A1 | 26-07-2012 |
| WO 03076442 A1 | 18-09-2003 | AT 346070 T | 15-12-2006 |
| | | AU 2003215325 A1 | 22-09-2003 |
| | | BR 0308243 A | 11-01-2005 |
| | | CA 2477967 A1 | 18-09-2003 |
| | | CN 1639165 A | 13-07-2005 |
| | | DE 60309848 T2 | 16-05-2007 |
| | | DK 1483265 T3 | 19-03-2007 |
| | | EP 1483265 A1 | 08-12-2004 |
| | | ES 2276048 T3 | 16-06-2007 |
| | | IL 163781 A | 15-04-2010 |
| | | JP 4414232 B2 | 10-02-2010 |
| | | JP 2005526072 A | 02-09-2005 |
| | | KR 20040087338 A | 13-10-2004 |
| | | PT 1483265 E | 31-01-2007 |
| | | US 2005090483 A1 | 28-04-2005 |
| | | US 2009105229 A1 | 23-04-2009 |
| | | US 2011207721 A1 | 25-08-2011 |
| | | US 2011294792 A1 | 01-12-2011 |
| | | WO 03076442 A1 | 18-09-2003 |
| WO 2008051405 A1 | 02-05-2008 | AU 2007309569 A1 | 02-05-2008 |
| | | CA 2667003 A1 | 02-05-2008 |
| | | EP 2083623 A1 | 05-08-2009 |
| | | JP 2010506917 A | 04-03-2010 |
| | | US 2010204236 A1 | 12-08-2010 |
| | | WO 2008051405 A1 | 02-05-2008 |
| US 2917511 A | 15-12-1959 | NONE | |
| US 3050525 A | 21-08-1962 | NONE | |
| WO 2012065297 A1 | 24-05-2012 | US 2013280245 A1 | 24-10-2013 |
| | | WO 2012065297 A1 | 24-05-2012 |
| | | WO 2012065546 A1 | 24-05-2012 |
| US 2013150343 A1 | 13-06-2013 | US 2013150343 A1 | 13-06-2013 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 13 17 3962

This annex lists the patent family members relating to the patent documents cited in the above-mentioned European search report. The members are as contained in the European Patent Office EDP file on
The European Patent Office is in no way liable for these particulars which are merely given for the purpose of information.

27-03-2014

| Patent document cited in search report | Publication date | Patent family member(s) | Publication date |
|---|---|---|---|
| | | WO 2013083604 A1 | 13-06-2013 |
| US 4244953 A | 13-01-1981 | NONE | |
| US 2011098471 A1 | 28-04-2011 | CN 102131811 A | 20-07-2011 |
| | | TW 201004958 A | 01-02-2010 |
| | | US 2011098471 A1 | 28-04-2011 |
| | | WO 2009157423 A1 | 30-12-2009 |
| WO 2010019899 A1 | 18-02-2010 | AR 072936 A1 | 29-09-2010 |
| | | AU 2009281822 A1 | 18-02-2010 |
| | | CA 2731108 A1 | 18-02-2010 |
| | | CN 102124005 A | 13-07-2011 |
| | | CO 6351724 A2 | 20-12-2011 |
| | | DO P2011000050 A | 15-03-2011 |
| | | EA 201170295 A1 | 30-08-2011 |
| | | EC SP11010889 A | 29-04-2011 |
| | | EP 2313407 A1 | 27-04-2011 |
| | | GE P20125652 B | 25-09-2012 |
| | | JP 2012500215 A | 05-01-2012 |
| | | KR 20110039383 A | 15-04-2011 |
| | | MA 32615 B1 | 01-09-2011 |
| | | NZ 590617 A | 25-05-2012 |
| | | PE 02752011 A1 | 08-05-2011 |
| | | TW 201014857 A | 16-04-2010 |
| | | US 2010063054 A1 | 11-03-2010 |
| | | UY 32049 A | 26-03-2010 |
| | | WO 2010019899 A1 | 18-02-2010 |
| WO 2011060235 A1 | 19-05-2011 | EP 2501232 A1 | 26-09-2012 |
| | | US 2012225885 A1 | 06-09-2012 |
| | | WO 2011060235 A1 | 19-05-2011 |
| WO 2008057855 A2 | 15-05-2008 | EP 2089389 A2 | 19-08-2009 |
| | | US 2010190820 A1 | 29-07-2010 |
| | | WO 2008057855 A2 | 15-05-2008 |
| WO 2004043940 A1 | 27-05-2004 | AT 350374 T | 15-01-2007 |
| | | AU 2003290577 A1 | 03-06-2004 |
| | | BR 0315796 A | 13-09-2005 |
| | | CA 2504735 A1 | 27-05-2004 |
| | | DE 60310991 T2 | 18-10-2007 |
| | | EC SP055783 A | 11-08-2005 |
| | | EP 1562925 A1 | 17-08-2005 |
| | | ES 2278213 T3 | 01-08-2007 |
| | | IS 7817 A | 20-04-2005 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 13 17 3962

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2014

| Patent document cited in search report | Publication date | Patent family member(s) | Publication date |
|---|---|---|---|
| | | JP 4413863 B2 | 10-02-2010 |
| | | JP 2006509839 A | 23-03-2006 |
| | | KR 20050072481 A | 11-07-2005 |
| | | MA 27556 A1 | 03-10-2005 |
| | | MX PA05004890 A | 22-07-2005 |
| | | US 2005222140 A1 | 06-10-2005 |
| | | WO 2004043940 A1 | 27-05-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012028335 A1


### Non-patent literature cited in the description


- **WANG et al.** *Biochem. Biophys Res.Commun.,* 2012, vol. 421, 312-317
- Remington's Pharmaceutical Sciences. Mack Publishing Co,
- *Methods in Enzymology,* 2011, vol. 493, 300-319